# EUROPEAN PATENT APPLICATION

(11) **EP 1 612 204 A1**
(43) Date of publication of application: **04.01.2006**
(21) Application number: 04724752.3
(22) Date of filing: 31.03.2004
(51) Int. Cl.: C07C 251/86, C07D 211/70, C07D 213/53, C07D 233/61, C07D 263/32, C07D 277/10, C07D 277/66, C07D 295/12, C07D 401/12, C07D 413/12, C07D 417/12, C07D 471/04, C07D 487/04, C07D 513/04

(54) **HYDRAZONE DERIVATIVE**

(30) Priority: 31.03.2003 JP 2003094257
(71) Applicant: DAIICHI PHARMACEUTICAL CO., LTD., Chuo-ku, Tokyo 103-8234 (JP)
(72) Inventor: KAWAGOE, Keiichi, Daiichi Pharmaceutical Co. Ltd., Edogawa-ku, Tokyo 134-8630 (JP); MOTOKI, Kayoko, c/o Daiichi Pharmaceutical Co. Ltd, Edogawa-ku, Tokyo 134-8630 (JP); ODAGIRI, Takashi, Daiichi Pharmaceutical Co. Ltd., Edogawa-ku, Tokyo 134-8630 (JP); SUZUKI, Nobuyuki, Daiichi Pharmaceutical Co. Ltd., Edogawa-ku, Tokyo 134-8630 (JP); CHEN, Chun-Jen, c/o Daiichi Pharmaceutical Co. Ltd, Edogawa-ku, Tokyo 134-8630 (JP); MIMURA, Tetsuya, Daiichi Pharmaceutical Co. Ltd., Edogawa-ku, Tokyo 134-8630 (JP)
(74) Representative: Reitstötter - Kinzebach
(86) International application number: PCT/JP2004/004607
(87) International publication number: WO 2004/087641

(57) **Abstract**

A compound represented by the following formula (I): 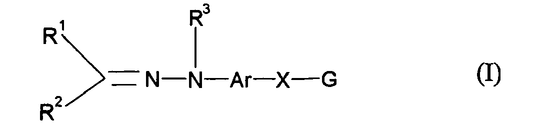 wherein R¹ represents hydrogen, aryl which may have a substituent, a saturated or unsaturated 5- to 7-membered heterocyclic group which may have a substituent, etc.; R² represents hydrogen, aryl which may have a substituent, a saturated or unsaturated 5- to 7-membered heterocyclic group which may have a substituent, *etc.;* R³ represents hydrogen, *etc.;* Ar represents a divalent group derived from aromatic hydrocarbon, *etc.;* X represents a single bond, linear or branched alkylene having from 1 to 3 carbon atoms which may have a substituent, etc.; and G represents halogen, a saturated or unsaturated 5- or 6-membered cyclic hydrocarbon group which may have a substituent, a saturated or unsaturated 5- to 7-membered heterocyclic group which may have a substituent, *etc*., a salt thereof or a solvate thereof; and an agent for inhibiting aggregation and/or deposition of an amyloid protein or an amyloid-like protein, which comprises the compound, a salt thereof or a solvate thereof

## Description

### Technical Field

The present invention relates to hydrazone derivatives which have the action to inhibit aggregation and/or deposition of amyloid protein or amyloid-like protein.

### Background of the Invention

Amyloidosis is a general term for diseases in which a special fibrous and stable protein aggregate called amyloid is accumulated, and, in human, various diseases are included depending on the amyloid-forming protein and its accumulating region (e.g., Alzheimer disease, Down syndrome, Creutzfeldt-Jacob disease, diabetes mellitus type II, dialysis amyloidosis, AA amyloidosis, Gerstmann Straussler Scheinker syndrome, Maxwell's syndrome, localized atrial amyloid, medullary carcinoma of thyroid, skin amyloidosis, localized nodular amyloidosis, AL amyloidosis, AH amyloidosis, familial amyloid polyneuropathy, senile systemic amyloidosis, cerebrovascular amyloidosis, familial Mediterranean fever, *etc.).* In addition, amyloidosis caused by prion protein is broadly observed in animals, and disease names, such as bovine spongiform encephalopathy and scrapie, are given for respective animal species. Amyloid is defined as a branchless fibrous protein aggregate of about 10 nm in width, which is stained with Congo red and generates green polarized light under polarization microscope observation, and classically, it means only those which are accumulated in the extracellular moiety (cf. *Puchtler et al., J. Histochem. Cytochem.,* 10, 355-364 (1963)).

However, since a large number of diseases in which a protein aggregate which coincides with the definition of amyloid is accumulated inside the cells have been found in recent years (e.g., Parkinson disease, tauopathy, ALS, CAG repeat disease, *etc.),* it has been proposed that these diseases may be combined with the amyloidosis and generally referred to as a name conformation disease (cf. Carrell *et al., Lancet,* 350, 134-138 (1997)). Regarding the protein which forms amyloid, about 20 kinds such as β protein, prion protein, tau protein, α-cinuclein and the like are known, and these proteins have a common characteristic of being rich in β-sheet structure and it is considered that they do not exert toxicity as monomers but cause organ diseases when they are agglicated (cf. Pile *et al*., Brain Res., 563, 311-314, 1991, and Lorenzo *et al., Proc. Natl. Acad. Sci. USA,* 91, 12243 (1994)). In addition, it is known that the initial formation of a short aggregate is the rate-determining step of the amyloid formation process, and when this is formed, a reaction mode in which elongation of the fibrous aggregate quickly progresses using this as the aggregation nucleus ("nucleus- dependent aggregation reaction") occurs (cf. Joseph *et al., Cell,* 73, 1055-1058 (1993)).

Current definite diagnosis of conformation disease is mainly based on the clinical symptoms and the like while alive, but it is necessary for the complete definite diagnosis to histopathologically verify accumulation of amyloid or amyloid-like aggregate by biopsy while alive or pathologic autopsy after death. Also it is known by pathologic study using pathologic autopsy cases that this aggregate accumulation is progressing before the appearance of definite symptoms in any disease (cf. Braak *et al*., *Acta Neuropathol*., 82, 239-259 (1991)). When Alzheimer disease which occurs in 5 to 10% of aged persons of 65 years or more and shows progressive dementia is taken into consideration as an example, a method for evaluating reduction of the cognition function (ADAS (Alzheimer's disease assessment scale), MMSE (mini mental state examination) or Hasegawa dementia scale) is generally used as a clinical diagnosis method, and this is sometimes evaluated by synthesizing the results of inspection of cerebral atrophy findings and the like through image diagnosis (MRI (magnetic resonance imaging) or CT (computed tomography), inspection of cerebrospinal fluid and the like. However, definite diagnosis of Alzheimer disease is not sufficient by these methods, and under the present situation, the diagnosis is defined basically by carrying out pathologic autopsy after death (cf. Khachturian *et al., Arch. Neurol.,* 42, 1097-1105 (1985)). It is shown as a result of pathologic study that accumulation of amyloid as a pathologic change in the most earliest stage in the brain of Alzheimer disease and nerve degeneration accompanied thereby are started in 30 to 40 years before the occurrence of definite clinical symptoms, and it is known that the pathologic image in the brain is already progressed considerably at the time when clinical symptoms started to appear (cf. Braak *et al*., *Acta Neuropathol*., 82, 239-259 (1991)). Thus, it is pointed out that the reason why therapeutic effects of drugs (brain function improver, *etc.)* are fairly limited in the clinical field is the delay of treatment starting time by the current diagnostic methods (cf. Gauthier *et al*., *Prog. Neuropsychopharmacol. Biol*. *Psychiatry,* 25, 73-89 (2001), and Sramek *et al*., *Ann. Pharmacother.,* 34, 1179-1188 (2000).

Based on such present situation, studies are in progress on the development of a new diagnostic method for detecting progress of a disease before the appearance of definite symptoms, for carrying out effective treatment. Recently, a case has been reported in which it was successful in labeling a protein or compound having affinity for amyloid, with a radioisotope, in administering it, and in detecting distribution of the isotope-labeled body bonded to amyloid from the outside of the human body by SPECT (single photon emission computed tomography) or PET (positron emission tomography). Specifically, there is a case in which peripheral amyloid accumulation is detected with an amyloid-binding protein ¹²³I-labeled SAP (serum amyloid P component) using a y camera (cf. Hawkins *et al., Lancet,* 1413-1418 (1988), and Lovat *et al., Gut,* 42, 727-734 (1998)), and there is a report stating that accumulation of amyloid of β protein or tau protein distributing in the brain of an Alzheimer patient was detected by PET using an ¹⁸F-labeled compound of amyloid-binding FDDNP (2-(1,1-dicyanopropen-2-yl)-6-(2-fluoroethyl)-methylamino)-naphthalene) as a probe (cf. Kooresb *et al., Am. J. Geriatr. Psychiatry,* 10, 24-36 (2002)).

However, the former case is sharply limited in terms of clinical use and applicable disorders, because it uses human blood preparations as the material, and SAP does not shift into the brain by peripheral administration (cf. Lovat *et al*., *Alzheimer Disease and Associated Disorders,* 12(3), 208-210 (1998)). In the latter case, there are many non-specific tissue bonding, so that concern is directed toward the development of a compound having a binding characteristic of more higher specificity.

In the case of conformation disease, it is considered that inhibition of the formation and tissue deposition of amyloid, desirably re-dissolution thereof, is an effective therapeutic method, but there is no broadly accepted therapeutic agent which can be used for this purpose, so that it is the present situation that only symptomatic therapy is carried out for all diseases. At the experimental level, studies have been carried out on the use of an agent capable of inhibiting formation of amyloid as a therapeutic agent for amyloidosis, using an agent which binds to amyloid or a protein constituting the same (cf. Kisilivsky *et al., Nature Medicine,* 4, 772-773 (1998), Soto *et al, Nature Medicine,* 4, 882-886 (1998), and Tomiyama *et al., J. Biol. Chem.,* 271, 6839-6844 (1996)). It is possible that a compound capable of specifically binding to amyloid can become a therapeutic agent for various conformation diseases in human and animals by inhibiting formation of amyloid and inhibiting binding of the formed amyloid to cells and tissues and further dissolving it (cf. Burgevin *et al., Neuro Report,* 5, 2429-2432 (1994), and it is also possible to use the compound as an *in vivo* or *in vitro* diagnostic agent for conveniently inspecting accumulation of amyloid in human and animals *in vivo* or *in vitro,* by labeling it (isotope labeling, biotin labeling or the like) by a certain method and using a device for detecting the label (cf. Klunk *et al., Neurobiol Aging,* 16, 514-548 (1995)).

### Disclosure of the Invention

The present invention provides a hydrazone derivative which has the action to inhibit aggregation and/or deposition of amyloid protein or amyloid-like protein.

As a result of intensive studies, the present inventors have found a compound which has the action to inhibit aggregation of an amyloid(-like) protein and to inhibit binding of the formed aggregate to cells and is useful as a preventive and/or therapeutic agent for diseases caused by accumulation of a specific fibrous and stable protein aggregate called amyloid, and further found a compound which can be used as an *in vivo* or *in vitro* diagnostic agent for conveniently inspecting accumulation of amyloid in human and animals *in vivo* or *in vitro,* by labeling it (isotope labeling, biotin labeling, etc.) by a certain method and using a device for detecting the label, thereby accomplishing the present invention.

The compound of the present invention can be used as therapeutic and diagnostic agents for Alzheimer disease in which amyloid(-like) protein is concerned, as well as Down syndrome, Creutzfeldt-Jacob disease, diabetes mellitus type II, dialysis amyloidosis, AA amyloidosis, Gerstmann Straussler Scheinker syndrome, Maxwell's syndrome, localized atrial amyloid, medullary carcinoma of thyroid, skin amyloidosis, localized nodular amyloidosis, AL amyloidosis, AH amyloidosis, familial amyloid polyneuropathy, senile systemic amyloidosis, cerebrovascular amyloidosis, familial Mediterranean fever, Parkinson disease, tauopathy, ALS, CAG repeat disease and the like conformation diseases.

That is, the present invention provides a compound represented by the following formula (I): 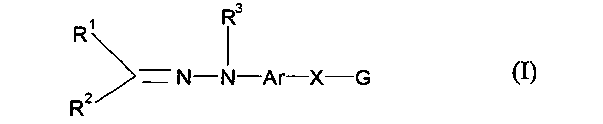 wherein R¹ and R² each independently represents hydrogen, alkyl, alkenyl, alkynyl, aralkyl, amino, alkylamino, cyano, halogen, halogenoalkyl, halogenoalkenyl, halogenoalkynyl, carboxyl, alkoxycarbonyl, carbamoyl, *N*-alkylcarbamoyl, *N*,*N*-dialkylcarbamoyl, *N*-hydroxyalkylcarbamoyl, aryl which may have a substituent, a saturated or unsaturated 5- to 7-membered heterocyclic group which may have a substituent, a saturated or unsaturated bicyclic or tricyclic condensed heterocyclic group which may have a substituent, arylalkenyl which may have a substituent, saturated or unsaturated hetero ring-alkenyl which may have a substituent, or saturated or unsaturated bicyclic or tricyclic condensed hetero ring-alkenyl which may have a substituent, wherein the substituent is one substituent or 2 or 3 substituents, which are the same or different, selected from the following Group (A):
Group (A):
   halogen, hydroxyl, alkyl, alkoxy, halogenoalkyl, cyano, nitro, hydroxyalkyl, carboxyl, alkoxycarbonyl, carboxyalkoxy, alkoxycarbonylalkoxy, aralkyloxy, *N*-alkylaminoalkylcarbonyl, *N,N-*dialkylaminoalkylcarbonyl, carboxyalkyl, alkoxycarbonylalkoxy, morpholinocarbonylalkoxy, mercapto, alkylthio, aminosulfonyl, *N*-alkylaminosulfonyl, *N,N*-dialkylaminosulfonyl, sulfo, alkylsulfonyl, alkylsulfonylalkyl, tetrazolyl, trialkyltin, trialkylsilyl, aminosulfonylalkyl, *N*-alkylaminosulfonylalkyl, *N,N*-dialkylaminosulfonylalkyl, aralkyl, alkylsulfonylamino, *N*-alkylaminosulfonylamino, *N,N*-dialkylaminosulfonylamino, *N*-alkylaminoacylamino and *N,N*-dialkylaminoacylamino,
   a group represented by the following formula (II):

      -A¹-Y¹ (II)
   wherein A¹ represents a single bond or linear, branched or cyclic alkylene having from 1 to 6 carbon atoms which may be substituted with halogen or hydroxyl; and Y¹ represents a saturated or unsaturated 5- to 7-membered heterocyclic group which may have a substituent,
   wherein the substituent on Y¹ is one substituent or 2 or 3 substituents, which are the same or different, selected from the group consisting of halogen, alkyl, halogenoalkyl, carboxyl, alkoxycarbonyl, aminoalkyl, *N*-alkylamino, *N,N* dialkylamino, *N*-alkylaminoalkyl, *N,N-*dialkylaminoalkyl, *N*-alkyl-*N*-alkoxycarbonylamino and *N*-alkyl-*N*-alkoxycarbonylaminoalkyl,
   a group represented by the following formula (III)

      -A²-(C=O)-Y² (III)
   wherein A² represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl, in which the alkylene binds to the carbonyl in the group; and Y² represents a saturated or unsaturated 5- to 7-membered heterocyclic group which may have a substituent,
   wherein the substituent on Y² represents one substituent or 2 or 3 substituents, which are the same or different, selected from the group consisting of halogen, alkyl, halogenoalkyl, carboxyl, alkoxycarbonyl, aminoalkyl, *N*-alkylamino, *N*,*N-*dialkylamino, *N*-alkylaminoalkyl, *N*,*N-*dialkylaminoalkyl, *N*-alkyl-*N*-alkoxycarbonylamino and *N*-alkyl-*N*-alkoxycarbonylaminoalkyl,
   a group represented by the following formula (IV)

      -A³-N(R⁴)(R⁵) (IV)
   wherein A³ represents a single bond, linear, branched or cyclic alkylene having from 1 to 6 carbon atoms which may be substituted with halogen or hydroxyl, linear, branched or cyclic-O-alkylene having from 1 to 6 carbon atoms which may be substituted with halogen or hydroxyl, in which the alkylene binds to the nitrogen atom in the group, or linear, branched or cyclic-(C=O)-alkylene having from 1 to 6 carbon atoms which may be substituted with halogen or hydroxyl, in which the alkylene binds to the nitrogen atom in the group; and R⁴ and R⁵ each independently represents hydrogen, alkyl, hydroxyalkyl, halogenoalkyl, acyl, alkoxycarbonyl, alkylsulfonyl, *N*-alkylaminosulfonyl, *N,N-*dialkylaminosulfonyl, *N*-alkylaminoalkylcarbonyl, *N,N-*dialkylaminoalkylcarbonyl or alkyldiphenylsilyloxyalkyl, and
   a group represented by the following formula (V)

      -A⁴-(C=O)-N(R⁶)(R⁷) (V)
   wherein A⁴ represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl, in which the alkylene binds to the carbonyl in the group; and
   R⁶ and R⁷ each independently represents hydrogen, alkyl, hydroxyalkyl, halogenoalkyl, acyl, alkoxycarbonyl, alkylsulfonyl, *N*-alkylaminosulfonyl, *N,N-*dialkylaminosulfonyl, *N*-alkylaminoalkylcarbonyl, *N,N-*dialkylaminoalkylcarbonyl or alkyldiphenylsilyloxyalkyl;
   R³ represents hydrogen, alkyl which may have substituent, acyl or alkoxycarbonyl;
   Ar represents a divalent group derived from aromatic hydrocarbon, a saturated or unsaturated 5- to 7-membered hetero ring or a saturated or unsaturated bicyclic or tricyclic condensed hetero ring, which may have one substituent or 2 or 3 substituents, which are the same or different, selected from the following Group (B):
Group (B):
   halogen, hydroxyl group, alkyl, alkoxy, halogenoalkyl, cyano, amino, nitro, alkylamino, hydroxyalkyl, carboxyl, alkoxycarbonyl, carbamoyl, mercapto, alkylthio, aminosulfonyl, *N*-alkylaminosulfonyl, *N,N* dialkylaminosulfonyl, sulfo, trialkyltin and trialkylsilyl;
   X represents a single bond, linear or branched alkylene having from 1 to 3 carbon atoms which may have a substituent, linear or branched alkenylene having from 1 to 3 carbon atoms which may have a substituent, linear or branched alkynylene having from 1 to 3 carbon atoms which may have a substituent or carbonyl; and
   G represents halogen, halogenoalkyl, halogenoalkenyl, halogenoalkynyl, alkoxy, alkoxycarbonyl, *N*-alkylamino, *N*,*N*-dialkylamino, a saturated or unsaturated 5- or 6-membered cyclic hydrocarbon group which may have a substituent, a saturated or unsaturated bicyclic or tricyclic condensed hydrocarbon group which may have a substituent, a saturated or unsaturated 5- to 7-membered heterocyclic group which may have a substituent, or a saturated or unsaturated bicyclic or tricyclic condensed heterocyclic group which may have a substituent, wherein the substituent represents one substituent or 2 or 3 substituents, which are the same or different, selected from the following Group (C):
Group (C):
   halogen, hydroxyl, alkyl, alkoxy, halogenoalkyl, halogenoalkenyl, halogenoalkoxy, cyano, amino, nitro, *N*-alkylamino, *N*,*N*-dialkylamino, *N*-alkylaminoalkyl, *N*,*N*-dialkylaminoalkyl, hydroxyalkyl, carboxyl, carboxyalkyl, alkoxycarbonyl, carbamoyl, mercapto, alkylthio, aminosulfonyl, *N*-alkylaminosulfonyl, *N*,*N*-dialkylaminosulfonyl, oxo, trialkyltin and trialkylsilyl,
   a salt thereof or a solvate thereof.

Also, the present invention provides a radioactive diagnostic agent useful as an image diagnosis probe for a disease in which amyloid is accumulated, which comprises a compound in which any one of the substituents R¹, R², R³, Ar and G of formula (I) is labeled with a radiation-releasing isotope, a salt thereof or a solvate thereof.

Furthermore, the present invention provides a medicament which comprises a compound represented by formula (I), a salt thereof or a solvate thereof; an agent for inhibiting aggregation and/or deposition of an amyloid protein or an amyloid-like protein; an agent for preventing and/or treating a conformation disease; and an agent for preventing and/or treating a disease caused by accumulation of amyloid. Also, the present invention provides an agent for preventing and/or treating Alzheimer disease, Down syndrome, Creutzfeldt-Jacob disease, diabetes mellitus type II, dialysis amyloidosis, AA amyloidosis, Gerstmann Straussler Scheinker syndrome, Maxwell's syndrome, localized atrial amyloid, medullary carcinoma of thyroid, skin amyloidosis, localized nodular amyloidosis, AL amyloidosis, AH amyloidosis, familial amyloid polyneuropathy, senile systemic amyloidosis, cerebrovascular amyloidosis, familial Mediterranean fever, Parkinson disease, tauopathy, ALS or CAG repeat disease.

In addition, the present invention provides a method for preventing and/or treating the above-described disease, which comprises administering a compound represented by formula (I), a salt thereof or a solvate thereof; and a method for diagnosing accumulation of amyloid, which comprises administering the above-described radioactive diagnosing agent and detecting a radiation-releasing isotope.

### Best Method for Carrying Out the Invention

The substituents in the compound represented by formula (I) are described below.

### <Regarding R¹ and R²>

R¹ and R² each independently represents hydrogen, alkyl, alkenyl, alkynyl, aralkyl, amino, alkylamino, cyano, halogen, halogenoalkyl, halogenoalkenyl, halogenoalkynyl, carboxyl, alkoxycarbonyl, carbamoyl, *N*-alkylcarbamoyl, *N*,*N*-dialkylcarbamoyl, *N*-hydroxyalkylcarbamoyl, aryl which may have a substituent, a saturated or unsaturated 5- to 7-membered heterocyclic group which may have a substituent, a saturated or unsaturated bicyclic or tricyclic condensed heterocyclic group which may have a substituent, arylalkenyl which may have a substituent, saturated or unsaturated hetero ring-alkenyl which may have a substituent, or saturated or unsaturated bicyclic or tricyclic condensed hetero ring-alkenyl which may have a substituent.

Herein, the alkyl represents linear, branched or cyclic alkyl having from 1 to 6 carbon atoms, and examples include methyl, ethyl, isopropyl, cyclopropyl, butyl, *tert*-butyl and the like.

The alkenyl represents linear or branched alkenyl having from 2 to 6 carbon atoms and having one double bond, and examples include vinyl, allyl, propenyl and the like.

The alkynyl represents linear or branched alkynyl having from 2 to 6 carbon atoms and having one triple bond, and examples include ethynyl, propynyl and the like.

The alkylamino represents a group in which amino is substituted with one of the above-described alkyl having from 1 to 6 carbon atoms, and examples include methylamino, ethylamino and the like.

The halogen represents a fluorine atom, a chlorine atom, a bromine atom or an iodine atom.

The halogenoalkyl represents a group in which the above-described alkyl group having from 1 to 6 carbon atoms is substituted with one halogen atom or 2 or 3 halogen atoms which are the same or different, as described above, and examples include chloromethyl, 1-bromoethyl, trifluoromethyl and the like.

The halogenoalkenyl represents a group in which the above-described alkenyl group having from 2 to 6 carbon atoms is substituted with one halogen atom or 2 or 3 halogen atoms which are the same or different, as described above, and examples include 2-chlorovinyl, 2-bromoallyl and the like.

The halogenoalkynyl represents a group in which the above-described alkynyl group having from 2 to 6 carbon atoms is substituted with one halogen atom or 2 or 3 halogen atoms which are the same or different, as described above, and examples include 2-chloroethynyl, 2-bromopropynyl and the like.

The alkoxycarbonyl represents a group having from 2 to 7 carbon atoms constituted by a linear, branched or cyclic alkoxy having from 1 to 6 carbon atoms, such as methoxy or ethoxy, and carbonyl, and examples include methoxycarbonyl, ethoxycarbonyl and the like.

The *N*-alkylcarbamoyl represents a group in which carbamoyl is substituted with one of the above-described alkyl having from 1 to 6 carbon atoms, and examples include *N*-methylcarbamoyl and *N*-ethylcarbamoyl and the like.

The *N,N-*dialkylcarbamoyl represents a group in which carbamoyl is substituted with two of the above-described alkyl having from 1 to 6 carbon atoms, which are the same or different, and examples include *N,N-*dimethylcarbamoyl, *N,N-*diethylcarbamoyl, *N*-ethyl-*N*-methylcarbamoyl and the like.

The *N*-hydroxyalkylcarbamoyl represents a group in which carbamoyl is substituted with one hydroxyalkyl group, wherein the above-described alkyl having from 1 to 6 carbon atoms is substituted with one hydroxyl group, and examples include *N*-hydroxymethylcarbamoyl, *N*-(2-hydroxyethyl)carbamoyl and the like.

The aryl represents aryl having from 6 to 14 carbon atoms, and examples include phenyl, naphthyl, anthryl, phenanthryl, biphenylyl and the like.

The saturated or unsaturated 5- to 7-membered heterocyclic group represents a group in which a hetero ring having at least one hetero atom selected from the group consisting of an oxygen atom, a sulfur atom and a nitrogen atom is a divalent group, and examples include furyl, pyrrolyl, thienyl, pyrazolyl, imidazolyl, pyrazolinyl, oxazolyl, isoxazolyl, oxazolinyl, thiazolyl, thiazolinyl, thiadiazolyl, furazanyl, pyranyl, pyridyl, tetrahydropyridyl, pyrimidinyl, pyrazinyl, pyridazinyl, pyrrolidinyl, piperazinyl, piperidinyl, oxazinyl, oxadiazinyl, morpholinyl, thiazinyl, thiadiazinyl, thiomorpholinyl, tetrazolyl, triazolyl, triazinyl, azepinyl, diazepinyl, triazepinyl and the like. According to the present invention, furyl, pyrrolyl, thienyl, pyrazolyl, imidazolyl, oxazolyl, isoxazolyl, thiazolyl, pyridyl, pyrimidinyl, pyrazinyl, triazinyl and the like are preferable.

The saturated or unsaturated bicyclic or tricyclic condensed heterocyclic group represents a group in which a saturated or unsaturated bicyclic or tricyclic condensed hetero ring is a monovalent group, and the saturated or unsaturated bicyclic or tricyclic condensed hetero ring represents the following (1) to (3):
(1) a bicyclic or tricyclic condensed hetero ring formed by condensation of 2 or 3 saturated or unsaturated 5- to 7-membered hetero rings which are the same or different,
(2) a bicyclic or tricyclic condensed hetero ring formed by condensation of 1 saturated or unsaturated 5- to 7-membered hetero ring with 1 to 2 saturated or unsaturated 5- or 6-membered cyclic hydrocarbon, and
(3) a tricyclic condensed hetero ring formed by condensation of 2 saturated or unsaturated 5- to 7-membered hetero rings with 1 saturated or unsaturated 5- or 6-membered cyclic hydrocarbon.

The above-described saturated or unsaturated 5- to 7-membered hetero ring represents a hetero ring having at least one hetero atom selected from the group consisting of an oxygen atom, a sulfur atom and a nitrogen atom, and examples include furan, pyrrole, thiophene, pyrazole, imidazole, oxazole, oxazolidine, thiazole, thiadiazole, furazane, pyran, pyridine, pyrimidine, pyridazine, pyrrolidine, piperazine, piperidine, oxazine, oxadiazine, morpholine, thiazine, thiadiazine, thiomorpholine, tetrazole, triazole, triazine, thiadiazine, oxadiazine, azepine, diazepine, triazepine, thiazepine, oxazepine and the like. Also, the saturated or unsaturated 5- or 6-membered cyclic hydrocarbon includes cyclopentane, cyclopentene, cyclohexane, cyclohexene, cyclohexadiene, benzene and the like.

Examples of the saturated or unsaturated bicyclic or tricyclic condensed heterocyclic group include indolyl, indolinyl, isoindolyl, isoindolinyl, indazolyl, quinolyl, dihydroquinolyl, tetrahydroquinolyl, isoquinolyl, tetrahydroisoquinolyl, 4H-quinolizinyl, quinazolinyl, dihydroquinazolinyl, tetrahydroquinazolinyl, quinoxalinyl, tetrahydroquinoxalinyl, cinnolinyl, tetrahydrocinnolinyl, indolizinyl, tetrahydroindolizinyl, benzothiazolyl, tetrahydrobenzothiazolyl, benzoxazolyl, benzoisothiazolyl, benzoisooxazolyl, benzoimidazolyl, naphthyridinyl, tetrahydronaphthyridinyl, thienopyridyl, tetrahydrothienopyridyl, thiazolopyridyl, tetrahydrothiazolopyridyl, thiazolopyridazinyl, tetrahydrothiazolopyridazinyl, pyrrolopyridyl, dihydropyrrolopyridyl, tetrahydropyrrolopyridyl, pyrrolopyrimidinyl, dihydropyrrolopyrimidinyl, pyridopyrimidinyl, tetrahydropyridopyrimidinyl, pyranothiazolyl, dihydropyranothiazolyl, furopyridyl, tetrahydrofuropyridyl, oxazolopyridyl, tetrahydrooxazolopyridyl, oxazolopyridazinyl, tetrahydrooxazolopyridazinyl, pyrrolothiazolyl, dihydropyrrolothiazolyl, pyrrolooxazolyl, dihydropyrrolooxazolyl, thienopyrrolyl, thiazolopyrimidinyl, thiazolooxazolyl, imidazothiazolyl, imidazooxazolyl, imidazopyrimidinyl, imidazopyridyl, tetrahydroimidazopyridyl, pyrazinopyridazinyl, imidazotriazinyl, oxazolopyridyl, benzooxepinyl, benzoazepinyl, tetrahydrobenzoazepinyl, benzodiazepinyl, benzotriazepinyl, thienoazepinyl, tetrahydrothienoazepinyl, thienodiazepinyl, thienotriazepinyl, thiazoloazepinyl, tetrahydro thiazoloazepinyl and the like. The condensation form of the above-described condensed heterocyclic group is not particularlyl limited. As the saturated or unsaturated bicyclic or tricyclic condensed heterocyclic group, the case of the above-described (2) and (3) is preferable, and (2) is more preferable. In the (2), a case in which a bicyclic condensed hetero ring formed by the condensation of one saturated or unsaturated 5-to 7-membered hetero ring with one saturated or unsaturated 5- or 6-membered cyclic hydrocarbon is a monovalent group is preferable, and a case in which a bicyclic condensed hetero ring formed by the condensation of one saturated or unsaturated 5- to 7-membered hetero ring with one benzene ring is a monovalent group is more preferable. According to the present invention, isoindolinyl, quinolyl, tetrahydroquinolyl, isoquinolyl, tetrahydroisoquinolyl, benzothiazolyl, benzoxazolyl, benzoimidazolyl, thienopyridyl, thiazolopyridyl, tetrahydrothiazolopyridyl, pyrrolopyridyl, pyrrolopyrimidinyl, oxazolopyridyl, tetrahydrooxazolopyridyl, imidazothiazolyl, imidazooxazolyl, imidazopyrimidinyl, imidazopyridyl, tetrahydroimidazopyridyl and the like are preferable, and tetrahydroquinolyl, tetrahydrothiazolopyridyl, imidazothiazolyl, imidazooxazolyl, imidazopyrimidinyl, imidazopyridyl, tetrahydroimidazopyridyl and the like are particularly preferable.

The arylalkenyl represents a group constituted by the above-described aryl and an alkenylene having from 2 to 6 carbon atoms, and examples include styryl and the like.

The saturated or unsaturated heterocyclic-alkenyl represents a group which is constituted by the above-described saturated or unsaturated heterocyclic group and alkenylene having from 2 to 6 carbon atoms, and examples include thienylethenyl, pyridylethenyl and the like.

The saturated or unsaturated bicyclic or tricyclic condensed heterocyclic-alkenyl represents a group which is constituted by the above-described saturated or unsaturated bicyclic or tricyclic condensed heterocyclic group and alkenylene having from 2 to 6 carbon atoms, and examples include benzofurylethenyl, indolylethenyl and the like.

The above-described aryl, saturated or unsaturated 5- to 7-membered heterocyclic group, saturated or unsaturated bicyclic or tricyclic condensed heterocyclic group, arylalkenyl, saturated or unsaturated heterocyclic-alkenyl and saturated or unsaturated bicyclic or tricyclic condensed heterocyclic-alkenyl may have one substituent or 2 or 3 substituents, which are the same or different, selected from the Group (A), and these substituents are explained below.

The Group (A) includes halogen, hydroxyl, alkyl, alkoxy, halogenoalkyl, cyano, nitro, hydroxyalkyl, carboxyl, alkoxycarbonyl, carboxyalkoxy, alkoxycarbonylalkoxy, aralkyloxy, *N*-alkylaminoalkylcarbonyl, *N*,*N*-dialkylaminoalkylcarbonyl, carboxyalkyl, alkoxycarbonylalkoxy, morpholinocarbonylalkoxy, mercapto, alkylthio, aminosulfonyl, *N*-alkylaminosulfonyl, *N*,*N*-dialkylaminosulfonyl, sulfo, alkylsulfonyl, alkylsulfonylalkyl, tetrazolyl, trialkyltin, trialkylsilyl, aminosulfonylalkyl, *N*-alkylaminosulfonylalkyl, *N,N*-dialkylaminosulfonylalkyl, aralkyl, alkylsulfonylamino, *N*-alkylaminosulfonylamino, *N*,*N*-dialkylaminosulfonylamino, *N*-alkylaminoacylamino and *N*,*N-*dialkylaminoacylamino,
a group represented by the following formula (II):

-A¹-Y¹ (II)

(wherein A¹ represents a single bond or linear, branched or cyclic alkylene having from 1 to 6 carbon atoms which may be substituted with halogen or hydroxyl; and Y¹ represents a saturated or unsaturated 5- to 7-membered heterocyclic group which may have a substituent,
wherein the substituent on Y¹ represents one substituent or 2 or 3 substituents, which are the same or different, selected from the group consisting of halogen, alkyl, halogenoalkyl, carboxyl, alkoxycarbonyl, aminoalkyl, *N*-alkylamino, *N,N*-dialkylamino, *N*-alkylaminoalkyl, *N,N*-dialkylaminoalkyl, *N*-alkyl-*N-*alkoxycarbonylamino and N-alkyl-N-alkoxycarbonylaminoalkyl),
a group represented by the following formula (III):

-A²-(C=O)-Y² (III)

(wherein A² represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl (wherein the alkylene binds to the carbonyl in the group); and Y² represents a saturated or unsaturated 5- to 7-membered heterocyclic group which may have a substituent,
wherein the substituent on Y² represents one substituent or 2 or 3 substituents, which are the same or different, selected from the group consisting of halogen, alkyl, halogenoalkyl, carboxyl, alkoxycarbonyl, aminoalkyl, *N*-alkylamino, *N*,*N*-dialkylamino, *N*-alkylaminoalkyl, *N*,*N*-dialkylaminoalkyl, *N*-alkyl-*N*-alkoxycarbonylamino and *N*-alkyl-*N*-alkoxycarbonylaminoalkyl),
a group represented by the following formula (IV):

-A³-N(R⁴)(R⁵) (IV)

(wherein A³ represents a single bond, linear, branched or cyclic alkylene having from 1 to 6 carbon atoms which may be substituted with halogen or hydroxyl, linear, branched or cyclic-O-alkylene having from 1 to 6 carbon atoms which may be substituted with halogen or hydroxyl (wherein the alkylene binds to the nitrogen atom in the group) or linear, branched or cyclic-(C=O)-alkylene having from 1 to 6 carbon atoms which may be substituted with halogen or hydroxyl (wherein the alkylene binds to the nitrogen atom in the group), and R⁴ and R⁵ each independently represents hydrogen, alkyl, hydroxyalkyl, halogenoalkyl, acyl, alkoxycarbonyl, alkylsulfonyl, *N-*alkylaminosulfonyl, *N*,*N*-dialkylaminosulfonyl, *N*-alkylaminoalkylcarbonyl, *N*,*N*-dialkylaminoalkylcarbonyl or alkyldiphenylsilyloxyalkyl), and
a group represented by the following formula (V):

-A⁴-(C=O)-N(R⁶)(R⁷) (V)

(wherein A⁴ represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl (wherein the alkylene binds to the carbonyl in the group), and
R⁶ and R⁷ each independently represents hydrogen, alkyl, hydroxyalkyl, halogenoalkyl, acyl, alkoxycarbonyl, alkylsulfonyl, *N*-alkylaminosulfonyl, *N*,*N*-dialkylaminosulfonyl, *N*-alkylaminoalkylcarbonyl, *N*,*N-*dialkylaminoalkylcarbonyl or alkyldiphenylsilyloxyalkyl).

In the Group (A), the halogen represents a fluorine atom, a chlorine atom, a bromine atom or an iodine atom in the same manner as described above.

The alkyl represents a linear, branched or cyclic alkyl having from 1 to 6 carbon atoms, and examples include methyl, ethyl, isopropyl, cyclopropyl, butyl, *tert*-butyl and the like in the same manner as described above.

The alkoxy represents linear, branched or cyclic alkoxy having from 1 to 6 carbon atoms, and examples include methoxy, ethoxy, *tert*-butoxy and the like.

The halogenoalkyl represents a group in which the above-described alkyl having from 1 to 6 carbon atoms is substituted with one halogen atom or 2 or 3 halogen atoms which are the same or different, and examples include chloromethyl, 1-bromoethyl, trifluoromethyl and the like in the same manner as described above.

The hydroxyalkyl represents a group in which the above-described alkyl having from 1 to 6 carbon atom is substituted with one hydroxyl group, and examples include hydroxymethyl, 1-hydroxyethyl and the like.

The alkoxycarbonyl represents a group having from 2 to 7 carbon atoms constituted by the above-described alkoxy having from 1 to 6 carbon atoms and carbonyl, and examples include methoxycarbonyl, ethoxycarbonyl and the like.

The aminoalkyl represents a group in which the above-described alkyl having from 1 to 6 carbon atoms is substituted with one amino group, and examples include aminomethyl, aminoethyl and the like.

The carboxyalkoxy represents a group in which the above-described alkoxy having from 1 to 6 carbon atoms is substituted with one carboxy group, and examples include carboxymethoxy, 1-carboxyethoxy and the like.

The alkoxycarbonylalkoxy represents a group in which the above-described alkoxy having from 1 to 6 carbon atoms is substituted with one of the above-described alkoxycarbonyl having from 2 to 7 carbon atoms, and examples include methoxycarbonylmethoxy, ethoxycarbonylmethoxy and the like.

The aralkyloxy represents a group constructed by the above-described aralkyl and oxygen atom, and examples include benzyloxy and the like.

The *N*-alkylaminoalkylcarbonyl represents a group constructed by *N*-alkylaminoalkyl in which the above-described alkyl having from 1 to 6 carbon atoms is substituted with one *N*-alkylamino group, wherein amino is substituted with one of the alkyl having from 1 to 6 carbon atoms, and carbonyl, and examples include *N-*methylaminomethylcarbonyl, *N*-ethylaminomethylcarbonyl and the like.

The *N,N-*dialkylaminoalkylcarbonyl represents a group constructed by *N,N*-dialkylaminoalkyl in which the above-described alkyl having from 1 to 6 carbon atoms is substituted with one *N,N-*dialkylamino group, wherein amino is substituted with two of the alkyl having from 1 to 6 carbon atoms, which are the same or different, and carbonyl, and examples include *N,N*-dimethylaminomethylcarbonyl, *N,N*-ethylmethylaminomethylcarbonyl and the like.

The carboxyalkyl represents a group in which above-described alkyl having from 1 to 6 carbon atoms is substituted with one carboxy group, and examples include carboxymethyl, 1-carboxyethyl and the like.

The alkoxycarbonylalkoxy represents a group in which the above-described alkoxy having from 1 to 6 carbon atoms is substituted with one of the above-described alkoxycarbonyl having from 2 to 7 carbon atoms, and examples include methoxycarbonylmethoxy, ethoxycarbonylmethoxy and the like.

The morpholinocarbonylalkoxyalkyl represents a group in which the above-described alkoxy having from 1 to 6 carbon atoms is substituted with one morpholinocarbonyl group constituted by morpholino and carbonyl, and examples include morpholinocarbonylmethoxy, morpholinocarbonylethoxy and the like.

The alkylthio represents a group constituted by the above-described alkyl having from 1 to 6 carbon atoms and sulfur, and examples include methylthio, ethylthio and the like.

The aminosulfonyl represents a group constructed by amino and sulfonyl, i.e., -SO₂NH₂.

The *N*-alkylaminosulfonyl represents a group in which the above-described aminosulfonyl is substituted with one of the above-described alkyl having from 1 to 6 carbon atoms, and examples include *N*-methylaminosulfonyl, *N*-ethylaminosulfonyl and the like.

The *N*,*N*-dialkylaminosulfonyl represents a group in which the above-described aminosulfonyl is substituted with two of above-described alkyl having from 1 to 6 carbon atoms, which are the same or different, and examples include *N,N-*dimethylaminosulfonyl, *N,N-*diethylaminosulfonyl and the like.

The sulfo represents -SO₃H.

The alkylsulfonyl represents a group constituted by the above-described alkyl having from 1 to 6 carbon atoms and sulfonyl, and examples include methylsulfonyl, ethylsulfonyl and the like.

The alkylsulfonylalkyl represents a group in which the above-described alkyl having from 1 to 6 carbon atoms is substituted with one of the above-described alkylsulfonyl, and examples include methylsulfonylmethyl, ethylsulfonylmethyl and the like.

The trialkyltin represents a group in which tin is substituted with three of the above-described alkyl having from 1 to 6 carbon atoms, which are the same or different, and examples include trimethyltin, tributyltin and the like.

The trialkylsilyl represents a group in which silicon is substituted with three of the above-described alkyl having from 1 to 6 carbon atoms, which are the same or different, and examples include trimethylsilyl, triethylsilyl and the like.

The aminosulfonylalkyl represents a group in which the above-described alkyl having from 1 to 6 carbon atoms is substituted with one of the above-described aminosulfonyl, and examples include aminosulfonylmethyl, aminosulfonylethyl and the like.

The *N*-alkylaminosulfonylalkyl represents a group in which the amino group in the above-described aminosulfonylalkyl is substituted with one of the above-described alkyl having from 1 to 6 carbon atoms, and examples include *N*-methylaminosulfonylmethyl, *N*-ethylaminosulfonylmethyl and the like.

The *N,N*-dialkylaminosulfonylalkyl represents a group in which the amino group in the above-described aminosulfonylalkyl is substituted with two of the above-described alkyl having from 1 to 6 carbon atoms, which are the same or different, and examples include *N,N*-dimethylaminosulfonylmethyl, *N*-ethyl-*N*-ethylaminosulfonylmethyl and the like.

The aralkyl represents a group in which the above-described alkyl group having from 1 to 6 carbon atoms is substituted with one of the above-described aryl, and examples include benzyl, phenethyl, 1-naphthylmethyl, 2-naphthylmethyl and the like.

The alkylsulfonylamino represents a group in which amino is substituted with one of the above-described alkylsulfonyl, and examples include methylsulfonylamino, ethylsulfonylamino and the like.

The *N*-alkylaminosulfonylamino represents a group in which amino is substituted with one of the above-described *N*-alkylaminosulfonyl, and examples include *N*-methylaminosulfonylamino, *N*-ethylaminosulfonylamino and the like.

The *N*,*N*-dialkylaminosulfonylamino represents a group in which amino is substituted with one of the above-described *N*,*N*-dialkylaminosulfonyl, and examples include *N*,*N*-dimethylaminosulfonylamino, *N*-ethyl-*N*-methylaminosulfonylamino and the like.

The *N*-alkylaminoacylamino represents a group in which amino is substituted with one of the above-described *N*-alkylaminoalkylcarbonyl, and examples include *N*-methylaminomethylcarbonylamino, *N*-ethylaminomethylcarbonylamino and the like.

The *N*,*N*-dialkylaminoacylamino represents a group in which amino is substituted with one of the above-described *N,N-*dialkylaminoalkylcarbonyl, and examples include *N,N*-dimethylaminomethylcarbonylamino, *N*-ethyl-*N*-methylaminomethylcarbonylamino and the like.

The alkylene in A¹ of the group represented by the following formula (II):

-A¹-Y¹ (II)

(in the group, A¹ represents a single bond or linear, branched or cyclic alkylene having from 1 to 6 carbon atoms which may be substituted with halogen or hydroxyl, and Y¹ represents saturated or unsaturated 5- to 7-membered heterocyclic group which may have a substituent,
wherein the substituent on Y¹ represents one substituent or 2 or 3 substituents, which are the same or different, selected from the group consisting of halogen, alkyl, halogenoalkyl, carboxyl, alkoxycarbonyl, aminoalkyl, *N*-alkylamino, *N,N-*dialkylamino, *N*-alkylaminoalkyl, *N,N-*dialkylaminoalkyl, *N-*alkyl-*N*-alkoxycarbonylamino and *N*-alkyl-*N*-alkoxycarbonylaminoalkyl)
includes methylene, ethylene, trimethylene, propylene, tetramethylene, pentamethylene, hexamethylene and the like.

The saturated or unsaturated 5- to 7-membered heterocyclic group in Y¹ represents a group in which a hetero ring having at least one hetero atom selected from the group consisting of an oxygen atom, a sulfur atom and a nitrogen atom is a monovalent group, and examples include furyl, pyrrolyl, thienyl, pyrazolyl, imidazolyl, pyrazolinyl, oxazolyl, isoxazolyl, oxazolinyl, thiazolyl, thiazolinyl, thiadiazolyl, furazanyl, pyranyl, pyridyl, pyrimidinyl, pyrazinyl, pyridazinyl, pyrrolidinyl, piperazinyl, piperidinyl, oxazinyl, oxadiazinyl, morpholinyl, thiazinyl, thiadiazinyl, thiomorpholinyl, tetrazolyl, triazolyl, triazinyl, azepinyl, diazepinyl, triazepinyl and the like.

The halogen, alkyl, halogenoalkyl and alkoxycarbonyl as the substituents on Y¹ have the same meanings as described above.

The *N*-alkylamino represents a group in which amino is substituted with one of the above-described alkyl having from 1 to 6 carbon atoms, and examples include methylamino, ethylamino and the like.

The *N,N-*dialkylamino represents a group in which amino is substituted with two of the above-described alkyl having from 1 to 6 carbon atoms, which are the same or different, and examples include *N,N*-dimethylamino, *N,N-* ethylmethylamino and the like.

The *N*-alkylaminoalkyl represents a group in which the above-described alkyl having from 1 to 6 carbon atoms is substituted with one of the above-described *N*-alkylamino, and examples include *N*-methylaminomethyl, 1-(*N*-methylamino)ethyl and the like.

The *N,N-*dialkylaminoalkyl represents a group in which the above-described alkyl having from 1 to 6 carbon atoms is substituted with one of the above-described *N,N*-dialkylamino, and examples include *N,N-*dimethylaminomethyl, *N,N-*ethylmethylaminomethyl and the like.

The *N*-alkoxycarbonyl-*N*-alkylamino represents a group in which amino is substituted with the above-described alkoxycarbonyl having from 2 to 7 carbon atoms and with the above-described alkyl having from 1 to 6 carbon atoms, and examples include *N*-methoxycarbonyl-*N*-methylamino, *N*-ethoxycarbonyl-*N*-methylamino and the like.

The *N*-alkoxycarbonyl-*N*-alkylaminoalkyl represents a group in which the above-described alkyl having from 1 to 6 carbon atoms is substituted with one of the above-described *N*-alkoxycarbonyl-*N*-alkylamino, and examples include *N*-methoxycarbonyl-*N*-methylaminomethyl, *N*-ethoxycarbonyl-*N*-methylaminomethyl and the like.

The alkylene in A² of the group represented by the following formula (III):

-A²-(C=O)-Y² (III)

(wherein A² represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl (wherein the alkylene binds to the carbonyl in the group); and Y² represents a saturated or unsaturated 5- to 7-membered heterocyclic group which may have a substituent,
wherein the substituent on Y² represents one substituent or 2 or 3 substituents, which are the same or different, selected from the group consisting of halogen, alkyl, halogenoalkyl, carboxyl, alkoxycarbonyl, aminoalkyl, *N*-alkylamino, *N,N-*dialkylamino, *N*-alkylaminoalkyl, *N,N-*dialkylaminoalkyl, *N*-alkyl-*N*-alkoxycarbonylamino and *N-*alkyl-*N*-alkoxycarbonylaminoalkyl)
includes methylene, ethylene, trimethylene, propylene, tetramethylene, pentamethylene, hexamethylene and the like, and the-O-alkylene includes -O-methylene, -O-ethylene and the like.

The saturated or unsaturated 5- to 7-membered heterocyclic group in Y² represents the same saturated or unsaturated 5- to 7-membered heterocyclic group in Y¹. In addition, the substituent on Y² also represents the same substituent.

The alkylene in A³ of the group represented by the following formula (IV):

-A³-N(R⁴)(R⁵) (IV)

(wherein A³ represents a single bond, linear, branched or cyclic alkylene having from 1 to 6 carbon atoms which may be substituted with halogen or hydroxyl, linear, branched or cyclic-O-alkylene having from 1 to 6 carbon atoms which may be substituted with halogen or hydroxyl (wherein the alkylene binds to the nitrogen atom in the group) or linear, branched or cyclic-(C=O)-alkylene having from 1 to 6 carbon atoms which may be substituted with halogen or hydroxyl (wherein the alkylene binds to the nitrogen atom in the group); and
R⁴ and R⁵ each independently represents hydrogen, alkyl, hydroxyalkyl, halogenoalkyl, acyl, alkoxycarbonyl, alkylsulfonyl, *N-*alkylaminosulfonyl, *N*,*N*-dialkylaminosulfonyl, *N*-alkylaminoalkylcarbonyl, *N,N*-dialkylaminoalkylcarbonyl or alkyldiphenylsilyloxyalkyl)
includes methylene, ethylene, trimethylene, propylene, tetramethylene, pentamethylene, hexamethylene and the like, the -O-alkylene includes -O-methylene, -O-ethylene and the like, and the-(C=O)-alkylene includes -(C=O)-methylene, -(C=O)-ethylene and the like.

The alkyl, hydroxyalkyl, halogenoalkyl, alkoxycarbonyl, alkylsulfonyl, alkylsulfonyl, *N*-alkylaminosulfonyl, *N,N*-dialkylaminosulfonyl, *N*-alkylaminoalkylcarbonyl and *N,N-*dialkylaminoalkylcarbonyl in R⁴ and R⁵ represents the same groups described above.

The acyl represents linear or branched alkanoyl having from 1 to 6 carbon atoms, such as formyl, acetyl, propionyl or butyryl; aroyl having from 7 to 15 carbon atoms, such as benzoyl or naphthoyl; or arylalkanoyl (e.g., phenacetyl, etc.) in which alkanoyl is substituted with one of the above-described aryl.

The alkyldiphenylsilyloxyalkyl represents a group in which a group, wherein silicon is substituted with two phenyl groups and one of the above-described alkyl having from 1 to 6 carbon atoms, binds to alkylene via an oxygen atom, and examples include 2-(*tert*-butyldiphenylsilyloxy)ethyl and the like.

The alkylene in A⁴ of the group represented by the following formula (V):

-A⁴-(C=O)-N(R⁶)(R⁷) (V)

(in the group, A⁴ represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl (wherein the alkylene binds to the carbonyl in the group); and
R⁶ and R⁷ each independently represents hydrogen, alkyl, hydroxyalkyl, halogenoalkyl, acyl, alkoxycarbonyl, alkylsulfonyl, *N*-alkylaminosulfonyl, *N*,*N*-dialkylaminosulfonyl, *N*-alkylaminoalkylcarbonyl, *N,N*-dialkylaminoalkylcarbonyl or alkyldiphenylsilyloxyalkyl)
includes methylene, ethylene, trimethylene, propylene, tetramethylene, pentamethylene, hexamethylene and the like, and the -O-alkylene includes -O-methylene, -O-ethylene and the like.

The respective groups in R⁶ and R⁷ have the same meanings as those in R⁴ and R⁵.

In the present invention, R¹ and R² are preferably hydrogen, alkyl, amino, cyano, halogen, halogenoalkenyl, carboxyl, alkoxycarbonyl, carbamoyl, *N*,*N*-dialkylcarbamoyl, *N*-hydroxyalkylcarbamoyl, aryl which may have a substituent, a saturated or unsaturated 5- to 7-membered heterocyclic group which may have a substituent, a saturated or unsaturated bicyclic or tricyclic condensed heterocyclic group which may have a substituent or the like.

Also, the substituent which may be substituted on the aryl, saturated or unsaturated 5- to 7-membered heterocyclic group, saturated or unsaturated bicyclic or tricyclic condensed heterocyclic group, arylalkenyl, saturated or unsaturated heterocyclic-alkenyl, or saturated or unsaturated bicyclic or tricyclic condensed heterocyclic-alkenyl is preferably halogen, hydroxyl, alkyl, alkoxy, hydroxyalkyl, carboxyl, alkoxycarbonyl, carboxyalkoxy, alkoxycarbonylalkoxy, NN-dialkylaminoalkylcarbonyl, carboxyalkyl, aminosulfonyl, alkylsulfonylalkyl, aminosulfonylalkyl, *N*,*N*-dialkylaminosulfonylalkyl, aralkyl, alkylsulfonylamino, *NN-*dialkylaminosulfonylamino, *N*,*N* dialkylaminoacylamino or the group represented by the formulae (II), (III), (IV) or (V).

Also, the substituent on Y¹ in the group represented by formula (II) is preferably alkyl, aminoalkyl, *N,N*-dialkylamino or the like.

In the group represented by formula (III), A² is preferably a single bond or -O-alkylene having from 1 to 6 carbon atoms, and the substituent on Y² is preferably alkyl.

In the group represented by formula (IV), preferable R⁴ and R⁵ are each independently preferably hydrogen, alkyl, hydroxyalkyl, halogenoalkyl, acyl, alkoxycarbonyl, alkylsulfonyl, *N,N* dialkylaminosulfonyl, *N,N*-dialkylaminoalkylcarbonyl, alkyldiphenylsilyloxyalkyl or the like.

In the group represented by formula (V), preferably R⁶ and R⁷ are each independently hydrogen, alkyl, hydroxyalkyl or the like.

### <Regarding R³>

R³ represents hydrogen, alkyl which may have a substituent, acyl or alkoxycarbonyl. Herein, the alkyl, acyl and alkoxycarbonyl have the same meanings as described in the <Regarding R¹ and R²>. Examples of the substituent on the alkyl include halogen, hydroxyl, alkoxy, carboxyl, alkoxycarbonyl, amino, carbamoyl, *N*-alkylcarbamoyl, *N*,*N*-dialkylcarbamoyl, *N*-alkylamino, *N*,*N-*dialkylamino and the like. These groups have the same meanings as described in the <Regarding R¹ and R²>. According to the present invention, R³ is preferably hydrogen.

### <Regarding Ar>

Ar represents a divalent group derived from aromatic hydrocarbon, a saturated or unsaturated 5- to 7-membered hetero ring or a saturated or unsaturated bicyclic or tricyclic condensed hetero ring.

Herein, the aromatic hydrocarbon includes benzene, biphenyl, p-terphenyl, diphenylmethane, indene, naphthalene, tetralin, anthracene and the like.

The saturated or unsaturated 5- to 7-membered hetero ring and saturated or unsaturated bicyclic or tricyclic condensed hetero ring have the same meanings as those described in the <Regarding R¹ and R²>.

According to the present invention, a divalent group derived from aromatic hydrocarbon or a saturated or unsaturated 5- to 7-membered hetero ring is preferable as Ar. Particularly, a divalent group derived from aromatic hydrocarbon is preferable, and phenylene is most preferable. As the phenylene, it may be any one of o-phenylene, m-phenylene and p-phenylene, and p-phenylene is particularly preferable.

The above-described divalent group derived from aromatic hydrocarbon, a saturated or unsaturated 5- to 7-membered hetero ring or a saturated or unsaturated bicyclic or tricyclic condensed hetero ring may have one substituent or 2 or 3 substituents, which are the same or different, selected from the Group (B).

The Group (B) includes halogen, hydroxyl, alkyl, alkoxy, halogenoalkyl, cyano, amino, nitro, alkylamino, hydroxyalkyl, carboxyl, alkoxycarbonyl, carbamoyl, mercapto, alkylthio, aminosulfonyl, *N*-alkylaminosulfonyl, *N*,*N*-dialkylaminosulfonyl, sulfo, trialkyltin and trialkylsilyl, and these groups have the same meanings as those described above in the description of R¹, R² and R³.

According to the present invention, among the Group (B), halogen, hydroxyl, alkoxy, halogenoalkyl, amino, hydroxyalkyl and the like are preferable.

### <Regarding X>

X represents a single bond, linear or branched alkylene having from 1 to 3 carbon atoms which may have a substituent, linear or branched alkenylene having from 1 to 3 carbon atoms which may have a substituent, linear or branched alkynylene having from 1 to 3 carbon atoms which may have a substituent, or carbonyl.

Herein, the alkylene having from 1 to 3 carbon atoms includes methylene, ethylene, trimethylene, propylene and the like.

The alkenylene having from 1 to 3 carbon atoms includes vinylene, propenylene and the like.

The alkynylene having from 1 to 3 carbon atoms includes ethynylene, propynylene and the like.

The alkylene, alkenylene and alkynylene may have a substituent, and examples of the substituent include halogen such as a fluorine atom, a chlorine atom, a bromine atom and an iodine atom, and hydroxyl and the like.

According to the present invention, X is preferably a single bond or linear or branched alkylene having from 1 to 3 carbon atoms which may have a substituent.

### <Regarding G>

G represents halogen, halogenoalkyl, halogenoalkenyl, halogenoalkynyl, alkoxy, alkoxycarbonyl, *N*-alkylamino, *N,N-*dialkylamino, a saturated or unsaturated 5- or 6-membered cyclic hydrocarbon group which may have a substituent, a saturated or unsaturated bicyclic or tricyclic condensed hydrocarbon group which may have a substituent, a saturated or unsaturated 5- to 7-membered heterocyclic group which may have a substituent, or a saturated or unsaturated bicyclic or tricyclic condensed heterocyclic group which may have a substituent.

Herein, the saturated or unsaturated 5- or 6-membered cyclic hydrocarbon group includes cyclopentyl, cyclopentenyl, cyclohexyl, cyclohexadienyl, phenyl and the like.

The saturated or unsaturated bicyclic or tricyclic condensed hydrocarbon group includes indenyl, indanyl, tetrahydronaphthyl, naphthyl and the like.

The saturated or unsaturated 5- to 7-membered heterocyclic group and the saturated or unsaturated bicyclic or tricyclic condensed heterocyclic group have the same meanings as those described in R¹, R² and R³.

The above-described groups may have one substituent or 2 or 3 substituents, which are the same or different, selected from the following Group (C), and these substituents are described below.

The Group (C) includes halogen, hydroxyl, alkyl, alkoxy, halogenoalkyl, halogenoalkenyl, halogenoalkoxy, cyano, amino, nitro, *N*-alkylamino, *N,N-*dialkylamino, *N*-alkylaminoalkyl, *N,N-*dialkylaminoalkyl, hydroxyalkyl, carboxyl, carboxyalkyl, alkoxycarbonyl, carbamoyl, mercapto, alkylthio, aminosulfonyl, *N*-alkylaminosulfonyl, *N,N*-dialkylaminosulfonyl, oxo, trialkyltin and trialkylsilyl.

Herein, the halogen, alkyl, alkoxy, halogenoalkyl, halogenoalkenyl, *N*-alkylamino, *N*,*N*-dialkylamino, *N*-alkylaminoalkyl, *N,N-*dialkylaminoalkyl, hydroxyalkyl, carboxyl, carboxyalkyl, alkoxycarbonyl, carbamoyl, mercapto, alkylthio, aminosulfonyl, *N*-alkylaminosulfonyl, *N,N*-dialkylaminosulfonyl, trialkyltin and trialkylsilyl have the same meanings as those described in R¹, R², R³ and Ar.

The halogenoalkoxy represents a group in which the above-described alkoxy having from 1 to 6 carbon atoms is substituted with one halogen atom or 2 or 3 halogen atoms, which are the same or different, and examples include chloromethoxy and the like.

In the present invention, G is preferably halogen, halogenoalkenyl, alkoxy, alkoxycarbonyl, *N*,*N*-dialkylamino, saturated or unsaturated 5- or 6-membered cyclic hydrocarbon group, a saturated or unsaturated 5- to 7-membered heterocyclic group or the like, and more specifically a fluorine atom, an iodine atom, 2-fluoroethyl, 3-fluoropropyl, methoxy, oxazolyl, pyridyl, oxadiazolyl, imidazopyridyl, imidazothiazolyl, benzothiazolyl or the like.

In addition, halogen, hydroxyl, alkyl, halogenoalkyl, halogenoalkenyl, halogenoalkoxy, *N*,*N-*dialkylamino, *N*,*N-*dialkylaminoalkyl, hydroxyalkyl, carboxyalkyl, oxo, trialkyltin, trialkylsilyl and the like are preferable as the substituents on the saturated or unsaturated 5- or 6-membered cyclic hydrocarbon group, the saturated or unsaturated bicyclic or tricyclic condensed hydrocarbon group or the saturated or unsaturated 5- to 7-membered heterocyclic group.

The compound of the present invention represented by formula (I) includes all of the individual stereoisomer based on C=N of hydrazone and carbon-carbon double bond and mixture thereof and racemate, racemic mixture, single enantiomer, diastereomer mixture, individual diastereomer and the like optical or geometrical isomers and mixtures thereof.

Salts of the compound of the present invention represented by formula (I) are not particularly limited, so long as they are pharmaceutically acceptable salts, and specific examples include mineral acid salts such as hydrochloride, hydrobromide, hydroiodide, phosphate, nitrate and sulfate; organic sulfonic acid salts such as benzoate, methanesulfonate, 2-hydroxyethanesulfonate and *p*-toluenesulfonate; and organic carboxylic acid salts such as acetate, propanoate, oxalate, malonate, succinate, glutarate, adipate, tartarate, maleate, malate and mandelate. In addition, when the compound of the present invention represented by formula (I) has an acidic group, it may become a salt of an alkali metal ion or an alkaline earth metal ion. Solvates are not particularly limited, so long as they are pharmaceutically acceptable, and specific examples include hydrate, ethanol solvate and the like.

The compound of the present invention represented by formula (I) can be prepared by various methods, and an example of the production methods is described below. Also, in the reaction, it may be carried out by protecting substituents with protecting groups, if necessary, and the converting order of respective substituents should not be particularly limited.

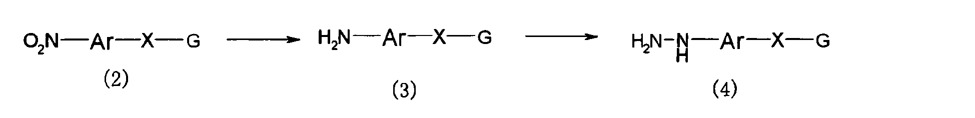

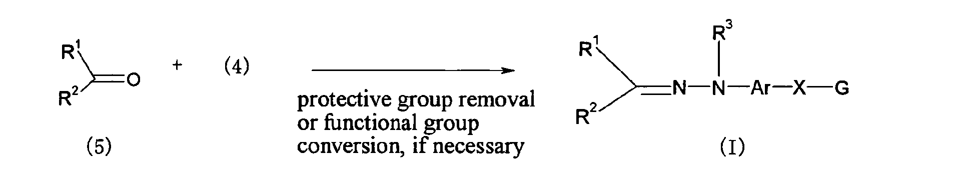

In the reaction scheme, R¹, R², R³, Ar, X and G have the same meanings as defined above.

The compound (I) of the present invention can be prepared by reacting a hydrazine compound represented by formula (4) with an aldehyde compound or ketone compound represented by formula (5), and subsequently removing protecting groups and converting functional groups, if necessary.

In general, the reaction is carried out in a solvent at room temperature or under heating, but the reaction smoothly progresses by carrying out the reaction with heating under reflux, depending on the kinds of the aldehyde compound and ketone compound, and it is more advantageous to carrying out the reaction using a dehydrator.

The solvent includes organic solvents which do not react with the substrate, product, reagent and the like, for example, various solvents such as ethanol, methanol, ether, tetrahydrofuran, benzene, toluene, xylene, dichloroethane, dichloromethane, chloroform, carbon tetrachloride, dioxane, dimethoxymethane, dimethoxyethane, ethyl acetate, acetonitrile, *N*,*N-*dimethylformamide and dimethyl sulfoxide, and mixed solvents thereof The solvent is preferably ethanol, methanol, benzene, toluene and the like and mixed solvents containing these solvents. The protecting groups can be removed in accordance with a usual method, and as an example of functional group conversion, in the case of a *tert*-butoxycarbonyl group as a protecting group of a nitrogen atom, the compound (I) of the present invention can be produced by using hydrochloric acid or trifluoroacetic acid.

The compound (I) of the present invention prepared by the above-described synthetic process can be isolated and purified as its free form or a salt thereof. Isolation and purification can be carried out by general chemical operations such as applying extraction, evaporation, crystallization, filtration, recrystallization, and various types of chromatography.

The free compound or a salt thereof obtained in this manner can be converted into other salt by carrying out a usual salt formation reaction.

An intermediate (hydrazine compound (4)) of the compound (I) of the present invention can be produced from an amino compound represented by formula (3). The intermediate can be produced in accordance with the usual way by reacting the amino compound (3) with sodium nitrite or isoamyl nitrite under cooling, at room temperature or under heating, using, as a solvent, an aqueous acidic solution such as hydrochloric acid, or a mixed solvent of ethanol, methanol, tetrahydrofuran, dioxane, ethyl acetate *N,N*-dimethylformamide and the like, and then using a reducing agent such as tin chloride, sodium sulfite, triphenylphosphine, zinc or sodium borohydride.

An intermediate (amino compound (3)) of the compound (I) of the present invention can be produced from a nitro compound represented by formula (2). The intermediate can be produced by catalytic reduction of the nitro compound (2) in the presence of a catalyst such as palladium-carbon, Raney nickel or platinum, using, as a solvent, ethanol, methanol, tetrahydrofuran, dioxane, ethyl acetate *N,N-*dimethylformamide, water or the like or a mixed solvent thereof Alternatively, the intermediate can be produced by reduction reaction in the presence of a metal such as tin, zinc or iron in a tin chloride or acidic solution using, as a solvent, ethanol, methanol, tetrahydrofuran, dioxane, ethyl acetate *N,N-*dimethylformamide, water or the like or a mixed solvent thereof,

The compound (I) of the present invention labeled with a radiation-releasing isotope is described below.

The radioactive element which can be used for labeling the compound (I) of the present invention includes ¹¹C, ¹³N, ¹⁵O, ¹⁸F, ⁶⁷Ga, ^{99m}Tc, ¹¹¹In, ¹²²I, ¹²³I, ¹²⁴I, ¹²⁵I, ¹³¹I, ¹³³Xe, ²⁰¹Ti and the like, and preferably examples include ¹¹C, ¹³N, ¹⁵O, ¹⁸F, and radioactive iodine atom such as ¹²²I, ¹²³I, ¹²⁴I, ¹²⁵I and ¹³¹I.

An example of the method for producing the compound (I) of the present invention labeled with a radioactive iodine atom is described below.

The compound (I) of the present invention labeled with a radioactive iodine atom can be produced by reacting an alkali metal radioactive iodine compound, such as a sodium compound of radioactive iodine or a potassium compound of radioactive iodine, with the compound (I) of the present invention having an iodine atom, a trialkyltin group and/or a trialkylsilyl silyl group as a substituent.

Since the reaction varies between a case in which the compound (I) of the present invention labeled with a radiation-releasing isotope has a iodine atom as a substituent and a case in which it has a trialkyltin group or a trialkylsilyl silyl group, this is described below.

That is, when it has an iodine atom as a substituent, the non-radioactive iodine atom can be converted into a radioactive iodine atom by reacting it with an alkali metal radioactive iodine compound under acidic conditions. When it has a trialkyltin group or a trialkylsilyl silyl group as a substituent, the compound (I) of the present invention labeled with a radioactive iodine atom can be produced by reacting it with an alkali metal radioactive iodine compound under acidic conditions, and further reacting it with an oxidizing agent such as chloramine T, hydrogen peroxide or peracetic acid.

In addition, regarding the compound (I) of the present invention labeled with ¹¹C, ¹³N, ¹⁵O, ¹⁸F or the like, an appropriate atom in the compound (I) of the present invention may be simply substituted with such a radiation-releasing isotope. Also, since various labeling methods are known, the compound can be produced in accordance with a known method.

When the thus obtained compound (I) of the present invention labeled with a radiation-releasing isotope is used as a radioactive medicament, it is preferable to purify it by removing unreacted radioactive ions and insoluble impurities using membrane filters, columns packed with various packing materials, HPLC and the like.

Preferred examples of the compound of the present invention represented by formula (I) include the compounds which are shown later in Examples, salts of the compounds and solvates thereof, as well as the compounds shown in the following Tables, salts of the compounds and solvates thereof.

In the tables, Me represents methyl, and Et represents ethyl.

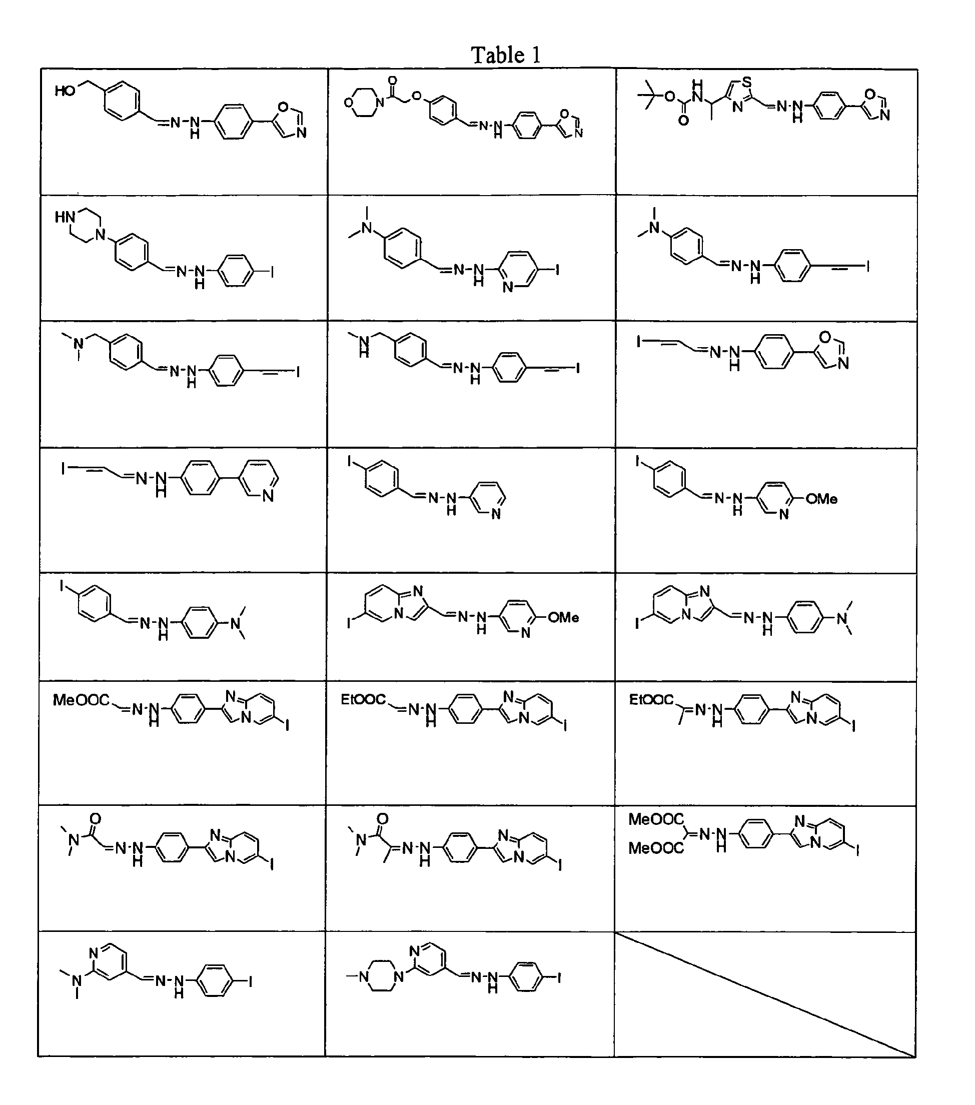

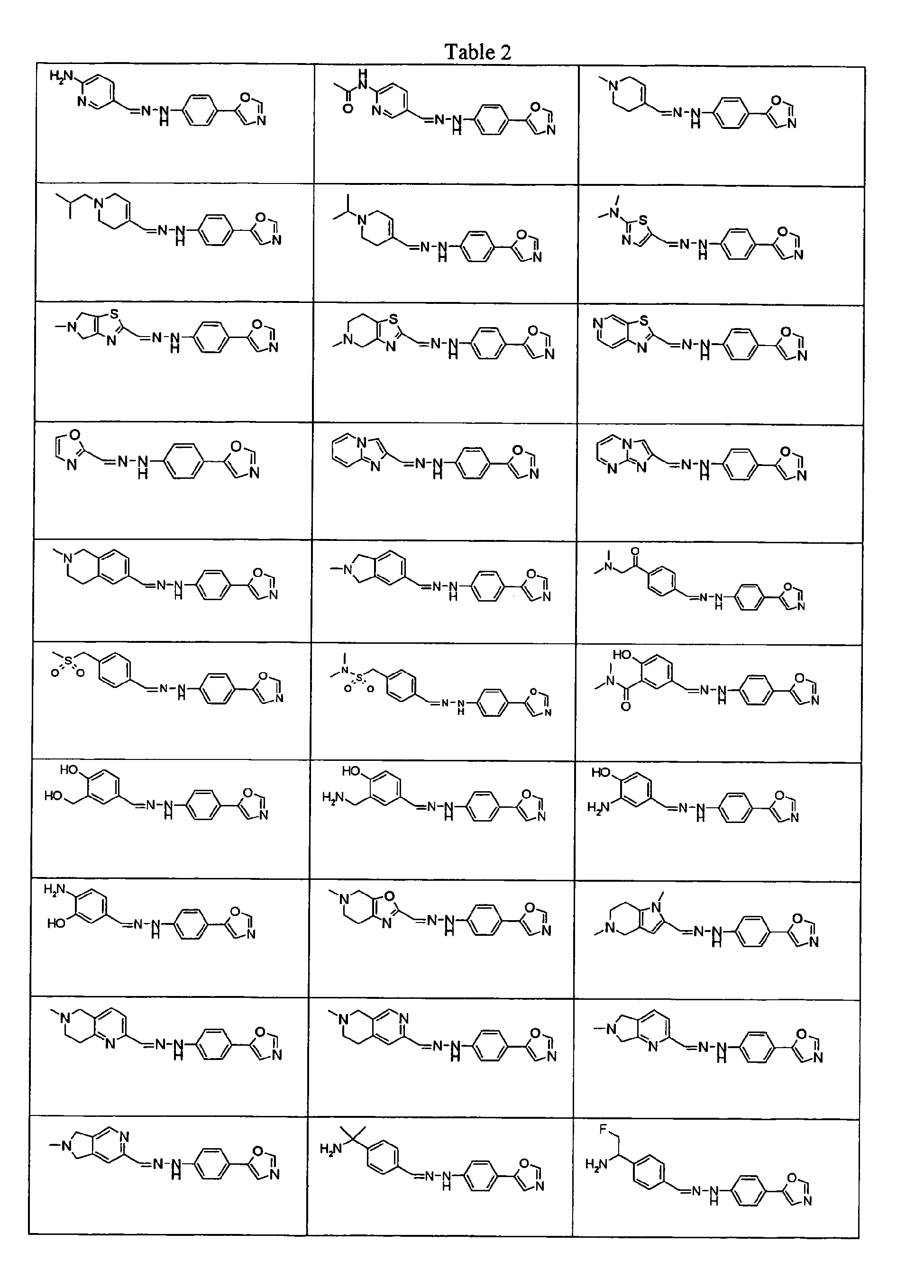

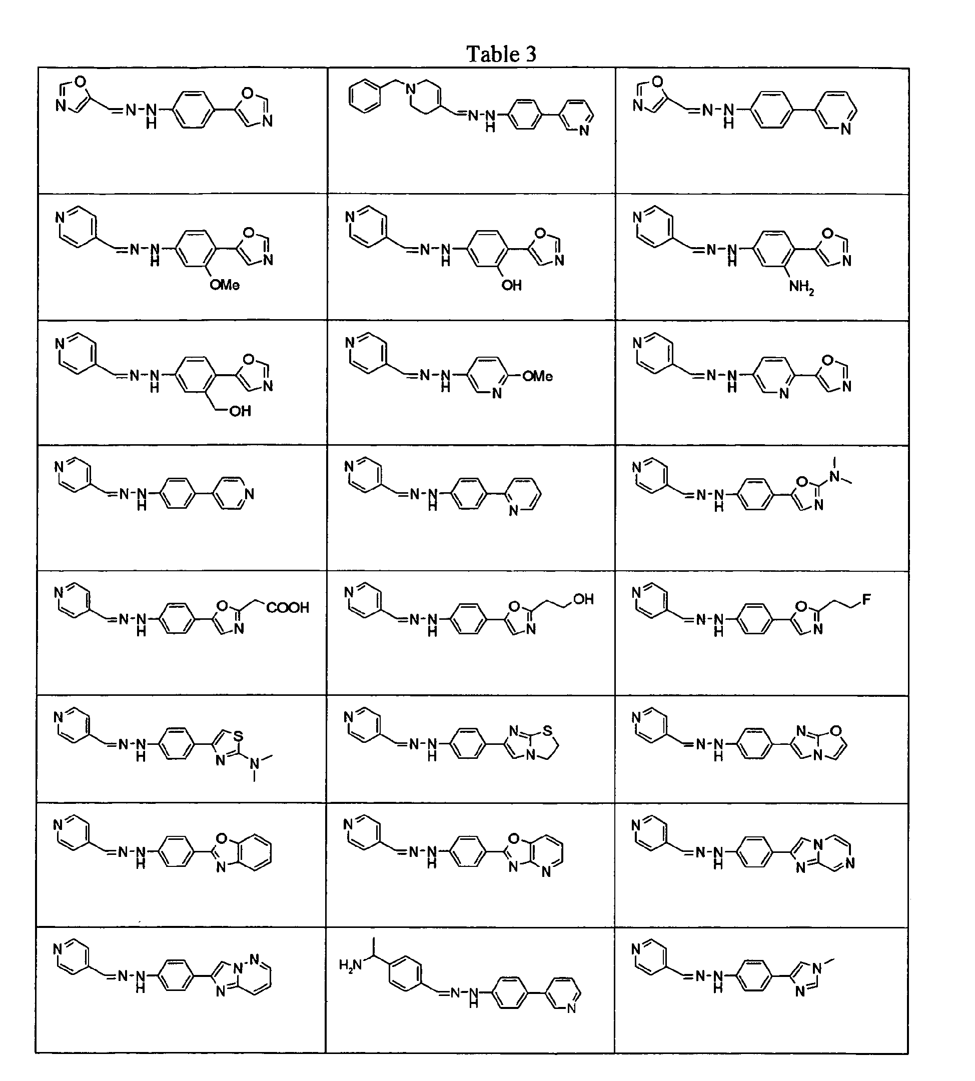

When the compound of the present invention represented by formula (I), a salt thereof or a solvate thereof (hereinafter also referred to as "compound of the present invention") is used as a medicament, or when the compound of the present invention represented by formula (I) labeled with a radiation-releasing isotope, a salt thereof or a solvate thereof (hereinafter also referred to as "labeled compound of the present invention") is used as a medicament or a radioactive diagnostic agent, these can be orally or parenterally administered to human and animals, they can be administered alone, but it is general to make them into pharmaceutical preparations. The dosage form is selected based on the usage or the target disease. As the dosage forms for oral administration, tablets, capsules powders, internal solutions can be exemplified, and injections, eye drops, suppositories, suspensions, ointments, cataplasmas, liniments, lotions, aerosols, plasters and the like can be exemplified as the dosage forms for parenteral administration.

Preparation of these dosage forms is generally carried out by an optional method well known in the technical field of manufacturing pharmacy, by mixing with one or plural pharmaceutically acceptable carriers, and specifically, it may be carried out by optionally using pharmaceutical additives such as an excipient, a binder, a disintegrating agent, a fluidizing agent, a suspending agent, a moisture keeping agent and a solubilization assisting agent, within such a range that effects of the compound of the present invention are not spoiled.

Dose of the compound of the present invention and the labeled compound of the present invention may be optionally decided based on the kind and degree of each disease, administration method, compound to be administered, and age, sex and body weight of each patient. For example, in the case of oral administration, from about 0.1 mg to about 1000 mg per day per adult can be exemplified. Regarding the administration time, before meals, during meals, after meals, before going to bed and the like can be exemplified, and the administration may be carried out once or dividing into several times.

In addition, in the case of the labeled compound of the present invention, it may be optionally decided also by taking measuring conditions of radiation imaging devices such as SPECT and PET into consideration. For example, it is from 37 to 555 MBq, preferably from 111 to 370 MBq as the radioactivity.

The labeled compound of the present invention can also be used as an *in vivo* or *in vitro* diagnostic agent for conveniently inspecting *in vivo* or *in vitro* accumulation of amyloid of human and animals. Specifically, the diagnosis can be carried out by administering the labeled compound of the present invention to a subject such as human, an animal, or a human or animal cell or tissue, and using a radiation imaging device such as SPECT or PET which detects the radioactive label.

The present invention is described below in more detail based on Reference Examples, Examples and Test Examples, although the present invention is not limited to the mixture.

The following abbreviations are used in the descriptions of Reference Examples and Examples.
- (Boc)₂O:: di-tert-butyl dicarbonate
- THF:: tetrahydrofuran
- DMF:: dimethylformamide
- DMSO:: dimethyl sulfoxide
- EDC·HCl:: 1-ethyl-3-(3-dimethylaminopropyl)carbodiimide hydrochloride
- HOBt:: 1-hydroxybenzotriazole
- NMM:: *N*-methylmorpholine
- AIBN:: 2,2'-azobisisobutyronitrile
- DMAP:: 4-dimethylaminopyridine

### Reference Example 1

### 1-(4-Nitrophenyl)imidazole

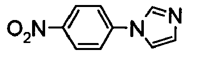

4-Chloronitrobenzene (5.0 g) and imidazole (10.8 g) were heat-melted at 150°C and stirred for 15 hours. The reaction solution was poured into ice water (200 ml) and vigorously stirred for 1 hour. The insoluble material was collected by filtration and washed with water and ethanol to obtain the title compound (4.37 g) as a brown solid.
¹H-NMR (400 MHz, CDCl₃) δ: 7.26 (1H, br s), 7.38 (1H, br s), 7.58 (2H, d, J=7.0 Hz), 7.98 (1H, s), 8.38 (2H, d, J=7.0 Hz).
ESI-MS m/z: 190 (M+H)⁺

### Reference Example 2

### 4-(Imidazol-1-yl)phenylamine

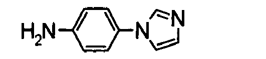

Under hydrogen atmosphere, an ethanol solution (80 ml) of 1-(4-nitrophenyl)imidazole (1.47 g) and 20% palladium hydroxide-carbon (300 mg) was stirred at room temperature for 5 hours. The catalyst was filtered, and the filtrate was concentrated under reduced pressure and then crystallized by adding hexane to obtain the title compound (1.17 g) as a yellow solid.
¹H-NMR (400 MHz, CD₃OD) δ: 6.78 (2H, d, J=9.0 Hz), 7.07 (1H, s), 7.20 (2H, d, J=8.8 Hz), 7.36 (1H, s), 7.89 (1H, s).
FAB-MS m/z: 160 (M+H)⁺.

### Reference Example 3

### 4-(Imidazol-1-yl)phenylhydrazine

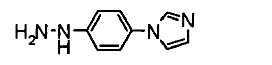

1-(4-Aminophenyl)imidazole (1.97 g) was dissolved in concentrated hydrochloric acid (15 ml) and water (30 ml), and an aqueous solution (6 ml) of sodium nitrite (1.02 g) was slowly added dropwise to the solution at 0°C. After stirring for 30 minutes, a concentrated hydrochloric acid solution (3 ml) of tin chloride dihydrate (5.90 g) was added to the solution, followed by stirring at room temperature for 1 hour. The reaction solution was alkalified by adding an aqueous solution of 20% potassium hydroxide, chloroform:methanol = 9:1 (500 ml) solution was added to the mixture and filtered through celite. The organic layer was dried over anhydrous sodium sulfate, the solvent was evaporated, and the thus obtained solid was washed with diethyl ether to obtain the title compound (932 mg) as a yellow solid.
¹H-NMR (400 MHz, DMSO-d₆) δ: 3.32 (2H, br s), 4.13 (1H, br s), 6.84 (2H, d, J=8.8 Hz), 7.02 (1H, s), 7.30 (2H, d, J=8.8 Hz), 7.51 (1H, s), 7.99 (1H, s).
ESI-MS m/z: 175 (M+H)⁺

### Reference Example 4

### 4-(Oxazol-5-yl)phenylamine

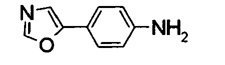

1-Nitro-4-(oxazol-5-yl)benzene (1.0 g) and 10% Pd-C (0.15 g) were added to ethanol (150 ml), followed by catalytic reduction at atmospheric pressure for 2 hours. The catalyst was removed by filtration, and the filtrate was concentrated under reduced pressure to obtain the title compound (0.82 g) as a crystalline solid.
¹H-NMR (400 MHz, CDCl₃) δ: 3.83 (2H, br),6.71 (2H, d, J=8.3 Hz), 7.15 (1H, s), 7.45 (2H, d, J=8.33 Hz), 7.83 (1H, s).

### Reference Example 5

### 4-(Oxazol-5-yl)phenylhydrazine

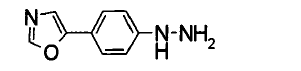

4-(Oxazol-5-yl)phenylamine (0.51 g) was added to water (2.5 ml) and dissolved by adding concentrated hydrochloric acid (5 ml) dropwise. Water (2 ml) solution of sodium nitrite (0.79 g) was added dropwise to the mixture under ice-cooling. After stirring for 30 minutes, a concentrated hydrochloric acid (5 ml) solution of tin chloride dihydrate (1.8 g) was added dropwise to the mixture, and the mixture was allowed to warm to room temperature. The mixture was alkalified with concentrated aqueous ammonia and extracted with chloroform to obtain the title compound (0.30 g) as a crystalline solid.
¹H-NMR (400 MHz, CDCl₃) δ: 3.62 (2H, br s), 5.33 (1H, br s), 6.88 (2H, d, J=8.57 Hz), 7.18 (1H, s), 7.52 (2H, d, J=8.57 Hz), 7.84 (1H, s).

### Reference Example 6

### 2-(4-Nitrophenyl)-4,5-dihydrothiazole

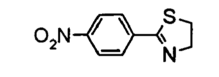

Potassium carbonate (3.19 g) was added to an ethanol solution (30 ml) of 4-cyanonitrobenzene (1.14 g) and 2-mercaptoethylamine hydrochloride (874 mg) at room temperature, followed by heating under reflux for 14 hours. The solvent was evaporated, and the thus obtained residue was diluted with ethyl acetate (300 ml) and washed twice with water (150 ml). The mixture was dried over anhydrous sodium sulfate and then purified by flash silica gel column chromatography (hexane:ethyl acetate = 4:1 to 1:1) to obtain the title compound (550 mg) as a white solid.
¹H-NMR (400 MHz, CDCl₃) δ: 3.50 (2H, t, J=8.3 Hz), 4.52 (2H, t, J=8.3 Hz), 7.99 (2H, br d, J=8.8 Hz), 8.27 (2H, br d, J=8.8 Hz).
ESI-MS m/z: 209 (M+H)⁺

### Reference Example 7

### 4-(4,5-Dihydrothiazol-2-yl)phenylamine

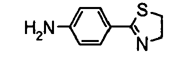

Zinc powder (863 mg) and ammonium chloride (706 mg) were added to an ethanol solution (20 ml) of 2-(4-nitrophenyl)-4,5-dihydrothiazole (550 mg), followed by stirring at room temperature for 14 hours. After celite filtration, the solvent was evaporated and the residue was recrystallized from hexane to obtain the title compound (472 mg) as a white solid.
¹H-NMR (400 MHz, CD₃OD) δ: 3.43 (2H, t, J=8.3 Hz), 4.35 (2H, t, J=8.3 Hz), 6.93 (2H, d, J=8.8 Hz), 7.66 (2H, d, J=8.8 Hz).
ESI-MS m/z: 178M⁺.

### Reference Example 8

### 3-(Oxazol-5-yl)nitrobenzene

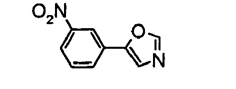

3-Nitrobenzaldehyde (10 g) and p-toluenesulfonylmethyl isocyanide (12.9 g) were dissolved in methanol (120 ml), potassium carbonate (11.0 g) was added to the mixture at room temperature and heated under reflux for 1.5 hours. After evaporation of the solvent, crystallization was carried out by adding water (300 ml), and the solid was washed with water, ethanol and hexane to obtain the title compound (8.79 g) as a yellow solid.
¹H-NMR (400 MHz, CDCl₃) δ: 7.53 (1H, s), 7.64 (1H, t, J=7.8 Hz), 7.97 (1H, d, J=7.8 Hz), 8.00 (1H, s), 8.20 (1H, d, J=10.5 Hz), 8.52 (1H, br s).
FAB-MS m/z: 191 (M+H)⁺

### Reference Example 9

### 3-(Oxazol-5-yl)phenylamine

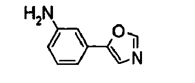

3-(Oxazol-5-yl)nitrobenzene (3.56 g) was dissolved in ethanol (80 ml) and ethyl acetate (80 ml), 5% palladium-carbon (1.9 g) was added to the mixture and stirred at room temperature for 15 hours in an atmosphere of hydrogen. After filtration of the catalyst, the solvent was evaporated, and the thus obtained crystals were washed with hexane to obtain the title compound (2.80 g) as a white solid.
¹H-NMR (400 MHz, CDCl₃) δ: 3.77 (2H, br s), 6.66 (1H, d, J=7.8 Hz), 6.98 (1H, br s), 7.05 (1H, br d, J=7.9 Hz), 7.20 (1H, t, J=8.1 Hz), 7.30 (1H, s), 7.88 (1H, s).
FAB-MS m/z: 161 (M+H)⁺.

### Reference Example 10

### 2-(Oxazol-5-yl)nitrobenzene

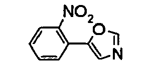

2-Nitrobenzaldehyde (10 g) and p-toluenesulfonylmethyl isocyanide (12.9 g) were dissolved in methanol (120 ml), potassium carbonate (11.0 g) was added to the mixture and heated under reflux for 2 hours. After evaporation of the solvent, crystallization was carried out by adding water (300 ml), and the solid was washed with water, hexane and ethanol and dried to obtain the title compound (8.55 g) as a yellow solid.
¹H-NMR (400 MHz, CDCl₃) δ: 7.41 (1H, s), 7.55 (1H, ddd, J=1.4 Hz, 7.9 Hz, 7.8 Hz), 7.67 (1H, ddd, J=1.3 Hz, 7.9 Hz, 7.8 Hz), 7.72 (1H, dd, J=1.4 Hz, 7.8 Hz), 7.86 (1H, dd, J=1.2 Hz, 8.0 Hz), 7.97 (1H, s).
FAB-MS m/z: 191 (M+H)⁺

### Reference Example 11

### 2-(Oxazol-5-yl)phenylamine

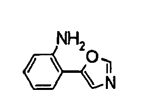

5% Palladium-carbon (500 mg) was added to an ethanol solution (30 ml) of 2-(oxazol-5-yl)nitrobenzene (1.01 g), followed by stirring at room temperature for 15 hours in an atmosphere of hydrogen. After removing the catalyst by filtration, the solvent was evaporated, and the residue was purified by flash silica gel column chromatography (hexane:acetone = 1:1) to obtain the title compound (777 mg) as a pale yellow solid.
¹H-NMR (400 MHz, CDCl₃) δ: 4.19 (2H, br s), 6.78 (1H, d, J=8.3 Hz), 6.83 (1H, t, J=10.2 Hz), 7.18 (1H, t, J=8.1 Hz), 7.31 (1H, s), 7.48 (1H, d, J=6.6 Hz), 7.95 (1H, s).

### Reference Example 12

### 1-(4-Nitrophenyl)pyrazole

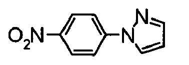

4-Chloronitrobenzene (6.0 g) and pyrazole (25.9 g) were heat-melted at 210°C and stirred for 7 days. The reaction solution was diluted with ethyl acetate (400 ml), washed with water (100 ml) three times and dried over anhydrous sodium sulfate. After evaporation of the solvent, the residue was purified by flash silica gel column chromatography (hexane:ethyl acetate = 1:1), the thus obtained solid was dissolved in acetone (100 ml), water (5 ml) was added to the mixture and stirred overnight, and the precipitated crystals were collected by filtration to obtain the title compound (4.32 g) as a yellow solid.
¹H-NMR (400 MHz, CDCl₃) δ: 6.56 (1H, br s), 7.80 (1H, s), 7.89 (2H, d, J=9.0 Hz), 8.04 (1H, s), 8.35 (2H, d, J=8.3 Hz).
ESI-MS m/z: 190 (M+H)⁺

### Reference Example 13

### 4-(Pyrazol-1-yl)phenylamine

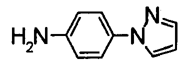

Under hydrogen atmosphere, an ethanol solution (80 ml) of 1-(4-nitrophenyl)pyrazole (2.91 g) and 5% palladium-carbon (1.4 g) was stirred at room temperature for 24 hours. After filtration of the catalyst, the filtrate was concentrated under reduced pressure and then purified by flash silica gel column chromatography (hexane:ethyl acetate = 1:1) to obtain the title compound (2.45 g) as a colorless oil.
¹H-NMR (400 MHz, CDCl₃) δ: 3.73 (2H, br s), 6.41 (1H, br s), 6.75 (2H, d, J=8.5 Hz), 7.44 (2H, d, J=8.5 Hz), 7.67 (1H, s), 7.78 (1H, s).

### Reference Example 14

### 4-(Pyridin-3-yl)phenylamine

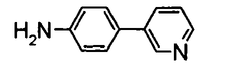

Under argon atmosphere, 3-pyridyl-diethyl borane (1.5 g) and water (1 drop) were added to a THF solution (60 ml) of 4-aminobromobenzene (855 mg), tetrakistriphenylphosphine palladium (672 mg), tetrabutylammonium bromide (937 mg) and potassium hydroxide (979 mg), followed by heating under reflux for 14 hours. After concentration of the reaction solution, the mixture was diluted with ethyl acetate (300 ml) and washed twice with water (100 ml). After drying over anhydrous sodium sulfate, the solvent was evaporated, and the residue was purified by flash silica gel column chromatography (hexane:ethyl acetate = 1:1 to ethyl acetate) to obtain the title compound (465 mg) as a white solid.
¹H-NMR (400 MHz, CDCl₃) δ: 3.79 (2H, br s), 6.77 (2H, d, J=6.6 Hz), 7.30 (1H, dd, J=4.9 and 7.8 Hz), 7.40 (2H, d, J=6.6 Hz), 7.80 (1H, dd, J=3.9 Hz, 7.9 Hz), 8.50 (1H, d; J=4.7 Hz), 8.79 (1H, s).
FAB-MS m/z: 171 (M+H)⁺.

### Reference Example 15

### 4-(Pyridin-3-yl)phenylhydrazine

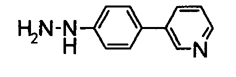

4-(Pyridin-3-yl)phenylamine (214 mg) was dissolved in hydrochloric acid (4 ml) and water (2 ml), and an aqueous solution (2 ml) of sodium nitrite (95 mg) was added dropwise to the solution over 30 minutes at 0°C. After stirring at the same temperature for 1 hour, a hydrochloric acid solution (2 ml) of tin(II) chloride dihydrate (709 mg) was added to the mixture, followed by stirring at room temperature for 1 hour. The reaction solution was alkalified by adding an aqueous solution of 20 wt% potassium hydroxide and extracted with chloroform: methanol = 9:1 (100 ml). After drying over anhydrous sodium sulfate, the solvent was evaporated, and the thus obtained solid was washed with diethyl ether to obtain the title compound (183 mg) as a pale yellow solid.
¹H-NMR (400 MHz, CDCl₃) δ: 3.64 (2H, br s), 5.31 (1H, br s), 6.93 (2H, d, J=8.9 Hz), 7.31 (1H, dd, J=4.9 Hz, 7.8 Hz), 7.48 (2H, d, J=8.8 Hz), 7.82 (1H, dd, J=1.7 Hz, 7.9 Hz), 8. 51 (1H, dd, J=1.5 Hz, 4.9 Hz), 8.81 (1H, d, J=2.4 Hz).
ESI-MS m/z: 186 (M+H)⁺

### Reference Example 16

### 2-(4-Nitrophenyl)[1,3,4]oxadiazole

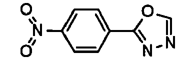

A mixture of 4-nitrobenzoic acid hydrazide (5.06 g) and orthoformic acid triethyl ester (100 ml) was stirred for 21 hours under reflux. After cooling, the reaction mixture was concentrated under reduced pressure, and the thus obtained residue was washed with diethyl ether and then collected by filtration. By recrystallizing the crude product from ethanol, the title compound (4.77 g) was obtained.
¹H-NMR (400 MHz, CDCl₃) δ: 8.31 (2H, m), 8.40 (2H, m), 8.61 (1H, s).

### Reference Example 17

### 4-([1,3,4]Oxadiazol-2-yl)phenylamine

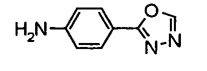

2-(4-Nitrophenyl)-[1,3,4]oxadiazole (4.37 g) and 5% Pd-C (2.2 g) were added to ethanol (100 ml)-ethyl acetate (175 ml), followed by catalytic reduction at atmospheric pressure for 7 hours. By removing the catalyst by filtration and concentrating the filtrate under reduced pressure, the title compound (3.53 g) was obtained as a crystalline solid.
¹H-NMR (400 MHz, CDCl₃) δ: 4.08 (2H, br s), 6.74 (2H, d, J=8.3 Hz), 7.87 (2H, d, J=8.5 Hz), 8.36 (1H, s).

### Reference Example 18

### 4-([1,3,4]Oxadiazol-2-yl)phenylhydrazine

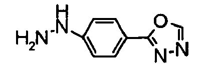

4-([1,3,4]Oxadiazol-2-yl)phenylamine (0.81 g) was dissolved in concentrated hydrochloric acid (7.5 ml) and water (3.8 ml), and an aqueous solution (3 ml) of sodium nitrite (414 mg) was added dropwise to the solution under ice-cooling. After stirring for 30 minutes, a concentrated hydrochloric acid solution (5 ml) of tin chloride dihydrate (2.71 g) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (61 mg) as a yellow solid.
¹H-NMR (400 MHz, CD₃OD) δ: 6.94 (2H, d, J=9.0 Hz), 7.83 (2H, d, J=9.0 Hz), 8.85 (1H, s).

### Reference Example 19

### 2-Methyl-5-(4-nitrophenyl)[1,3,4]oxadiazole

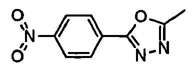

A mixture of 4-nitrobenzoic acid hydrazide (5.04 g) and orthoacetic acid triethyl ester (100 ml) was stirred for 24 hours under reflux. After cooling, the reaction mixture was concentrated under reduced pressure, and the thus obtained residue was washed with diisopropyl ether and then collected by filtration. The crude product was washed with ethanol and diisopropyl ether and then dried to obtain the title compound (4.28 g) as a crystalline solid.
¹H-NMR (400 MHz, CDCl₃) δ: 2.68 (3H, s), 8.23 (2H, d, J=8.5 Hz), 8.37 (2H, d, J=8.5 Hz), 8.61 (1H, s).

### Reference Example 20

### 4-(5-Methyl[1,3,4]oxadiazol-2-yl)phenylamine

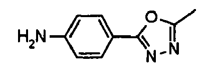

2-Methyl-5-(4-nitrophenyl)[1,3,4]oxadiazole (4.11 g) and 5% Pd-C (2.1 g) were added to ethanol (100 ml)-ethyl acetate (200 ml), followed by catalytic reduction at atmospheric pressure for 7 hours. By removing the catalyst by filtration and concentrating the filtrate under reduced pressure, the title compound (3.43 g) was obtained as a crystalline solid.
¹H-NMR (400 MHz, CDCl₃) δ: 2.57 (3H, s), 7.03 (2H, br s), 6.72 (2H, d, J=8.6 Hz), 7.81 (2H, d, J=8.6 Hz).

### Reference Example 21

### 4-(5-Methyl[1,3,4]oxadiazol-2-yl)phenylhydrazine

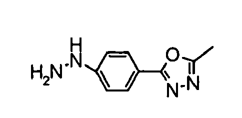

4-(5-Methyl[1,3,4]oxadiazol-2-yl)phenylamine (0.88 g) was dissolved in concentrated hydrochloric acid (7.5 ml) and water (3.8 ml), and an aqueous solution (3 ml) of sodium nitrite (414 mg) was added dropwise to the mixture under ice-cooling. After stirring for 30 minutes, a concentrated hydrochloric acid solution (5 ml) of tin chloride dihydrate (2.71 g) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (659 mg) as a yellow solid.
¹H-NMR (400 MHz, CDCl₃) δ: 2.58 (3H, s), 3.67 (2H, br s), 5.53 (1H, br s), 6.89 (2H, d, J=9.0 Hz), 7.88 (2H, d, J=9.0 Hz).

### Reference Example 22

### N-hydroxy-4-nitrobenzamidine

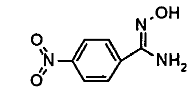

Potassium carbonate (12.44 g) was added to a methanol (300 ml) solution of 4-nitrobenzcyanide (4.44 g) and hydroxylamine hydrochloride (6.25 g), and the mixture was stirred under reflux for 14 hours. After cooling and subsequent removal of the insoluble material by filtration, the filtrate was concentrated under reduced pressure, and water was added to the thus obtained residue. After extraction with ethyl acetate, the organic layer was washed with water and saturated brine and dried over sodium sulfate. The solvent was evaporated under reduced pressure, and the thus obtained residue was washed with diisopropyl ether and n-hexane and then dried to obtain the title compound (4.80 g) as a crystalline solid.
¹H-NMR (400 MHz, DMSO-d₆) δ: 6.06 (2H, s), 7.94 (2H, d, J=9.0 Hz), 8.22 (2H, d, J=9.0 Hz), 10.13 (1H, s).

### Reference Example 23

### 5-Methyl-3-(4-nitrophenyl)[1,2,4]oxadiazole

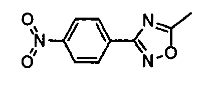

A mixture of *N*-hydroxy-4-nitrobenzamidine (1.0 g) and acetic anhydride (30 ml) was stirred under reflux for 11 hours. After cooling, the reaction mixture was concentrated under reduced pressure, and to the thus obtained residue was added saturated aqueous sodium bicarbonate solution and extracted with ethyl acetate. The organic layer was washed with water and saturated brine and dried over sodium sulfate. The solvent was evaporated under reduced pressure, the thus obtained residue was purified by silica gel column chromatography, and the fraction obtained from the eluate of dichloromethane:methanol = 100:1 was concentrated under reduced pressure to obtain the title compound (690 mg) as a crystalline solid.
¹H-NMR (400 MHz, CDCl₃) δ: 2.70 (3H, s), 8.18-8.40 (4H, m).

### Reference Example 24

### 3-(4-Nitrophenyl)[1,2,4]oxadiazole

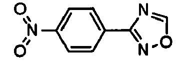

A mixture of *N*-hydroxy-4-nitrobenzamidine (1.0 g) and orthoformic acid triethyl ester (20 ml) was stirred under reflux for 24 hours. After cooling, the reaction mixture was concentrated under reduced pressure, and the thus obtained residue was washed with diisopropyl ether and ethanol and then collected by filtration and dried to obtain the title compound (520 mg) as a crystalline solid.
¹H-NMR (400 MHz, CDCl₃) δ: 8.28-8.45 (4H, m), 8.85 (1H, s).

### Reference Example 25

### 4-(5-Methyl[1,2,4]oxadiazol-3-yl)phenylamine

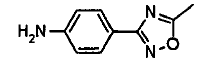

Zinc (1.91 g) was added to a methanol solution (40 ml) of 5-methyl-3-(4-nitrophenyl)[1,2,4]oxadiazole (600 mg) and ammonium chloride (781 mg), followed by stirring under reflux for 1 hour. After cooling, the reaction mixture was concentrated under reduced pressure, a saturated aqueous sodium bicarbonate solution and methylene chloride were added to the thus obtained residue, and then the mixture was filtered through celite. The organic layer was dried over sodium sulfate and then the solvent was evaporated under reduced pressure to obtain the title compound (481 mg) as a crystalline solid.
¹H-NMR (400 MHz, CDCl₃) δ: 2.62 (3H, s), 3.94 (2H, br s), 6.73 (2H, d, J=8.5 Hz), 7.85 (2H, d, J=8.7 Hz).

### Reference Example 26

### 4-([1,2,4]Oxadiazol-3-yl)phenylamine

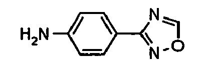

Zinc (1.47 g) was added to a methanol solution (40 ml) of 3-(4-nitrophenyl)[1,2,4]oxadiazole (430 mg) and ammonium chloride (602 mg), followed by stirring under reflux for 1 hour. After cooling, the reaction mixture was concentrated under reduced pressure, a saturated aqueous sodium bicarbonate solution and methylene chloride were added to the thus obtained residue, and then the mixture was filtered through celite. The organic layer was dried over sodium sulfate, the solvent was evaporated under reduced pressure, the thus obtained residue was purified by silica gel column chromatography, and then the fraction obtained from the eluate of dichloromethane:methanol = 20:1 was concentrated under reduced pressure to obtain the title compound (344 mg) as a crystalline solid.
¹H-NMR (400 MHz, CDCl₃) δ: 3.98 (2H, br s), 6.74 (2H, d, J=8.3 Hz), 7.91 (2H, d, J=8.3 Hz), 8.66 (1H, s).

### Reference Example 27

### 4-(5-Methyl[1,2,4]oxadiazol-3-yl)phenylhydrazine

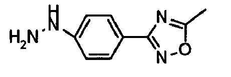

4-(5-Methyl[1,2,4]oxadiazol-3-yl)phenylamine (480 mg) was dissolved in concentrated hydrochloric acid (6.0 ml) and water (3.0 ml), and an aqueous solution (2 ml) of sodium nitrite (227 mg) was added dropwise to the mixture under ice-cooling. After stirring for 40 minutes, a concentrated hydrochloric acid solution (3 ml) of tin chloride dihydrate (1.48 g) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (452 mg) as a yellow solid.
¹H-NMR (400 MHz, CDCl₃) δ: 2.62 (3H, s), 3.65 (2H, br s), 5.44 (1H, br s), 6.88 (2H, d, J=9.0 Hz), 7.93 (2H, d, J=9.0 Hz).

### Reference Example 28

### 4-([1,2,4]oxadiazol-3-yl)phenylhydrazine

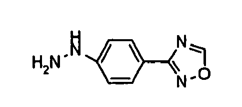

4-([1,2,4]Oxadiazol-3-yl)phenylamine (287 mg) was dissolved in concentrated hydrochloric acid (6.0 ml) and water (3.0 ml), and an aqueous solution (2 ml) of sodium nitrite (148 mg) was added dropwise to the mixture under ice-cooling. After stirring for 40 minutes, a concentrated hydrochloric acid solution (2 ml) of tin chloride dihydrate (963 mg) was added to the mixture, followed by stirring at room temperature for 2 hours. The reaction solution was alkalified by adding aqueous ammonia (28%), chloroform: methanol = 10:1 solution was added to the mixture and then filtered through celite. An organic layer of the filtrate was dried over anhydrous sodium sulfate, and the solvent was evaporated to obtain the title compound (220 mg) as a yellow solid.
¹H-NMR (400 MHz, CDCl₃) δ: 3.66 (2H, br s), 5.48 (1H, br s), 6.90 (2H, d, J=8.3 Hz), 7.98 (2H, d, J=8.3 Hz), 8.68 (1H, s).

### Reference Example 29

### N-(4-Methyliminomethylphenyl)acetamide

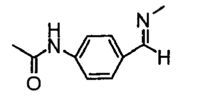

An aqueous methylamine solution (40%, 1.24 g) was added to an ethanol (30 ml) solution of 4-formylphenylacetamide (1.63 g), and the mixture was stirred under reflux for 1 hour. After cooling, the reaction solution was concentrated under reduced pressure, and chloroform was added to the thus obtained residue. The solvent was evaporated under reduced pressure to obtain the title compound (1.77 g) as a crystalline solid.
¹H-NMR (400 MHz, DMSO-d₆) δ: 2.06 (3H, s), 3.39 (3H, d, J=1.5 Hz), 7.64 (4H, s), 8.24 (1H, d, J=1.5 Hz), 10.10 (1H, s).

### Reference Example 30

### N-[4-(3-Methyl-3H-imidazol-4-yl)phenyl]acetamide

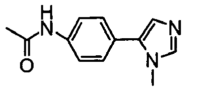

p-Toluenesulfonylmethyl isocyanide (2.66 g) was added to a methanol solution (40 ml) of *N*-(4-methyliminomethylphenyl)acetamide (1.20 g) and potassium carbonate (1.88 g), followed by stirring under reflux for 2 hours. p-Toluenesulfonylmethyl isocyanide (1.33 g) was added to the mixture, followed by further stirring under reflux for 2 hours. After cooling, the reaction solution was concentrated under reduced pressure, and to the thus obtained residue was added water and extracted with chloroform. The organic layer was washed with water and then dried over sodium sulfate. The solvent was evaporated under reduced pressure, the thus obtained residue was purified by silica gel column chromatography, and the fraction obtained from the eluate of dichloromethane:methanol = 10:1 was concentrated under reduced pressure to obtain the title compound (804 mg) as a crystalline solid.
¹H-NMR (400 MHz, CDCl₃) δ: 2.21 (3H, s), 3.65 (3H, s), 7.06 (1H, s), 7.32 (2H, d, J=8.5 Hz), 7.51 (1H, s), 7.63 (2H, d, J=8.5 Hz), 8.56 (1H, br s).

### Reference Example 31

### 4-(3-Methyl-3H-imidazol-4-yl)phenylamine

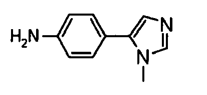

An aqueous solution of 20% sodium hydroxide was added to a DMSO solution (20 ml) of *N*-[4-(3-methyl-3H-imidazol-4-yl)phenyl]acetamide (708 mg), followed by stirring at 120°C for 2 hours. After cooling, the reaction solution was concentrated under reduced pressure, and to the thus obtained residue was added water and extracted with chloroform. The organic layer was washed with water and then dried over sodium sulfate. The solvent was evaporated under reduced pressure, the thus obtained residue was purified by silica gel column chromatography, and the fraction obtained from the eluate of dichloromethane:methanol = 10:1 was concentrated under reduced pressure. The thus obtained product was washed with diisopropyl ether and then dried to obtain the title compound (480 mg) as a crystalline solid.
¹H-NMR (400 MHz, CDCl₃) δ: 3.61 (3H, s), 3.80 (2H, br s), 6.73 (2H, d, J=8.1 Hz), 6.99 (1H, s), 7.16 (2H, d, J=8.1 Hz), 7.46 (1H, br s).

### Reference Example 32

### 4-(3-Methyl-3H-imidazol-4-yl)phenylhydrazine

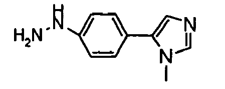

4-(3-Methyl-3H-imidazol-4-yl)phenylamine (430 mg) was dissolved in concentrated hydrochloric acid (4.0 ml), water (2.0 ml) and THF (2.0 ml), and an aqueous solution (2 ml) of sodium nitrite (206 mg) was added dropwise to the mixture under ice-cooling. After stirring for 40 minutes, a concentrated hydrochloric acid solution (3 ml) of tin chloride dihydrate (1.34 g) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (289 mg) as a yellow solid.
¹H-NMR (400 MHz, CDCl₃) δ: 3.24 (2H, br s), 3.62 (3H, s), 5.34 (1H, br s), 6.88 (2H, d, J=8.8 Hz), 7.01 (1H, s), 7.24 (2H, d, J=8.8 Hz), 7.47 (1H, s).

### Reference Example 33

### N-(Methoxycarbonyl)oxy-4-nitrobenzamidine

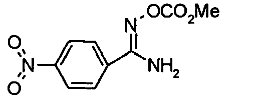

Methyl chlorocarbonate (0.47 ml) was added to methylene chloride (10 ml)-THF (10 ml) solution of *N*-hydroxy-4-nitrobenzamidine (1.0 g) and pyridine (0.67 ml) under ice-cooling, and the mixture was stirred at room temperature for 17 hours. The reaction solution was concentrated under reduced pressure, water was added to the resulting residue, and the thus obtained crystals were collected by filtration. The product was washed with diethyl ether and methanol and then dried to obtain the title compound (979 mg) as a crystalline solid.
¹H-NMR (400 MHz, DMSO-d₆) δ: 3.79 (3H, s), 7.12 (2H, br s), 7.96 (2H, d, J=9.0 Hz), 8.30 (2H, d, J=8.8 Hz).

### Reference Example 34

### 3-(4-Nitrophenyl)-4H-[1,2,4]oxadiazol-5-one

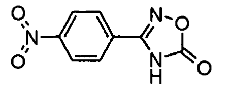

A pyridine (30 ml) solution of *N*-(methoxycarbonyl)oxy-4-nitrobenzamidine (0.89 g) was heated under reflux for 7 hours with stirring. After cooling, the reaction mixture was poured into ice water and acidified with concentrated hydrochloric acid. The thus precipitated crystals were collected by filtration, washed with water, ethanol and diethyl ether and then dried to obtain the title compound (715 mg) as a crystalline solid.
¹H-NMR (400 MHz, DMSO-d₆) δ: 8.07 (2H, d, J=9.0 Hz), 8.43 (2H, d, J=9.0 Hz), 13.28 (1H, s).

### Reference Example 35

### 4-Methyl-3-(4-nitrophenyl)-4H-[1,2,4]oxadiazol-5-one

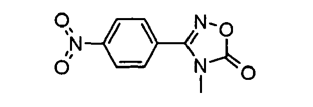

Sodium hydride (60% in paraffin liquid, 79 mg) was added to DMF (10 ml) solution of 3-(4-nitrophenyl)-4H-[1,2,4]oxadiazol-5-one (0.28 g) under ice-cooling, followed by stirring at room temperature for 20 minutes. Methyl iodide (280 mg) was added to the reaction mixture under ice-cooling, followed by stirring at room temperature for 3 hours. The reaction solution was concentrated under reduced pressure, and to the thus obtained residue was added water and extracted with chloroform. The organic layer was washed with water and then dried over magnesium sulfate. After evaporation of the solvent, the thus obtained crystals were washed with diisopropyl ether and then dried to obtain the title compound (208 mg) as a crystalline solid.
¹H-NMR (400 MHz, CDCl₃) δ: 3.39 (3H, s), 7.87 (2H, d, J=8.5 Hz), 8.44 (2H, d, J=8.3 Hz).

### Reference Example 36

### 4-(4-Methyl-5-oxo-4,5-dihydro[1,2,4]oxadiazol-3-yl)phenylamine

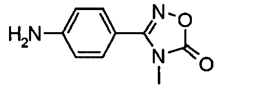

Zinc powder (455 mg) was added to a methanol solution (20 ml) of 4-methyl-3-(4-nitrophenyl)-4H-[1,2,4]oxadiazol-5-one (154 mg) and ammonium chloride (186 mg), followed by stirring under reflux for 1 hour. After cooling, the reaction mixture was filtered through celite, the filtrate was concentrated under reduced pressure, and a saturated aqueous sodium bicarbonate solution was added to the thus obtained residue. The mixture was extracted with chloroform-methanol (10:1), the organic layer was dried over sodium sulfate, and the solvent was evaporated under reduced pressure to obtain the title compound (118 mg) as a crystalline solid.
¹H-NMR (400 MHz, CDCl₃) δ: 3.32 (3H, s), 4.08 (2H, br s), 6.76 (2H, d, J=8.5 Hz), 7.40 (2H, d, J=8.5 Hz).

### Reference Example 37

### 4-(4-Methyl-5-oxo-4,5-dihydro[1,2,4]oxadiazol-3-yl)phenylhydrazine

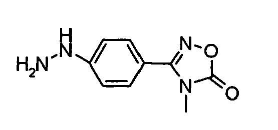

4-(4-Methyl-5-oxo-4,5-dihydro[1,2,4]oxadiazol-3-yl)phenylamine (100 mg) was dissolved in concentrated hydrochloric acid (2.0 ml) and water (2.0 ml), and an aqueous solution (1 ml) of sodium nitrite (43 mg) was added dropwise to the mixture under ice-cooling. After stirring for 40 minutes, a concentrated hydrochloric acid solution (1.5 ml) of tin chloride dihydrate (282 mg) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (53 mg) as a yellow solid.
¹H-NMR (400 MHz, CDCl₃-CD₃OD) δ: 3.33 (3H, s), 6.94 (2H, d, J=8.5 Hz), 7.46 (2H, d, J=8.8 Hz).

### Reference Example 38

### (E)-5-[2-(4-nitrophenyl)vinyl]oxazole

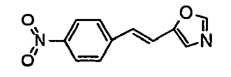

4-Nitrocinnamaldehyde (590 mg) and p-toluenesulfonylmethyl isocyanide (650 mg) were dissolved in methanol (40 ml), to the solution potassium carbonate (553 mg) was added at room temperature and heated under reflux for 1.5 hours. After evaporation of the solvent, water (300 ml) was added to the mixture, and the mixture was extracted with ethyl acetate. The organic layer was washed with water and saturated brine and then dried over magnesium sulfate. The solvent was evaporated, the thus obtained residue was purified by silica gel column chromatography, and the fraction obtained from the eluate of n-hexane:ethyl acetate = 1:1 was concentrated under reduced pressure to obtain the title compound (394 mg) as a crystalline solid.
¹H-NMR (400 MHz, CDCl₃) δ: 7.07 (1H, d, J=16.4 Hz), 7.15 (1H, d, J=16.4 Hz), 7.20 (1H, s), 7.61 (1H, d, J=8.8 Hz), 7. 91 (1H, s), 8.23 (2H, d, J=8. 8 Hz).

### Reference Example 39

### (E)-4-[2-(Oxazol-5-yl)vinyl]phenylamine

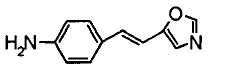

Zinc powder (1.10 g) was added to a methanol solution (50 ml) of (*E*)-5-[2-(4-nitrophenyl)vinyl]oxazole (364 mg) and ammonium chloride (449 mg), followed by stirring under reflux for 30 minutes. After cooling, the reaction mixture was filtered through celite, the filtrate was concentrated under reduced pressure, and a saturated aqueous sodium bicarbonate solution was added to the thus obtained residue. The mixture was extracted with chloroform-methanol (10:1), the organic layer was dried over sodium sulfate, and then the solvent was evaporated under reduced pressure to obtain the title compound (314 mg) as a crystalline solid.
¹H-NMR (400 MHz, DMSO-d₆) δ: 5.41 (2H, s), 6.55 (2H, d, J=8.3 Hz), 6.80 (1H, d, J=16.4 Hz), 6.91 (1H, d, J=16.4 Hz), 7.09 (1H, s), 7.26 (2H, d, J=8. Hz), 8.27 (1H, s).

### Reference Example 40

### (E)-4-[2-(Oxazol-5-yl)vinyl]phenylhydrazine

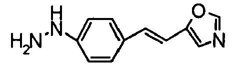

(*E*)-4-[2-(Oxazol-5-yl)vinyl]phenylamine (220 mg) was dissolved in concentrated hydrochloric acid (4.0 ml), water (2.0 ml) and THF (2.0 ml), and an aqueous solution (2 ml) of sodium nitrite (98 mg) was added dropwise to the mixture under ice-cooling. After stirring for 40 minutes, a concentrated hydrochloric acid solution (2 ml) of tin chloride dihydrate (639 mg) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (178 mg) as a crystalline solid.
¹H-NMR (400 MHz, CDCl₃) δ: 3.60 (2H, br s), 5.31 (1H, br s), 6.74 (1H, d, J=16.4 Hz), 6.81 (2H, d, J=8.3 Hz), 6.99 (1H, s), 7.02 (1H, d, J=16.4 Hz), 7.37 (2H, d, J=8.5 Hz), 7.80 (1H, s).

### Reference Example 41

### 2-(4-Nitrophenyl)imidazo[1,2-a]pyridine

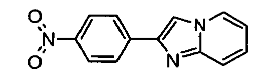

2-Aminopyridine (471 mg) was added to an acetone (30 ml) solution of 2-bromo-1-(4-nitrophenyl)ethanone (1.22 g), followed by stirring under reflux for 6 hours. After cooling, the thus precipitated crystals were collected by filtration, washed with diisopropyl alcohol and then dried to obtain the title compound (770 mg) as a crystalline solid.
¹H-NMR (400 MHz, CDCl₃-CD₃OD) δ: 6.70 (1H, t, J=6.8 Hz), 7.29 (1H, t, J=6.8 Hz), 7.64 (1H, d, J=9.3 Hz), 8.06 (1H, s), 8.09 (2H, d, J=8.3 Hz), 8.21 (1H, d, J=6.8 Hz), 8.30 (2H, d, J=8.3 Hz).

### Reference Example 42

### 4-(Imidazo[1,2-a]pyridin-2-yl)phenylamine and

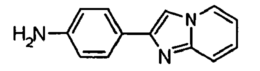

### 4-(5,6,7,8-Tetrahydroimidazo[1,2-a]pyridin-2-yl)phenylamine

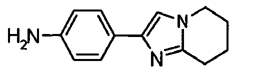

5% Pd-C (370 mg) was added to a methanol (150 ml)-THF (150 ml) solution of 2-(4-nitrophenyl)imidazo[1,2-a]pyridine (740 mg), followed by catalytic reduction under hydrogen pressure for 5 hours. The catalyst was removed by filtration, the filtrate was concentrated under reduced pressure, the thus obtained residue was purified by silica gel column chromatography, and 4-(imidazo[1,2-a]pyridin-2-yl)phenylamine (439 mg) was obtained as a crystalline solid by concentrating the low polar fraction obtained from the eluate of chloroform:methanol = 10:1 under reduced pressure, and 4-(5,6,7,8-tetrahydroimidazo[1,2-a]pyridin-2-yl)phenylamine (185 mg) as a crystalline solid by concentrating the high polar fraction under reduced pressure.
4-(Imidazo[1,2-a]pyridin-2-yl)phenylamine:
¹H-NMR (400 MHz, DMSO-d₆) δ: 5.21 (2H, br s), 6.61 (2H, m), 6.81 (1H, m), 7.16 (1H, m), 7.48 (1H, dd, J=9.0 Hz, 0.5 Hz), 7.62 (2H, m), 8.11 (1H, d, J=0.8 Hz), 8.44 (1H, d, J=6.8 Hz).
4-(5,6,7,8-Tetrahydroimidazo[1,2-a]pyridin-2-yl)phenylamine:
¹H-NMR (400 MHz, CDCl₃) δ: 1.90-2.00 (4H, m), 2.91 (2H, t, J=6.3 Hz), 3.62 (2H, br s), 3.95 (2H, t, J=5.7 Hz), 6.68 (2H, d, J=8.5 Hz), 6.91 (1H, s), 7.53 (2H, d, J=8.5 Hz).

### Reference Example 43

### 4-(Imidazo[1,2-a]pyridin-2-yl)phenylhydrazine

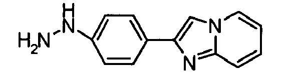

4-(Imidazo[1,2-a]pyridin-2-yl)phenylamine (170 mg) was dissolved in concentrated hydrochloric acid (4.0 ml), water (2.0 ml) and THF (4.0 ml), and an aqueous solution (2 ml) of sodium nitrite (67 mg) was added dropwise to the mixture under ice-cooling. After stirring for 40 minutes, a concentrated hydrochloric acid solution (2 ml) of tin chloride dihydrate (439 mg) was added to the mixture, followed by stirring at room temperature for 3 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (180 mg) as a crystalline solid.
¹H-NMR (400 MHz, CDCl₃) δ: 3.60 (2H, br s), 5.30 (1H, br s), 6.74 (1H, t, J=7.4 Hz), 6.88 (2H, d, J=8.3 Hz), 7.13 (1H, t, J=7.9 Hz), 7.59 (1H, d, J=9.3 Hz), 7.75 (1H, s), 7.84 (2H, d, J=8.3 Hz), 8.08 (1H, d, J=6.1 Hz).

### Reference Example 44

### 4-(5,6,7, 8-Tetrahydroimidazo[1,2-a]pyridin-2-yl)phenylhydrazine

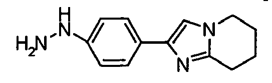

4-(5,6,7,8-Tetrahydroimidazo[1,2-a]pyridin-2-yl)phenylamine (133 mg) was dissolved in concentrated hydrochloric acid (4.0 ml) and water (2.0 ml), and an aqueous solution (2 ml) of sodium nitrite (52 mg) was added dropwise to the mixture under ice-cooling. After stirring for 40 minutes, a concentrated hydrochloric acid solution (2 ml) of tin chloride dihydrate (336 mg) was added to the mixture, followed by stirring at room temperature for 2 hours. The reaction solution was alkalified by adding aqueous ammonia (28%), chloroform:methanol = 10:1 solution was added to the mixture and then filtered through celite. An organic layer of the filtrate was dried over anhydrous sodium sulfate, and the solvent was evaporated to obtain the title compound (127 mg) as a crystalline solid.
¹H-NMR (400 MHz, CDCl₃) δ: 1.94-2.00 (4H, m), 2.93 (2H, t, J=6.2 Hz), 3.96 (2H, t, J=5.8 Hz), 5.17 (1H, m), 6.81 (2H, d, J=8.3 Hz), 6.95 (1H, s), 7.62 (2H, d, J=8.3 Hz).

### Reference Example 45

### 4-(6-Methylbenzothiazol-2-yl)phenylhydrazine

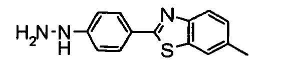

2-(4-Aminophenyl)-6-methylbenzothiazole (1.25 g) was dissolved in hydrochloric acid (8 ml) and water (4 ml), and an aqueous solution (4 ml) of sodium nitrite (395 mg) was added dropwise to the mixture at 0°C over 30 minutes. The mixture was stirred at the same temperature for 2 hours, and then a hydrochloric acid solution (4 ml) of tin(II) chloride dihydrate (2.93 g) was added to the mixture, followed by stirring at room temperature for 1 hour. The reaction mixture was alkalified by adding an aqueous solution of 20 wt% potassium hydroxide and extracted with chloroform:methanol = 9:1 (300 ml). After drying over anhydrous sodium sulfate, the solvent was evaporated, and the thus obtained solid was washed with diethyl ether to obtain the title compound (621 mg) as a yellowish brown solid.
¹H-NMR (400 MHz, DMSO-d₆) δ: 2.41 (3H, s), 4.18 (2H, s), 6.85 (2H, d, J=8.8 Hz), 7.25 (1H, d, J=8.3 Hz), 7.45 (1H, s), 7.77 (4H, m).
ESI-MS m/z: 256 (M+H)⁺.

### Reference Example 46

### 6-Iodo-2-(4-nitrophenyl)imidazo[1,2-a]pyridine

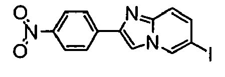

2-Bromo-1-(4-nitrophenyl)ethanone (4.85 g) and 2-amino-5-iodopyridine (4.38 g) were dissolved in acetone (80 ml) and heated under reflux at 70°C for 3 hours. The reaction solution was poured into a saturated aqueous sodium bicarbonate solution (500 ml), followed by stirring at room temperature for 3 hours and then the mixture was filtered. Then, the thus obtained solid was washed with water, ethanol and diethyl ether to obtain the title compound (6.40 g) as a yellow solid.
¹H-NMR (400 MHz, DMSO-d₆) δ: 7.47 (2H, s), 8.20 (2H, d, J=9.0 Hz), 8.29 (2H, d, J=9.0 Hz), 8.53 (1H, s), 8.95 (1H, s).
ESI-MS m/z: 366 (M+H)⁺

### Reference Example 47

### 4-(6-Iodoimidazo[1,2-a]pyridin-2-yl)phenylamine

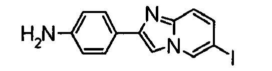

6-Iodo-2-(4-nitrophenyl)imidazo[1,2-a]pyridine (6.30 g) was dissolved in THF (10 ml), to the solution tin chloride dihydrate (19.5 g) was added and heated at 80°C under reflux for 3 hours. After evaporation of the large portion of THF by concentration under reduced pressure, a saturated aqueous sodium bicarbonate solution (300 ml) and ethyl acetate (300 ml) were added to the mixture. After celite filtration, the organic layer was dried over anhydrous sodium sulfate, the solvent was evaporated, the residue was purified by flash silica gel column chromatography (hexane: ethyl acetate = 1:1 to ethyl acetate), and the thus obtained solid was washed with diethyl ether to obtain the title compound (3.12 g) as a yellowish brown solid.
¹H-NMR (400 MHz, DMSO-d₆) δ: 5.25 (2H, br s), 6.59 (2H, d, J=8.3 Hz), 7.33 (2H, s), 7.59 (2H, d, J=8.3 Hz), 8.03 (1H, s), 8. 81 (1H, s).
ESI-MS m/z: 336 (M+H)⁺

### Reference Example 48

### 4-(6-Iodoimidazo[1,2-a]pyridin-2-yl)phenylhydrazine

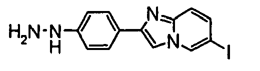

4-(6-Iodoimidazo[1,2-a]pyridin-2-yl)phenylamine (1.48 g) was dissolved in concentrated hydrochloric acid (12 ml) and water (24 ml), and an aqueous solution (6 ml) of sodium nitrite (366 mg) was slowly added dropwise to the mixture at 0°C. After stirring for 30 minutes, a concentrated hydrochloric acid solution (3 ml) of tin chloride dihydrate (2.00 g) was added to the mixture, followed by stirring at room temperature for 1 hour. The reaction mixture was alkalified by adding 28% aqueous ammonia, chloroform: methanol = 9:1 (500 ml) solution was added and then filtered through celite. The organic layer was dried over anhydrous sodium sulfate, the solvent was evaporated, and the thus obtained solid was washed with diethyl ether to obtain the title compound (595 mg) as a yellow solid.
¹H-NMR (400 MHz, DMSO-d₆) δ: 3.32 (2H, br s), 4.69 (1H, br s), 6.62 (2H, d, J=8.6 Hz), 7.35 (2H, s), 7.69 (2H, d, J=8.6 Hz), 8.08 (1H, s), 8.83 (1H, s).
ESI-MS m/z: 351 (M+H)⁺

### Reference Example 49

### N-(2-Hydroxyethyl)-4-nitrobenzamide

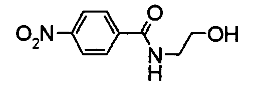

2-Aminoethanol (1.97 g) was dissolved in THF (20 ml), and a THF (20 ml) solution of 4-nitrobenzoyl chloride (5.99 g) was added dropwise to the mixture at 0°C. After stirring at the same temperature for 1 hour, a saturated aqueous sodium bicarbonate solution (100 ml) was added to the mixture, followed by stirring at room temperature for 1 hour. The mixture was extracted with chloroform (100 ml) twice and dried over anhydrous sodium sulfate. The solvent was evaporated, and the residue was recrystallized from ethanol to obtain the title compound (2.26 g) as colorless needle crystals.
¹H-NMR (400 MHz, CD₃OD) δ: 3.31 (1H, br s), 3.53 (2H, t, J=5.9 Hz), 3.72 (2H, t, J=5.9 Hz), 8.03 (2H, d, J=9.0 Hz), 8.31 (2H, d, J=9.0 Hz).
FAB-MS m/z: 211 (M+H)⁺.

### Reference Example 50

### N-(2-Chloroethyl)-4-nitrobenzamide

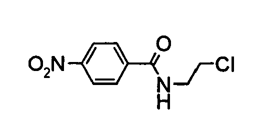

*N*-(2-Hydroxyethyl)-4-nitrobenzamide (2.12 g) was dissolved in dichloromethane (50 ml), and thionyl chloride (879 µl) was added to the mixture at 0°C. The mixture was stirred at room temperature for 3 days, and then water (100 ml) was added to the mixture, extracted with chloroform (100 ml) twice and dried over anhydrous sodium sulfate. The solvent was evaporated, and the thus obtained solid was washed with hexane-isopropyl ether (1:1) to obtain the title compound (2.23 g) as a white solid.
¹H-NMR (400 MHz, CDCl₃) δ: 3.76-3.87 (4H, m), 6.61 (1H, br s), 7.97 (2H, d, J=6.7 Hz), 8.32 (2H, d, J=6.8 Hz).
ESI-MS m/z: 211 (M+H)⁺.

### Reference Example 51

### 2-(4-Nitrophenyl)-4,5-dihydrooxazole

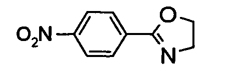

A THF (20 ml) solution of *N*-(2-chloroethyl)-4-nitrobenzamide (2.23 g) was added dropwise to a THF (20 ml) solution of sodium hydride (468 mg), followed by stirring at 50°C for 1 hour. After quenching the reaction mixture with methanol, THF was evaporated and the residue was diluted with ethyl acetate (150 ml). The mixture was washed with a saturated aqueous ammonium chloride solution, a saturated aqueous sodium bicarbonate solution and saturated brine, and dried over anhydrous sodium sulfate. After concentration under reduced pressure, the thus obtained solid was washed with diethyl ether and hexane to obtain the title compound (1.67 g) as a white solid.
¹H-NMR (400 MHz, CDCl₃) δ: 4.12 (2H, t, J=9.5 Hz), 4.49 (2H, t, J=9.7 Hz), 8.12 (2H, d, J=7.1 Hz), 8.26 (2H, d, J=7.1 Hz).
ESI-MS m/z: 193 (M+H)⁺.

### Reference Example 52

### 4-(4, 5-Dihydrooxazol-2-yl)phenylamine

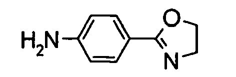

2-(4-Nitrophenyl)-4,5-dihydrooxazole (1.19 g) was dissolved in ethyl acetate (50 ml) and ethanol (50 ml) and 5% palladium-carbon (about 500 mg) was added to the solution, followed by stirring at room temperature for 3 days under hydrogen atmosphere. The catalyst was filtered, the filtrate was concentrated under reduced pressure, and the thus obtained solid was washed with diethyl ether to obtain the title compound (671 mg) as a white solid.
¹H-NMR (400 MHz, DMSO-d₆) δ: 3.83 (2H, t, J=9.3 Hz), 4.29 (2H, t, J=9.3 Hz), 5.64 (2H, s), 6.53 (2H, d, J=8.8 Hz), 7.50 (2H, d, J=8.6 Hz).
ESI-MS m/z: 163 (M+H)⁺.

### Reference Example 53

### 4-[N-(2-Hydroxyethyl)-N-methylamino]benzonitrile

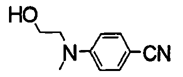

*N*-Methylethanolamine (1.86 g) was added to a dimethyl sulfoxide solution (30 ml) of 4-fluorobenzonitrile (2.00 g) and sodium carbonate (3.42 g), followed by stirring overnight at 100°C. The reaction solution was cooled to room temperature, water was added to the mixture, extracted with ethyl acetate and then dried over sodium sulfate. The solvent was evaporated, the thus obtained residue was purified by silica gel column chromatography, and the fraction obtained from the eluate of n-hexane:ethyl acetate = 1:2 was concentrated under reduced pressure to obtain the title compound (2.40 g) as a colorless oil.
¹H-NMR (400 MHz, CDCl₃) δ: 3.07 (3H, s), 3.56 (2H, t, J=5.9 Hz), 3.83 (2H, q, J=5.9 Hz), 6.70 (2H, d, J=9.2 Hz), 7.42 (2H, d, J=9.2 Hz).
ESI-MS m/z: 177 (M+H)⁺.

### Reference Example 54

### Methanesulfonic acid 2-[N-(4-cyanophenyl)-N-methylamino]ethyl ester

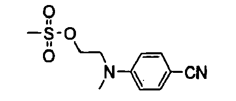

Methanesulfonyl chloride (2.0 ml) was added dropwise to a dichloromethane solution (20 ml) of 4-[*N*-(2-hydroxyethyl)-*N*-methylamino]benzonitrile (1.52 g) and triethylamine (3.6 ml) at 0°C, followed by stirring at the same temperature for 30 minutes. To the reaction solution was added a saturated aqueous ammonium chloride solution, extracted with dichloromethane and then dried over sodium sulfate. The solvent was evaporated, the thus obtained residue was purified by silica gel column chromatography, and the title compound (2.40 g) was obtained from the eluate of n-hexane:ethyl acetate = 1:1 as a white solid.
¹H-NMR (400 MHz, CDCl₃) δ: 2.97 (3H, s), 3.09 (3H, s), 3.78 (2H, t, J=5.9 Hz), 4.37 (2H, t, J=5.9 Hz), 6,71 (2H, d, J=9.0 Hz), 7.48 (2H, d, J=9.0 Hz).
ESI-MS m/z: 255 (M⁺+H).

### Reference Example 55

### 4-[N-(2-Fluoroethyl)-N-methylamino]benzonitrile

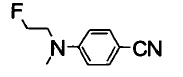

A THF (1 M) solution (43.0 ml) of tetrabutylammonium fluoride was added dropwise to a THF solution (30 ml) of methanesulfonic acid 2-[*N*-(4-cyanophenyl)-*N*-methylamino]ethyl ester (2.20 g), followed by heating under reflux for 2 hours. The reaction solution was cooled to room temperature, water was added to the mixture, extracted with ethyl acetate and then dried over sodium sulfate. The solvent was evaporated, the thus obtained residue was purified by silica gel column chromatography, and the title compound (780 mg) was obtained from the eluate of n-hexane:ethyl acetate = 2:1 as a colorless oil.
¹H-NMR (400 MHz, CDCl₃) δ: 3.05 (3H, s), 3.65 (1H, t, J=5.0 Hz), 3.71 (1H, t, J=5.0 Hz), 4.53 (1H, t, J=5.0 Hz), 4.64 (1H, t, J=5.0 Hz), 6.65 (2H, d, J=9.3 Hz), 7.41 (2H, d, J=9.3 Hz).

### Reference Example 56

### 4-[N-(2-Fluoroethyl)-N-methylamino]benzaldehyde

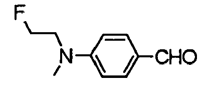

Diisobutylaluminum hydride (5.6 ml, 0.93 M hexane solution) was added dropwise to a THF solution (60 ml) of 4-[*N*-(2-fluoroethyl)-*N*-methylamino]benzonitrile (780 mg) at -78°C, followed by stirring at the same temperature for 2 hours. Methanol (1.0 ml) was added dropwise to the reaction solution, and concentrated hydrochloric acid (4.0 ml) was subsequently added to the mixture, followed by stirring overnight at room temperature. The reaction solution was extracted with diethyl ether and then washed with water and saturated brine and dried over sodium sulfate. The solvent was evaporated, the thus obtained residue was purified by silica gel column chromatography, and the fraction obtained from the eluate of n-hexane:ethyl acetate = 10:3 was concentrated under reduced pressure to obtain the title compound (582 mg) as a colorless oil.
¹H-NMR (400 MHz, CDCl₃) δ: 3.14 (3H, s), 3.73 (1H, t, J=4.9 Hz), 3.80 (1H, t, J=4.9 Hz), 4.58 (1H, t, J=4.9 Hz), 4.70 (1H, t, J=4. 9 Hz), 6.74 (2H, d, J=8.8 Hz), 7. 75 (2H, d, J=8.8 Hz), 9.76 (1H, s).
ESI-MS m/z: 181M⁺.

### Reference Example 57

### 4-[N-(2-Hydroxyethyl)-N-methylamino]benzaldehyde

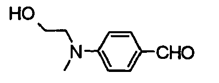

2-Methylaminoethanol (1.65 g) was added to a DMF solution (20 ml) of 4-fluorobenzaldehyde (1.24 g) and sodium carbonate (2.07 g), followed by stirring at 90°C for 4 days. The reaction solution was poured into ice water and extracted with ethyl acetate, and the organic layer was washed with water and saturated brine and dried over magnesium sulfate. The solvent was evaporated, the thus obtained residue was purified by silica gel column chromatography, and the fraction obtained from the eluate of methylene chloride:methanol = 30:1 was concentrated under reduced pressure to obtain the title compound (1.15 g).
¹H-NMR (400 MHz, CDCl₃) δ: 2.28 (1H, br s), 3.12 (3H, s), 3.62 (2H, t, J=5.9 Hz), 3.86 (2H, m), 6.75 (2H, d, J=9.0 Hz), 7.70 (2H, d, J=8.8 Hz), 9.70 (1H, s).

### Reference Example 58

### 4-(Dimethylaminomethyl)benzaldehyde diethylacetal

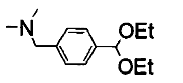

Dimethylamine (8.6 ml, 2.0 M tetrahydrofuran solution) was added dropwise to a methanol solution of terephthalaldehyde monodiethylacetal (3.00 g) at 0°C, followed by stirring at room temperature for 1 hour. After evaporation of methanol, the residue obtained by drying under reduced pressure was again dissolved in methanol, and sodium borohydride was added to the mixture at 0°C, followed by stirring at the same temperature for 30 minutes. Water was added to the reaction solution, methanol was evaporated, and the residue was extracted with ethyl acetate. The extract was dried over sodium sulfate, the solvent was evaporated, the thus obtained residue was purified by silica gel column chromatography, and the fraction obtained from the eluate of dichloromethane:methanol = 10:1 was concentrated under reduced pressure to obtain the title compound (1.95 g) as a colorless solid.
¹H-NMR (400 MHz, CDCl₃) δ: 1.23 (6H, t, J=7.1 Hz), 2.23 (6H, s), 3.42 (2H, s), 3.51-3.64 (4H, m), 5.49 (1H, s), 7.29 (2H, d, J=8.3 Hz), 7.41 (2H, d, J=8.3 Hz).
ESI-MS m/z: 237M⁺.

### Reference Example 59

### 4-(Dimethylaminomethyl)benzaldehyde

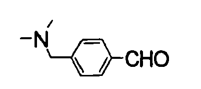

A hydrochloric acid-methanol solution (10 ml) was added to a methanol solution (2 ml) of 4-(dimethylaminomethyl)benzaldehyde diethylacetal (1.0 g) at 0°C, followed by stirring overnight at room temperature. To the residue obtained by evaporating the solvent was added a saturated aqueous sodium bicarbonate solution, extracted with diethyl ether and dried over sodium sulfate to obtain the title compound (634 mg) as a colorless oil.
¹H-NMR (400 MHz, CDCl₃) δ: 2.26 (6H, s), 3.49 (2H, s), 7.49 (2H, d, J=8.3 Hz), 7.84 (2H, d, J=8.3 Hz), 10.00 (1H, s).
ESI-MS m/z: 164 (M+H)⁺.

### Reference Example 60

### 4-(Morpholinomethyl)benzonitrile

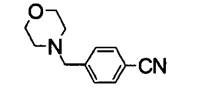

Morpholine (1.30 ml) was added to a DMF solution (10 ml) of 4-(bromomethyl)benzonitrile (1.00 g) and sodium carbonate (1.06 g), followed by stirring at 50°C for 1 hour. The reaction solution was cooled to room temperature, water was added to the mixture, extracted with ethyl acetate, and then dried over sodium sulfate. The solvent was evaporated to obtain the title compound (1.00 g) as a colorless oil.
¹H-NMR (400 MHz, CDCl₃) δ: 2.44 (4H, t, J=4.4 Hz), 3.54 (2H, s), 3.71 (4H, t, J=4.4 Hz), 7.46 (2H, d, J=8.3 Hz), 7.61 (2H, d, J=8.3 Hz).
ESI-MS m/z: 203 (M+H)⁺.

### Reference Example 61

### 4-(Morpholinomethyl)benzaldehyde

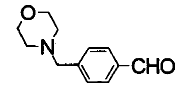

Diisobutylaluminum hydride (18.9 ml, 0.93 M hexane solution) was added dropwise to a THF solution (60 ml) of 4-(morpholinomethyl)benzonitrile (1.00 mg) at -78°C, followed by stirring at the same temperature for 2 hours. Methanol (5.0 ml) was added dropwise to the reaction solution, and concentrated hydrochloric acid (6.7 ml) was subsequently added to the mixture, followed by stirring overnight at room temperature. A saturated aqueous sodium bicarbonate solution was added to the reaction solution, and the resulting precipitate was removed by filtration through celite. The filtrate was extracted with ethyl acetate, washed with water and saturated brine and dried over sodium sulfate. The solvent was evaporated to obtain the title compound (670 mg) as a colorless oil.
¹H-NMR (400 MHz, CDCl₃) δ: 2.46 (4H, t, J=4.6 Hz), 3.57 (2H, s), 3.72 (4H, t, J=4.6 Hz), 7.52 (2H, d, J=8.3 Hz), 7.84 (2H, d, J=8.3 Hz), 10.00 (1H, s).
ESI-MS m/z: 205M⁺.

### Reference Example 62

### Methyl 4-[N-(tert-butoxycarbonyl)aminomethyl]benzoate

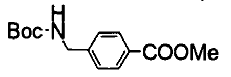

(Boc)₂O (3.4 ml) was added to a dichloromethane-water (1:1, v/v) solution (40 ml) of methyl 4-(aminoethyl)benzoate hydrochloride (1.00 g) and sodium carbonate (3.43 g) at 0°C, followed by stirring for 2 hours. The dichloromethane layer was extracted and then dried over sodium sulfate. The solvent was evaporated, the thus obtained residue was purified by silica gel chromatography, and the fraction obtained from the eluate of n-hexane:ethyl acetate = 10:2 was concentrated under reduced pressure to obtain the title compound (2.54 g) as a colorless oil.
¹H-NMR (400 MHz, CDCl₃) δ: 1.46 (9H, s), 3.90 (3H, s), 4.36 (2H, s), 5.06 (1H, bs), 7.34 (2H, d, J=8.1 Hz), 7.98 (2H, d, J=8.1 Hz).
ESI-MS m/z: 265M⁺.

### Reference Example 63

### tert-Butyl 4-hydroxymethylbenzylcarbamate

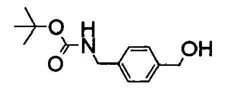

Diisobutylaluminum hydride (25.7 ml, 0.93 M hexane solution) was added dropwise to a THF solution (40 ml) of methyl 4*-*[*N-*(*tert-*-butoxycarbonyl)aminomethyl]benzoate (2.54 g) at -78°C, followed by stirring at the same temperature for 2 hours. A saturated aqueous ammonium chloride solution (10 ml) and diethyl ether were added dropwise to the reaction solution, followed by stirring at room temperature for 1 hour. Magnesium sulfate was added to the reaction solution, followed by further stirring for 1 hour. After removal of the resulting precipitate by filtration trough celite, the filtrate was concentrated, the thus obtained residue was purified by silica gel column chromatography, and the title compound (1.22 g) was obtained as a colorless oil from the fraction of the eluate of n-hexane: ethyl acetate = 10:3.
¹H-NMR (400 MHz, CDCl₃) δ: 1.43 (9H, s), 4.29 (2H, d, J=5.6 Hz), 4.67 (2H, d, J=5.9 Hz), 4.87 (1H, bs), 7.25 (2H, d, J=8.1 Hz), 7.32 (2H, d, J=8.1 Hz).

### Reference Example 64

### tert-Butyl 4-formylbenzylcarbamate

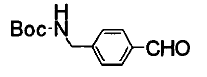

Manganese dioxide (1.22 g) was added to a chloroform solution (20 ml) *of tert-*-butyl 4-hydroxymethylbenzylcarbamate (1.22 g), followed by heating under reflux for 1 hour. After celite filtration, the filtrate was concentrated, the thus obtained residue was purified by silica gel column chromatography, and the fraction obtained from the eluate of n-hexane:ethyl acetate = 2:1 was concentrated under reduced pressure to obtain the title compound (965 mg) as a colorless oil.
¹H-NMR (400 MHz, CDCl₃) δ: 1.46 (9H, s), 4.39 (2H, d, J=5.6 Hz), 5.16 (1H, bs), 7.44 (2H, d, J=8.1 Hz), 7.81 (2H, d, J=8.1 Hz), 9.98 (1H, s).
ESI-MS m/z: 236 (M+H)⁺.

### Reference Example 65

### 3-(Dimethylaminomethyl)benzonitrile

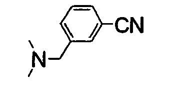

Dimethylamine (7.7 ml) was added to a DMF solution (10 ml) of 3-(bromomethyl)benzonitrile (1.00 g), followed by stirring at room temperature for 10 hours. Water was added to the reaction solution, extracted with dichloromethane and dried over sodium sulfate. The solvent was evaporated, the thus obtained residue was purified by silica gel column chromatography, and the title compound (820 mg) was obtained as a yellowish brown oil from the eluate of dichloromethane: methanol = 10:1.
¹H-NMR (400 MHz, CDCl₃) δ: 2.24 (6H, s), 3.44 (2H, s), 7.42 (1H, t, J=7.6 Hz), 7.54 (2H, bs), 7.63 (1H, s).
ESI-MS m/z: 161 (M+H)⁺.

### Reference Example 66

### 3-(Dimethylaminomethyl)benzaldehyde

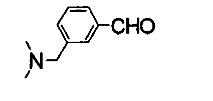

Diisobutylaluminum hydride (16.5 ml, 0.93 M hexane solution) was added dropwise to a THF solution (60 ml) of 3-(dimethylaminomethyl)benzonitrile (820 mg) at -78°C, followed by stirring at the same temperature for 2 hours. Methanol (5.0 ml) was added dropwise to the reaction solution, and silica gel was subsequently added to the mixture, followed by stirring overnight at room temperature. The solvent was evaporated, the thus obtained residue was purified by silica gel column chromatography, and the fraction obtained from the eluate of dichloromethane:methanol = 10:1 was concentrated under reduced pressure to obtain the title compound (259 mg) as a colorless oil.
¹H-NMR (400 MHz, CDCl₃) δ: 2.26 (6H, s), 3.50 (2H, s), 7.49 (1H, t, J=7.9 Hz), 7.60 (1H, d, J=7.9 Hz), 7.80 (1H, d, J=7.9 Hz), 7.83 (1H, s), 10.02 (1H, s).
ESI-MS m/z: 163 (M+H)⁺.

### Reference Example 67

### 2-(Dimethylaminomethyl)benzonitrile

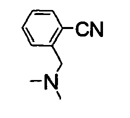

Dimethylamine (7.7 ml) was added to a DMF solution (10 ml) of 2-(bromomethyl)benzonitrile (1.00 g), followed by stirring at room temperature for 10 hours. Water was added to the reaction solution, extracted with dichloromethane and dried over sodium sulfate. The solvent was evaporated, the thus obtained residue was purified by silica gel column chromatography, and the title compound (830 mg) was obtained as a yellowish brown oil from the eluate of dichloromethane:methanol = 10:1.
¹H-NMR (400 MHz, CDCl₃) δ: 2.30 (6H, s), 3.63 (2H, s), 7.36 (1H, dt, J=2.3 and 7.8 Hz), 7.54-7.56 (2H, m), 7.64 (1H, d, J=7.8 Hz).
ESI-MS m/z: 161 (M+H)⁺.

### Reference Example 68

### 2-(Dimethylaminomethyl)benzaldehyde

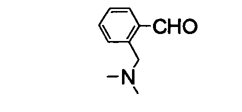

Diisobutylaluminum hydride (16.7 ml, 0.93 M hexane solution) was added dropwise to a THF solution (20 ml) of 2-(dimethylaminomethyl)benzonitrile (830 mg) at -78°C, followed by stirring at the same temperature for 2 hours. Methanol (5.0 ml) was added dropwise to the reaction solution, and silica gel was subsequently added to the mixture, followed by stirring overnight at room temperature. The solvent was evaporated, the thus obtained residue was purified by silica gel column chromatography, and the fraction obtained from the eluate of dichloromethane:methanol = 10:1 was concentrated under reduced pressure to obtain the title compound (166 mg) as a yellowish brown oil.
¹H-NMR (400 MHz, CDCl₃) δ: 2.25 (6H, s), 3.76 (2H, s), 7.39 (1H, t, J=7.8 Hz), 7.42 (1H, d, J=7.8 Hz), 7.51 (1H, t, J=7.8 Hz), 7.87 (1H, d, J=7.8 Hz), 10.37 (1H, s).
ESI-MS m/z: 164 (M+H)⁺.

### Reference Example 69

### Methyl 4-[N-(2-hydroxyethyl)-N-methylaminomethyl]benzoate

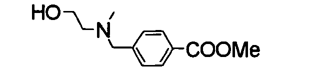

*N*-methylethanolamine (1.2 g) was added to a DMF solution (40 ml) of methyl 4-(bromomethyl)benzoate (3.00 g) and sodium carbonate (2.71 g), followed by stirring overnight at room temperature. Water was added to the reaction solution, extracted with ethyl acetate and dried over sodium sulfate. The solvent was evaporated, the thus obtained residue was purified by silica gel column chromatography, and the title compound (1.53 g) was obtained as a yellowish brown oil from the eluate of dichloromethane:methanol = 10:1.
¹H-NMR (400 MHz, CDCl₃) δ: 2.23 (3H, s), 2.61 (2H, t, J=5.4 Hz), 2.70 (1H, br s), 3.61 (2H, s), 3.63 (2H, t, J=5.4 Hz), 3.91 (3H, s), 7.37 (2H, d, J=8.3 Hz), 7.99 (2H, d, J=8.3 Hz).
ESI-MS m/z: 224 (M+H)⁺.

### Reference Example 70

### Methyl 4-{N-[2-(t-butyldiphenylsilyloxy)ethyl]-N-methylaminomethyl}benzoate

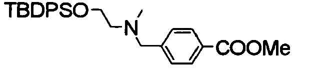

t-Butylchlorodiphenylsilane (1.75 ml) was added dropwise to a DMF solution (20 ml) of methyl 4-[*N*-(2-hydroxyethyl)-*N*-methylaminomethyl]benzoate (1.00 g) and imidazole (457 mg) at 0°C, followed by stirring overnight. Water was added to the reation solution, extracted with diethyl ether and dried over sodium sulfate. The solvent was evaporated, the thus obtained residue was purified by silica gel column chromatography, and the fraction obtained from the eluate of n-hexane:ethyl acetate = 10:3 was concentrated under reduced pressure to obtain the title compound (2.07 g) as a colorless oil.
¹H-NMR (400 MHz, CDCl₃) δ: 1.04 (9H, s), 2.21 (3H, s), 2.60 (2H, t, J=6.1 Hz), 3.58 (2H, s), 3.78 (2H, t, J=6.1 Hz), 3.91 (3H, s), 7.35-7.42 (8H, m), 7.67 (4H, dd, J=1.5 and 7.8 Hz), 7.95 (2H, d, J=8.3 Hz).
ESI-MS m/z: 462 (M+H)⁺.

### Reference Example 71

### 4-{N-[2-(t-butyldiphenylsilyloxy)ethyl]-N-methylaminomethyl}benzyl alcohol

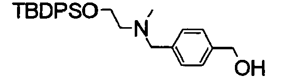

Diisobutylaluminum hydride (14.5 ml, 0.93 M hexane solution) was added dropwise to a THF solution (40 ml) of methyl 4-{*N*-[2-(t-butyldiphenylsilyloxy)ethyl]-*N*-methylaminomethyl}benzoate (2.07 g) at -78°C, followed by stirring at the same temperature for 2 hours. A saturated aqueous ammonium chloride solution (5.7 ml) and diethyl ether were added dropwise to the reaction solution, followed by stirring at room temperature for 1 hour. Magnesium sulfate was added to the reaction solution, followed by further stirring for 1 hour. After removing the resulting precipitate by celite filtration, the filtrate was concentrated, and the thus obtained residue was purified by silica gel column chromatography, and the fraction obtained from the eluate of n-hexane:ethyl acetate = 2:1 was concentrated under reduced pressure to obtain the title compound (1.70 g) as a colorless oil.
¹H-NMR (400 MHz, CDCl₃) δ: 1.04 (9H, s), 2.20 (3H, s), 2.60 (2H, t, J=6.4 Hz), 3.52 (2H, s), 3.78 (2H, t, J=6.4 Hz), 4.67 (2H, s), 7.26 (2H, d, J=7.9 Hz), 7.34-7.41 (8H, m), 7.67 (4H, dd, J=1.5 and 7.8 Hz).
ESI-MS m/z: 435 (M+H)⁺.

### Reference Example 72

### 4-{N-[2-(t-Butyldiphenylsilyloxy)ethyl]-N-methylaminomethyl}benzaldehyde

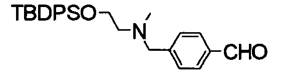

Manganese dioxide (1.70 g) was added to a chloroform solution (40 ml) of 4-{*N*-[2-(t-butyldiphenylsilyloxy)ethyl]-*N*-methylaminomethyl}benzyl alcohol (1.70 g), followed by heating under reflux for 2 hours. After celite filtration, the filtrate was concentrated, the thus obtained residue was purified by silica gel column chromatography, and the fraction obtained from the eluate of n-hexane:ethyl acetate = 10:3 was concentrated under reduced pressure to obtain the title compound (1.51 g) as a colorless oil.
¹H-NMR (400 MHz, CDCl₃) δ: 1.04 (9H, s), 2.22 (3H, s), 2.61 (2H, t, J=5.8 Hz), 3.61 (2H, s), 3.79 (2H, t, J=5.8 Hz), 7.34-7.42 (6H, s), 7.47 (2H, d, J=8.1 Hz), 7.67 (4H, dd, J=1.5 and 8.1 Hz), 7.79 (2H, d, J=8.1 Hz), 9.99 (1H, s).
ESI-MS m/z: 433 (M⁺+H).

### Reference Example 73

### Methyl 4-[N-(2-fluoroethyl)-N-methylaminomethyl]benzoate

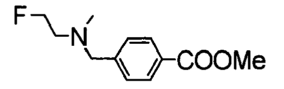

Methanesulfonyl chloride (2.3 ml) was added dropwise to a dichloromethane solution (40 ml) of methyl 4-[*N*-(2-hydroxyethyl)-*N*-methylaminomethyl]benzoate (2.18 g) and triethylamine (4.1 ml) at -78°C, followed by stirring at the same temperature for 30 minutes. A saturated aqueous ammonium chloride solution was added to the reaction solution, extracted with dichloromethane and then dried over sodium sulfate. The solvent was evaporated, the thus obtained residue was purified by silica gel column chromatography, and the fraction obtained from the eluate of dichloromethane:methanol = 10:1 was concentrated and used in the next reaction.

Tetrabutylammonium fluoride (44.3 ml) was added dropwise to a tetrahydrofuran solution (30 ml) of the above-described residue, followed by heating overnight under reflux. The reaction solution was cooled to room temperature and then water was added to the mixture, extracted with ethyl acetate and dried over sodium sulfate. The solvent was evaporated, the thus obtained residue was purified by silica gel column chromatography, and the title compound (689 mg) was obtained as a colorless oil from the eluate of n-hexane:ethyl acetate = 1:1.
¹H-NMR (400 MHz, CDCl₃) δ: 2.30 (3H, s), 2.70 (1H, t, J=4.9 Hz), 2.77 (1H, t, J=4.9 Hz), 3.63 (2H, s), 3.91 (3H, s), 4.50 (1H, t, J=4.9 Hz), 4.61 (1H, t, J=4.9 Hz), 7.40 (2H, d, J=8.5 Hz), 7.98 (2H, d, J=8.5 Hz).
ESI-MS m/z: 226 (M+H)⁺.

### Reference Example 74

### 4-[N-(2-Fluoroethyl)-N-methylaminomethyl]benzyl alcohol

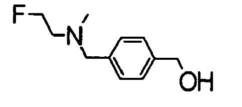

Diisobutylaluminum hydride (8.2 ml, 0.93 M hexane solution) was added dropwise to a THF solution (15 ml) of methyl 4-[*N*-(2-fluoroethyl)-*N*-methylaminomethyl]benzoate (689 mg) at -78°C, followed by stirring at the same temperature for 2 hours. A saturated aqueous ammonium chloride solution (3 ml) and diethyl ether were added dropwise to the reaction solution, followed by stirring at room temperature for 1 hour. Magnesium sulfate was added to the reaction solution, followed by further stirring for 1 hour. After removing the resulting precipitate by celite filtration, the filtrate was concentrated, the thus obtained residue was purified by silica gel column chromatography, and the fraction obtained from the eluate of n-hexane:ethyl acetate = 10:1 was concentrated under reduced pressure to obtain the title compound (430 mg) as a colorless oil.
¹H-NMR (400 MHz, CDCl₃) δ: 2.30 (3H, s), 2.70 (1H, t, J=4.9 Hz), 2.75 (1H, t, J=4.9 Hz), 3.58 (2H, s), 4.49 (1H, t, J=4.9 Hz), 4.62 (1H, t, J=4.9 Hz), 4.68 (2H, s), 7.32 (4H, s).
ESI-MS m/z: 197M⁺.

### Reference Example 75

### 4-[N-(2-Fluoroethyl)-N-methylaminomethyl]benzaldehyde

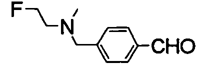

Manganese dioxide (430 mg) was added to a chloroform solution (10 ml) of 4-[*N*-(2-fluoroethyl)-*N*-methylaminomethyl]benzyl alcohol (430 mg), followed by heating under reflux for 1.5 hours. After celite filtration of the catalyst, the filtrate was concentrated, the thus obtained residue was purified by silica gel column chromatography, and the fraction obtained from the eluate of n-hexane:ethyl acetate = 1:1 was concentrated under reduced pressure to obtain the title compound (198 mg) as a colorless oil.
¹H-NMR (400 MHz, CDCl₃) δ: 2.32 (3H, s), 2.72 (1H, t, J=4.9 Hz), 2.79 (1H, t, J=4.9 Hz), 3.67 (2H, s), 4.51 (1H, t, J=4.9 Hz), 4.63 (1H, t, J=4.9 Hz), 7.52 (2H, d, J=8.3 Hz), 7.84 (2H, d, J=8.3 Hz), 10.00 (1H, s).
ESI-MS m/z: 195 (M)⁺.

### Reference Example 76

### 4-Cyanophenylacetic acid

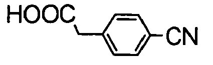

A catalytic amount of ruthenium chloride (28 mg) and sodium periodate (5.80 g) were added to a carbon tetrachloride-acetonitrile-water (2:2:3) solvent solution (20 ml) of 4-(2-hydroxyethyl)benzonitrile (1.00 g), followed by stirring overnight at room temperature. The reaction solution was extracted with dichloromethane and dried over sodium sulfate to obtain the title compound (755 mg) as a colorless oil.
¹H-NMR (400 MHz, CDCl₃) δ: 3.73 (2H, s), 7.40 (2H, d, J=8.3 Hz), 7.63 (2H, d, J=8.3 Hz).

### Reference Example 77

### 4-Formylphenylacetic acid

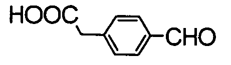

Diisobutylaluminum hydride (5.0 ml, 0.93 M hexane solution) was added dropwise to a THF solution (10 ml) of 4-cyanophenylacetic acid (500 mg) at -78°C, followed by stirring at the same temperature for 2 hours. Methanol (5.0 ml) was added dropwise to the reaction solution and subsequently mixed with silica gel, followed by stirring overnight at room temperature. The solvent was evaporated, the thus obtained residue was purified by silica gel column chromatography, and the fraction obtained from the eluate of dichloromethane: methanol = 10:1 was concentrated under reduced pressure to obtain the title compound (220 mg) as a colorless oil.
¹H-NMR (400 MHz, CDCl₃) δ: 3.76 (2H, s), 7.47 (2H, d, J=8.1 Hz), 7.87 (2H, d, J=8.1 Hz), 10.01 (1H, s).

### Reference Example 78

### 4-(4-tert-Butoxycarbonylpiperazin-1-yl)benzaldehyde

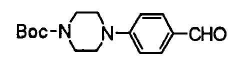

1-*tert*-Butoxycarbonylpiperazine (3.00 g) was added to a DMF solution (40 ml) of 4-fluorobenzaldehyde (2.00 g) and sodium carbonate (3.34 g), followed by stirring overnight at 90°C. The reaction solution was cooled to room temperature, water was added to the mixture, extracted with ethyl acetate and then dried over sodium sulfate. The solvent was evaporated, the thus obtained residue was purified by silica gel column chromatography, and the fraction obtained from the eluate of n-hexane: ethyl acetate = 10:3 was concentrated under reduced pressure to obtain the title compound (3.21 g) as a white solid.
¹H-NMR (400 MHz, CDCl₃) δ: 1.49 (9H, s), 3.39 (4H, t, J=4.9 Hz), 3.59 (4H, t, J=4.9 Hz), 6.91 (2H, d, J=8.8 Hz), 7.76 (2H, d, J=8.8 Hz), 9.79 (1H, s).
ESI-MS m/z: 291 (M+H)⁺.

### Reference Example 79

### Ethyl 2-methylthiazole-4-carboxylate

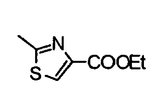

Ethyl bromopyruvate (7.79 g) was added to an acetonitrile solution (100 ml) of thioacetamide (3.00 g), followed by heating under reflux for 4 hours. After cooling the reaction solution, a saturated aqueous sodium bicarbonate solution was added to the mixture, acetonitrile was evaporated, and then the mixture was extracted with ethyl acetate. The extract was washed with water and brine and dried over sodium sulfate. Then, the solvent was evaporated, the thus obtained residue was purified by silica gel column chromatography, and the fraction obtained from the eluate of n-hexane:ethyl acetate = 2:1 was concentrated under reduced pressure to obtain the title compound (4.74 g) as a white solid.
¹H-NMR (400 MHz, CDCl₃) δ: 1.40 (3H, t, J=7.1 Hz), 2.77 (3H, s), 4.42 (2H, q, J=7.1 Hz), 8.05 (1H, s).
ESI-MS m/z: 171(M)⁺.

### Reference Example 80

### Ethyl 2-dimethylaminomethylthiazole-4-carboxylate

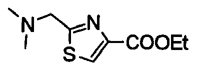

*N*-Bromosuccinimide (1.14 g) and a catalytic amount of AIBN were added to a carbon tetrachloride solution (20 ml) of ethyl 2-methylthiazole-4-carboxylate (1.00 g), followed by heating under reflux for 3 hours. After cooling the reaction solution to room temperature, the resulting precipitate was removed by filtration, the filtrate was concentrated, and the thus obtained residue was used in the subsequent reaction without purification.

The above-described residue was dissolved in dichloromethane (30 ml), and triethylamine (16.3 ml) and dimethylamine hydrochloride (714 mg) were added to the mixture at 0°C, followed by stirring overnight at room temperature. A saturated aqueous ammonium chloride solution was added to the reacton solution, extracted with dichloromethane and dried over sodium sulfate. The solvent was evaporated, the thus obtained residue was purified by silica gel column chromatography, and the fraction obtained from the eluate of dichloromethane:methanol = 100:3 was concentrated under reduced pressure to obtain the title compound (420 mg) as a colorless oil.
¹H-NMR (400 MHz, CDCl₃) δ: 1.41 (3H, t, J=7.1 Hz), 2.38 (6H, s), 3.83 (2H, s), 4.43 (2H, q, J=7.1 Hz), 8.16 (1H, s).

### Reference Example 81

### 2-Dimethylaminomethylthiazole-4-carboxyaldehyde

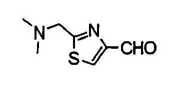

Diisobutylaluminum hydride (6.3 ml) was added dropwise to a tetrahydrofuran solution (10 ml) of ethyl 2-dimethylaminomethylthiazole-4-carboxylate (420 mg) at 0°C, followed by stirring at the same temperature for 2 hours. To the reaction solution, a saturated aqueous ammonium chloride solution and diethyl ether were added, followed by stirring at room temperature for 30 minutes, and then magnesium sulfate was added to the mixture, followed by stirring for 30 minutes. The resulting precipitate was removed by filtration, the filtrate was evaporated, and the thus obtained residue was used in the subsequent reaction without purification.

The above-described residue was dissolved in chloroform (5 ml), manganese dioxide (300 mg) was added to the solution and heated under reflux for 2 hours. After cooling the reaction solution to room temperature, the reaction mixture was filtered, the filtrate was concentrated, the thus obtained residue was purified by silica gel column chromatography, and the fraction obtained from the eluate of dichloromethane:methanol = 100:5 was concentrated under reduced pressure to obtain the title compound (173 mg) as a colorless oil.
¹H-NMR (400 MHz, CDCl₃) δ: 2.39 (6H, s), 3.83 (2H, s), 8.18 (1H, s), 9.99 (1H, s).

### Reference Example 82

### 2-Iodo-N-methoxy-N-methyl-4-nitrobenzamide

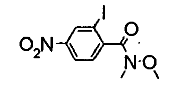

An aqueous solution (8 ml) of sodium nitrite (2.27 g) was added to an aqueous solution (40 ml) of 4-nitroanthranilic acid (5.00 g) under ice-cooling. Subsequently, an aqueous solution (6 ml) of potassium iodide (5.47 g) was added to the mixture, followed by stirring at room temperature for 20 minutes, and then the mixture was heated to 75°C, followed by further stirring for 10 minutes. After completion of the reaction, sodium hydrogen sulfite was added to the mixture under ice-cooling, and the mixture was extracted with dichloromethane-methanol (10:1, v/v). The organic layer was washed with water and saturated brine and dried over sodium sulfate. The residue obtained by evaporating the solvent was again dissolved in water and the same operation described in the above was repeated, and the thus obtained residue was used in the subsequent reaction without purification.

The above-described residue was dissolved in dichloromethane, and *N*,*O*-dimethylhydroxylamine hydrochloride (3.21 g), HOBt (4.45 g), EDC-HCl (6.31 g) and *N*-methylmorpholine (3.6 ml) were added to the solution at 0°C, followed by stirring overnight at room temperature. To the reaction solution was adde a saturated aqueous sodium bicarbonate solution, extracted with dichloromethane, washed with 1 N hydrochloric acid, water and saturated brine and dried over sodium sulfate. The solvent was evaporated, the thus obtained residue was purified by silica gel column chromatography, and the fraction obtained from the eluate of n-hexane:ethyl acetate = 2:1 was concentrated under reduced pressure to obtain the title compound (6.57 g) as a colorless solid.
¹H-NMR (400 MHz, CDCl₃) δ: 3.41 (3H, s), 3.49 (3H, s), 7.44 (1H, d, J=8.5 Hz), 8.26 (1H, d, J=2.0 and 8.5 Hz), 8.67 (1H, d, J=2.0 Hz).

### Reference Example 83

### 2-Iodo-4-nitrobenzaldehyde

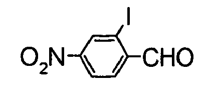

Diisobutylaluminum hydride (52.6 ml) was added dropwise to a THF solution (120 ml) of 2-iodo-*N*-methoxy-*N*-methyl-4-nitrobenzamide (6.57 g) at -78°C, followed by stirring at the same temperature for 1 hour. A saturated aqueous ammonium chloride solution (20.6 ml) was added dropwise to the reaction solution, and then the mixture was stirred at room temperature for 1 hour, and magnesium sulfate and diethyl ether were added to the mixture, followed by further stirring for 1 hour. After celite filtration, the solvent was evaporated, the thus obtained residue was purified by silica gel column chromatography, and the fraction obtained from the eluate of n-hexane: ethyl acetate = 10:1 was concentrated under reduced pressure to obtain the title compound (3.96 g).
¹H-NMR (400 MHz, CDCl₃) δ: 8.02 (1H, d, J=8.5 Hz), 8.30 (1H, dd, J=2.0 and 8.5 Hz), 8.79 (1H, d, J=2.0 Hz), 10.14 (1H, s).

### Reference Example 84

### 5-(2-Iodo-4-nitrophenyl)oxazole

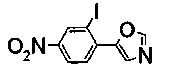

Potassium carbonate (2.37 g) was added to a methanol solution (80 ml) of 2-iodo-4-nitrobenzaldehyde (3.69 g) and p-toluenesulfonylmethyl isocyanide (3.35 g), followed by heating overnight under reflux. The solvent was evaporated under reduced pressure, the thus obtained residue was purified by silica gel column chromatography, and the fraction obtained from the eluate of n-hexane:ethyl acetate = 10:3 was concentrated under reduced pressure and then dried under reduced pressure to obtain the title compound (3.32 g) as a colorless oil.
¹H-NMR (400 MHz, CDCl₃) δ: 7.82 (1H, d, J=8.5 Hz), 8.07 (1H, s), 8.14 (1H, s), 8.27 (1H, dd, J=2.0 and 8.5 Hz), 8.83 (1H, d, J=2.0 Hz).

### Reference Example 85

### 3-Iodo-4-(oxazol-5-yl)phenylamine

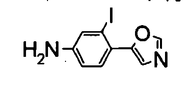

Tin chloride dihydrate (9.48 g) was added to an ethanol solution (100 ml) of 5-(2-iodo-4-nitrophenyl)oxazole (3.32 g), followed by stirring at 90°C for 2 hours. A saturated aqueous sodium bicarbonate solution was added to the reaction solution, the solvent was evaporated, and the thus obtained residue was extracted with ethyl acetate and washed with water and saturated brine. After drying over sodium sulfate, the solvent was evaporated, the thus obtained concentrated residue was purified by silica gel column chromatography, and the fraction obtained from the eluate of n-hexane:ethyl acetate = 10:3 was concentrated under reduced pressure to obtain the title compound (2.56 g) as a yellow oil.
¹H-NMR (400 MHz, CDCl₃) δ: 3.90 (2H, br s), 6.69 (1H, d, J=8.5 Hz), 7.28 (1H, s), 7.30 (1H, d, J=8.5 Hz), 7.54 (1H, s), 7.90 (1H, s).
ESI-MS m/z: 287 (M+H)⁺.

### Reference Example 86

### 3 -Iodo-4-(oxazol-5-yl)phenylhydrazine

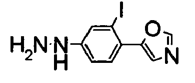

Concentrated hydrochloric acid (20 ml) was added to an aqueous solution (15 ml) of 3-iodo-4-(oxazol-5-yl)phenylamine (2.56 g) at 0°C, and an aqueous solution (10 ml) of sodium nitrite (680 mg) was subsequently added slowly dropwise to the solution, followed by stirring under ice-cooling for 30 minutes. A hydrochloric acid solution (20 ml) of tin chloride (5.04 g) was added dropwise to the reaction solution, and then the mixture was stirred at room temperature for 2 hours. The reaction solution was alkalified with an aqueous solution of 20% potassium hydroxide, and then the mixture was extracted with chloroform-methanol (10:1, v/v). The extract was dried over sodium sulfate, and then the residue obtained by evaporating the solvent was washed with diethyl ether and dried to obtain the title compound (1.40 g) as a red solid.
¹H-NMR (400 MHz, CDCl₃) δ: 3.50 (2H, br s), 5.40 (1H, br s), 6.84 (1H, d, J=8.5 Hz), 7.38 (1H, dd, J=2.5 and 8.5 Hz), 7.46 (1H, s), 7.55 (1H, d, J=2.5 Hz), 7.90 (1H, s).
ESI-MS m/z: 302 (M+H)⁺.

### Reference Example 87

### 4-(tert-Butyldimethylsilyloxy)methyl-2-iodophenylamine

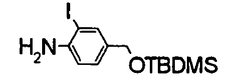

Diisobutylaluminum hydride (46.6 ml, 0.93 M hexane solution) was added dropwise to a THF solution (60 ml) of methyl 4-amino-3-iodobenzoate (3.0 g) at -78°C, followed by stirring at the same temperature for 1 hour. A saturated aqueous ammonium chloride solution and diethyl ether were added to the reaction solution, followed by stirring at room temperature for 1 hour. Magnesium sulfate was added to the reaction solution, followed by further stirring for 1 hour. After celite filtration of the precipitate, the filtrate was concentrated, and the thus obtained residue was used in the subsequent reaction.

Imidazole (480 mg) was added to a DMF solution (30 ml) of the above-described residue (1.46 g) and *tert*-butylchlorodimethylsilane (1.06 g) at 0°C, followed by stirring at room temperature for 1 hour. To the reaction solution were added water and diethyl ether and extracted with diethyl ether, and the extract was dried over sodium sulfate. The solvent was evaporated, the thus obtained residue was purified by silica gel column chromatography, and the fraction obtained from the eluate of n-hexane:ethyl acetate = 100:5 was concentrated under reduced pressure to obtain the title compound (2.02 g) as a colorless oil.
¹H-NMR (400 MHz, CDCl₃) δ: 0.10 (6H, s), 0.94 (9H, s), 4.69 (2H, s), 7.35 (1H, d, J=8.5 Hz), 7.82 (1H, s), 8.14 (1H, d, J=8.5 Hz), 8.25 (2H, bs).
ESI-MS m/z: 364 (M+H)⁺.

### Reference Example 88

### 2,2,2-Trifluoro-N-(4-hydroxymethyl-2-iodophenyl)acetamide

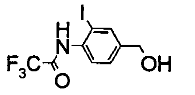

Triethylamine (1.2 ml) was added to a dichloromethane solution (40 ml) of 4-(*tert*-butyldimethylsilyloxy)methyl-2-iodoaniline (2.02 g) and trifluoroacetic anhydride (1.2 ml) at 0°C, followed by stirring at room temperature for 4 hours. To the reaction solution was added 1 N hydrochloric acid, extracted with dichloromethane and then the extract was washed with a saturated aqueous sodium bicarbonate solution, water and saturated brine. The extract was dried over sodium sulfate, and then the residue (2.60 g) obtained by evaporating the solvent was used in the subsequent reaction without purification.

n-Tetrabutylammonium fluoride (8.5 ml, 1.0 M, solution in THF) was added dropwise at 0°C to a THF solution of the above-described residue (2.60 g), followed by stirring overnight at room temperature. The solvent was evaporated, the thus obtained residue was purified by silica gel column chromatography, and the fraction obtained from the eluate of n-hexane:ethyl acetate = 10:3 was concentrated under reduced pressure to obtain the title compound (1.68 g) as a colorless oil.
¹H-NMR (400 MHz, CDCl₃) δ: 4.60 (2H, s), 7.29 (1H, dd, J=1.7 and 8.3 Hz), 7.80 (1H, d, J=1.7 Hz), 7.97 (1H, d, J=8.3 Hz).

### Reference Example 89

### 2,2,2-Trifluoro-N-(4-formyl-2-iodophenyl)acetamide

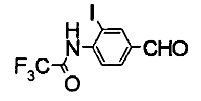

Manganese dioxide (1.47 g) was added to a chloroform solution (15 ml) of 2,2,2-trifluoro-*N*-(4-hydroxymethyl-2-iodophenyl)acetamide (1.47 g), followed by heating under reflux for 1 hour. After celite filtration, the filtrate was concentrated, the thus obtained residue was purified by silica gel column chromatography, and the fraction obtained from the eluate of n-hexane:ethyl acetate = 10:1 was concentrated under reduced pressure to obtain the title compound (1.09 g) as a colorless oil.
¹H-NMR (400 MHz, CDCl₃) δ: 7.93 (1H, dd, J=1.6 and 8.5 Hz), 8.37 (1H, d, J=1.6 Hz), 8.49 (1H, d, J=8.5 Hz), 8.51 (1H, br s), 9.92 (1H, s).
ESI-MS m/z: (N4+H)⁺.

### Reference Example 90

### 2-Iodo-4-(oxazol-5-yl)phenylamine

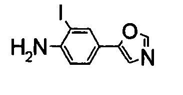

Potassium carbonate (550 mg) was added to a methanol solution (30 ml) of 2,2,2-trifluoro-*N*-(4-formyl-2-iodophenyl)acetamide (1.24 g) and p-toluenesulfonylmethyl isocyanide (780 mg), followed by heating overnight under reflux. After cooling the reaction solution, the solvent was evaporated, the thus obtained residue was purified by silica gel column chromatography, and the fraction obtained from the eluate of n-hexane:ethyl acetate = 10:3 was concentrated under reduced pressure to obtain the title compound (883 mg) as a colorless oil.
¹H-NMR (400 MHz, CDCl₃) δ: 4.31 (2H, br s), 6.74 (1H, d, J=8.5 Hz), 7.15 (1H, s), 7.40 (1H, dd, J=1.9 and 8.5 Hz), 7.83 (1H, s), 7.92 (1H, d, J=1.9 Hz).
ESI-MS m/z: 287 (M+H)⁺.

### Reference Example 91

### 3-Iodo-4-(oxazol-5-yl)phenylhydrazine

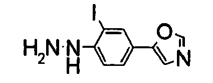

Concentrated hydrochloric acid (3 ml) was added to an aqueous solution (2 ml) of 2-iodo-4-(oxazol-5-yl)aniline (400 mg) at 0°C, and an aqueous solution (1 ml) of sodium nitrite (106 mg) was subsequently added slowly dropwise to the mixture, followed by stirring under ice-cooling for 30 minutes. A hydrochloric acid solution (3 ml) of tin chloride (790 mg) was added dropwise to the reaction solution, and then the mixture was stirred at room temperature for 1.5 hours. The reaction solution was alkalified with an aqueous solution of 20% potassium hydroxide, and then the mixture was extracted with chloroform-methanol (10:1, v/v). The extract was dried over sodium sulfate, and then the residue obtained by evaporating the solvent was washed with diethyl ether and dried to obtain the title compound (194 mg) as a red solid.
¹H-NMR (400 MHz, CDCl₃) δ: 3.71 (2H, bs), 5.70 (1H, bs), 7.07 (1H, d, J=8.5 Hz), 7.15 (1H, s), 7.55 (1H, dd, J=1.7 and 8.5 Hz), 7.83 (1H, s), 7.92 (1H, d, J=1.7 Hz).

### Reference Example 92

### 3-Iodo-N,4-dimethyl-N-methoxybenzamide

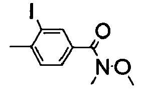

*N*,*O*-Dimethylhydroxylamine hydrochloride (1.23 g), HOBt (1.86 g), EDC·HCl (2.63 g) and NMM (1.5 ml) were added to a dichloromethane solution (60 ml) of 3-iodo-4-methylbenzoic acid (3.0 g) at 0°C, followed by stirring overnight at room temperature. To the reaction solution was added 1 N hydrochloric acid, extracted with dichloromethane and dried over sodium sulfate. The solvent was evaporated, the thus obtained residue was purified by silica gel column chromatography, and the fraction obtained from the eluate of n-hexane:ethyl acetate = 2:1 was concentrated under reduced pressure to obtain the title compound (3.19 g) as a colorless solid.
¹H-NMR (400 MHz, CDCl₃) δ: 2.46 (3H, s), 3.34 (3H, s), 3.56 (3H, s), 7.26 (1H, d, J=7.8 Hz), 7.60 (1H, dd, J=1.5 and 7.8 Hz), 8.15 (1H, d, J=1.5 Hz).
ESI-MS m/z: 306 (M+H)⁺.

### Reference Example 93

### 4-Bromomethyl-3-iodo-N-methoxy-N-methylbenzamide

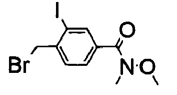

*N*-Bromosuccinimide (1.40 g) and a catalytic amount of AIBN were added to a carbon tetrachloride solution (40 ml) of 3-iodo-*N*,4-dimethyl-*N*-methoxybenzamide (2.0 g), followed by heating under reflux for 2 hours. The precipitate was removed by celite filtration, the filtrate was concentrated under reduced pressure, the thus obtained residue was purified by silica gel column chromatography, and the title compound (867 mg) was obtained as a yellowish brown solid from the eluate of n-hexane:ethyl acetate = 2:1.
¹H-NMR (400 MHz, CDCl₃) δ: 3.35 (3H, s), 3.56 (3H, s), 4.59 (2H, s), 7.49 (1H, d, J=8.0 Hz), 7.65 (1H, dd, J=1.5 and 8.0 Hz), 8.16 (1H, d, J=1.5 Hz).
ESI-MS m/z: 385 (M+H)⁺.

### Reference Example 94

### 4-Dimethylaminomethyl-3-iodo-N-methoxy-N-methylbenzamide

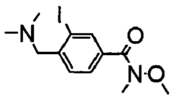

Dimethylamine (1.7 ml, 2.0 M THF solution) was added to a DMF solution (10 ml) of 4-bromomethyl-3-iodo-N-methoxy-N-methylbenzamide (860 mg), followed by stirring overnight at room temperature. To the reaction solution was added water, extracted with dichloromethane and then dried over sodium sulfate. The solvent was evaporated, the thus obtained residue was purified by silica gel column chromatography, and the fraction obtained from the eluate of dichloromethane:methanol = 100:2 was concentrated under reduced pressure to obtain the title compound (490 mg) as a colorless oil.
¹H-NMR (400 MHz, CDCl₃) δ: 2.31 (6H, s), 3.35 (3H, s), 3.48 (2H, s), 3.56 (3H, s), 7.43 (1H, d, J=8.6 Hz), 7.65 (1H, dd, J=1.5 and 8.6 Hz), 8.16 (1H, d, J=1.5 Hz).
ESI-MS m/z: 349 (M+H)⁺.

### Reference Example 95

### 4-(Dimethylaminomethyl)-3-iodobenzaldehyde

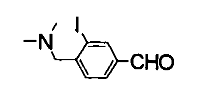

Diisobutylaluminum hydride (3.8 ml) was added to a THF solution (10 ml) of 4-dimethylaminomethyl-3-iodo-*N*-methoxy-*N*-methylbenzamide (490 mg) at -78°C, followed by stirring at the same temperature for 1 hour. A saturated aqueous ammonium chloride solution (1.5 ml) was added dropwise to the reaction solution, and then the mixture was stirred at room temperature for 1 hour, and magnesium sulfate and diethyl ether were added to the mixture, followed by further stirring for 1 hour. After celite filtration, the solvent was evaporated, the thus obtained residue was purified by silica gel column chromatography, and the fraction obtained from the eluate of dichloromethane:methanol = 100:2 was concentrated under reduced pressure to obtain the title compound (314 mg).
¹H-NMR (400 MHz, CDCl₃) δ: 2.32 (6H, s), 3.51 (2H, s), 7.59 (1H, d, J=7.8 Hz), 7.83 (1H, dd, J=1.5 and 7.8 Hz), 8.31 (1H, d, J=1.5 Hz), 9.91 (1H, s).
ESI-MS m/z: 290 (M+H)⁺.

### Reference Example 96

### (3-Iodopyridin-4-yl)methanol

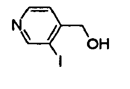

Fuming sulfuric acid (60%, SO₃, 60 ml) was added to 4-picoline (9.73 ml) by portions under ice-cooling, followed by stirring at the same temperature for 8 hours. Iodine (20.3 g) was added to the mixture under ice-cooling, followed by stirring at the same temperature for 15 minutes and then stirring at 90°C for 24 hours. Ice was added to the reaction solution by portions under ice-cooling, and an aqueous solution of 40% sodium hydroxide was further added to the mixture. After celite filtration of the reaction mixture, the filtrate was extracted with diethyl ether and dried over sodium sulfate. The starting materials and 3-iodo-4-picoline were evaporated under reduced pressure from the residue obtained by evaporating the solvent. The thus obtained residue was purified by silica gel column chromatography, and the fraction obtained from the eluate of dichloromethane: methanol = 20:1 to 10:1 was concentrated under reduced pressure to obtain the title compound (1.66 g) as a crystalline solid.
¹H-NR4R (400 MHz, CDCl₃) δ: 4.40 (2H, d, J=5.6 Hz), 5.69 (1H, m), 7.49 (1H, d, J=4.9 Hz), 8.52 (1H, d, J=4.9 Hz), 8.78 (1H, s).

### Reference Example 97

### 3-Iodopyridine-4-carboxyaldehyde

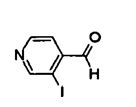

Manganese dioxide (391 mg) was added to a chloroform solution (15 ml) of (3-iodopyridin-4-yl)methanol (211 mg), followed by stirring at 70°C for 3 hours. After cooling, the reaction solution was celite-filtered, the filtrate was concentrated, the thus obtained residue was purified by silica gel column chromatography, and the fraction obtained from the eluate of dichloromethane:methanol = 10:1 was concentrated under reduced pressure to obtain the title compound (180 mg) as a crystalline solid.
¹H-NMR (400 MHz, CDCl₃) δ: 7.69 (1H, d, J=4.9 Hz), 8.69 (1H, d, J=4.9 Hz), 9.11 (1H, s), 10.06 (1H, s).

### Reference Example 98

### 2-Iodo-4-pyridinecarboxylic acid

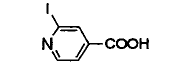

Hydroiodic acid (4.0 ml) was added to a 2-butanone solution (150 ml) of 2-chloroisonicotinic acid (5.0 g) and sodium iodide (17.0 g), followed by heating under reflux for 6 hours. After cooling, the reaction solution was filtered, the filtrate was concentrated under reduced pressure, and the residue was dissolved in water. The mixture was alkalified with 1 N NaOH, and then the insoluble substance was removed by filtration, and an aqueous sodium sulfite solution was added to the filtrate. The reaction solution was acidified by adding 1 N HCl and concentrated hydrochloric acid, and then the resulting precipitate was collected by filtration. The product was washed with isopropyl alcohol and diisopropyl ether and then dried to obtain the title compound (4.8 g) as a crystalline solid.
¹H-NMR (400 MHz, DMSO-d₆) δ: 7.82 (1H, dd, J=5.1 Hz, 1.2 Hz), 8.15 (1H, d, J=0.5 Hz), 8.55 (1H, d, J=4.9 Hz), 13.91 (1H, br s).
FAB-MS m/z: 250 (N4+H)⁺.

### Reference Example 99

### 2-Iodo-N-methoxy-N-methyl-4-pyridinecarboxamide

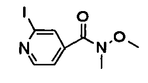

*N,O*-Dimethylhydroxylamine hydrochloride (102.4 mg) was added to a dichloromethane solution (20 ml)-DMF solution (10 ml) of 2-iodo-4-pyridinecarboxylic acid (249 mg), EDC·HCl (249 mg) and DMAP (281 mg) under ice-cooling, and the mixture was stirred at room temperature for 15 hours. The reaction solution was concentrated under reduced pressure, chloroform (100 ml) was added to the residue, and the organic layer was washed with a saturated aqueous ammonium chloride solution, water and a saturated aqueous sodium bicarbonate solution and dried over sodium sulfate. The solvent was evaporated, the thus obtained residue was purified by silica gel column chromatography, and the fraction obtained from the eluate of dichloromethane:methanol = 20:1 was concentrated under reduced pressure to obtain the title compound (260 mg) as a pale yellow oil.
¹H-NMR (400 MHz, CDCl₃) δ: 3.36 (3H, s), 3.56 (3H, s), 7.49 (1H, dd, J=4.9 Hz, 1.3 Hz), 7.95 (1H, s), 8.44 (1H, d, J=4.9 Hz).

### Reference Example 100

### 2-Iodo-4-pyridinecarboxyaldehyde

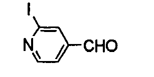

Diisobutylaluminum hydride (1.15 ml) was added dropwise to a tetrahydrofuran solution (5 ml) of 2-iodo-*N*-methoxy-*N*-methyl-4-pyridinecarboxamide (260 mg) under ice-cooling, followed by stirring at the same temperature for 1 hour. To the reaction solution were added methanol, a saturated aqueous ammonium chloride solution and diethyl ether, the mixture stirred at room temperature for 30 minutes, to the mixture was added magnesium sulfate and further stirred for 30 minutes. The resulting precipitate was removed by filtration, the filtrate was concentrated under reduced pressure, the thus obtained residue was purified by silica gel column chromatography, and the fraction obtained from the eluate of dichloromethane:methanol = 20:1 was concentrated under reduced pressure to obtain the title compound (198 mg).
¹H-NMR (400 MHz, CDCl₃) δ: 7.67 (1H, d, J=4.8 Hz), 8.12 (1H, s), 8.63 (1H, d, J=4.6 Hz), 9.99 (1H, s).

### Reference Example 101

### 2-Fluoro-N-methoxy-N-methyl-4-pyridinecarboxamide

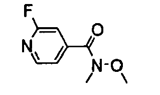

N,O-Dimethylhydroxylamine hydrochloride (830 mg), HOBt (1.15 g), EDC·HCl (1.63 g) and NMM (935 µl) were added to a dichloromethane solution (20 ml) of 2-fluoro-4-pyridinecarboxylic acid (1.0 g) at 0°C, followed by stirring overnight at room temperature. To the reaction solution was added 1 N hydrochloric acid, extracted with dichloromethane and dried over sodium sulfate. The solvent was evaporated, the thus obtained residue was purified by silica gel column chromatography, and the fraction obtained from the eluate of n-hexane:ethyl acetate = 2:1 was concentrated under reduced pressure to obtain the title compound (1.30 g) as a colorless solid.
¹H-NMR (400 MHz, CDCl₃) δ: 3.38 (3H, s), 3.56 (3H, s), 7.18 (1H, d, J=1.3 Hz), 7.42 (1H, dd, J=1.3 and 4.9 Hz), 8.30 (1H, d, J=4.9 Hz).

### Reference Example 102

### N-Methoxy-N-methyl-2-(4-methylpiperazin-1-yl)-4-pyridinecarboxamide

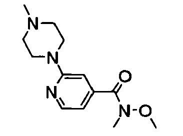

*N*-Methylpiperazine (326 µg) was added to a DMF solution (10 ml) of 2-fluoro-*N*-methoxy-*N*-methyl-4-pyridine carboxamide (500 mg) and potassium carbonate (563 mg), followed by stirring overnight at 90°C. The reaction solution was cooled to room temperature, water was added to the mixture, extracted with ethyl acetate and then dried over sodium sulfate. The solvent was evaporated, the thus obtained residue was purified by silica gel column chromatography, and the fraction obtained from the eluate of dichloromethane:methanol = 100:5 was concentrated under reduced pressure to obtain the title compound (544 mg) as a white solid.
¹H-NMR (400 MHz, CDCl₃) δ: 2.35 (3H, s), 2.52 (4H, t, J=5.1 Hz), 3.34 (3H, s), 3.58 (3H, s), 3.60 (4H, t, J=5.1 Hz), 6.77 (1H, d, J=4.9 Hz), 6.83 (1H, s), 8.22 (1H, d, J=4.9 Hz).
ESI-MS m/z: 265 (N4+H)⁺.

### Reference Example 103

### 1-Methyl-1H-indole-2,3-dione

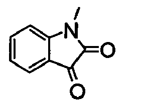

A DMF solution (7 ml) of isatin (1.0 g) was added dropwise to a DMF solution (20 ml) of sodium hydride (408 mg) at 0°C. After stirring at the same temperature for 1 hour, methyl iodide (635 µl) was added dropwise to the mixture, followed by stirring at room temperature for 1 hour. After adding a small amount of methanol, the solvent was evaporated, and the residue was diluted with water (100 ml), extracted with ethyl acetate (300 ml), washed with saturated brine (100 ml) and dried over anhydrous sodium sulfate. The solvent was evaporated, and the residue was purified by flash silica gel column chromatography (hexane:ethyl acetate = 3:2) to obtain the title compound (261 mg) as a reddish orange solid.
¹H-NMR (400 MHz, CDCl₃) δ: 3.26 (3H, s), 6.89 (1H, d, J=8.6 Hz), 7.13 (1H, dd, J=6.6 and 8.6 Hz), 7.60 (2H, m).
ESI-MS m/z: 162 (M+H)⁺

### Reference Example 104

### N'-[4-(Oxazol-5-yl)phenyl]hydrazide isonicotinate

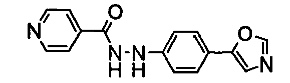

4-(Oxazol-5-yl)phenylhydrazine (206 mg) and isonicotinic acid (145 mg) were dissolved in dichloromethane (6 ml) and DMF (2 ml), to the mixture EDC-HCl (226 mg) was added at -15°C and stirred for 2 hours. After evaporation of the solvent, to the residue was added water (30 ml), extracted with ethyl acetate (30 ml) three times and dried over anhydrous sodium sulfate. The solvent was evaporated, the residue was purified by flash silica gel column chromatography (chloroform:methanol = 20:1 to 9:1), and the thus obtained solid was washed with diethyl ether to obtain the title compound (191 mg) as a pale yellow solid.
¹H-NMR (400 MHz, CDCl₃) δ: 6.37 (1H, br s), 6.97 (2H, d, J=8.6 Hz), 7.23 (1H, s), 7.57 (2H, d, J=8.6 Hz), 7.70 (2H, d, J=6.1 Hz), 7.86 (1H, s), 7.99 (1H, br s), 8.83 (2H, d, J=6.1 Hz).
ESI-MS m/z: 281 (M+H)⁺

### Reference Example 105

### 4-(N'-Pyridin-4-ylmethylenehydrazino)benzoic acid

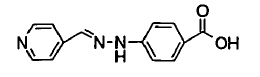

4-Pyridinecarboxyaldehyde (1.07 g) was added to an ethanol solution (80 ml) of 4-hydrazinobenzoic acid (1.52 g), followed by heating under reflux for 3 hours. The solvent was evaporated, and the thus obtained residue was washed with ethanol and diethyl ether and then dried to obtain the title compound (2.35 g) as a yellow solid.
¹H-NMR (400 MHz, DMSO-d₆) δ: 7.17 (2H, d, J=8.5 Hz), 7.61 (2H, d, J=6.4 Hz), 7.85 (2H, d, J=8.3 Hz), 7.90 (1H, s), 8.56 (2H, d, J=5.1 Hz), 11.15 (1H, s), 12.32 (1H, br s).

### Reference Example 106

### N-methoxy-N-methyl-4-sulfamoylbenzamide

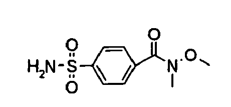

By using 4-sulfamoylbenzoic acid as the material and carrying out the operation in the same manner as in Reference Example 92, the title compound was obtained as a colorless solid.
¹H-NMR (400 MHz, CDCl₃) δ: 3.38 (3H, s), 3.53 (3H, s), 7.79 (2H, d, J=8.3 Hz), 7.96 (2H, d, J=8.3 Hz), 8.01 (2H, s).

### Reference Example 107

### 4-Formylbenzenesulfonamide

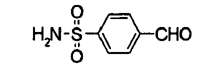

By using the compound obtained in Reference Example 106 and carrying out the operation in the same manner as in Reference Example 95, the title compound was obtained as a colorless oil.
¹H-NMR (400 MHz, CDCl₃) δ: 7.63 (2H, d, J=8.3 Hz), 7.88 (2H, d, J=8.3 Hz), 8.07 (2H, s), 10.07 (1H, s).

### Reference Example 108

### Ethyl 4-(methanesulfonylamino)benzoate

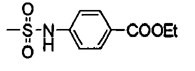

Methane sulfonyl chloride (1.4 ml) was added to a dichloromethane solution (20 ml) of ethyl 4-aminobenzoate (1.0 g) and pyridine (1.5 ml), followed by stirring at room temperature for 1 hour. To the reaction solution was added water, extracted with dichloromethane and then dried over sodium sulfate. The solvent was evaporated, the thus obtained residue was purified by silica gel column chromatography, and the fraction obtained from the eluate of n-hexane:ethyl acetate = 1:1 was concentrated under reduced pressure to obtain the title compound (1.16 g) as a colorless oil.
¹H-NMR (400 MHz, CDCl₃) δ: 1.39 (3H, t, J=7.1 Hz), 3.09 (3H, s), 4.38 (2H, q, J=7.1 Hz), 7.28 (2H, d, J=8.5 Hz), 7.50 (1H, br s), 8.03 (2H, d, J=8.5 Hz).

### Reference Example 109

### N-(4-Hydroxymethylphenyl)methanesulfonamide

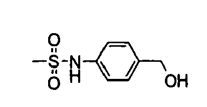

By using the compound obtained in Reference Example 108 and carrying out the operation in the same manner as in Reference Example 95, the title compound was obtained as a colorless oil.
¹H-NMR (400 MHz, CDCl₃) δ: 2.93 (3H, s), 3.36 (1H, s), 4.57 (2H, s), 7.22 (2H, d, J=8.8 Hz), 7.32 (2H, d, J=8.8 Hz).

### Reference Example 110

### N-(4-Formylphenyl)methanesulfonamide

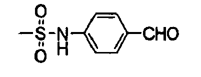

By using the compound obtained in Reference Example 109 and carrying out the operation in the same manner as in Reference Example 64, the title compound was obtained as white powder.
¹H-NMR (400 MHz, CDCl₃) δ: 3.08 (3H, s), 3.31 (1H, s), 7.38 (2H, d, J=8.8 Hz), 7.87 (2H, d, J=8.8 Hz), 9.87 (1H, s).

### Reference Example 111

### Ethyl 4-(N,N-dimethylaminosulfonylamino)benzoate

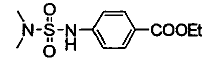

Dimethylsulfamoyl chloride (2.6 ml) was added to a dichloromethane solution (20 ml) of ethyl 4-aminobenzoate (1.0 g) and pyridine (1.5 ml), followed by stirring at room temperature for 1 hour. To the reaction solution was added water, extracted with dichloromethane and then dried over sodium sulfate. The solvent was evaporated, the thus obtained residue was purified by silica gel column chromatography, and the fraction obtained from the eluate of n-hexane:ethyl acetate = 1:1 was concentrated under reduced pressure to obtain the title compound (1.42 g) as a colorless oil.
¹H-NMR (400 MHz, CDCl₃) δ: 1.34-1.38 (3H, m), 2.81 (6H, s), 4.27-4.85 (2H, m), 4.85 (1H, s), 7.19 (1H, d, J=8. 8 Hz), 7.26 (1H, d, J=8.8 Hz), 7.90 (1H, d, J=8.8 Hz), 7.94 (1H, d, J=8.8 Hz).

### Reference Example 112

### N-(4-Hydroxymethylphenyl)-N',N'-dimethylaminosulfonamide

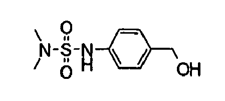

By using the compound obtained in Reference Example 111 and carrying out the operation in the same manner as in Reference Example 95, the title compound was obtained as a brown oil.
¹H-NMR (400 MHz, CDCl₃) δ: 2.74 (6H, s), 3.29 (1H, s), 4.54 (2H, s), 7.20 (2H, d, J=8.3 Hz), 7.27 (2H, d, J=8.3 Hz).

### Reference Example 113

### N-(4-Formylphenyl)-N,N'-dimethylaminosulfonamide

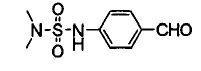

By using the compound obtained in Reference Example 112 and carrying out the operation in the same manner as in Reference Example 64, the title compound was obtained as white powder.
¹H-NMR (400 MHz, CDCl₃) δ: 2.82 (6H, s), 5.48 (1H, s), 7.33 (2H, d, J=8.5 Hz), 7.82 (2H, d, J=8.5 Hz), 9.84 (1H, s).

### Reference Example 114

### 4-[2-(N,N-Dimethylamino)ethoxy]benzaldehyde hydrochloride

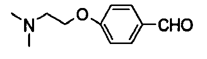

2-Dimethylaminoethanol (730 mg), triphenylphosphine (2.20 g) and diisopropyl diazocarboxylate (1.60 ml) were added to a THF solution (20 ml) of 4-hydroxybenzaldehyde (1.0 g), followed by stirring for 1 hour. The solvent was evaporated, the thus obtained residue was purified by silica gel column chromatography, and the fraction obtained from the eluate of n-hexane:ethyl acetate = 1:1 was concentrated under reduced pressure. The thus obtained residue was added to a saturated methanol hydrochloric acid solution (5 ml) at 0°C, followed by stirring at room temperature for 1 hour. The solvent was evaporated and then the residue was dried under reduced pressure to obtain the title compound (316 mg) as brown oil.
¹H-NMR (400 MHz, CDCl₃) δ: 2.35 (6H, s), 2.77 (2H, t, J=5.6 Hz), 4.16 (2H, t, J=5.6 Hz), 7.02 (2H, d, J=8.8 Hz), 7.82 (2H, d, J=8.8 Hz), 9.87 (1H, s).

### Reference Example 115

### Methyl 5-formyl-2-hydroxybenzoate

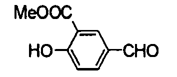

Trimethylsilyldiazomethane (12.0 ml, 2.0 M THF solution) was added to a THF solution (40 ml) of 5-formylsalicylic acid (2.0 g), followed by stirring at room temperature for 2 hours. The solvent was evaporated, the thus obtained residue was purified by silica gel column chromatography, and the fraction obtained from the eluate of n-hexane:ethyl acetate = 10:1 was concentrated under reduced pressure to obtain the title compound (918 mg) as colorless oil.
¹H-NMR (400 MHz, CDCl₃) δ: 4.01 (3H, s), 7.09 (1H, d, J=8.8 Hz), 7.99 (1H, dd, J=2.2, 8.8 Hz), 8.37 (1H, d, J=2.2 Hz), 9.88 (1H, s), 11.34 (1H, s).

### Reference Example 116

### Methyl 5-formyl-2-hydroxy-3-iodobenzoate

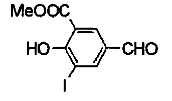

Benzyltrimethylammonium dichloroiodide (1.65 g) and potassium carbonate (3.70 g) were added to a dichloromethane-methanol mixed solution (5:2, v/v) (14 ml) of methyl 5-formyl-2-hydroxybenzoate (800 mg), followed by stirring overnight at room temperature. After completion of the reaction, the solvent was evaporated, and the thus obtained residue was acidified with 1 N hydrochloric acid, extracted with dichloromethane and then washed with water and saturated brine. The extract was dried over sodium sulfate, the solvent was evaporated, the thus obtained residue was purified by silica gel column chromatography, and the fraction obtained from the eluate of n-hexane: ethyl acetate = 10:1 was concentrated under reduced pressure to obtain the title compound (1.07 g) as a white solid.
¹H-NMR (400 MHz, CDCl₃) δ: 4.04 (3H, s), 8.36 (1H, d, J=2.0 Hz), 8.45 (1H, d, J=2.0 Hz), 9.81 (1H, s), 12.21 (1H, s).

### Reference Example 117

### 2-Dimethylamino-N-(4-hydroxymethylphenyl)acetamide

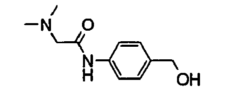

HOBt (1.32 g), EDC·HCl (1.87 g) and *N*-methylmorpholine (1.1 ml) were added to a dichloromethane solution (20 ml) of 4-aminobenzyl alcohol (1.0 g) and *N,N*-dimethylglycine hydrochloride (1.13 g), followed by stirring at room temperature for 2 hours. To the reaction solution was added a saturated aqueous sodium bicarbonate solution, extracted with dichloromethane, washed with water and saturated brine and then dried over sodium sulfate. The solvent was evaporated, the thus obtained residue was purified by silica gel column chromatography, and the fraction obtained from the eluate of n-hexane:ethyl acetate = 2:1 was concentrated under reduced pressure to obtain the title compound (239 mg) as a colorless solid.
¹H-NMR (400 MHz, CDCl₃) δ: 2.36 (6H, s), 3.04 (2H, s), 4.61 (2H, s), 7.30 (2H, d, J=8.3 Hz), 7.54 (2H, d, J=8.3 Hz), 9.11 (1H, br s).

### Reference Example 118

### 2-Dimethylamino-N-(4-formylphenyl)acetamide

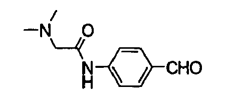

By using the compound obtained in Reference Example 117 and carrying out the operation in the same manner as in Reference Example 64, the title compound was obtained as white powder.
¹H-NMR (400 MHz, CDCl₃) δ: 2.39 (6H, s), 3.10 (2H, s), 7.79 (2H, d, J=8.5 Hz), 7.85 (2H, d, J=8.5 Hz), 9.44 (1H, br s), 9.91 (1H, s).

### Reference Example 119

### Methyl [(tert-butoxycarbonylmethylamino)methyl]benzoate

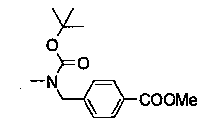

Methylamine (44 ml, 2.0 M THF solution) was added dropwise to a THF solution (10 ml) of methyl 4-(bromomethyl)benzoate, followed by stirring for 1 hour. After removing the resulting crystals by filtration, the filtrate was concentrated, and the thus obtained residue was dissolved in THF (80 ml). Triethylamine (4.9 ml) and (Boc)₂O (5.7 g) were added to the mixture at 0°C, followed by stirring for 1 hour. After completion of the reaction, to the reaction solution was added water, extracted with ethyl acetate and then dried over sodium sulfate. The solvent was evaporated, the thus obtained residue was purified by silica gel column chromatography, and the fraction obtained from the eluate of n-hexane:ethyl acetate = 10:1 was concentrated under reduced pressure to obtain the title compound (4.0 g) as colorless oil.
¹H-NMR (400 MHz, CDCl₃) δ: 1.45 (9H, s), 2.81 (3/2H, s), 2.87 (3/2H, s), 3.91 (3H, s), 4.47 (2H, br s), 7.28 (2H, d, J=8.1 Hz), 8.00 (2H, d, J=8.1 Hz).

### Reference Example 120

### tert-Butyl (4-hydroxymethylbenzyl)methylcarbamate

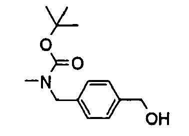

By using the compound obtained in Reference Example 119 and carrying out the operation in the same manner as in Reference Example 95, the title compound was obtained as colorless oil.
¹H-NMR (400 MHz, CDCl₃) δ: 1.48 (9H, s), 2.83 (3H, s), 4.42 (2H, s), 4.69 (2H, s), 7.24 (2H, d, J=7.8 Hz), 7.34 (2H, d, J=7.8 Hz).

### Reference Example 121

### tert-Butyl (4-formylbenzyl)methylcarbamate

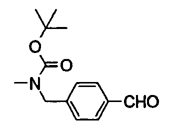

By using the compound obtained in Reference Example 120 and carrying out the operation in the same manner as in Reference Example 64, the title compound was obtained as colorless oil.
¹H-NMR (400 MHz, CDCl₃) δ: 1.45 (9/2H, s), 1.50 (9/2H, s), 2.84 (3/2H, s), 2.89 (3/2H, s), 4.50 (2H, br s), 7.38 (2H, d, J=8.1 Hz), 7.85 (2H, d, J=8.1 Hz), 10.01 (1H, s).

### Reference Example 122

### tert-Butyl methyl{4-[4-(oxazol-5-yl)phenylhydrazonomethyl]benzyl}carbamate

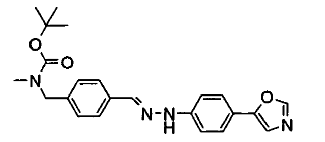

By using the compound obtained in Reference Example 121 and carrying out the operation in the same manner as in Example 31, the title compound was obtained as colorless oil.
¹H-NMR (400 MHz, DMSO-d₆) δ: 1.42 (9H, s), 2.77 (3H, s), 4.38 (2H, s), 7.14 (2H, d, J=8.8 Hz), 7.23 (1H, d, J=8.1 Hz), 7.43 (1H, s), 7.57 (2H, d, J=8.8 Hz), 7.65 (2H, d, J=8.1 Hz), 7.89 (1H, s), 8.31 (1H, s), 10.57 (1H, s).

### Reference Example 123

### 4-Fluoro-N-methoxy-N-methyl-3-nitrobenzamide

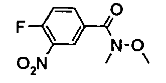

By using 4-fluoro-3-nitrobenzoic acid as the material and carrying out the operation in the same manner as in Reference Example 92, the title compound was obtained as a colorless solid.
¹H-NMR (400 MHz, CDCl₃) δ: 3.40 (3H, s), 3.58 (3H, s), 7.35 (1H, d, J=8.1 Hz), 8.04-8.08 (1H, m), 8.51 (1H, dd, J=1.9 Hz, 8.1 Hz).

### Reference Example 124

### 3-Amino-4-fluoro-N-methoxy-N-methylbenzamide

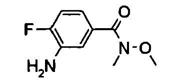

By using the compound obtained in Reference Example 123 and carrying out the operation in the same manner as in Reference Example 11, the title compound was obtained as colorless oil.
¹H NMR (400 MHz, CDCl₃) δ: 3.35 (3H, s), 3.57 (3H, s), 3.82 (2H, s), 7.03-7.22 (3H, m).

### Reference Example 125

### 4-Fluoro-3-iodo-N-methoxy-N-methylbenzamide

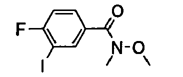

Concentrated hydrochloric acid (4 ml) was added to an aqueous solution (20 ml) of 3-amino-4-fluoro-N-methoxy-N-methylbenzamide (1.80 g) at O°C, followed by stirring for 10 minutes. An aqueous solution (4 ml) of sodium nitrite (940 mg) was added dropwise to the mixture at the same temperature, followed by stirring for 10 minutes. An aqueous solution (3 ml) of potassium iodide (2.26 g) was added to the mixture, followed by stirring at room temperature for 30 minutes and then at 75°C for 1 hour. The reaction solution was cooled to 0°C, sodium hydrogen sulfite was added to the mixture, extracted with chloroform and then dried over sodium sulfate. The solvent was evaporated to obtain the title compound (740 mg) as colorless oil.
¹H-NMR (400 MHz, CDCl₃) δ: 3.36 (3H, s), 3.55 (3H, s), 7.08 (1H, d, J=7.5 Hz), 7.69-7.73 (1H, m), 8.15 (1H, dd, J=1.9 Hz, 5.9 Hz).

### Reference Example 126

### 4-Fluoro-3-iodobenzaldehyde

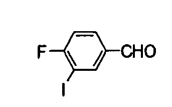

By using the compound obtained in Reference Example 125 and carrying out the operation in the same manner as in Reference Example 95, the title compound was obtained.
¹H-NMR (400 MHz, CDCl₃) δ: 7.21 (1H, t, J=7.8 Hz), 7.85-7.89 (1H, m), 8.29 (1H, dd, J=2.0 Hz, 6.1 Hz), 9.90 (1H, s).

### Reference Example 127

### tert-Butyl 4-(4-formyl-2-iodophenyl)piperazine-1-carboxylate

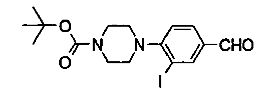

1-Boc-piperazine (362 mg) and potassium carbonate (537 mg) were added to a DMF solution (10 ml) of 4-fluoro-3-iodobenzaldehyde (486 mg), followed by stirring at 90°C for 3 nights. To the reaction solution was added water, extracted with ethyl acetate and then dried over sodium sulfate. The solvent was evaporated, the thus obtained residue was purified by silica gel column chromatography, and the fraction obtained from the eluate of n-hexane:ethyl acetate = 10:3 was concentrated under reduced pressure to obtain the title compound (164 mg) as colorless oil.
¹H-NMR (400 MHz, CDCl₃) δ: 1.49 (9H, s), 3.06 (4H, t, J=4.9 Hz), 3.66 (4H, t, J=4.9 Hz), 7.06 (1H, d, J=8.3 Hz), 7.83 (1H, dd, J=2.0 Hz, 8.3 Hz), 8.34 (1H, d, J=2.0 Hz), 9.84 (1H, s).

### Reference Example 128

### tert-Butyl 4-{2-iodo-4-[4-(oxazol-5-yl)phenylhydrazonomethyl]phenyl)piperazine-1-carboxylate

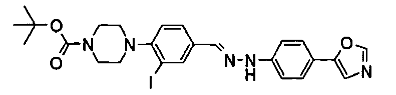

By using the compound obtained in Reference Example 127 and carrying out the operation in the same manner as in Example 31, the title compound was obtained as colorless amorphous.
¹H-NMR (400 MHz, DMSO-d₆) δ: 1.43 (9H, s), 2.90 (4H, br s), 3.50 (4H, br s), 7.13 (4H, d, J=8.5 Hz), 7.42 (1H, s), 7.57 (1H, d, J=8.8 Hz), 7.64 (1H, d, J=8.8 Hz), 7.80 (1H, s), 8.15 (1H, s), 8.31 (1H, s), 10.58 (1H, s).

### Reference Example 129

### 3-Iodo-N-methoxy-N-methyl-4-methylaminomethylbenzamide

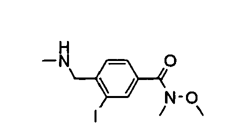

N-Bromosuccinimide (2.17 g) and a catalytic amount of AIBN were added to a carbon tetrachloride solution (70 ml) of 4-methyl-3-iodo-N-methoxy-N-methylbenzamide (3.38 g), followed by heating under reflux for 2 hours. *N*-Bromosuccinimide (2.0 g) and AIBN were further added to the mixture, followed by heating under reflux for 1 hour. The precipitate was celite-filtered, the filtrate was concentrated under reduced pressure, the thus obtained residue was purified by silica gel column chromatography, and a mixture (2.45 g) of the material and the desired product was obtained from the eluate of n-hexane:ethyl acetate = 10:1, and the desired compound (867 mg) from the eluate of n-hexane:ethyl acetate = 2:1 as a yellowish brown solid. The compound was used in the subsequent reaction without purification.

Methylamine (38 ml, 2.0 M THF solution) was added to a THF solution (40 ml) of the above-described mixture (2.45 g), followed by stirring overnight at room temperature. The solvent was evaporated, the thus obtained residue was purified by silica gel column chromatography, and the fraction obtained from the eluate of dichloromethane:methanol = 10:1 was concentrated under reduced pressure to obtain the title compound (1.45 g) as colorless oil.
¹H-NMR (400 MHz, CDCl₃) δ: 2.47 (3H, s), 3.35 (3H, s), 3.55 (3H, s), 3.79 (2H, s), 7.40 (1H, d, J=8.1 Hz), 7.66 (1H, dd, J=1.5 Hz, 8.1 Hz), 8.1 (1H, d, J=1.5 Hz).

### Reference Example 130

### tert-Butyl [2-iodo-4-(methoxymethylcarbamoyl)benzyl]methylcarbamate

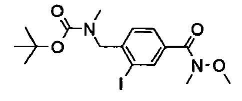

Triethylamine (1.8 ml) and (Boc)₂O (1.89 g) were added to a dichloromethane solution (30 ml) of 3-iodo-*N*-methoxy-*N*-methyl-4-methylaminomethylbenzamide (1.45 g), followed by stirring overnight at room temperature. By adding water to the reaction solution, the dichloromethane layer was separated and washed with saturated brine. After drying over sodium sulfate, the solvent was evaporated, the thus obtained residue was purified by silica gel column chromatography, and the fraction obtained from the eluate of n-hexane:ethyl acetate = 2:1 was concentrated under reduced pressure to obtain the title compound (1.10 g) as colorless oil.
¹H-NMR (400 MHz, CDCl₃) δ: 1.41 (9/2H, s), 1.51 (9/2H, s), 2.87 (3/2H, s), 2.92 (3/2H, s), 3.36 (3H, s), 3.71 (3H, s), 4.42 (2/2H, s), 4.48 (2/2H, s), 7.13 (1H, d, J=8.1 Hz), 7.67 (1H, d, J=8.1 Hz), 8.16 (1H, s).

### Reference Example 131

### tert-Butyl (4-formyl-2-iodobenzyl)methylcarbamate

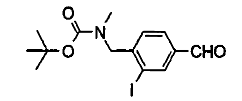

By using the compound obtained in Reference Example 130 and carrying out the operation in the same manner as in Reference Example 95, the title compound was obtained.
¹H-NMR (400 MHz, CDCl₃) δ: 1.40 (9/2H, s), 1.52 (9/2H, s), 2.91 (3/2H, s), 2.95 (3/2H, s), 4.44 (2/2H, s), 4.50 (2/2H, s), 7.26 (1H, d, J=7.6 Hz), 7.85 (1H, d, J=7.6 Hz), 8.33 (1H, s), 9.93 (1H, s).

### Reference Example 132

### Ethyl 2-(tert-butyldiphenylsilyloxymethyl)thiazole-4-carboxylate

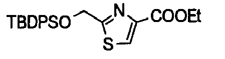

Ethyl bromopyruvate (8.90 g) was added to an acetonitrile solution (200 ml) of 2-(*tert*-butyldiphenylsilyloxy)thioacetamide (15.0 g), followed by heating overnight under reflux. After cooling the reaction solution, a saturated aqueous sodium bicarbonate solution was added to the mixture, acetonitrile was evaporated, and then the residue was extracted with ethyl acetate. The extract was washed with water and brine and dried over sodium sulfate, and then the solvent was evaporated, and the thus obtained residue was used as such in the subsequent reaction.

The above-described residue was dissolved in DMF (300 ml), *tert*-butylchlorodiphenylsilane (14.2 ml) and imidazole (3.72 g) were added to the solution at 0°C and stirred at room temperature for 4 hours. To the reaction solution was added water, extracted with ethyl acetate and dried over sodium sulfate. The solvent was evaporated, the thus obtained residue was purified by silica gel column chromatography, and the fraction obtained from the eluate of n-hexane:ethyl acetate = 10:1 was concentrated under reduced pressure to obtain the title compound (14.0 g) as colorless oil.
¹H-NMR (400 MHz, CDCl₃) δ: 1.13 (9H, s), 1.26 (3H, t, J=7.1 Hz), 4.40 (2H, q, J=7.1 Hz), 5.01 (2H, s), 7.37-7.47 (6H, m), 7.67 (4H, dd, J=1.4 Hz, 7.9 Hz), 8.16 (1H, s).

### Reference Example 133

### [2-(tert-Butyldiphenylsilyloxymethyl)thiazol-4-yl]methanol

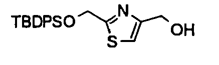

By using the compound obtained in Reference Example 132 and carrying out the operation in the same manner as in Reference Example 95, the title compound was obtained as colorless oil.
¹H-NMR (400 MHz, CDCl₃) δ: 1.24 (9H, s), 4.71 (2H, d, J=6.1 Hz), 4.96 (2H, s), 7.15 (1H, s), 7.37-7.45 (6H, m), 7.68-7.70 (4H, m).

### Reference Example 134

### 2-(tert-Butyldiphenylsilyloxymethyl)-4-trityloxymethylthiazole

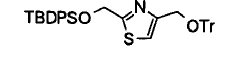

Triphenylmethyl chloride (2.05 g) and triethylamine (1.00 ml) were added to a dichloromethane solution (30 ml) of [2-(*tert*-butyldiphenylsilyloxymethyl)thiazol-4-yl]methanol (1.41 g), followed by stirring overnight. To the reaction solution was added a saturated aqueous ammonium chloride solution, extracted with dichloromethane and then dried over sodium sulfate. The solvent was evaporated, the thus obtained residue was purified by silica gel column chromatography, and the fraction obtained from the eluate of n-hexane:ethyl acetate = 100:3 was concentrated under reduced pressure to obtain the title compound (2.07 g) as colorless oil.
¹H-NMR (400 MHz, CDCl₃) δ: 1.12 (9H, s), 4.94 (2H, s), 5.27 (2H, s), 7.20-7.42 (16H, m), 7.49 (6H, d, J=7.1 Hz), 7.68 (4H, d, J=7.1 Hz).

### Reference Example 135

### (4-Trityloxymethylthiazol-2-yl)methanol

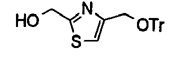

By using the compound obtained in Reference Example 134 and carrying out the operation in the same manner as in Example 65, the title compound was obtained as colorless oil.
¹H-NMR (400 MHz, CDCl₃) δ: 4.30 (2H, s), 4.87 (2H, s), 7.24-7.32 (9H, m), 7.35 (1H, s), 7.50 (6H, d, J=6.9 Hz).

### Reference Example 136

### 4-Trityloxymethylthiazole-2-carboxyaldehyde

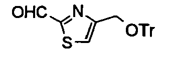

By using the compound obtained in Reference Example 135 and carrying out the operation in the same manner as in Reference Example 64, the title compound was obtained as colorless oil.
¹H-NMR (400 MHz, CDCl₃) δ: 4.45 (2H, s), 7.26-7.39 (9H, m), 7.50 (6H, d, J=8.3 Hz), 7.80 (1H, s), 9.93 (1H, s).

### Reference Example 137

### 4-Trityloxymethylthiazole-2-carboxyaldehyde 4-(oxazol-5-yl)phenylhydrazone

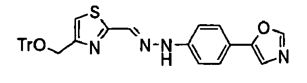

By using the compound obtained in Reference Example 136 and carrying out the operation in the same manner as in Example 31, the title compound was obtained as a mixture of isomers.
Isomer A: ¹H-NMR (400 MHz, CDCl₃) δ: 4.32 (2H, s), 7.16 (2H, d, J=8.5 Hz), 7.20-7.39 (10H, m), 7.50 (6H, d, J=7.4 Hz), 7.58 (2H, d, J=8.5 Hz), 7.86 (2H, s), 8.18 (1H, s).
Isomer B: ¹H-NMR (400 MHz, CDCl₃) δ: 4.42 (2H, s), 7.20-7.43 (14H, m), 7.55 (6H, d, J=7.4 Hz), 7.66 (1H, d, J=7.1 Hz), 7.85 (2H, s), 7.91 (1H, s), 12.87 (1H, s).

### Reference Example 138

### 2-(tert-Butyldiphenylsilyloxymethyl)thiazole-4-carboxyaldehyde

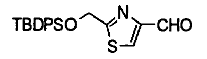

By using the compound obtained in Reference Example 133 and carrying out the operation in the same manner as in Reference Example 64, the title compound was obtained as colorless oil.
¹H-NMR (400 MHz, CDCl₃) δ:1.14 (9H, s), 5.06 (2H, s), 7.36-7.47 (6H, m), 7.62-7.72 (4H, m), 8.15 (1H, s), 9.94 (1H, s).

### Reference Example 139

### 2-Hydroxymethylthiazole-4-carboxyaldehyde

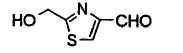

By using the compound obtained in Reference Example 138 and carrying out the operation in the same manner as in Example 65, the title compound was obtained as colorless oil.
¹H-NMR (400 MHz, CDCl₃) δ: 5.30 (2H, s), 8.18 (1H, s), 10.00 (1H, s).

### Reference Example 140

### 2-Dimethylamino-N-methoxy-N-methyl-4-pyridinecarboxamide

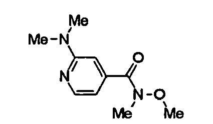

By using the compound obtained in Reference Example 101 and dimethylamine, and carrying out the operation in the same manner as in Reference Example 102, the title compound was obtained as colorless oil.
¹H-NMR (400 MHz, CDCl₃) δ: 3.11 (6H, s), 3.34 (3H, s), 3.69 (3H, s), 6.70 (2H, s), 8.19 (1H, s).

### Reference Example 141

### 2-Dimethylamino-4-pyridinecarboxyaldehyde

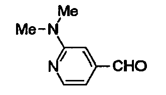

By using the compound obtained in Reference Example 140 and carrying out the operation in the same manner as in Reference Example 95, the title compound was obtained as colorless oil.
¹H-NMR (400 MHz, CDCl₃) δ: 3.15 (6H, s), 6.89 (1H, s), 6.92 (1H, d, J=4.6 Hz), 8.36 (1H, d, J=4.6 Hz), 9.96 (1H, s).

### Reference Example 142

### 6-Fluoro-3-pyridinecarboxylic acid

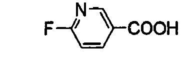

Potassium permanganate (17.8 g) was added to an aqueous solution (200 ml) of 2-fluoro-5-methylpyridine (5.0g) at room temperature, followed by heating under reflux for 4 hours. The reaction solution was cooled to room temperature, and the starting material was evaporated under reduced pressure. After removing the insoluble material by filtration, the filtrate was acidified by adding concentrated hydrochloric acid, extracted with a chloroform-methanol (10:1) mixed solution and then dried over sodium sulfate, and the solvent was evaporated to obtain the title compound (1.54 g) as a white solid.
¹H-NMR (400 MHz, CDCl₃) δ: 7.06 (1H, dd, J=2.9 Hz, 8.5 Hz), 8.48 (1H, ddd, J=1.9 Hz, 2.9 Hz, 8.5 Hz), 8.99 (1H, d, J=1.9 Hz).

### Reference Example 143

### 6-Fluoro-N-methoxy-N-methylnicotinamide

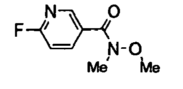

By using the compound obtained in Reference Example 142 and carrying out the operation in the same manner as in Reference Example 92, the title compound was obtained as a colorless solid.
¹H-NMR (400 MHz, CDCl₃) δ: 3.40 (3H, s), 3.57 (3H, s), 6.98 (1H, dd, J=2.2 Hz, 8.7 Hz), 8.19 (1H, ddd, J=2.2 Hz, 2.9 Hz, 8.7 Hz), 8.66 (1H, d, J=2.2 Hz).

### Reference Example 144

### 6-Fluoro-3-pyridinecarboxyaldehyde

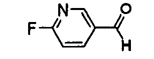

By using the compound obtained in Reference Example 143 and carrying out the operation in the same manner as in Reference Example 95, the title compound was obtained as colorless oil.
¹H-NMR (400 MHz, CDCl₃) δ: 7.11 (1H, dd, J=2.4 Hz, 8.3 Hz), 8.32 (1H, dt, J=2.4 Hz, 7.6 Hz), 8.76 (1H, s), 10.09 (1H, s).

### Reference Example 145

### 6-Dimethylamino-N-methoxy-N-methylnicotinamide

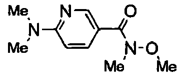

By using the compound obtained in Reference Example 143 and dimethylamine hydrochloride, and carrying out the operation in the same manner as in Reference Example 102, the title compound was obtained as colorless oil.
¹H-NMR (400 MHz, CDCl₃) δ: 3.15 (6H, s), 3.35 (3H, s), 3.61 (3H, s), 6.48 (1H, d, J=9.1 Hz), 7.93 (1H, dd, J=2.4 Hz, 9.1 Hz), 8.71 (1H, d, J=2.4 Hz).

### Reference Example 146

### 6-Dimethylamino-3-pyridinecarboxyaldehyde

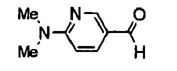

By using the compound obtained in Reference Example 145 and carrying out the operation in the same manner as in Reference Example 95, the title compound was obtained as colorless oil.
¹H-NMR (400 MHz, CDCl₃) δ: 3.21 (6H, s), 6.55 (1H, dd, J=0.5 Hz, 9.1 Hz), 7.91 (1H, ddd, J=0.5 Hz, 2.4 Hz, 9.1 Hz), 8.55 (1H, d, J=2.2 Hz), 9.76 (1H, s).

### Reference Example 147

### N-Methoxy-N-methyl-6-(4-methylpiperazin-1-yl)nicotinamide

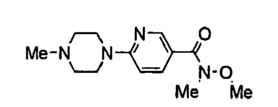

By using the compound obtained in Reference Example 143 and carrying out the operation in the same manner as in Reference Example 102, the title compound was obtained as colorless oil.
¹H-NMR (400 MHz, CDCl₃) δ: 2.35 (3H, s), 2.51 (4H, t, J=4.9 Hz), 3.35 (3H, s), 3.60 (3H, s), 3.67 (4H, t, J=4.9 Hz), 6.60 (1H, d, J=9.1 Hz), 7.94 (1H, dd, J=2.4 Hz, 9.1 Hz), 8.69 (1H, d, J=2.4 Hz).

### Reference Example 148

### 6-(4-Methylpiperazin-1-yl)pyridine-3 -carboxyaldehyde

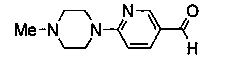

By using the compound obtained in Reference Example 147 and carrying out the operation in the same manner as in Reference Example 95, the title compound was obtained as colorless oil.
¹H-NMR (400 MHz, CDCl₃) δ: 2.35 (3H, s), 2.51 (4H, t, J=5.1 Hz), 3.77 (4H, t, J=5.1 Hz), 6.66 (1H, d, J=9.0 Hz), 7.91 (1H, dd, J=2.1 Hz, 9.0 Hz), 8.55 (1H, d, J=2.1 Hz), 9.77 (1H, s).

### Reference Example 149

### 1-Trityl-1H-imidazol-2-yl-carboxyaldehyde

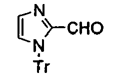

By using 2-imidazolecarboxyaldehyde and carrying out the operation in the same manner as in Reference Example 134, the title compound was obtained as a white solid.
¹H-NMR (400 MHz, CDCl₃) δ: 7.02 (1H, s), 7.10-7.12 (6H, m), 7.29 (1H, s), 7.32-7.34 (9H, m), 9.22 (1H, s).

### Reference Example 150

### N-{2-[2-(4-Methoxycarbonyl)phenyl]ethyl}di-tert-butyliminodicarboxylate

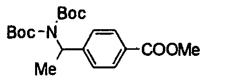

Di-*tert*-butyliminodicarboxylate (1.33 g) and triphenylphosphine (1.60 g) were added to a THF solution (20 ml) of methyl 4-(1-hydroxyethyl)benzoate (1.00 g), followed by stirring. Diisopropyl azadicarboxylate (1.20 ml) was added dropwise to the mixture, followed by stirring overnight at room temperature. The solvent was evaporated, the thus obtained residue was purified by silica gel column chromatography, and the fraction obtained from the eluate of n-hexane:ethyl acetate = 20:1 was concentrated under reduced pressure to obtain the title compound (1.51 g) as colorless oil.
¹H-NMR (400 MHz, CDCl₃) δ: 1.39 (18H, s), 1.72 (3H, d, J=7.8 Hz), 3.91 (3H, s), 5.55 (1H, q, J=7.8 Hz), 7.41 (2H, d, J=8.5 Hz), 7.99 (2H, d, J=8.5 Hz).

### Reference Example 151

### N-{2-[2-(4-Hydroxymethyl)phenyl]ethyl}di-tert-butyliminodicarboxylate

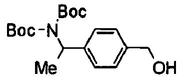

By using the compound obtained in Reference Example 150 and carrying out the operation in the same manner as in Reference Example 95, the title compound was obtained as colorless oil.
¹H-NMR (400 MHz, CDCl₃) δ: 1.38 (18H, s), 1.69 (3H, d, J=7.3 Hz), 4.65 (2H, d, J=5.6 Hz), 5.49 (1H, q, J=7.3 Hz), 7.30 (2H, d, J=8.3 Hz), 7.31 (2H, d, J=8.3 Hz).

### Reference Example 152

### N-{2-[2-(4-Formyl)phenyl]ethyl}di-tert-butyliminodicarboxylate

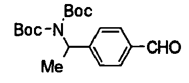

By using the compound obtained in Reference Example 151 and carrying out the operation in the same manner as in Reference Example 64, the title compound was obtained as colorless oil.
¹H-NMR (400 MHz, CDCl₃) δ: 1.40 (18H, s), 1.74 (3H, d, J=7.1 Hz), 5.56 (1H, q, J=7.1 Hz), 7.51 (2H, d, J=8.1 Hz), 7.85 (2H, d, J=8.1 Hz), 10.00 (1H, s).

### Reference Example 153

### N-[1-(4-{N-[4-(Oxazol-5-yl)phenyl]hydrazonomethyl}phenyl)-1-ethyl]di-tert-butyliminodicarboxylate

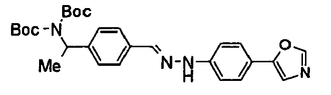

By using the compound obtained in Reference Example 152 and carrying out the operation in the same manner as in Example 31, the title compound was obtained as a yellow solid.
¹H-NMR (400 MHz, CDCl₃) δ: 1.38 (18H, s), 1.71 (3H, d, J=6.8 Hz), 5.53 (1H, q, J=6.8 Hz), 7.15 (2H, d, J=7.1 Hz), 7.21 (1H, d, J=1.0 Hz), 7.36 (2H, d, J=7.6 Hz), 7.58 (2H, dd, J=1.0 Hz, 7.6 Hz), 7.60 (2H, d, J=7.1 Hz), 7.67 (1H, s), 8.85 (1H, d, J=1.0 Hz).

### Reference Example 154

### 5-Formyl-2-hydroxy-3-iodobenzoic acid

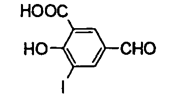

1 N Sodium hydroxide (4 ml) was added to a methanol/THF mixed solution (5 ml, 1:1) of methyl 5-formyl-2-hydroxy-3-iodobenzoate (340 mg) at 0°C, followed by stirring at room temperature for 3 hours. After completion of the reaction, the solvent was evaporated, and the thus obtained residue was acidified by adding 1 N hydrochloric acid and extracted with dichloromethane. After drying the organic layer over sodium sulfate, the solvent was evaporated to obtain the title compound (324 mg) as yellowish brown powder.
¹H-NMR (400 MHz, DMSO-d₆) δ: 8.32 (1H, s), 8.40 (1H, s), 9.79 (1H, s).

### Reference Example 155

### Ethyl 1-benzyl-1,2,3,6-tetrahydropyridine-4-carboxylate

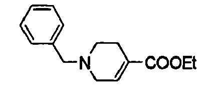

Benzyl bromide (2.0 ml) was added to an ethanol solution (5 ml) of isonicotinic acid ethyl ester (2.0 g), followed by stirring with heating at 80°C for 1 hour. After cooling the reaction solution to room temperature, n-hexane was added to the mixture, followed by stirring at room temperature for 1 hour. The resulting crystals were collected by filtration and then dissolved in ethanol (80 ml). Sodium borohydride (543 mg) was added to the mixture in small portions at 0°C, followed by stirring at the same temperature for 30 minutes. To the reaction solution was added water, extracted with dichloromethane and washed with saturated brine. After drying the extract over sodium sulfate, the solvent was evaporated, the thus obtained residue was purified by silica gel column chromatography, and the fraction obtained from the eluate of n-hexane:ethyl acetate = 10:3 was concentrated under reduced pressure to obtain the title compound (2.31 g) as brown oil.
¹H-NMR (400 MHz, CDCl₃) δ: 1.28 (3H, t, J=7.3 Hz), 2.39-2.44 (2H, m), 2.61 (2H, t, J=5.8 Hz), 3.13 (2H, q, J=2.9 Hz), 3.61 (2H, s), 4.19 (2H, q, J=7.3 Hz), 6.86-6.88 (1H, m), 7.25-7.35 (5H, m).

### Reference Example 156

### 1-Benzyl-1,2,3,6-tetrahydropyridine-4-carboxyaldehyde

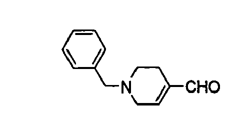

Diisobutylaluminum hydride (25.7 ml, 0.93 M hexane solution) was added dropwise to a THF solution (40 ml) of ethyl 1-benzyl-1,2,3,6-tetrahydropyridine-4-carboxylate (2.0 g) at -78°C, followed by stirring at the same temperature for 2 hours. A saturated aqueous ammonium chloride solution and diethyl ether were added dropwise to the reaction solution, followed by stirring at room temperature for 30 minutes. To the reaction solution was added magnesium sulfate and further stirred for 30 minutes. The resulting precipitate was removed by celite filtration, the filtrate was evaporated, and the thus obtained residue was used in the subsequent reaction without purification.

The above-described residue was dissolved in chloroform (40 ml), manganese dioxide (2.0 g) was added to the mixture and heated under reflux for 3 hours. After removing the precipitate by filtration, the filtrate was concentrated, the thus obtained residue was purified by silica gel column chromatography, and the fraction obtained from the eluate of n-hexane:ethyl acetate = 10:3 was concentrated under reduced pressure to obtain the title compound (635 mg) as brown oil.
¹H-NMR (400 MHz, CDCl₃) δ: 2.33-2.36 (2H, m), 2.60 (2H, t, J=5.6 Hz), 3.22-3.26 (2H, m), 3.63 (2H, s), 6.69-6.70 (1H, m), 7.23-7.38 (5H, m), 9.45 (1H, s).

### Reference Example 157

### Ethyl 6-iodoimidazo[1,2-a]pyridine-2-carboxylate

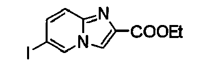

Bromopyruvic acid ethyl ester (1.4 ml) was added to an acetonitrile solution (60 ml) of 2-amino-5-iodopyridine (2.38 g), followed by heating overnight under reflux. After cooling the reaction solution, a saturated aqueous sodium bicarbonate solution was added to the mixture, acetonitrile was evaporated, and the residue was extracted with ethyl acetate. The extract was washed with water and brine and dried over sodium sulfate, and then the solvent was evaporated, the thus obtained residue was purified by silica gel column chromatography, and the fraction obtained from the eluate of n-hexane:ethyl acetate = 10:3 was concentrated under reduced pressure to obtain the title compound (1.74 g) as a white solid.
¹H-NMR (400 MHz, CDCl₃) δ: 1.44 (3H, t, J=7.1 Hz), 4.46 (2H, q, J=7.1 Hz), 7.41 (1H, dd, J=1.5 Hz, 9.5 Hz), 7.48 (1H, d, J=9.5 Hz), 8.17 (1H, s), 8.39 (1H, s).

### Reference Example 158

### 6-Iodoimidazo[1,2-a]pyridine-2-carboxyaldehyde

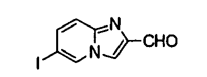

By using the compound obtained in Reference Example 157 and carrying out the operation in the same manner as in Reference Example 95, the title compound was obtained.
¹H-NMR (400 MHz, CDCl₃) δ: 7.52 (1H, d, J=9.5 Hz), 7.56 (1H, d, J=9.5 Hz), 8.54 (1H, s), 9.03 (1H, d, J=1.0 Hz), 10.04 (1H, d, J=1.0 Hz).

### Reference Example 159

### 4-(4-Dimethylaminopiperidin-1-yl)-3-iodobenzaldehyde

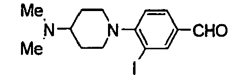

By using the compound obtained in Reference Example 126 and 4-dimethylaminopiperidine, and carrying out the operation in the same manner as in Reference Example 127, the title compound was obtained as colorless oil.
¹H-NMR (400 MHz, CDCl₃) δ: 1.75-1.84 (2H, m), 1.95-2.02 (2H, m), 2.36 (6H, s), 2.73 (2H, t, J=11.8 Hz), 3.47-3.52 (3H, m), 7.06 (1H, d, J=8.3 Hz), 7.80 (1H, dd, J=1.7 Hz, 8.3 Hz), 8.32 (1H, d, J=1.7 Hz), 9.82 (1H, s).

### Reference Example 160

### Ethyl bromopyridin-4-ylacetate

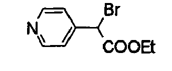

Ethyl 4-pyridylacetate (5.0 g) was dissolved in an acetic acid solution (40 ml) of about 30% HBr, and bromine (1.72 ml) was added dropwise to the mixture at room temperature, followed by stirring for 30 minutes. After concentration under reduced pressure, the residue was diluted with ethyl acetate (500 ml), washed with a saturated aqueous sodium bicarbonate solution (200 ml) and dried over anhydrous sodium sulfate. A 1 N hydrochloric acid-ethanol solution (30 ml) was added to the mixture, to the residue concentrated under reduced pressure was added ethyl acetate, and the thus obtained crystals were washed with diethyl ether to obtain the title compound (7.68 g) as a pale yellow solid.
¹H-NMR (400 MHz, DMSO-d₆) δ: 1.18 (3H, t, J=7.1 Hz), 4.21 (2H, q, J=7.0 Hz), 6.27 (1H, s), 7.98 (2H, d, J=6.6 Hz), 8.89 (2H, d, J=6.4 Hz).

### Reference Example 161

### Ethyl oxopyridin-4-ylacetate

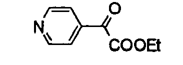

Ethyl bromopyridin-4-ylacetate (7.66 g) was dissolved in acetonitrile (250 ml), and an aqueous solution (60 ml) of sodium azide (3.91 g) was added dropwise to the solution at room temperature. After stirring for 12 hours and subsequent evaporation of acetonitrile, copper(II)sulfate pentahydrate (500 mg) was added to the mixture, followed by stirring at room temperature for 1 hour. Ethyl acetate (300 ml) was added to the reaction mixture, and then the organic layer was washed with water (150 ml) twice and dried over anhydrous sodium sulfate. 1 N hydrochloric acid-ethanol (60 ml) was added to the mixture, followed by concentration under reduced pressure, and the resulting solid was washed with diethyl ether to obtain the title compound (1.97 g) as a pale crimson solid.
¹H-NMR (400 MHz, CDCl₃) δ: 1.11 (3H, t, J=7.1 Hz), 4.07 (2H, q, J=7.1 Hz), 8.04 (2H, d, J=5.6 Hz), 8.88 (2H, d, J=5.4 Hz).

### Reference Example 162

### 4-(Oxazol-5-yl)benzenediazonium tetrafluoroborate

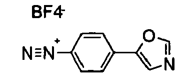

4-(Oxazol-5-yl)phenylamine (1.0 g) was dissolved in water (6 ml) and concentrated hydrochloric acid (2 ml), and an aqueous solution (1 ml) of sodium nitrite (474 mg) was added dropwise to the mixture at 0°C over 30 minutes. The mixture was stirred at the same temperature for 1 hour and then sodium borofluoride (2.74 g) was added to the mixture, followed by stirring for 1 hour. The resulting insoluble material was collected by filtration and washed with water, ethanol and diethyl ether to obtain the title compound (47.6 mg) as a pale yellow solid.
¹H-NMR (400 MHz, DMSO-d₆) δ: 8.25 (1H, s), 8.28 (2H, d, J=9.3 Hz), 8.72 (2H, d, J=9.2 Hz), 8.77 (1H, s).

### Reference Example 163

### Ethyl benzimidate hydrochloride

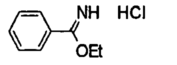

Benzonitrile (20.0 g) was dissolved in about 8 N hydrochloric acid-ethanol (50 ml), followed by stirring at 0°C for 3 hours. The mixture was allowed to stand still in a refrigerator (5°C) for 2 days and diethyl ether (30 ml) was added to the mixture, and the thus precipitate was collected by filtration and washed with diethyl ether to obtain the title compound (31.0 g) as colorless prisms.
¹H-NMR (400 MHz, DMSO-d₆) δ: 1.46 (3H, t, J=7.1 Hz), 4.66 (2H, q, J=7.1 Hz), 7.62 (2H, t, J=7.6 Hz), 7.79 (1H, t, J=7.5 Hz), 8.15 (2H, d, J=8.6 Hz), 12.02 (2H, br s).

### Reference Example 164

### 2-Fluoro-N-methoxy-N-methyl-4-nitrobenzamide

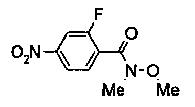

By using 2-fluoro-4-nitrobenzoic acid and carrying out the operation in the same manner as in Reference Example 92, the title compound was obtained as a colorless solid.
¹H-NMR (400 MHz, CDCl₃) δ: 3.40 (3H, s), 3.54 (3H, s), 7.63 (1H, d, J=6.9 Hz), 8.00 (1H, d, J=7.8 Hz), 8.10 (1H, d, J=7.8 Hz).

### Reference Example 165

### 5-(2-Fluoro-4-nitrophenyl)oxazole

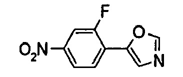

Diisobutylaluminum hydride (43.0 ml, 0.93 M hexane solution) was added dropwise to a THF solution (60 ml) of 2-fluoro-N-methoxy-N-methyl-4-nitrobenzamide (3.66 g) at -78°C, followed by stirring at the same temperature for 1 hour. A saturated aqueous ammonium chloride solution (22 ml) was added dropwise to the reaction solution, followed by stirring at room temperature for 30 minutes, and magnesium sulfate and diethyl ether were added to the mixture, followed by stirring for 30 minutes. After celite filtration, the residue obtained by evaporating the solvent was purified by silica gel column chromatography, the fraction obtained from the eluate of n-hexane:ethyl acetate = 20:1 was concentrated under reduced pressure, and the thus obtained residue was used as such in the subsequent reaction.

Potassium carbonate (2.45 g) was added to a methanol solution (60 ml) of the above-described residue (2.73 g) and p-toluenesulfonylmethyl isocyanide (3.47 g), followed by heating under reflux for 3 hours. After cooling the reaction solution, the solvent was evaporated, the thus obtained residue was purified by silica gel column chromatography, and the fraction obtained from the eluate of n-hexane:ethyl acetate = 2:1 was concentrated under reduced pressure to obtain the title compound (1.98 g) as colorless oil.
¹H-NMR (400 MHz, CDCl₃)δ:7.73 (1H, d, J=4.1 Hz), 7.97 (1H, t, J=7.1 Hz), 8.07 (1H, dd, J=2.2 Hz, 10.1 Hz), 8.08 (1H, s), 8.16 (1H, ddd, J=0.5 Hz, 2.2 Hz, 10.1 Hz).

### Reference Example 166

### 3-Fluoro-4-(oxazol-5-yl)aniline

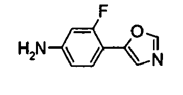

By using the compound obtained in Reference Example 165 and carrying out the operation in the same manner as in Reference Example 85, the title compound was obtained as yellow oil.
¹H-NMR (400 MHz, CDCl₃) δ: 3.93 (2H, br s), 6.45 (1H, dd, J=2.2 Hz, 12.5 Hz), 6.51 (1H, dd, J=2.2 Hz, 8.3 Hz), 7.28 (1H, d, J=3.7 Hz), 7.51 (1H, t, J=8.3 Hz), 7.85 (1H, s).

### Reference Example 167

### 3-Fluoro-4-(oxazol-5-yl)phenylhydrazine

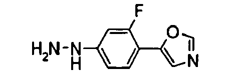

By using the compound obtained in Reference Example 166 and carrying out the operation in the same manner as in Reference Example 86, the title compound was obtained as a red solid.
¹H-NMR (400 MHz, DMSO-d₆) δ: 4.18 (2H, s), 6.64 (1H, dd, J=2.0 Hz, 8.8 Hz), 6.67 (1H, d, J=14.6 Hz), 7.19 (1H, d, J=3.4 Hz), 7.42 (1H, br s), 7.45 (1H, d, J=8.8 Hz), 8.34 (1H, s).

### Reference Example 168

### tert-Butyl 1-{2-[3-iodo-4-(oxazol-5-yl)phenylhydrazonomethyl]thiazol-4-yl}ethylcarbamate

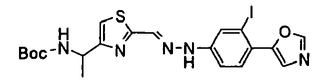

*tert*-Butyl 1-(2-formylthiazol-4-yl)ethyl]carbamate (170 mg) was added to an ethanol solution (5 ml) of 3-iodo-4-(oxazol-5-yl)phenylhydrazine (200 mg), followed by heating overnight under reflux. Diethyl ether was added to the residue obtained by evaporating the solvent, and the resulting powde was collected by filtration, washed with diethyl ether and dried to obtain the title compound (278 mg) as a mixture of isomers.
¹H-NMR (400 MHz, CDCl₃) δ: 1.38 (9H, s), 1.48 (3H, d, J=7.1 Hz), 4.70-4.78 (0.5H, m), 4.87-4.99 (0.5H, m), 7.15 (0.5H, d, J=8.5 Hz), 7.27 (0.5H, s), 7.32 (0.5H, d, J=8.5 Hz), 7.38-7.63 (4H, m), 7.67 (0.5H, d, J=2.2 Hz), 7.95 (0.5H, s), 8.05 (0.5H, s), 8.45 (0.5H, s), 8.47 (0.5H, s), 11.16 (0.5H, s), 13.08 (0.5H, s).

### Reference Example 169

### tert-Butyl 4-{4-[3-iodo-4-(oxazol-5-yl)phenylhydrazonomethyl]phenyl}piperazine-1-carboxylate

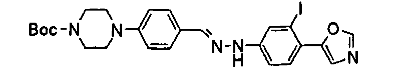

By using the compounds obtained in Reference Example 78 and Reference Example 86, and carrying out the operation in the same manner as in Reference Example 168, the title compound was obtained as a yellow solid.
¹H-NMR (400 MHz, CDCl₃) δ: 1.43 (9H, s), 3.19 (4H, t, J=4.9 Hz), 3.47 (4H, t, J=4.9 Hz), 6.98 (2H, d, J=8.3 Hz), 7.11 (1H, d, J=8.5 Hz), 7.40 (1H, d, J=8.5 Hz), 7.50 (1H, s), 7.54 (2H, d, J=8.3 Hz), 7.64 (1H, s), 7.83 (1H, s), 8.42 (1H, s), 10.44 (1H, s).

### Reference Example 170

### 6-Bromo-2-(4-nitrophenyl)imidazo[1,2-a]pyridine

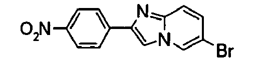

By using 2-amino-5-bromopyridine and carrying out the operation in the same manner as in Reference Example 46, the title compound was obtained as a crystalline solid.
¹H-NMR (400 MHz, DMSO-d₆) δ: 7.43 (1H, dd, J=6.8 Hz, 2.0 Hz), 7.62 (1H, d, J=9.8 Hz), 8.22 (2H, d, J=9.0 Hz), 8.31 (2H, d, J=9.0 Hz), 8.59 (1H, s), 8.93 (1H, d, J=1.7 Hz).

### Reference Example 171

### 4-(6-Bromoimidazo[1,2-a]pyridin-2-yl)phenylamine

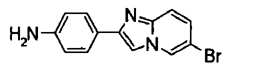

Tin chloride dihydrate (18.5 g) was added to a THF (300 ml) solution of 6-bromo-2-(4-nitrophenyl)imidazo[1,2-a]pyridine (5.22 g), followed by heating under reflux for 1 hour while stirring. After cooling, the mixture was concentrated under reduced pressure, aqueous ammonia (28%) and chloroform:methanol = 10:1 were added to the residue, and then the mixture was celite-filtrated. The organic layer was dried over sodium sulfate, and then the crystals precipitated by evaporating the solvent under reduced pressure were dried to obtain the title compound (2.81 g) as a crystalline solid.
¹H-NMR (400 MHz, DMSO-d₆) δ: 5.28 (2H, s), 6.61 (2H, d, J=8.3 Hz), 7.27 (1H, dd, J=9.5 Hz, 1.2 Hz), 7.48 (1H, d, J=9.5 Hz), 7.62 (2H, d, J=8.3 Hz), 8.11 (1H, s), 8.80 (1H, s).

### Reference Example 172

### 6-Chloro-2-(4-nitrophenyl)imidazo[1,2-a]pyridine

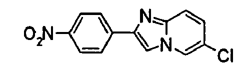

By using 2-amino-5-chloropyridine and carrying out the operation in the same manner as in Reference Example 46, the title compound was obtained as a yellow solid.
¹H-NMR (DMSO-d₆) δ: 7.35 (1H, dd, J=9.6 Hz, 2.1 Hz), 7.67 (1H, d, J=9.8 Hz), 8.22 (2H, d, J=8.3 Hz), 8.30 (2H, d, J=8.5 Hz), 8.59 (1H, s), 8.86 (1H, d, J=2.0 Hz).

### Reference Example 173

### 4-(6-Chloroimidazo[1,2-a]pyridin-2-yl)phenylamine

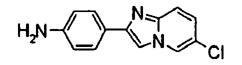

By using the compound obtained in Reference Example 172 and carrying out the operation in the same manner as in Reference Example 171, the title compound was obtained as a yellowish brown solid.
¹H-NMR (400 MHz, DMSO-d₆) δ: 5.26 (2H, br s), 6.60 (2H, d, J=8.8 Hz), 7.20 (1H, dd, J=9.5 Hz, 2.2 Hz), 7.52 (1H, d, J=9.5 Hz), 7.58 (2H, d, J=8.6 Hz), 8.08 (1H, s), 8.72 (1H, d, J=1.2 Hz).

### Reference Example 174

### 4-(6-Chloroimidazo[1,2-a]pyridin-2-yl)phenylhydrazine

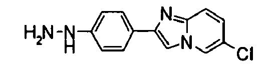

By using the compound obtained in Reference Example 173 and carrying out the operation in the same manner as in Reference Example 86, the title compound was obtained as a gray solid.
¹H-NMR (400 MHz, DMSO-d₆) δ: 4.21 (2H, br s), 6.82 (2H, d, J=8.6 Hz), 6.91 (1H, br s), 7.20 (1H, dd, J=9.4 Hz, 2.1 Hz), 7.53 (1H, d, J=9.5 Hz), 7.69 (2H, d, J=8.3 Hz), 8.12 (1H, s), 8.72 (1H, d, J=2.0 Hz).

### Reference Example 175

### 6-Fluoro-2-(4-nitrophenyl)imidazo[1,2-a]pyridine

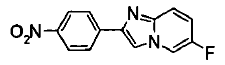

By using 2-amino-5-fluoropyridine and carrying out the operation in the same manner as in Reference Example 46, the title compound was obtained as a yellow solid.
¹H-NMR (400 MHz, DMSO-d₆) δ: 7.39 (1H, dd, J=10.0 Hz, 5.6 Hz), 7.70 (1H, dd, J=10.0 Hz, 5.4 Hz), 8.22 (2H, d, J=9.0 Hz), 8.29 (2H, d, J=9.0 Hz), 8.62 (1H, s), 8.81 (1H, d, J=2.7 Hz).

### Reference Example 176

### 4-(6-Fluoroimidazo[1,2-a]pyridin-2-yl)phenylamine

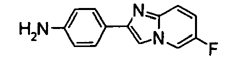

By using the compound obtained in Reference Example 175 and carrying out the operation in the same manner as in Reference Example 171, the title compound was obtained as a yellow solid.
¹H-NMR (400 MHz, DMSO-d₆) δ: 5.24 (2H, s), 6.59 (2H, d, J=8.6 Hz), 7.22 (1H, t, J=9.6 Hz), 7.53 (1H, dd, J=5.2 Hz, 9.8 Hz), 7.60 (2H, d, J=8.5 Hz), 8.10 (1H, s), 8.67 (1H, br s).

### Reference Example 177

### 6-(4-Nitrophenyl)imidazo[2, 1-b]thiazole

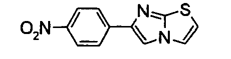

By using 2-aminothiazole and carrying out the operation in the same manner as in Reference Example 46, the title compound was obtained as a crystalline solid.
¹H-NMR (400 MHz, DMSO-d₆) δ: 7.35 (1H, d, J=4.4 Hz), 8.00 (1H, d, J=4.4 Hz), 8.10 (2H, d, J=8.3 Hz), 8.26 (2H, d, J=8.5 Hz), 8.51 (1H, s).

### Reference Example 178

### 4-(Imidazo[2,1-b]thiazol-6-yl)phenylamine

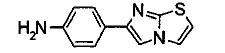

6-(4-Nitrophenyl)imidazo[2,1-b]thiazole (0.40 g), 5% Pd-C (0.4 g) and concentrated hydrochloric acid (2.0 ml) were added to DMF (150 ml)-methanol (50 ml), followed by catalytic reduction for 23 hours under hydrogen atmosphere. The catalyst was removed by filtration, the filtrate was concentrated under reduced pressure, the thus obtained residue was purified by silica gel column chromatography, and the fraction obtained from the eluate of dichloromethane:methanol = 20:1 was concentrated under reduced pressure and then dried to obtain the title compound (0.18 g) as a crystalline solid.
¹H-NMR (400 MHz, DMSO-d₆) δ: 5.12 (2H, s), 6.57 (2H, d, J=8.5 Hz), 7.17 (1H, d, J=4.4 Hz), 7.48 (2H, d, J=8. Hz), 7.86 (1H, d, J=4.4 Hz), 7.91 (1H, s).

### Reference Example 179

### 4-(Imidazo[1,2-a]pyrimidin-2-yl)phenylamine

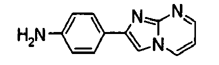

An acetone solution (250 ml) of 2-bromo-1-(4-nitrophenyl)ethanone (10.3 g) and 2-aminopyrimidine (4.0 g) was heated at 70°C under reflux for 3 hours. After evaporation of the solvent, methanol (200 ml) was added to the mixture, followed by heating at 70°C under reflux for 14 hours. After concentration of the mixture under reduced pressure, a saturated aqueous sodium bicarbonate solution was added to the residue, and the resulting solid was collected by filtration and washed with water, ethanol, ethyl acetate and diethyl ether to obtain a yellow solid.

Tin chloride dihydrate (28.5 g) was added to a THF suspension (300 ml) of the above-described solid, followed by heating at 70°C under reflux for 4 hours. The solvent was evaporated, and the residue was alkalified by adding a saturated aqueous sodium bicarbonate solution, filtered through celite and washed with chloroform:methanol = 5:1. The organic layer was dried over anhydrous sodium sulfate and then concentrated under reduced pressure, and the thus obtained solid was washed with diethyl ether to obtain the title compound (3.23 g) as a yellow solid.
¹H-NMR (400 MHz, DMSO-d₆) δ: 5.31 (2H, br s), 6.62 (2H, d, J=8.5 Hz), 6.96 (1H, dd, J=6.8 Hz, 4.2 Hz), 7.66 (2H, d, J=8.5 Hz), 8.08 (1H, s), 8.41 (1H, dd, J=4.1 Hz, 2.0 Hz), 8.86 (1H, dd, J=6.6 Hz, 2.0 Hz).

### Reference Example 180

### 4-(Imidazo[1,2-a]pyrimidin-2-yl)phenylhydrazine

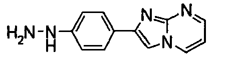

By using the compound obtained in Reference Example 179 and carrying out the operation in the same manner as in Reference Example 86, the title compound was obtained as a yellowish brown solid.
¹H-NMR (400 MHz, DMSO-d₆) δ: 6.87 (2H, d, J=8.5 Hz), 6.98 (1H, dd, J=6.6 Hz, 4.2 Hz), 7.23 (1H, br s), 7.3 (2H, br s), 7.76 (2H, d, J=8.5 Hz), 8.15 (1H, s), 8.43 (1H, d, J=2.2 Hz), 8.89 (1H, dd, J=6.6 Hz, 2.0 Hz).

### Reference Example 181

### 2-(4-Nitrophenyl)-6-methoxybenzothiazole

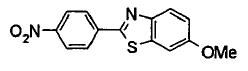

A 50 wt% potassium hydroxide solution (200 ml) of 2-amino-6-methoxybenzothiazole (18.4 g) was heated at 100°C under reflux for 16 hours. The reaction solution was cooled to 0°C and then acidified by adding acetic acid, and the resulting crystals were collected by filtration. By washing the crystals with water and ethanol, a yellowish brown solid (12.4 g) was obtained.

An ethanol solution (200 ml) of the above-described solid and 4-nitrobenzaldehyde (12.1 g) was heated at 80°C under reflux for 18 hours. By washing the resulting solid with ethanol, water and ethyl acetate, the title compound (14.0) was obtained as a yellowish brown solid.
¹H-NMR (400 MHz, CDCl₃) δ: 3.74 (3H, s), 6.76 (1H, dd, J=8.8 Hz, 2.7 Hz), 7.19 (1H, d, J=8. 8 Hz), 8.14 (2H, d, J=8. 8 Hz), 8.34 (2H, d, J=9.0 Hz), 8.60 (1H, s).

### Reference Example 182

### 2-(4-Nitrophenyl)-6-hydroxybenzothiazole

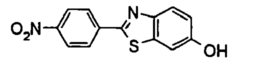

2-(4-Nitrophenyl)-6-methoxybenzothiazole (7.8 g) was dissolved in dichloromethane (200 ml), a 1 M tribromoborane-dichloromethane solution (54 ml) was added dropwise to the solution at -78°C, and then the reaction temperature was allowed to warm to room temperature overnight, followed by stirring for 15 hours. After quenching with methanol, an aqueous solution (500 ml) of 20 wt% potassium hydroxide was added to the mixture to remove the organic layer, and then the water layer was acidified by adding concentrated hydrochloric acid and extracted with chloroform:methanol = 4:1 (500 ml) twice. The organic layer was dried over anhydrous sodium sulfate, the solvent was evaporated, and the residue was purified by flash silica gel column chromatography (hexane:ethyl acetate = 3:1 to 1:1) to obtain the title compound (4.21 g) as a yellow solid.
¹H-NMR (400 MHz, DMSO-d₆) δ: 7.05 (1H, dd, J=8.8 Hz, 1.2 Hz), 7.47 (1H, d, J=1.7 Hz), 7.93 (1H, d, J=8.8 Hz), 8.24 (2H, d, J=7.8 Hz), 8.35 (2H, d, J=7.8 Hz), 10.10 (1H, br s).

### Reference Example 183

### 4-(6-Hydroxybenzothiazol-2-yl)phenylamine

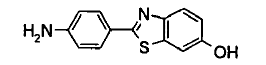

By using the compound obtained in Reference Example 182 and carrying out the operation in the same manner as in Reference Example 9, the title compound was obtained as a pale yellow solid.
¹H-NMR (400 MHz, DMSO-d₆) δ: 5.75 (2H, d, J=6.1 Hz), 6.63 (2H, d, J=8.5 Hz), 6.89 (1H, dd, J=8.9 Hz, 2.3 Hz), 7.30 (1H, d, J=2.4 Hz), 7.64 (2H, d, J=8.7 Hz), 7.67 (1H, d, J=8.7 Hz), 9.68 (1H, br s).

### Reference Example 184

### 2-Amino-5-iodopyrimidine

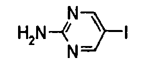

Iodine (21.7 g) and o-periodic acid (6.5 g) were added to a suspension of 2-aminopyrimidine (19 g) in acetic acid (200 ml), sulfuric acid (2.5 ml) and water (30 ml), followed by stirring at 90°C for 20 hours. To the reaction solution was added 10% aqueous sodium thiosulfate solution (300 ml) and extracted with dichloromethane (300 ml) twice. The solid obtained by drying over anhydrous sodium sulfate was recrystallized from water to obtain the title compound (4.1 g) as a pale yellow solid.
¹H-NMR (400 MHz, DMSO-d₆) δ: 6.82 (2H, s), 8.34 (2H, s).

### Reference Example 185

### 4-(6-Iodoimidazo[1,2-a]pyrimidin-2-yl)phenylamine

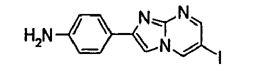

A THF (50 ml) suspension of 2-amino-5-iodopyrimidine (1.51 g) and 2-bromo-1-(4-nitrophenyl)ethanone (1.67 g) was heated under reflux for 3 days. The solid obtained by evaporating the solvent was washed with a saturated aqueous sodium bicarbonate solution and water to obtain a yellow solid (1.31 g).

A THF suspension (50 ml) of the above-described solid and tin chloride dihydrate (2.02 g) was heated under reflux for 5 hours. The solvent was evaporated, and then to the residue was added a saturated aqueous sodium bicarbonate solution (300 ml) and extracted with chloroform:methanol = 5:1 (300 ml) twice. After drying the extract over anhydrous sodium sulfate, the solvent was evaporated, and the residue was purified by flash silica gel column chromatography (chloroform:methanol = 20:1) to obtain the title compound (475 mg) as a yellow solid.
¹H-NMR (400 MHz, DMSO-d₆) δ: 5.34 (2H, d, J=7.6 Hz), 6.61 (2H, d, J=8.5 Hz), 7.65 (2H, d, J=8.5 Hz), 7.99 (1H, s), 8.48 (1H, d, J=2.2 Hz), 9.22 (1H, d, J=2.2 Hz).

### Reference Example 186

### Trifluoroacetic acid N-[4-(6-iodoimidazo[1,2-a]pyridin-2-yl)phenyl]-N'-pyridin-4-ylmethylenehydrazide

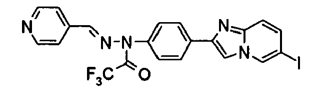

Trifluoroacetic anhydride (105 ml) was added to a THF suspension (8 ml) of 4-pyridinecarboxyaldehyde 4-(6-iodoimidazo[1,2-a]pyridin-2-yl)phenylhydrazone (132 mg) at 0°C, followed by stirring for 2 hours. A small amount of methanol was added to the mixture and then the solvent was evaporated. The thus obtained residue was diluted with ethyl acetate (60 ml) and washed with a saturated aqueous sodium bicarbonate solution and saturated brine (30 ml for each), and the organic layer was dried over anhydrous sodium sulfate. The solvent was evaporated, and the residue was purified by flash silica gel column chromatography (hexane:acetone = 2:1) to obtain the title compound (108 mg) as a reddish brown solid.
¹H-NMR (400 MHz, DMSO-d₆) δ: 7.47 (2H, s), 7.54 (2H, d, J=8.0 Hz), 7.64 (2H, d, J=5.4 Hz), 7.67 (1H, s), 8.21 (2H, d, J=8.1 Hz), 8.46 (1H, s), 8.65 (2H, d, J=3.9 Hz), 8.95 (1H, s).

### Reference Example 187

### Trifluoroacetic acid N-[4-(6-tributylstannylimidazo[1,2-a]pyridin-2-yl)phenyl]-N'-pyridin-4-ylmethylenehydrazide

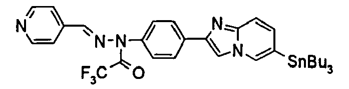

Hexabutylditin (382 ml) and tetrakis(triphenylphosphine)palladium (43.5 mg) were added to a 1,4-dioxane solution (10 ml) of trifluoroacetic acid *N*-[4-(6-iodoimidazo[1,2-a]pyridin-2-yl)phenyl]-*N'*-pyridin-4-ylmethylenehydrazide (101 mg), followed by stirring at 100°C for 2 hours. The solvent was evaporated, and the residue was purified by flash silica gel column chromatography (hexane:acetone = 2:1) to obtain the title compound (27.3 mg) as yellow oil.
¹H-NMR (400 MHz, CDCl₃) δ: 0.90-0.94 (9H, m), 1.12-1.16 (6H, m), 1.31-1.41 (6H, m), 1.53-1.62 (6H, m), 7.24-7.35 (3H, m), 7.43 (1H, s), 7.48 (2H, d, J=6.1 Hz), 7.63 (1H, d, J=7.5 Hz), 7.92 (1H, s), 8.03 (1H, s), 8.19 (2H, d, J=8.6 Hz), 8.68 (2H, d, J=6.1 Hz).

### Reference Example 188

### tert-Butyl N-(4-iodophenyl)-N'-pyridin-4-ylmethylenehydrazinecarboxylate

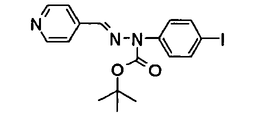

Dimethylaminopyridine (2.47 g) and (Boc)₂O (4.42 g) were added to a THF solution (60 ml) of 4-pyridinecarboxyaldehyde 4-iodophenylhydrazone (3.27 g) at 0°C, followed by stirring overnight. After completion of the reaction, to the reaction solution was added water, extracted with ethyl acetate and then dried over sodium sulfate. The solvent was evaporated, the thus obtained residue was purified by silica gel column chromatography, and the fraction obtained from the eluate of n-hexane:ethyl acetate = 10:2 was concentrated under reduced pressure to obtain the title compound (4.0 g) as colorless oil.
¹H-NMR (400 MHz, CDCl₃) δ: 1.44 (9H, s), 7.12 (2H, d, J=8.3 Hz), 7.29 (1H, s), 7.58 (2H, d, J=5.9 Hz), 7.92 (2H, d, J=8.3 Hz), 8.58 (2H, d, J=5.9 Hz).

### Reference Example 189

### tert-Butyl N-[4-(2-iodovinyl)phenyl]-N'-pyridin-4-ylmethylenehydrazinecarboxylate

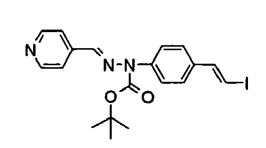

(E)-1,2-Bisbutylstannylethylene (515 mg) and a catalytic amount of dichloro bis(triphenylphosphine)palladium(II) were added to a toluene solution (5 ml) of *tert*-butyl *N-*-(4-iodophenyl)-*N*'-pyridin-4-ylmethylenehydrazinecarboxylate (300 mg), followed by heating under reflux for 1 hour. After removing the catalyst by filtration, the filtrate was concentrated, and the thus obtained residue was directly used in the subsequent reaction without purification.

The above-described residue was dissolved in dichloromethane (7 ml), and iodine (270 mg) was added to the solution, followed by stirring at room temperature for 1 hour. To the reaction solution was added water and the dichloromethane layer was extracted. The extract was washed with an aqueous solution of 0.1 mol/l sodium thiosulfate, water and saturated brine and then dried over sodium sulfate. The solvent was evaporated, the thus obtained residue was purified by silica gel column chromatography, and the fraction obtained from the eluate of n-hexane:ethyl acetate = 1:1 was concentrated under reduced pressure to obtain the title compound (173 mg) as colorless oil.
¹H-NMR (400 MHz, CDCl₃) δ: 1.50 (9H, s), 6.99 (1H, d, J=15.1 Hz), 7.16 (2H, d, J=8.3 Hz), 7.17 (1H, s), 7.45 (2H, d, J=8.3 Hz), 7.46 (1H, d, J=15.1 Hz), 7.50 (2H, d, J=8.3 Hz), 8.58 (2H, d, J=8.3 Hz).

### Reference Example 190

### 1-(Chloroethyl)-2-methyl-4-(4-nitrophenyl)-1H-imidazole

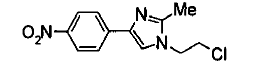

2-Bromo-1-(4-nitrophenyl)ethanone (6.0 g) was dissolved in THF (50 ml), and 2-methyloxazoline (2.07 ml) was added to the solution at room temperature, followed by stirring for 20 hours. A 7 N ammonia/methanol solution (10.5 ml) was added to the mixture, followed by stirring for 8 hours. The reaction solution was concentrated under reduced pressure, and the residue was diluted with ethyl acetate (300 ml) and washed twice with saturated brine (150 ml). The organic layer was dried over anhydrous sodium sulfate, concentrated under reduced pressure and then purified by flash silica gel column chromatography (ethyl acetate) to remove impurities, and then the product was suspended in dichloromethane (100 ml) and, under ice-cooling, thionyl chloride (1.19 ml) was added dropwise to the mixture. The reaction solution was concentrated, ethyl acetate (300 ml) was added to the residue and washed with a saturated aqueous sodium bicarbonate solution and saturated brine (150 ml for each). The organic layer was dried over anhydrous sodium sulfate and concentrated, and the thus obtained residue was purified by flash silica gel column chromatography (hexane:ethyl acetate = 1:1) to obtain the title compound (1.25 g) as a reddish brown solid.
¹H-NMR (400 MHz, CDCl₃) δ: 2.50 (3H, s), 3.80 (2H, t, J=6.2 Hz), 4.26 (2H, t, J=6.1 Hz), 7.35 (1H, s), 7.87 (2H, d, J=8.8 Hz), 8.22 (2H, d, J=9.0 Hz).

### Reference Example 191

### 4-[1-(2-Chloroethyl)-2-methyl-1 H-imidazol-4-yl]phenylamine

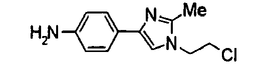

By using the compound obtained in Reference Example 190 and carrying out the operation in the same manner as in Reference Example 9, the title compound was obtained as a yellow solid.
¹H-NMR (400 MHz, CDCl₃) δ: 2.45 (3H, s), 3.65 (2H, br s), 3.74 (2H, t, J=6.5 Hz), 4.18 (2H, t, J=6.5 Hz), 6.69 (2H, d, J=8.1 Hz), 7.01 (1H, s), 7.53 (2H, d, J=8.0 Hz).

### Reference Example 192

### 3-Iodo-4,5-dimethoxybenzamide

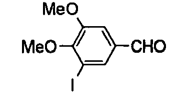

5-Iodovanillin (2.0 g) was dissolved in acetone (50 ml), and dimethyl sulfate (0.82 ml) and potassium carbonate (1.49 g) were added to the solution, followed by stirring at 60°C for 4 hours. After removing the large portion of acetone by concentration under reduced pressure, the mixture was diluted with ethyl acetate (200 ml) and washed with water (100 ml), and the organic layer was dried over anhydrous sodium sulfate. The solvent was evaporated, and the residue was purified by flash silica gel column chromatography (hexane:ethyl acetate = 4:1) to obtain the title compound (1.65 g) as a white solid.
¹H-NMR (400 MHz, CDCl₃)δ:3.93 (6H, s), 7.41 (1H, s), 7.85 (1H, s), 9.83 (1H, s).

### Reference Example 193

### 3-Methoxy-5-methylbromobenzene

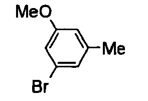

2-Methoxy-6-methylaniline (10 g) was dissolved in methanol (60 ml) and acetic acid (20 ml), and bromine (1.64 ml) was added dropwise to the mixture at room temperature, followed by stirring for 1 hour. The solvent was evaporated to obtain a residue as red oil.

The above-described residue was dissolved in acetic acid (70 ml), water (30 ml) and concentrated hydrochloric acid (8 ml), and an aqueous solution (20 ml) of sodium nitrite (5.05 g) was added dropwise to the mixture at 0°C. After stirring at the same temperature for 30 minutes, an aqueous solution (80 ml) of 50% phosphinic acid was added to the mixture, followed by stirring at room temperature for 3 days. After extraction with ethyl acetate (300 ml) and drying over anhydrous sodium sulfate, the solvent was evaporated, and the residue was purified by flash silica gel column chromatography (hexane:ethyl acetate = 60: 1) to obtain the title compound (3.92 g) as colorless oil.
¹H-NMR (400 MHz, CDCl₃) δ: 2.29 (3H, s), 3.77 (3H, s), 6.63 (1H, s), 6.85 (1H, s), 6.92 (1H, s).

### Reference Example 194

### 3-Bromo-5-methoxybenzoic acid

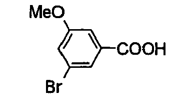

3-Methoxy-5-methylbromobenzene (3.65 g) was dissolved in pyridine (5 ml) and water (10 ml), and potassium permanganate (8.61 g) was added to the mixture at 70°C, followed by vigorously stirring for 3 days. Hot water (100 ml) was added to the mixture, followed by stirring at room temperature for 2 hours and then filtration through celite, and the insoluble material was washed with methanol. By adding ethyl acetate (200 ml), impurities were extracted into the organic layer, and the fraction obtained by concentrating the water layer under reduced pressure was recrystallized from diethyl ether to obtain the title compound (3.48 g) as a white solid.
¹H-NMR (400 MHz, DMSO-d₆) δ: 3.73 (3H, s), 6.98 (1H, s), 7.34 (1H, s), 7.51 (1H, s).

### Reference Example 195

### 3-Bromo-5,N-dimethoxy-N-methylbenzamide

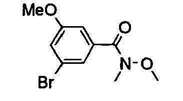

By using the compound obtained in Reference Example 194 and carrying out the operation in the same manner as in Reference Example 92, the title compound was obtained as colorless oil.
¹H-NMR (400 MHz, CDCl₃) δ: 3.35 (3H, s), 3.57 (3H, s), 3.82 (3H, s), 7.13 (2H, s), 7.38 (1H, s).

### Reference Example 196

### 3-Bromo-5-methoxybenzaldehyde

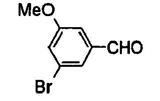

By using the compound obtained in Reference Example 195 and carrying out the operation in the same manner as in Reference Example 95, the title compound was obtained as white crystals.
¹H-NMR (400 MHz, CDCl₃)δ:3.87 (3H, s), 7.31 (1H, s), 7.32 (1H, s), 7.58 (1H, s), 9.91 (1H, s).

### Reference Example 197

### tert-Butyl {2-iodo-4-[4-(pyridin-3-yl)phenylhydrazonomethyl]benzyl}methylcarbamate

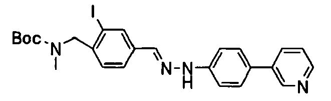

*tert*-Butyl *N*-(4-formyl-2-iodobenzyl)-*N*-methylcarbamate (244 mg) was added to an ethanol solution (30 ml) of 4-(pyridin-3-yl)phenylhydrazine (120 mg), followed by heating under reflux for 1 hour. The solvent was evaporated, the thus obtained residue was purified by silica gel column chromatography, and the fraction obtained from the eluate of dichloromethane:methanol = 20:1 was concentrated under reduced pressure to obtain the title compound (315 mg) as brown amorphous.
¹H-NMR (400 MHz, CDCl₃) δ: 1.43,1.52 (9H, s), 2.87,2.92 (3H, s), 4.42,4.49 (2H, s), 7.12 (1H, m), 7.22 (2H, d, J=8.5 Hz), 7.34 (1H, m), 7.53 (2H, d, J=8.5 Hz), 7.61 (2H, dd, J=8.1 Hz, 1.5 Hz), 7.86 (1H, m), 7.91 (1H, s), 8.12 (1H, s), 8.53 (1H, d, J=4.2 Hz), 8.84 (1H, s).

### Reference Example 198

### 4-Iodo-3-methylbenzonitrile

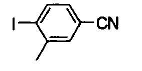

An aqueous solution (5 ml) of sodium nitrite (1.25 g) was added to an aqueous solution (20 ml) of 4-amino-3-methylbenzonitrile (2.0 g) under ice-cooling. Subsequently, an aqueous solution (5 ml) of potassium iodide (3.77 g) was added to the mixture, followed by stirring at room temperature for 2 hours and then stirred at 75°C for 1 hour. After completion of the reaction, sodium hydrogen sulfite was added to the mixture under ice-cooling, followed by extraction with dichloromethane. The organic layer was washed with saturated sodium thiosulfate, saturated sodium bicarbonate, water and saturated brine and then dried over sodium sulfate. The solvent was evaporated, the thus obtained residue was purified by silica gel column chromatography, and the fraction obtained from the eluate of n-hexane:ethyl acetate = 10:3 was concentrated under reduced pressure to obtain the title compound (2.22 g) as a white solid.
¹H-NMR (400 MHz, CDCl₃) δ: 2.46 (3H, s), 7.13 (1H, dd, J=1.5 Hz, 8.1 Hz), 7.48 (1H, d, J=1.5 Hz), 7.92 (1H, d, J=8.1 Hz).

### Reference Example 199

### tert-Butyl (5-cyano-2-iodobenzyl)methylcarbamate

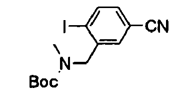

*N*-Bromosuccinimide (1.95 g) and a catalytic amount of AIBN were added to a carbon tetrachloride solution (40 ml) of 4-iodo-3-methylbenzonitrile (2.22 g), followed by heating under reflux for 3 days. The precipitate was filtered through celite, the filtrate was concentrated under reduced pressure, and the thus obtained concentrated residue was used as such in the subsequent reaction without purification.

Methylamine (20 ml, 2.0 M THF solution) was added to a THF solution (10 ml) of the above-described residue (2.36 g), followed by stirring overnight at room temperature. Triethylamine (2.0 ml) and (Boc)₂O (3.0 g) were added to a dichloromethane solution (10 ml) of the residue obtained by evaporating the solvent, followed by stirring overnight at room temperature. To the reaction solution was added water, and the dichloromethane layer was washed with saturated brine. After drying over sodium sulfate, the solvent was evaporated, the thus obtained residue was purified by silica gel column chromatography, and the fraction obtained from the eluate of n-hexane:ethyl acetate = 10:3 was concentrated under reduced pressure to obtain the title compound (368 mg) as colorless oil.
¹H-NMR (400 MHz, CDCl₃) δ: 1.42 (9/2H, s), 1.44 (9/2H, s), 2.92 (3H, br s), 4.44 (2H, br s), 7.24 (1H, d, J=8.1 Hz), 7.33 (1H, s), 7.97 (1H, d, J=8.1 Hz).

### Reference Example 200

### tert-Butyl (5-formyl-2-iodobenzyl)methylcarbamate

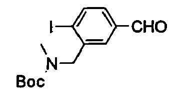

By using the compound obtained in Reference Example 199 and carrying out the operation in the same manner as in Reference Example 95, the title compound was obtained.
¹H-NMR (400 MHz, CDCl₃) δ: 1.42 (9/2H, s), 1.53 (9/2H, s), 2.93 (3H, s), 4.47 (2/2H, s), 4.50 (2/2H, s), 7.47 (1H.br s), 7.56 (1H, s), 8.04 (1H, s), 10.02 (1H, s).

### Reference Example 201

### 4-Bromomethyl-3-chlorobenzonitrile

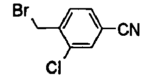

By using 3-chloro-4-methylbenzonitrile and carrying out the operation in the same manner as in Reference Example 93, the title compound was obtained as a yellowish brown solid.
¹H-NMR (400 MHz, CDCl₃) δ: 4.57 (2H, s), 7.56 (1H, d, J=1.7 Hz), 7.56 (1H, s), 7.68 (1H, d, J=1.7 Hz).

### Reference Example 202

### tert-Butyl (2-chloro-4-cyanobenzyl)methylcarbamate

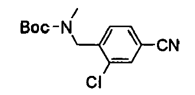

Methylamine (11 ml, 2.0 M THF solution) was added to a THF solution (20 ml) of 4-bromomethyl-3-chlorobenzonitrile (1.09 g), followed by stirring overnight at room temperature. Triethylamine (2.9 ml) and (Boc)₂O (3.0 g) were added to a dichloromethane solution (10 ml) of the residue obtained by evaporating the solvent, followed by stirring overnight at room temperature. To the reaction solution was added water, and the dichloromethane layer was washed with saturated brine. After drying over sodium sulfate, the solvent was evaporated, the thus obtained residue was purified by silica gel column chromatography, and the fraction obtained from the eluate of n-hexane:ethyl acetate = 10:3 was concentrated under reduced pressure to obtain the title compound (1.90 g) as colorless oil.
¹H-NMR (400 MHz, CDCl₃) δ: 1.33 (9/2H, s), 1.46 (9/2H, s), 2.83 (3/2H, s), 2.86 (3/2H, s), 4.47 (2/2H, s), 4.51 (2/2H, s), 7.23 (1H, br s), 7.49 (1H, d, J=7.1 Hz), 7.59 (1H, s).

### Reference Example 203

### tert-Butyl (2-chloro-4-formylbenzyl)methylcarbamate

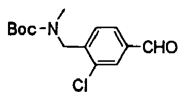

By using the compound obtained in Reference Example 202 and carrying out the operation in the same manner as in Reference Example 95, the title compound was obtained as a white solid.
¹H-NMR (400 MHz, CDCl₃) δ: 1.40 (9/2H, s), 1.51 (9/2H, s), 2.90 (3/2H, s), 2.95 (3/2H, s), 4.57 (2/2H, s), 4.62 (2/2H, s), 7.36 (1H, d, J=7.8 Hz), 7.77 (1H, d, J=7.8 Hz), 7.88 (1H, s), 9.97 (1H, s).

### Reference Example 204

### 3-Fluoro-N-methoxy-4,N-dimethylbenzamide

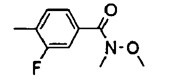

By using 3-fluoro-4-methylbenzoic acid and carrying out the operation in the same manner as in Reference Example 92, the title compound was obtained as a colorless solid.
¹H-NMR (400 MHz, CDCl₃) δ: 2.31 (3H, s), 3.53 (3H, s), 3.56 (3H, s), 7.21 (1H, t, J=7.8 Hz), 7.39 (2H, t, J=7.8 Hz).

### Reference Example 205

### tert-Butyl [2-fluoro-4-(N-methoxy-N-methylcarbamoyl)benzyl]methylcarbamate

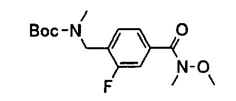

*N*-Bromosuccinimide (3.64 g) and a catalytic amount of AIBN were added to a carbon tetrachloride solution (80 ml) of 3-fluoro-*N*-methoxy-4,*N*-dimethylbenzamide (4.0 g), followed by heating under reflux for 3 hours. The precipitate was filtered through celite, the filtrate was concentrated under reduced pressure, the thus obtained concentrated residue was purified by silica gel column chromatography, and the fraction obtained from the eluate of n-hexane:ethyl acetate = 10:3 was concentrated under reduced pressure to obtain a mixture (1.44 g) of the starting material and a bromo compound. The mixture was used in the subsequent reaction without further purification.

Methylamine (12.6 ml, 2.0 M THF solution) was added to a THF solution (20 ml) of the above-described mixture (1.40 g), followed by stirring overnight at room temperature. Triethylamine (506 µl) and (Boc)₂O (530 mg) were added to a dichloromethane solution (10 ml) of the residue obtained by evaporating the solvent, followed by stirring overnight at room temperature. To the reaction solution was added water and the dichloromethane layer was washed with saturated brine. After drying over sodium sulfate, the solvent was evaporated, the thus obtained residue was purified by silica gel column chromatography, and the fraction obtained from the eluate of n-hexane:ethyl acetate = 2:1 was concentrated under reduced pressure to obtain the title compound (374 mg) as colorless oil.
¹H-NMR (400 MHz, CDCl₃) δ: 1.43 (9/2H, s), 1.46 (9/2H, s), 2.84 (3/2H, s), 2.88 (3/2H, s), 3.34 (3H, s), 3.74 (3H, s), 4.48 (2/2H, s), 4.50 (2/2H, s), 6.64 (1H, br s), 6.75 (1/2H, s), 6.77 (1/2H, s), 7.83 (1H, d, J=7.3 Hz).

### Reference Example 206

### 2,2,2-Trifluoro-N-{2-iodo-4-[4-(oxazol-5-yl)phenyl-2,2,2-trifluoroacetylhydrazonomethyl]benzy I} -N-methylacetamide

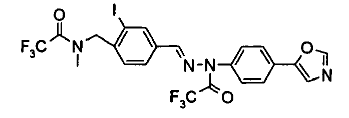

Trifluoroacetic anhydride (57.7 ml) was added to a THF solution (8 ml) of 3-iodo-4-(N-methylaminomethyl)benzaldehyde 4-(oxazol-5-yl)phenylhydrazone (71.7 mg) at 0°C, followed by stirring at room temperature for 1 hour. After adding a small amount of methanol, the solvent was evaporated. The thus obtained residue was diluted with chloroform: methanol = 9:1 (60 ml) and washed with a saturated aqueous sodium bicarbonate solution and saturated brine (30 ml for each), and the organic layer dried over anhydrous sodium sulfate. The residue obtained by evaporating the solvent was purified by flash silica gel column chromatography (chloroform:methanol = 40:1), the title compound (88.4 mg) was obtained as yellow oil.
¹H-NMR (400 MHz, CDCl₃) δ: 3.02 (0.9H, s), 3.11 (2.1H, s), 4.67 (0.7H, s), 4.74 (1.3H, s), 7.11-7.18 (3H, m), 7.59 (4H, m), 7.87 (2H, m), 8.19 (1H, d, J=7.3 Hz).

### Reference Example 207

### 2,2,2-Trifluoro-N-methyl-N-{4-[4-(oxazol-5-yl)phenylhydrazonomethyl]-2-trimethylstannylbenzyl} acetamide

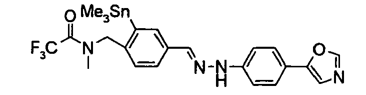

Hexamethylditin (94.9 mg) and tetrakis(triphenylphosphine)palladium (16.7 mg) were added to a dioxane solution (5 ml) of 2,2,2-trifluoro-*N*-{2-iodo-4-[4-(oxazol-5-yl)phenyl-2,2,2-trifluoroacetylhydrazonomethyl]benzyl}-*N*-methylacetamide (45.2 mg), followed by heating at 90°C under reflux for 1.5 hours under argon atmosphere. After evaporation of the solvent, the residue was purified by flash silica gel column chromatography (hexane:acetone = 4:1 to 2:1) to obtain the title compound (10.2 mg) as yellow oil.
¹H-NMR (400 MHz, CDCl₃) δ: 0.41 (9H, s), 2.98 (1H, s), 3.04 (2H, s), 4.65 (0.7H, s), 4.69 (1.3H, s), 7.14 (2H, d, J=8.9 Hz), 7.22 (1H, s), 7.26 (1H, d, J=2.7 Hz), 7.33 (1H, s), 7.48 (1H, m), 7.58 (2H, d, J=8.5 Hz), 7.63 (1H, m), 7.71-7.75 (1H, m), 7.86 (1H, s).

### Reference Example 208

### tert-Butyl 5-(N-methoxy-N-methylcarbamoyl)benzimidazole-1-carboxylate, tert-butyl 6-(N-methoxy-N-methylcarbamoyl)benzimidazole-1-carboxylate

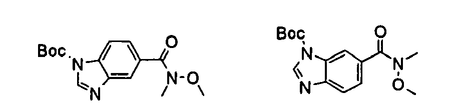

Benzimidazole-5-carboxylic acid (3.0 g) was dissolved in THF (90 ml), and (Boc)₂O (8.08 g) and DMAP (4.52 g) were added to the solution at room temperature, followed by stirring for 19 hours. After evaporation of the solvent, the residue was diluted with ethyl acetate (300 ml) and washed three times with a saturated aqueous ammonium chloride solution (100 ml). The organic layer was dried over anhydrous sodium sulfate and then the solvent was evaporated to obtain the residue (2.54 g).

The above-described residue (2.54 g) was dissolved in dichloromethane (50 ml), NMM (3.36 ml), HOBt-H₂O (1.78 g) and *N*,*O*-dimethylhydroxyamine hydrochloride (1.13 g) were added to the solution at 0°C, followed by stirring for 30 minutes, and then EDC-HCl (2.23 g) was added to the mixture, followed by stirring at room temperature for 6 hours. The solvent was evaporated, and the residue was diluted with ethyl acetate (200 ml), washed with a saturated aqueous ammonium chloride solution and saturated brine (100 ml for each) and dried over anhydrous sodium sulfate. The mixture was concentrated under reduced pressure and then purified by flash silica gel column chromatography (hexane:ethyl acetate = 1:1) to obtain the title compound (2.83 g) as a pale brown oil of a mixture of positional isomers (about 1:1).
¹H-NMR (400 MHz, CDCl₃) δ: 1.71 (9H, s), 3.40 (3H, s), 3.56 (1.5H, s), 3.58 (1.5H, s), 7.71 (0.5H, dd, J=8.4 Hz, 1.6 Hz), 7.78 (0.5H, dd, J=8. Hz, 1.7 Hz), 7.80 (0.5H, d, J=8. Hz), 8.02 (0.5H, d, J=8. Hz), 8.18 (0.5H, s), 8.38 (0.5H, s), 8.48 (0.5H, s), 8.51 (0.5H, s).

### Reference Example 209

### tert-Butyl 5-formylbenzimidazole-1-carboxylate, tert-butyl 6-formylbenzimidazole-1-carboxylate

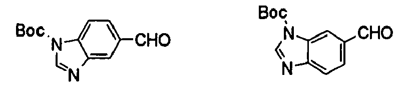

By using the compound obtained in Reference Example 208 and carrying out the operation in the same manner as in Reference Example 95, the title compound was obtained as a pale brown oil of a mixture of positional isomers (about 1:1).
¹H-NMR (400 MHz, CDCl₃) δ: 1.72 (9H, s), 7.45 (0.5H, dd, J=26.2,8.3 Hz), 7.75 (0.5H, d, J=8.1 Hz), 7. 81 (0.5H, s), 7.98 (0.5H, d, J=8. 8 Hz), 8.06 (0.5H, s), 8.15 (0.5H, d, J=8. 5 Hz), 8.30 (0.5H, s), 8.59 (0.5H, s), 10.12 (1H, s).

### Reference Example 210

### 3-Iodophenylhydrazine

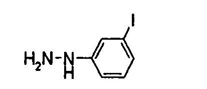

By using 3-iodoaniline and carrying out the operation in the same manner as in Reference Example 3, the title compound was obtained as a reddish brown solid.
¹H-NMR (400 MHz, CDCl₃)δ:3.54 (2H, br s), 5.17 (1H, br s), 6.74 (1H, dt, J=1.0 Hz, 8.1 Hz), 6.92 (1H, t, J=8.1 Hz), 7.12 (1H, dd, J=1.0 Hz, 8.1 Hz), 7.20 (1H, s).

### Reference Example 211

### 4-(6-Iodoimidazo[1,2-a]pyridin-2-yl)phenyldiazonium tetrafluoroborate

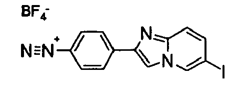

4-(6-Iodoimidazo[1,2-a]pyridin-2-yl)phenylamine (601 mg) was suspended in concentrated hydrochloric acid (2 ml) and water (6 ml), and an aqueous solution (1 ml) of sodium nitrite (136 mg) was slowly added dropwise to the suspension at 0°C. After stirring for 1 hour, sodium borofluoride (788 mg) was added to the mixture, and the thus formed insoluble subtance was collected by filtration and washed with water to obtain the title compound (720 mg) as a yellowish brown solid.
¹H-NMR (400 MHz, DMSO-d₆) δ: 7.51 (1H, d, J=9.4 Hz), 7.57 (1H, d, J=10.1 Hz), 8.49 (2H, d, J=8.8 Hz), 8.69 (2H, d, J=8.6 Hz), 8.71 (1H, s), 9.03 (1H, s).

### Example 1

### 4-Pyridinecarboxyaldehyde 4-(imidazol-1-yl)phenylhydrazone

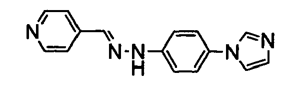

4-(Imidazol-1-yl)phenylamine (505.5 mg) was dissolved in water (5 ml) and concentrated hydrochloric acid (5 ml), and an aqueous solution (2 ml) of sodium nitrite (310 mg) was added dropwise to the solution at 0°C over 30 minutes. After stirring at the same temperature for 30 minutes, a concentrated hydrochloric acid solution (3 ml) of tin(II) chloride dihydrate (1.69 g) was added to the mixture, followed by stirring at room temperature for 30 minutes. The reaction solution was alkalified by adding an aqueous solution of 20% potassium hydroxide at 0°C and then extracted with chloroform: methanol = 9:1 (100 ml) twice. After drying the organic layer over anhydrous sodium sulfate, the solvent was evaporated to obtain a reddish brown residue (511 mg).

The thus obtained residue and 4-pyridinecarboxyaldehyde (276 µl) were dissolved in ethanol (10 ml) and heated under reflux for 15 hours. After evaporation of the solvent, the residue was purified by flash silica gel column chromatography (chloroform: methanol = 30:1) to obtain the title compound (419 mg) as a yellow solid.
¹H-NMR (400 MHz, CDCl₃) δ: 7.20 (1H, s), 7.22 (1H, s), 7.25 (2H, d, J=8.8 Hz), 7.32 (2H, d, J=8. 8 Hz), 7.52 (2H, d, J=4.7 Hz), 7.66 (1H, s), 7.79 (1H, s), 8.25 (1H, s), 8.61 (2H, d, J=4.7 Hz).
ESI-MS m/z: 264 (M+H)⁺

### Example 2

### 4-Pyridinecarboxyaldehyde 4-(4,5-dihydrothiazol-2-yl)phenylhydrazone

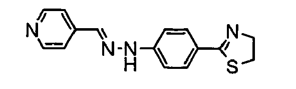

4-(4,5-dihydrothiazol-2-yl)phenylamine (195 mg) was dissolved in water (5 ml) and concentrated hydrochloric acid (5 ml), and an aqueous solution (2 ml) of sodium nitrite (90.6 mg) was slowly added dropwise to the solution at 0°C. After stirring at the same temperature for 30 minutes, a concentrated hydrochloric acid solution (2 ml) of tin(II) chloride dihydrate (494 mg) was added to the mixture, followed by stirring at room temperature for 30 minutes. The reaction solution was alkalified by adding an aqueous solution of 20% potassium hydroxide and extracted with chloroform: methanol = 9:1 (200 ml) twice. The mixture was dried over anhydrous sodium sulfate, and the solvent was evaporated to obtain a reddish brown residue. The residue and 4-pyridinecarboxyaldehyde (52.5 µl) were dissolved in ethanol (8 ml) and heated under reflux for 15 hours. The solvent was evaporated, and the residue was purified by flash silica gel column chromatography (chloroform:methanol = 30:1) to obtain the title compound (49.5 mg) as a yellow solid.
¹H-NMR (400 MHz, CDCl₃) δ: 3.40 (2H, t, J=8.1 Hz), 4.43 (2H, t, J=8.2 Hz), 7.14 (2H, d, J=8.8 Hz), 7.52 (2H, d, J=6.1 Hz), 7.64 (1H, s), 7.79 (2H, d, J=8.8 Hz), 8.08 (1H, s), 8.61 (2H, d, J=6.1 Hz).
ESI-MS m/z: 283 (M+H)⁺.

### Example 3

### 4-Pyridinecarboxyaldehyde 4-(oxazol-5-yl)phenylhydrazone

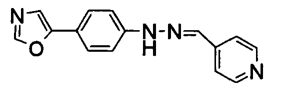

4-(Oxazol-5-yl)phenylhydrazine (1.0 g) and 4-pyridinecarboxyaldehyde (0.61 g) were heated overnight under reflux in ethanol (50 ml). After cooling, the precipitate was colleted by filtration and recrystallized from ethanol to obtain the title compound (1.03 g).
¹H-NMR (400 MHz, CDCl₃) δ: 7.20 (2H, d, J=8.56 Hz), 7.24 (1H, s), 7.52 (2H, J=5.39 Hz), 7.60 (2H, d, J=8.33 Hz), 7.63 (1H, s), 7.87 (1H, s), 8.06 (1H, br s), 8.60 (2H, d, J=5.39 Hz).
FAB-MS m/z: 265 (M+H)⁺

A part of the product was dissolved in ethanol, 10% hydrochloric acid-ethanol was added to the solution, and the precipitated crystals were collected by filtration to obtain the hydrochloride.
¹H-NMR (400 MHz, DMSO-d₆) δ: 7.38 (2H, d, J=8.82 Hz), 7.57 (1H, s), 7.70 (2H, d, J=8.82 Hz), 8.00 (1H, s), 8.18 (2H, d, J=6.86 Hz), 8.39 (1H, s), 8.74 (2H, d, J=6.86 Hz), 11.99 (1H, s).
FAB-MS m/z: 265 (M+H)⁺.

### Example 4

### N-[4-(Oxazol-5-yl)phenyl]-N'-pyridin-4-ylmethylenehydrazinecarboxylic acid tert-butyl ester

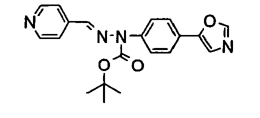

*tert*-Butoxycarboxylic anhydride (406 mg) and 4,4-dimethylaminopyridine (208 mg) were added at room temperature to a THF solution (50 ml) of 4-pyridinecarboxyaldehyde 4-(oxazol-5-yl)phenylhydrazone (410 mg), followed by stirring for 3 days. After evaporation of the solvent, the residue was purified by flash silica gel column chromatography (hexane: acetone = 3:1) to obtain the title compound (198 mg) as a yellow solid.
¹H-NMR (400 MHz, CDCl₃) δ: 1.51 (9H, s), 7.22 (1H, s), 7.27 (2H, d, J=8.6 Hz), 7.46 (1H, s), 7.48 (2H, d, J=5.9 Hz), 7.83 (2H, d, J=8.6 Hz), 7.98 (1H, s), 8.59 (2H, d, J=5.9 Hz).
ESI-MS m/z: 365 (M+H)⁺

### Example 5

### Acetic acid N-[4-(oxazol-5-yl)phenyl]-N'-pyridin-4-ylmethylenehydrazide

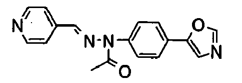

DMAP (41.0 mg) was added to a THF solution (10 ml) of 4-pyridinecarboxyaldehyde 4-(oxazol-5-yl)phenylhydrazone (59.1 mg) and acetic anhydride (31.7 µl) under ice-cooling, followed by stirring for 3 days. The solvent was evaporated, and the residue was diluted with ethyl acetate (120 ml), washed with a saturated aqueous ammonium chloride solution, a saturated aqueous sodium bicarbonate solution and saturated brine and dried over anhydrous sodium sulfate. After evaporation of the solvent, the residue was purified by preparative silica gel column chromatography (hexane:acetone = 1.5:1) to obtain the title compound (23.1 mg) as a white solid.
¹H-NMR (400 MHz, CDCl₃) δ: 2.64 (3H, s), 7.21 (1H, s), 7.25 (2H, d, J=8.6 Hz), 7.45 (2H, d, J=5.8 Hz), 7.46 (1H, s), 7.86 (2H, d, J=8.6 Hz), 7.98 (1H, s), 8.63 (2H, d, J=5.8 Hz).
ESI-MS m/z: 307 (M+H)⁺

### Example 6

### N-Methyl-N-[4-(oxazol-5-yl)phenyl]-N'-pyridin-4-ylmethylenehydrazine

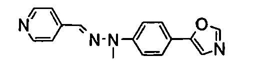

Methyl iodide (54.0 µl) was added at room temperature to a DMF solution (10 ml) of 4-pyridinecarboxyaldehyde 4-(oxazol-5-yl)phenylhydrazone (76.3 mg) and potassium carbonate (120 mg), followed by stirring at 80°C for 2 hours. The reaction solution was poured into water (100 ml), extracted with chloroform:methanol = 9:1 (100 ml) and dried over anhydrous sodium sulfate. After evaporation of the solvent, the residue was purified by preparative silica gel column chromatography (hexane:acetone = 1.5:1) to obtain the title compound (18.0 mg) as a yellow solid.
¹H-NMR (400 MHz, CDCl₃) δ: 3.50 (3H, s), 7.28 (1H, s), 7.42 (1H, s), 7.46 (2H, d, J=8.8 Hz), 7.56 (2H, d, J=6.1 Hz), 7.64 (2H, d, J=8.8 Hz), 7.90 (1H, s), 8.59 (2H, d, J=6.1 Hz).
ESI-MS m/z: 279 (M+H)⁺

### Example 7

### N-[4-(4-Iodooxazol-5-yl)phenyl]-N'-pyridin-4-ylmethylenehydrazinecarboxylic acid tert--butyl ester

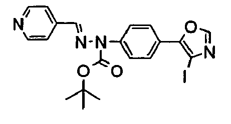

Under argon atmosphere, a 1 M lithium hexamethyldisilazide-THF solution (619 µl, 1.1 equiv) was added at -78°C to a THF solution (6 ml) of *N*-[4-(oxazol-5-yl)phenyl]-*N'*-pyridin-4-ylmethylenehydrazinecarboxylic acid *tert*-butyl ester (205 mg), followed by stirring at the same temperature for 1 hour. A THF solution (2 ml) of iodine (214 mg) was added to the mixture, followed by stirring at -78°C for 1 hour and at 0°C for 15 minutes. The reaction solution was diluted with ethyl acetate (90 ml), washed with a saturated aqueous sodium thiosulfate solution and saturated brine and dried over anhydrous sodium sulfate. The solvent was evaporated, and the residue was purified by flash silica gel column chromatography (hexane:acetone = 3:1) to obtain the title compound (114 mg) as a white solid.
¹H-NMR (400 MHz, CDCl₃) δ: 1.52 (9H, s), 7.24 (1H, s), 7.30 (2H, d, J=6.6 Hz), 7.49 (2H, d, J=4.6 Hz), 7.95 (1H, s), 8.18 (2H, d, J=6.6 Hz), 8.59 (2H, d, J=4.6 Hz).
ESI-MS m/z: 491 (M+H)⁺.

### Example 8

### 4-Pyridinecarboxyaldehyde 4-(4-iodooxazol-5-yl)phenylhydrazone

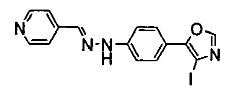

Trifluoroacetic acid (3 ml) was added at room temperature to a dichloromethane solution (10 ml) of *N*-[4-(4-iodooxazol-5-yl)phenyl]-*N'*-pyridin-4-ylmethylenehydrazinecarboxylic acid *tert*-butyl ester (113.5 mg), followed by stirring for 4 hours. After adding a saturated aqueous sodium bicarbonate solution (40 ml), the mixture was extracted with chloroform (40 ml) twice and dried over anhydrous sodium sulfate. The solvent was evaporated and the residue was recrystallized from diethyl ether to obtain the title compound (73.4 mg) as a yellow solid.
¹H-NMR (400 MHz, DMSO-d₆) δ: 7.25 (2H, d, J=8.8 Hz), 7.60 (2H, d, J=5.9 Hz), 7.80 (2H, d, J=8.8 Hz), 7.86 (1H, s), 8.42 (1H, s), 8.54 (2H, d, J=5.9 Hz), 11.06 (1H, s).
ESI-MS m/z: 391 (M+H)⁺

### Example 9

### 4-Pyridinecarboxyaldehyde 3-(oxazol-5-yl)phenylhydrazone

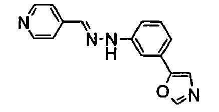

3-(Oxazol-5-yl)phenylamine (421 mg) was dissolved in water (8 ml) and concentrated hydrochloric acid (8 ml), and an aqueous solution (2 ml) of sodium nitrite (218 mg) was added dropwise to the solution at 0°C over 30 minutes. After stirring at the same temperature for 30 minutes, a concentrated hydrochloric acid (3 ml) solution of tin chloride dihydrate (1.19 g) was added dropwise to the mixture, followed by stirring at room temperature for 30 minutes. The reaction mixture was alkalified by adding an aqueous solution of 20% potassium hydroxide and extracted with methanol:chloroform = 1:9 (200 ml) twice. After drying over anhydrous sodium sulfate, the solvent was evaporated to obtain a yellow solid as the residue. The residue and 4-pyridinecarboxyaldehyde (227 µl) were dissolved in ethanol (8 ml) and heated under reflux for 15 hours. The solvent was evaporated, and the thus obtained solid was washed with ether to obtain the title compound (472 mg) as a yellow solid.
¹H-NMR (400 MHz, CDCl₃) δ: 7.11 (1H, d, J=7.9 Hz), 7.23 (1H, d, J=7.6 Hz), 7.36 (1H, t, J=7.8 Hz), 7.39 (1H, s), 7.46 (1H, s), 7.54 (2H, d, J=5.9 Hz), 7.65 (1H, s), 7.94 (1H, s), 8.04 (1H, s), 8.62 (2H, d, J=5.9 Hz).
ESI-MS m/z: 265 (M+H)⁺.

### Example 10

### 4-Pyridinecarboxyaldehyde 2-(oxazol-5-yl)phenylhydrazone

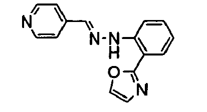

2-(Oxazol-5-yl)phenylamine (777 mg) was dissolved in water (10 ml) and concentrated hydrochloric acid (10 ml), and an aqueous solution (2 ml) of sodium nitrite (402 mg) was added to the solution at 0°C over 30 minutes. After stirring at the same temperature for 30 minutes, a concentrated hydrochloric acid solution (3 ml) of tin chloride dihydrate (2.19 g) was added to the mixture, followed by stirring at room temperature for 30 minutes. The reaction mixture was alkalified by adding an aqueous solution of 20% potassium hydroxide and extracted with chloroform:methanol = 9:1 (250 ml) twice. After drying over anhydrous sodium sulfate, the solvent was evaporated to obtain a reddish brown residue. The residue and 4-pyridinecarboxyaldehyde (346 µl) were dissolved in ethanol (8 ml) and heated under reflux for 15 hours. The solvent was evaporated, and then the residue was purified by flash silica gel column chromatography (chloroform:methanol = 30:1) to obtain the title compound (611 mg) as a yellow solid.
¹H-NMR (400 MHz, CDCl₃) δ: 7.00 (1H, t, J=7.8 Hz), 7.26 (1H, s), 7.40 (1H, t, J=8.0 Hz), 7.48 (1H, d, J=7.8 Hz), 7.53 (2H, d, J=6.1 Hz), 7.72 (1H, s), 7.79 (1H, d, J=8.3 Hz), 8.04 (1H, s), 8.62 (2H, d, J=6.1 Hz), 8.82 (1H, s).
ESI-MS m/z: 265 (M+H)⁺

### Example 11

### 4-Pyridinecarboxyaldehyde 4-(pyrazol-1-yl)phenylhydrazone

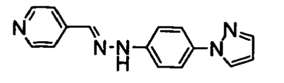

4-(Pyrazol-1-yl)phenylamine (646 mg) was dissolved in water (10 ml) and concentrated hydrochloric acid (10 ml), and an aqueous solution (4 ml) of sodium nitrite (336 mg) was added to the solution at 0°C over 30 minutes. After stirring at the same temperature for 30 minutes, a concentrated hydrochloric acid solution (3 ml) of tin chloride dihydrate (1.83 g) was added to the mixture, followed by stirring at room temperature for 30 minutes. The reaction mixture was alkalified by adding 20% aqueous solution of potassium hydroxide and extracted with chloroform: methanol = 9:1 (100 ml) twice. After drying over anhydrous sodium sulfate, the solvent was evaporated to obtain a reddish brown residue. The residue and 4-pyridinecarboxyaldehyde (324 µl) were dissolved in ethanol (8 ml) and heated under reflux for 15 hours. The solvent was evaporated, and then the residue was purified by flash silica gel column chromatography (chloroform: methanol = 10:1) to obtain the title compound (107 mg) as a reddish brown solid.
¹H-NMR (400 MHz, D₂O) δ: 6.35 (1H, s), 6.95 (2H, d, J=8.6 Hz), 7.14 (1H, s), 7.22 (2H, d, J=9.0 Hz), 7.47 (2H, d, J=5.8 Hz), 7.51 (1H, s), 7.76 (1H, s), 8.07 (2H, d, J=5.8 Hz).
ESI-MS m/z: 264 (M+H)⁺

### Example 12

### 4-Pyridinecarboxyaldehyde 4-([1,3,4]oxadiazol-2-yl)phenylhydrazone

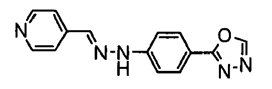

4-Pyridinecarboxyaldehyde (35 mg) was added to an ethanol solution (20 ml) of 4-([1,3,4]oxadiazol-2-yl)phenylhydrazine (58 mg), followed by heating under reflux for 1.5 hours. The solvent was evaporated, the thus obtained residue was purified by silica gel column chromatography, and the fraction obtained from the eluate of dichloromethane:methanol = 10:1 was concentrated under reduced pressure to obtain the title compound (64 mg) as a yellow solid.
¹H-NMR (400 MHz, DMSO-d₆) δ: 7.30 (2H, d, J=8.8 Hz), 7.64 (2H, d, J=5.9 Hz), 7.92 (3H, m), 8.57 (2H, d, J=5.9 Hz), 9.23 (1H, s), 11.23 (1H, s).
ESI-MS m/z: 266 (M+H)⁺.

### Example 13

### 4-Pyridinecarboxyaldehyde 4-(5-methyl[1,3,4]oxadiazol-2-yl)phenylhydrazone

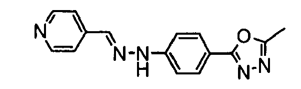

4-Pyridinecarboxyaldehyde (120 mg) was added to an ethanol solution (30 ml) of 4-(5-methyl[1,3,4]oxadiazol-2-yl)phenylhydrazine (212 mg), followed by heating under reflux for 1 hour. The solvent was evaporated, and the thus obtained residue was washed with ethanol and diethyl ether and then dried to obtain the title compound (277 mg) as a yellow solid.
¹H-NMR (400 MHz, DMSO-d₆) δ: 2.55 (3H, s), 7.28 (2H, d, J=8.8 Hz), 7.63 (2H, d, J=5.9 Hz), 7.85 (2H, d, J=8.8 Hz), 7.91 (1H, s), 8.57 (2H, d, J=5.9 Hz), 11.19 (1H, s).
FAB-MS m/z: 280 (M+H)⁺.

### Example 14

### 4-Pyridinecarboxyaldehyde 4-(5-methyl[1,2,4]oxadiazol-3-yl)phenylhydrazone

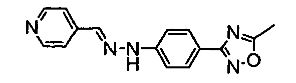

4-Pyridinecarboxyaldehyde (108 mg) was added to an ethanol solution (30 ml) of 4-(5-methyl[1,2,4]oxadiazol-3-yl)phenylhydrazine (192 mg), followed by heating under reflux for 1 hour. The solvent was evaporated, and the thus obtained residue was washed with diethyl ether and then dried to obtain the title compound (264 mg) as a yellow solid.
¹H-NMR (400 MHz, DMSO-d₆) δ: 2.64 (3H, s), 7.26 (2H, d, J=8.5 Hz), 7.63 (2H, d, J=5.9 Hz), 7.89 (2H, d, J=8.5 Hz), 7.90 (1H, s), 8.57 (2H, d, J=6.1 Hz), 11.14 (1H, s).
FAB-MS m/z: 280 (M+H)⁺.

### Example 15

### 4-Pyridinecarboxyaldehyde 4-([1,2,4]oxadiazol-3-yl)phenylhydrazone

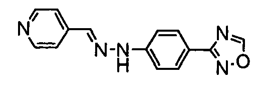

4-Pyridinecarboxyaldehyde (58.9 mg) was added to an ethanol solution (20 ml) of 4-([1,2,4]oxadiazol-2-yl)phenylhydrazine (96 mg), followed by heating under reflux for 1 hour. The solvent was evaporated, and the thus obtained residue was washed with diethyl ether and then dried to obtain the title compound (127 mg) as a yellow solid.
¹H-NMR (400 MHz, DMSO-d₆) δ: 7.28 (2H, d, J=8.8 Hz), 7.63 (2H, dd, J=4.4 Hz, 1.5 Hz), 7.91 (1H, s), 7.94 (2H, d, J=8.5 Hz), 8.57 (2H, dd, J=4.4 Hz, 1.5 Hz), 9.60 (1H, s), 11.15 (1H, s).
FAB-MS m/z: 266 (M+H)⁺.

### Example 16

### 4-Pyridinecarboxyaldehyde 4-(3-methyl-3H-imidazol-4-yl)phenylhydrazone

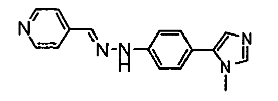

4-Pyridinecarboxyaldehyde (70.7 mg) was added to an ethanol solution (30 ml) of 4-(3-methyl-3H-imidazol-4-yl)phenylhydrazine (124 mg), followed by heating under reflux for 1 hour. The solvent was evaporated, the thus obtained residue was purified by silica gel column chromatography, and the fraction obtained from the eluate of dichloromethane:methanol = 10:1 to 5:1 was concentrated under reduced pressure to obtain the title compound (143 mg) as a yellow solid.
¹H-NMR (400 MHz, CDCl₃) δ: 3.66 (3H, s), 7.06 (1H, s), 7.21 (2H, d, J=8.5 Hz), 7.32 (2H, d, J=8.3 Hz), 7.52 (2H, d, J=5.9 Hz), 7.53 (1H, s), 7.66 (1H, s), 8.42 (1H, s), 8.60 (2H, d, J=6.1 Hz).
FAB-MS m/z: 278 (M+H)⁺.

### Example 17

### 4-Pyridinecarboxyaldehyde 4-(4-methyl-5-oxo-4,5-dihydro[1,2,4]oxadiazol-3-yl)phenylhydrazone

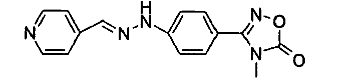

4-Pyridinecarboxyaldehyde (25.7 mg) was added to an ethanol solution (20 ml) of 4-(4-methyl-5-oxo-4,5-dihydro[1,2,4]oxadiazol-3-yl)phenylhydrazine (48 mg), followed by heating under reflux for 1 hour. The solvent was evaporated, and the thus obtained residue was washed with 20% water containing ethanol and then dried to obtain the title compound (40 mg) as a yellow solid.
¹H-NMR (400 MHz, DMSO-d₆) δ: 3.22 (3H, s), 7.29 (2H, d, J=8.8 Hz), 7.62 (2H, d, J=8.8 Hz), 7.64 (2H, d, J=6.3 Hz), 7.91 (1H, s), 8.56 (2H, d, J=5.6 Hz), 11.22 (1H, s).
FAB-MS m/z: 296 (M+H)⁺.

### Example 18

### N-[4-(4-Hydroxymethyloxazol-5-yl)phenyl]-N'-pyridin-4-ylmethylenehydrazinecarboxylic acid tert-butyl ester

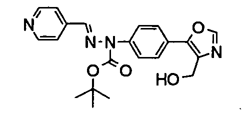

Paraformaldehyde (10 g) was heated at 160°C, the generated formaldehyde gas was bubbled into a THF solution (20 ml) of -78°C, and this solution was kept at the same temperature. On the other hand, at -78°C under argon atmosphere, a 1 M lithium hexamethyldisilazide-THF solution (3.2 ml) was added to a THF solution (16 ml) of *N*-[4-(oxazol-5-yl)phenyl]-N'-pyridin-4-ylmethylenehydrazinecarboxylic acid *tert*-butyl ester (968 mg), followed by stirring at the same temperature for 1 hour, and then a THF solution of formaldehyde was added dropwise to the mixture until the material on TLC almost disappeared. The reaction mixture was allowed to warm to 0°C and stirred for 30 minutes. Water (100 ml) was added to the mixture, and the mixture was extracted with chloroform (100 ml) twice and dried over anhydrous sodium sulfate. The solvent was evaporated, and then the residue was purified by flash silica gel column chromatography (hexane:acetone = 2:1 to 1:1) to obtain the title compound (175 mg) as yellow oil.
¹H-NMR (400 MHz, CDCl₃) δ: 1.52 (9H, s), 2.64 (1H, br s), 4.85 (2H, s), 7.23 (1H, s), 7.31 (2H, d, J=8.6 Hz), 7.49 (2H, d, J=6.1 Hz), 7.87 (2H, d, J=8.6 Hz), 7.95 (1H, s), 8.59 (2H, d, J=6.1 Hz).
ESI-MS m/z: 395 (M+H)⁺.

### Example 19

### 4-Pyridinecarboxyaldehyde 4-(4-hydroxymethyloxazol-5-yl)phenylhydrazone

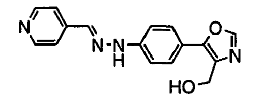

Trifluoroacetic acid (2 ml) was added to a dichloromethane solution (6 ml) of *N*-[4-(4-hydroxymethyloxazol-5-yl)phenyl]-*N*'-pyridin-4-ylmethylenehydrazinecarboxylic acid *tert*-butyl ester (47.2 mg) at room temperature, followed by stirring for 2 hours. After solvent evaporation and drying, the residue was dissolved in methanol (5 ml), triethylamine (1 ml) was added to the mixture and stirred at room temperature for 1 hour. The solvent was evaporated, and to the residue was added water (40 ml), extracted with chloroform: methanol = 9:1 (40 ml) twice and dried over anhydrous sodium sulfate. The solvent was evaporated and the thus obtained solid was washed with ether to obtain the title compound (12.0 mg) as a white solid.
¹H-NMR (400 MHz, DMSO-d₆) δ: 4.50 (2H, d, J=5.4 Hz), 5.25 (1H, t, J=5.4 Hz), 7.25 (2H, d, J=8.5 Hz), 7.62 (4H, m), 7.86 (1H, s), 8.29 (1H, s), 8.55 (2H, d, J=5.9 Hz), 11.03 (1H, s).
FAB-MS m/z: 295 (M+H)⁺

### Example 20

### 4-Pyridinecarboxyaldehyde 4-(pyridin-3-yl)phenylhydrazone

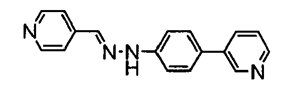

4-(Pyridin-3-yl)phenylhydrazine (58.6 mg) was dissolved in ethanol (8 ml), 4-pyridinecarboxyaldehyde (29.7 µl) was added to the solution and stirred at room temperature for 2 hours. The solvent was evaporated, the residue was purified by flash silica gel column chromatography (chloroform:methanol = 9: 1), and the thus obtained solid was washed with diethyl ether to obtain the title compound (74.2 mg) as a pale yellow solid.
¹H-NMR (400 MHz, DMSO-d₆) δ: 7.24 (2H, d, J=8.6 Hz), 7.42 (1H, dd, J=4.7 Hz, 7.8 Hz), 7.59 (2H, d, J=4.7 Hz), 7.65 (2H, d, J=8.6 Hz), 7.84 (1H, s), 8.00 (1H, ddd, J=1.5 Hz, 3.9, Hz, 7.8 Hz), 8.47 (1H, dd, J=1.5 Hz, 4.7 Hz), 8.53 (2H, d, J=4.6 Hz), 8.85 (1H, d, J=2.2 Hz), 10. 94 (1H, s).
ESI-MS m/z: 275 (M+H)⁺.

### Example 21

### 4-Pyridinecarboxyaldehyde 4-(6-methylbenzothiazol-2-yl)phenylhydrazone

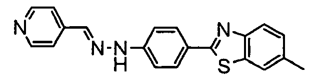

4-(6-Methylbenzothiazol-2-yl)phenylhydrazine (90.2 mg) was dissolved in ethanol (8 ml), 4-pyridinecarboxyaldehyde (34.9 µl) was added to the solution and stirred at 50°C for 2 hours. The solvent was evaporated and the thus obtained solid was washed with diethyl ether to obtain the title compound (71.2 mg) as a yellow solid.
¹H-NMR (400 MHz, DMSO-d₆) δ: 2.43 (3H, s), 7.26 (7H, d, J=8.8 Hz), 7.30 (1H, d, J=8.3 Hz), 7.62 (2H, d, J=8.1 Hz), 7.84 (2H, d, J=8.6 Hz), 7.90 (1H, s), 7.96 (2H, d, J=8.7 Hz), 8.56 (2H, d, J=5 . 9 Hz), 11.18 (1H, s).
ESI-MS m/z: 345 (M+H)⁺

### Example 22

### 4-(4-Methylpiperazin-1-yl)benzaldehyde 4-(6-methylbenzothiazol-2-yl)phenylhydrazone

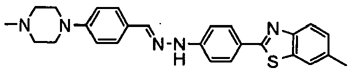

4-(4-Methylpiperazin-1-yl)benzaldehyde (87.4 mg) and 4-(6-methylbenzothiazol-2-yl)phenylhydrazine (92.7 mg) were dissolved in ethanol (8 ml) and heated at 60°C under reflux for 2 hours. After evaporation of the solvent, the residue was diluted with chloroform:methanol = 9:1 (100 ml), washed with a saturated aqueous sodium bicarbonate solution (50 ml) and dried over anhydrous sodium sulfate. The solvent was evaporated and the thus obtained solid was washed with diethyl ether and ethanol to obtain the title compound (92.6 mg) as a yellowish brown solid.
¹H-NMR (400 MHz, DMSO-d₆) δ: 2.22 (3H, s), 2.43 (4H, s), 3.20 (4H, s), 6.95 (2H, d, J=8.6 Hz), 7.13 (2H, d, J=8.6 Hz), 7.28 (1H, d, J=8.5 Hz), 7.52 (2H, d, J=8.5 Hz), 7.80 (1H, d, J=8.3 Hz), 7.83 (1H, s), 7.85 (1H, s), 7.89 (2H, d, J=8.8 Hz), 10.57 (1H, s).
ESI-MS m/z: 445 (M+H)⁺.

### Example 23

### 4-Pyridinecarboxyaldehyde 4-(4,5-dihydrooxazol-2-yl)phenylhydrazone

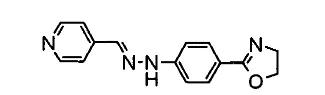

4-(4,5-Dihydrooxazol-2-yl)phenylamine (444 mg) was dissolved in water (6 ml) and concentrated hydrochloric acid (2 ml), and an aqueous solution (1 ml) of sodium nitrite (208 mg) was slowly added dropwise to the solution at 0°C. After stirring at the same temperature for 1 hour, a concentrated hydrochloric acid solution (1 ml) of tin chloride dihydrate (1.24 g) was added to the mixture, followed by stirring at room temperature for 1 hour. The reaction solution was alkalified by adding 20% aqueous solution of potassium hydroxide and extracted with chloroform: methanol = 9:1 (200 ml) twice. The mixture was dried over anhydrous sodium sulfate, and the solvent was evaporated to obtain a reddish brown residue. This residue and 4-pyridinecarboxyaldehyde (33.1 µl) were dissolved in ethanol (8 ml) and stirred at room temperature for 2 hours. After evaporation of the solvent, the residue was purified by flash silica gel column chromatography (chloroform:methanol = 30:1), and the thus obtained solid was washed with diethyl ether to obtain the title compound (21.6 mg) as a yellow solid.
¹H-NMR (400 MHz, DMSO-d₆) δ: 3.90 (2H, t, J=9.3 Hz), 4.33 (2H, t, J=9.5 Hz), 7.16 (2H, d, J=8.8 Hz), 7.59 (2H, d, J=6.1 Hz), 7.74 (2H, d, J=8.6 Hz), 7.86 (1H, s), 8.54 (2H, d, J=5.8 Hz), 11.09 (1H, s).
ESI-MS m/z: 267 (M+H)⁺.

### Example 24

### 4-Pyridinecarboxyaldehyde (E)-4-[2-(oxazol-5-yl)vinyl]phenylhydrazone

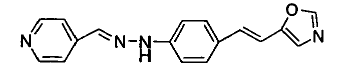

4-Pyridinecarboxyaldehyde (38.6 g) was added to an ethanol solution (25 ml) of (*E*)-4-[2-(oxazol-5-yl)vinyl]phenylhydrazine (70 mg), followed by heating under reflux for 1 hour. The solvent was evaporated, the thus obtained residue was purified by silica gel column chromatography, and the fraction obtained from the eluate of chloroform: methanol = 10:1 was concentrated under reduced pressure and dried to obtain the title compound (80 mg) as a crystalline solid.
¹H-NMR (400 MHz, DMSO-d₆) δ: 7.00 (2H, s), 7.13 (2H, d, J=8.5 Hz), 7.17 (1H, s), 7.50 (2H, d, J=8.5 Hz), 7.58 (2H, d, J=8.5 Hz), 7.82 (1H, s), 8.31 (1H, s), 8.53 (2H, d, J=5.6 Hz), 10.95 (1H, s).
FAB-MS m/z: 291 (M+H)⁺.

### Example 25

### 4-(Dimethylaminomethyl)benzaldehyde (E)-4-[2-(oxazol-5-yl)vinyl]phenylhydrazone

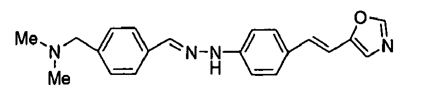

4-(N,N-Dimethylaminomethyl)benzaldehyde hydrochloride (98 mg) was added to an ethanol solution (30 ml) of (E)-4-[2-(oxazol-5-yl)vinyl]phenylhydrazine (96 mg), followed by heating under reflux for 1 hour. To the residue obtained by evaporating the solvent was added a saturated aqueous sodium bicarbonate solution and extracted with chloroform:methanol = 10:1. After drying the organic layer over sodium sulfate, the solvent was evaporated, the thus obtained residue was purified by silica gel column chromatography, and the fraction obtained from the lower layer eluate of chloroform:methanol:water = 15:3:1 was concentrated under reduced pressure and dried to obtain the title compound (25 mg) as a crystalline solid.
¹H-NMR (400 MHz, CDCl₃) δ: 2.27 (6H, s), 3.46 (2H, s), 6.76 (1H, d, J=16.4 Hz), 7.01 (1H, s), 7.04 (1H, d, J=16.1 Hz), 7.10 (2H, d, J=8.5 Hz), 7.32 (2H, d, J=8.1 Hz), 7.40 (2H, d, J=8.8 Hz), 7.62 (2H, d, J=8.1 Hz), 7.70 (1H, s), 7.74 (1H, s), 7.81 (1H, s).
FAB-MS m/z: 347 (M+H)⁺.

### Example 26

### 4-Pyridinecarboxyaldehyde 4-(imidazo[1,2-a]pyridin-2-yl)phenylhydrazone

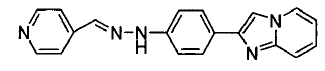

4-Pyridinecarboxyaldehyde (77 mg) was added to an ethanol solution (30 ml) of 4-(imidazo[1,2-a]pyridin-2-yl)phenylhydrazine (160 mg), followed by heating under reflux for 1 hour. The residue obtained by evaporating the solvent was purified by silica gel column chromatography, and the fraction obtained from the eluate of chloroform:methanol = 20:1 1 to 10:1 was concentrated under reduced pressure and dried to obtain the title compound (124 mg) as a crystalline solid.
¹H-NMR (400 MHz, DMSO-d₆) δ: 6.87 (1H, t, J=6.7 Hz), 7.21 (3H, m), 7.54 (1H, d, J=9.0 Hz), 7.61 (2H, d, J=4.9 Hz), 7.85 (2H, d, J=8.3 Hz), 7.89 (1H, s), 8.27 (1H, s), 8.50 (1H, dd, J=6.7 Hz, 0.9 Hz), 8.55 (2H, d, J=4.9 Hz), 10.93 (1H, s).
FAB-MS m/z: 314 (M+H)⁺.

### Example 27

### 4-Pyridinecarboxyaldehyde 4-(5,6,7,8-tetrahydroimidazo[1,2-a]pyridin-2-yl)phenylhydrazone

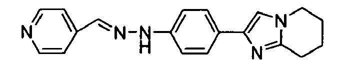

4-Pyridinecarboxyaldehyde (55 mg) was added to an ethanol solution (20 ml) of 4-(5,6,7,8-tetrahydroimidazo[1,2-a]pyridin-2-yl)phenylhydrazine (115 mg), followed by heating under reflux for 1 hour. The residue obtained by evaporating the solvent was purified by silica gel column chromatography, and the fraction obtained from the eluate of chloroform:methanol = 10: 1 was concentrated under reduced pressure and dried to obtain the title compound (86 mg) as a crystalline solid.
¹H-NMR (400 MHz, CDCl₃) δ: 1.94-2.00 (4H, m), 2.93 (2H, t, J=6.0 Hz), 3.97 (2H, t, J=5.7 Hz), 6.99 (1H, s), 7.12 (2H, d, J=8.5 Hz), 7.48 (2H, d, J=5.9 Hz), 7.57 (1H, s), 7.68 (2H, d, J=8.5 Hz), 8.25 (1H, s), 8.56 (2H, d, J=5.9 Hz).
EI-MS m/z: 317 (M)⁺.

### Example 28

### 4-Benzoylpyridine 4-(oxazol-5-yl)phenylhydrazone

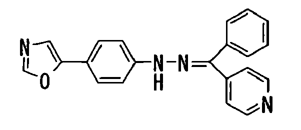

4-(Oxazol-5-yl)phenylhydrazine (0.10 g) and 4-benzoylpyridine (0.10 g) were added to ethanol (10 ml), followed by heating overnight under reflux. The mixture was concentrated under reduced pressure, and the residue was recrystallized from ethanol to obtain the title compound (0.019 g).
¹H-NMR (400 MHz, CDCl₃) δ: 7.14 (2H, d, J=8.82 Hz), 7.22 (1H, s), 7.30-7.40 (5H, m), 7.48 (1H, br s), 7.53-7.60 (4H, m), 7.85 (1H, s), 8.89 (2H, dd, J=5.88 and 1.22 Hz).
FAB-MS m/z: 341 (M+H)⁺

### Example 29

### 4-Dimethylaminobenzaldehyde 4-(oxazol-5-yl)phenylhydrazone

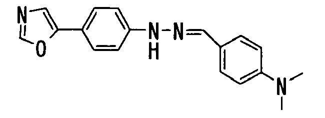

4-(Oxazol-5-yl)phenylhydrazine (0.46 g) and 4-dimethylaminobenzaldehyde (0.39 g) were added to ethanol (20 ml), followed by heating overnight under reflux. The mixture was concentrated under reduced pressure, and the residue was recrystallized from ethanol to obtain the title compound (0.19 g).
¹H-NMR (400 MHz, CDCl₃) δ: 3.01 (6H, s), 6.72 (2H, d, J=9.03 Hz), 7.12 (2H, d, J=8.79 Hz), 7.19 (1H, s), 7.50-7.60 (5H, m), 7.66 (1H, s), 7.85 (1H, s).
FAB-MS m/z: 307 (M+H)⁺

### Example 30

### Quinoline-4-carboxyaldehyde 4-(oxazol-5-yl)phenylhydrazone

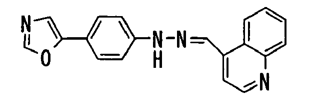

Quinoline-4-carboxyaldehyde (0.089 g) and 4-(oxazol-5-yl)phenylhydrazine (0.10 g) were added to ethanol (30 ml), followed by heating overnight under reflux. The mixture was concentrated under reduced pressure, and the residue was recrystallized from ethanol to obtain the title compound (0.04 g).
¹H-NMR (400 MHz, CDCl₃) δ: 7.25 (1H, s), 7.60-7.65 (4H, m), 7.75-7.80 (3H, m), 7.89 (1H, s), 8.16 (1H, d, J=8.06 Hz), 8.24 (1H, s), 8.32 (1H, s), 8.61 (1H, d, J=8.30 Hz), 8.94 (1H, d, J=4.39 Hz).
FAB-MS m/z: 315 (M+H)⁺

### Example 31

### 4-Acetylpyridine 4-(oxazol-5-yl)phenylhydrazone

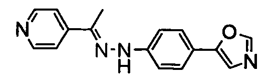

4-(Oxazol-5-yl)phenylhydrazine (50.0 mg) was dissolved in ethanol (8 ml) and 4-acetylpyridine (31.6 µl) was added to the solution, followed by heating under reflux for 3 hours. The solvent was evaporated, the residue was purified by flash silica gel column chromatography (hexane:acetone = 1:1), and the thus obtained solid was washed with ether to obtain the title compound (37.2 mg) as a reddish brown solid.
¹H-NMR (400 MHz, CDCl₃) δ: 2.23 (3H, s), 7.26 (3H, m), 7.61 (2H, d, J=8.6 Hz), 7.67 (2H, d, J=6.4 Hz), 7.68 (1H, s), 7.88 (1H, s), 8.61 (2H, d, J=6.3 Hz).
ESI-MS m/z: 278M⁺.

### Example 32

### Benzaldehyde 4-(oxazol-5-yl)phenylhydrazone

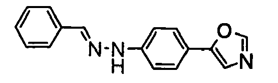

4-(Oxazol-5-yl)phenylhydrazine (59.6 mg) was dissolved in ethanol (8 ml), and benzaldehyde (34.7 µl) was added to the solution, followed by heating under reflux for 5 hours. The solvent was evaporated and the thus obtained solid was washed with diethyl ether and hexane to obtain the title compound (39.0 mg) as a reddish brown solid.
¹H-NMR (400 MHz, CDCl₃) δ: 7.17 (2H, d, J=8.8 Hz), 7.22 (1H, s), 7.33 (1H, s), 7.41 (2H, t, J=7.1 Hz), 7.58 (2H, d, J=8.8 Hz), 7.68 (2H, d, J=7.1 Hz), 7.73 (1H, s), 7.75 (1H, br s), 7.86 (1H, s).
FAB-MS m/z: 263M⁺.

### Example 33

### 4-Hydroxy-3-iodo-5-methoxybenzaldehyde 4-(oxazol-5-yl)phenylhydrazone

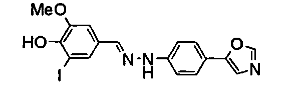

4-(Oxazol-5-yl)phenylhydrazine (82.0 mg) was dissolved in ethanol (8 ml), and 5-iodovanillin (130 mg) was added to the solution, followed by heating under reflux for 1 hour. The solvent was evaporated and the thus obtained solid was washed with diisopropyl ether to obtain the title compound (93.8 mg) as a reddish brown solid.
¹H-NMR (400 MHz, DMSO-d₆) δ: 3.89 (3H, s), 7.13 (2H, d, J=8.8 Hz), 7.31 (1H, s), 7.42 (1H, s), 7.52 (1H, s), 7.57 (2H, d, J=8.8 Hz), 7.76 (1H, s), 8.32 (1H, s), 9.76 (1H, s), 10.49 (1H, s).
ESI-MS m/z: 436 (M+H)⁺

### Example 34

### 5-Iodo-4-hydroxy-3-methoxybenzaldehyde 4-(imidazol-1-yl)phenylhydrazone

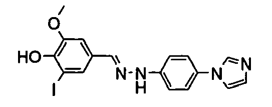

4-(Imidazol-1-yl)phenylhydrazine (107.3 mg) and 5-iodovanillin (171.3 mg) were dissolved in ethanol (8 ml) and heated at 60°C under reflux for 3 hours. After evaporation of the solvent, the residue was purified by flash silica gel column chromatography (chloroform:methanol = 30:1), and the thus obtained solid was washed with diethyl ether and ethanol to obtain the title compound (132 mg) as a pale yellow solid.
¹H-NMR (400 MHz, DMSO-d₆) δ: 3.87 (3H, s), 7.05 (1H, s), 7.13 (2H, d, J=8.8 Hz), 7.29 (1H, s), 7.42 (2H, d, J=8.8 Hz), 7.50 (1H, s), 7.56 (1H, s), 7.74 (1H, s), 8.05 (1H, s), 9.74 (1H, br s), 10.38 (1H, s).
ESI-MS m/z: 435 (M+H)⁺.

### Example 35

### 4-Hydroxy-3-methoxybenzaldehyde 4-(oxazol-5-yl)phenylhydrazone

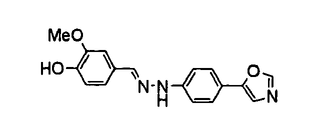

4-(Oxazol-5-yl)phenylhydrazine (82.9 mg) was dissolved in ethanol (8 ml), and vanillin (72.0 mg) was added to the solution, followed by heating under reflux for 1 hour. After evaporation of the solvent, the thus obtained solid was washed with diisopropyl ether to obtain the title compound (101 mg) as a reddish brown solid.
¹H-NMR (400 MHz, DMSO-d₆) δ: 3.84 (3H, s), 6.79 (1H, d, J=8.3 Hz), 7.03 (1H, d, J=8.3 Hz), 7.11 (2H, d, J=8. 8 Hz), 7.27 (1H, s), 7.42 (1H, s), 7.56 (2H, d, J=8.8 Hz), 7.81 (1H, s), 8.31 (1H, s), 9.26 (1H, s), 10.36 (1H, s).
ESI-MS m/z: 310 (M+H)⁺

### Example 36

### 3,4-Dimethoxybenzaldehyde 4-(oxazol-5-yl)phenylhydrazone

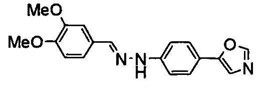

4-(Oxazol-5-yl)phenylhydrazine (74.9 mg) was dissolved in ethanol (8 ml), and 3,4-dimethoxybenzaldehyde (71.0 mg) was added to the solution, followed by heating under reflux for 1 hour. After evaporation of the solvent, the thus obtained solid was washed with diisopropyl ether to obtain the title compound (104 mg) as a reddish brown solid.
¹H-NMR (400 MHz, DMSO-d₆) δ: 3.92 (3H, s), 3.98 (3H, s), 6.87 (1H, d, J=8.3 Hz), 7.07 (1H, dd, J=1.7 and 8.3 Hz), 7.15 (2H, d, J=8.6 Hz), 7.21 (1H, s), 7.38 (1H, d, J=1.7 Hz), 7.57 (2H, d, J=8.6 Hz), 7.64 (1H, s), 7.68 (1H, s), 7.86 (1H, s).
ESI-MS m/z: 324 (M+H)⁺.

### Example 37

### 4-Hydroxybenzaldehyde 4-(oxazol-5-yl)phenylhydrazone

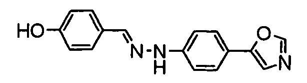

4-(Oxazol-5-yl)phenylhydrazine (68.8 mg) was dissolved in ethanol (8 ml), and 4-hydroxybenzaldehyde (48.0 mg) was added to the solution, followed by heating under reflux for 2 hours. After evaporation of the solvent, the thus obtained solid was washed with diisopropyl ether to obtain the title compound (104 mg) as a red solid.
¹H-NMR (400 MHz, DMSO-d₆) δ: 6.78 (2H, d, J=8.8 Hz), 7.08 (2H, d, J=8.8 Hz), 7.40 (1H, s), 7.48 (2H, d, J=8.6 Hz), 7.54 (2H, d, J=8.6 Hz), 7. 81 (1H, s), 8.30 (1H, s), 9.66 (1H, s), 10.30 (1H, s).
ESI-MS m/z: 280 (M+H)⁺.

### Example 38

### 3-Hydroxy-4-methoxybenzaldehyde 4-(oxazol-5-yl)phenylhydrazone

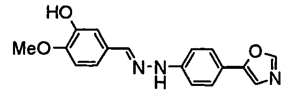

4-(Oxazol-5-yl)phenylhydrazine (49.5 mg) was dissolved in ethanol (8 ml), and 3-hydroxy-4-methoxybenzaldehyde (43.0 mg) was added to the solution, followed by heating under reflux for 12 hours. After evaporation of the solvent, the thus obtained solid was washed with diisopropyl ether to obtain the title compound (46.1 mg) as a red solid.
¹H-NMR (400 MHz, DMSO-d₆) δ: 3.86 (3H, s), 6.92 (1H, d, J=8.5 Hz), 6.96 (1H, d, J=8.5 Hz), 7.08 (2H, d, J=8.5 Hz), 7.19 (1H, s), 7.41 (1H, s), 7.55 (2H, d, J=8.5 Hz), 7.76 (1H, s), 8.30 (1H, s), 9.10 (1H, s), 10.36 (1H, s).
FAB-MS m/z: 310 (M+H)⁺

### Example 39

### 2-Pyridinecarboxyaldehyde 4-(oxazol-5-yl)phenylhydrazone

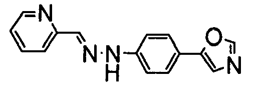

2-Pyridinecarboxyaldehyde (183 mg) was added to an ethanol solution (15 ml) of 4-(oxazol-5-yl)phenylhydrazine (300 mg), followed by heating overnight under reflux. After evaporation of the solvent, the thus obtained residue was collected by filtration and washed with ethanol to obtain the title compound (145 mg) as a red solid.
¹H-NMR (400 MHz, DMSO-d₆) δ: 7.19 (2H, d, J=8.5 Hz), 7.27 (1H, dd, J=4.9 and 7.3 Hz), 7.47 (1H, s), 7.61 (2H, d, J=8.5 Hz), 7.79 (1H, t, J=7.3 Hz), 7.92 (1H, s), 7.95 (1H, d, J=7.3 Hz), 8.34 (1H, s), 8.52 (1H, d, J=4.9 Hz), 10.91 (1H, s).
ESI-MS m/z: 265 (M+H)⁺

### Example 40

### 3-Pyridinecarboxyaldehyde 4-(oxazol-5-yl)phenylhydrazone

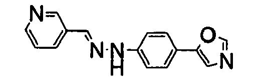

3-Pyridinecarboxyaldehyde (183 mg) was added to an ethanol solution (15 ml) of 4-(oxazol-5-yl)phenylhydrazine (300 mg), followed by heating overnight under reflux. After evaporation of the solvent, the thus obtained residue was collected by filtration and washed with ethanol to obtain the title compound (166 mg) as a red solid.
¹H-NMR (400 MHz, DMSO-d₆) δ: 7.18 (2H, d, J=8.5 Hz), 7.41 (1H, dd, J=4.6 and 7.8 Hz), 7.46 (1H, s), 7.59 (2H, d, J=8.5 Hz), 7.92 (1H, s), 8.08 (1H, d, J=7.8 Hz), 8.33 (1H, s), 8.48 (1H, d, J=4.6 Hz), 8.82 (1H, s), 10.80 (1H, s).
ESI-MS m/z: 265 (M⁺+H).

### Example 41

### 2-Pyrrolecarboxyaldehyde 4-(oxazol-5-yl)phenylhydrazone

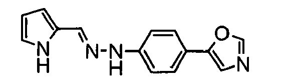

2-Pyrrolecarboxyaldehyde (109 mg) was added to an ethanol solution (15 ml) of 4-(oxazol-5-yl)phenylhydrazine (200 mg), followed by heating overnight under reflux. After evaporation of the solvent, the thus obtained residue was collected by filtration and washed with ethanol to obtain the title compound (154 mg) as a red solid.
¹H-NMR (400 MHz, CDCl₃) δ: 6.25 (1H, q, J=2.7 Hz), 6.34 (1H, br s), 6.89 (1H, br s), 7.07 (2H, d, J=8.6 Hz), 7.20 (1H, s), 7.53 (1H, s), 7.55 (2H, d, J=8.6 Hz), 7.64 (1H, s), 7.86 (1H, s), 9.00 (1H, br s).
ESI-MS m/z: 253 (M+H)⁺.

### Example 42

### 4-[N-(2-Hydroxyethyl)-N-methylamino]benzaldehyde 4-(oxazol-5-yl)phenylhydrazone

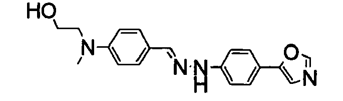

4-[*N*-(2-hydroxyethyl)-*N*-methylamino]benzaldehyde (1.15 g) was added to an ethanol solution (20 ml) of 4-(oxazol-5-yl)phenylhydrazine (1.12 g), followed by heating overnight under reflux. After evaporation of the solvent, the thus obtained residue was washed with ethanol to obtain the title compound (1.43 g) as a red solid.
¹H-NMR (400 MHz, CDCl₃) δ: 3.04 (3H, s), 3.55 (2H, t, J=5.5 Hz), 3.85 (2H, dd, J=4.9 and 5.5 Hz), 6.79 (2H, d, J=8.6 Hz), 7.13 (2H, d, J=8.6 Hz), 7.20 (1H, s), 7.55 (4H, d, J=8.6 Hz), 7.66 (1H, s), 7.85 (1H, s).
ESI-MS m/z: 337 (M+H)⁺.

### Example 43

### Thiazole-2-carboxyaldehyde 4-(oxazol-5-yl)phenylhydrazone

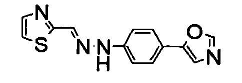

Thiazole-2-carboxyaldehyde (100 µl) was added to an ethanol solution (15 ml) of 4-(oxazol-5-yl)phenylhydrazine (200 mg), followed by heating overnight under reflux. After evaporation of the solvent, the thus obtained residue was purified by silica gel column chromatography, and the fraction obtained from the eluate of dichloromethane:methanol 100:3 was concentrated under reduced pressure to obtain the title compound (154 mg) as a red solid.
¹H-NMR (400 MHz, CDCl₃) δ: 7.18 (2H, d, J=8.8 Hz), 7.23 (1H, s), 7.28 (1H, d, J=3.4 Hz), 7.58 (2H, d, J=8.8 Hz), 7.78 (1H, d, J=3.4 Hz), 7.89 (1H, s), 8.00 (1H, s), 9.04 (1H, s).
ESI-MS m/z: 271 (M+H)⁺.

### Example 44

### 4-[4-(Oxazol-5-yl)phenylhydrazonomethyl]benzoic acid

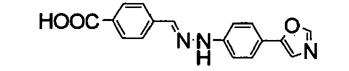

4-Formylbenzoic acid (257 mg) was added to an ethanol solution (10 ml) of 4-(oxazol-5-yl)phenylhydrazine (300 mg), followed by heating overnight under reflux. After evaporation of the solvent, the thus obtained residue was collected by filtration and then washed with ethanol to obtain the title compound (495 mg) as a red solid.
¹H-NMR (400 MHz, DMSO-d₆) δ: 7.19 (2H, d, J=8.6 Hz), 7.47 (1H, s), 7.61 (2H, d, J=8.6 Hz), 7.77 (2H, d, J=8.6 Hz), 7.75 (1H, s), 7.75 (2H, d, J=8.6 Hz), 8.34 (1H, s), 10.85 (1H, s).
ESI-MS m/z: 308 (N4+H)⁺.

### Example 45

### N,N-Dimethyl-4-[4-(oxazol-5-yl)phenylhydrazonomethyl]benzamide

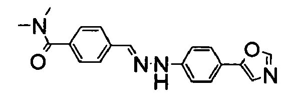

Dimethylamine (0.65 ml, 1.0 M THF solution), HOBt (106 mg) and EDC·HCl (150 mg) were added to a dichloromethane solution (10 ml) of 4-[4-(oxazol-5-yl)phenylhydrazonomethyl]benzoic acid (200 mg) at 0°C, followed by stirring overnight at room temperature. To the reaction solution was added water, extracted with dichloromethane and then dried over sodium sulfate. The solvent was evaporated, the thus obtained residue was purified by silica gel column chromatography, and the fraction obtained from the eluate of dichloromethane:methanol = 10:2 was concentrated under reduced pressure to obtain the title compound (140 mg) as a red solid.
¹H-NMR (400 MHz, CDCl₃) δ: 3.01 (3H, s), 3.12 (3H, s), 7.17 (2H, d, J=8.8 Hz), 7.22 (1H, s), 7.44 (2H, d, J=8.8 Hz), 7.58 (2H, d, J=8.8 Hz), 7.67 (1H, s), 7.67 (2H, d, J=8.8 Hz), 7.85 (1H, s), 7.97 (1H, s).
ESI-MS m/z: 335 (M⁺+H).

### Example 46

### tert-Butyl N-methyl-N-{2-[4-(oxazol-5-yl)phenylhydrazonomethyl]thiazol-4-ylmethyl}carbamate

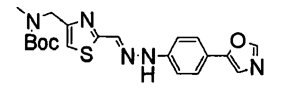

*tert-*Butyl *N*-(2-formylthiazol-4-ylmethyl)-*N*-methylcarbamate (150 mg) was added to an ethanol solution (5 ml) of 4-(oxazol-5-yl)phenylhydrazine (100 mg), followed by heating overnight under reflux. The solvent was evaporated, the thus obtained residue was purified by silica gel column chromatography, and the fraction obtained from the eluate of dichloromethane:methanol = 100:3 was concentrated under reduced pressure to obtain the title compound (92 mg) as a yellow solid.
¹H-NMR (400 MHz, CDCl₃) δ: 1.47 (9H, s), 2,96 (3H, s), 4.52 (2H, s), 7.00 (1H, bs), 7.17 (2H, d, J=8.6 Hz), 7.26 (1H, s), 7.58 (2H, d, J=8.6 Hz), 7.87 (1H, s), 7.91 (1H, s), 8.35 (1H, s).
ESI-MS m/z: 414 (M+H)⁺.

### Example 47

### 4-(N-Methylaminomethyl)thiazol-2-ylcarboxyaldehyde 4-(oxazol-5-yl)phenylhydrazone

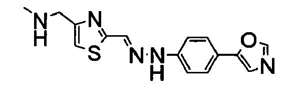

A hydrochloric acid-methanol solution (2 ml) was added to a methanol solution (0.5 ml) of *tert*-butyl *N*-methyl-*N*{2-[4-(oxazol-5-yl)phenylhydrazonomethyl]thiazol-4-ylmethyl}carbamate (92 mg) at 0°C, followed by stirring overnight at room temperature. The solvent was evaporated and to the thus obtained residue was added a saturated aqueous sodium bicarbonate solution and extracted with chloroform:methanol (10:1, v/v) solution. After drying over sodium sulfate, the solvent was evaporated and the thus obtained residue was washed with diethyl ether and then dried to obtain the title compound (35 mg) as a yellow solid.
¹H-NMR (400 MHz, CDCl₃) δ: 2.50 (3H, s), 3.87 (2H, s), 7.06 (1H, s), 7.17 (2H, d, J=8.6 Hz), 7.24 (1H, s), 7.59 (2H, d, J=8.6 Hz), 7.87 (1H, s), 7.92 (1H, s), 8.11 (1H, s).
ESI-MS m/z: 314 (M+H)⁺.

### Example 48

### 2-Dimethylaminomethylthiazole-4-carboxyaldehyde 4-(oxazol-5-yl)phenylhydrazone

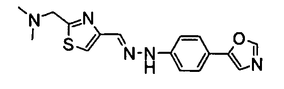

2-Dimethylaminomethylthiazole-4-carboxyaldehyde (160 mg) was added to an ethanol solution (5 ml) of 4-(oxazol-5-yl)phenylhydrazine (170 mg), followed by heating overnight under reflux. The solvent was evaporated, the thus obtained residue was purified by silica gel column chromatography, and the fraction obtained from the eluate of dichloromethane:methanol = 100:5 was concentrated under reduced pressure. Diethyl ether was added to the thus obtained residue, and the formed powder was collected by filtration, washed with diethyl ether and dried to obtain the title compound (80 mg) as a yellow solid.
¹H-NMR (400 MHz, CDCl₃) δ: 2.38 (6H, s), 3.81 (2H, s), 7.15 (2H, d, J=8.8 Hz), 7.21 (1H, s), 7.55 (2H, d, J=8.8 Hz), 7.56 (1H, s), 7.85 (1H, s), 7.87 (1H, s), 8.03 (1H, s).
ESI-MS m/z: 328 (M⁺+H).

### Example 49

### tert-Butyl 2-[4-(oxazol-5-yl)phenylhydrazonomethyl]-4, 5,6,7-tetrahydrothiazolo[5,4-c]pyridine-5-carboxylate

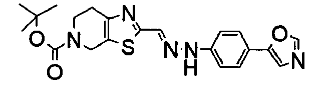

*tert*-Butyl 2-formyl-4,5,6,7-tetrahydrothiazolo[5,4-c]pyridine-5-carboxylate (200 mg) was added to an ethanol solution (5 ml) of 4-(oxazol-5-yl)phenylhydrazine (130 mg), followed by heating overnight under reflux. The solvent was evaporated, the thus obtained residue was purified by silica gel column chromatography, and the fraction obtained from the eluate of dichloromethane:methanol = 100:3 was concentrated under reduced pressure to obtain the title compound (140 mg) as a yellow solid.
¹H-NMR (400 MHz, CDCl₃) δ: 1.50 (9H, s), 2.87 (2H, br s), 3.75 (2H, br s), 4.66 (2H, br s), 7.16 (2H, d, J=8.6 Hz), 7.24 (1H, s), 7.58 (2H, d, J=8.6 Hz), 7.88 (2H, s), 8.40 (1H, br s).
ESI-MS m/z: 426 (M⁺+H).

### Example 50

### 4,5,6,7-Tetrahydrothiazolo[5,4-c]pyridine-2-carboxyaldehyde 4-(oxazol-5-yl)phenylhydrazone

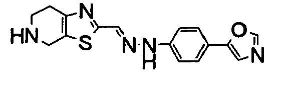

A hydrochloric acid-methanol solution (3 ml) was added to a methanol solution (0.5 ml) of *tert*-butyl 2-[4-(oxazol-5-yl)phenylhydrazonomethyl]-4,5,6,7-tetrahydrothiazolo[5,4-c]pyridine-5-carboxylate (140 mg) at 0°C, followed by stirring overnight at room temperature. The solvent was evaporated and to the thus obtained residue was added a saturated aqueous sodium bicarbonate solution and extracted with a chloroform:methanol (10:1, v/v) solution. After drying over sodium sulfate, the solvent was evaporated and the thus obtained residue was washed with methanol and diethyl ether and then dried to obtain the title compound (30 mg) as a yellow solid.
¹H-NMR (400 MHz, DMSO-d₆) δ: 2.65 (2H, t, J=5.7 Hz), 2.98 (2H, t, J=5.7 Hz), 3.89 (2H, s), 7.11 (2H, d, J=8.8 Hz), 7.47 (1H, s), 7.62 (2H, d, J=8.8 Hz), 8.01 (1H, s), 8.34 (1H, s), 11.03 (1H, s).

### Example 51

### 2-(5-Methyl-4,5,6,7-tetrahydrothiazolo[5,4-c]pyridine)carboxyaldehyde 4-(oxazol-5-yl)phenylhydrazone

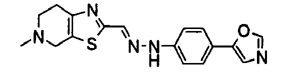

2-(5-Methyl-4,5,6,7-tetrahydrothiazolo[5,4-c]pyridine)carboxyaldehyde (171 mg) was added to an ethanol solution (10 ml) of 4-(oxazol-5-yl)phenylhydrazine (150 mg), followed by heating overnight under reflux. Dichloromethane was added to the residue obtained by evaporating the solvent, and the thus precipitated solid was collected by filtration, washed with diethyl ether and then dried to obtain the title compound (162 mg) as a yellow solid.
¹H-NMR (400 MHz, DMSO-d₆) δ: 2.38 (3H, s), 2.73 (4H, bs), 3.59 (2H, bs), 7.12 (2H, d, J=7.1 Hz), 7.47 (1H, d, J=7.6 Hz), 7.62 (2H, d, J=7.1 Hz), 7.99 (1H, d, J=2.9 Hz), 8.34 (1H, dd, J=2.9 and 7.6 Hz), 11.05 (1H, s).
ESI-MS m/z: 340 (M+H)⁺.

### Example 52

### 2-Hydroxy-5-[4-(oxazol-5-yl)phenylhydrazonomethyl]benzoic acid

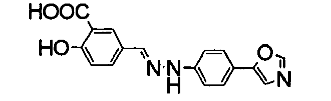

5-Formylsalicylic acid (142 mg) was added to an ethanol solution (10 ml) of 4-(oxazol-5-yl)phenylhydrazine (150 mg), followed by heating overnight under reflux. The residue obtained by evaporating the solvent was collected by filtration and then washed with ethanol to obtain the title compound (190 mg) as a yellow solid.
¹H-NMR (400 MHz, DMSO-d₆) δ: 7.00 (1H, d, J=8.6 Hz), 7.11 (2H, d, J=8.6 Hz), 7.41 (1H, d, J=3.9 Hz), 7.58 (2H, d, J=8.6 Hz), 7.88 (1H, s), 7.89 (1H, dd, J=2.0 and 8.6 Hz), 8.02 (1H, d, J=2.0 Hz), 8.28 (1H, t, J=3.9 Hz).
ESI-MS m/z: 324 (M⁺+H).

### Example 53

### 4-[N-(2-Fluoroethyl)-N-methylamino]benzaldehyde 4-(oxazol-5-yl)phenylhydrazone

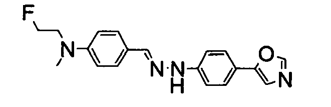

4-[*N*-(2-Fluoroethyl)-*N*-methylamino]benzaldehyde (207 mg) was added to an ethanol solution (10 ml) of 4-(oxazol-5-yl)phenylhydrazine (200 mg), followed by heating overnight under reflux. The residue obtained by evaporating the solvent was washed with diethyl ether to obtain the title compound (112 mg) as a yellow solid.
¹H-NMR (400 MHz, CDCl₃) δ: 3.07 (3H, s), 3.67 (1H, t, J=5.4 Hz), 3.73 (1H, t, J=5.4 Hz), 4.56 (1H, t, J=5.4 Hz), 4.68 (1H, t, J=5.4 Hz), 6.71 (2H, d, J=8.8 Hz), 7.12 (2H, d, J=8.8 Hz), 7.19 (1H, s), 7.54 (2H, d, J=8.8 Hz), 7.55 (2H, d, J=8.8 Hz), 7.65 (1H, s), 7.84 (1H, s).
ESI-MS m/z: 339 (M+H)⁺.

### Example 54

### 4-(Dimethylaminomethyl)benzaldehyde 4-(oxazol-5-yl)phenylhydrazone

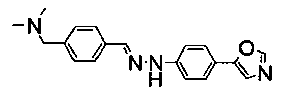

4-(Dimethylaminomethyl)benzaldehyde (186 mg) was added to an ethanol solution (5 ml) of 4-(oxazol-5-yl)phenylhydrazine (200 mg), followed by heating overnight under reflux. The residue obtained by evaporating the solvent was purified by silica gel column chromatography, and the fraction obtained from the eluate of dichloromethane:methanol = 10:1 was concentrated under reduced pressure and washed with diethyl ether and hexane to obtain the title compound (51 mg) as a light brown solid.
¹H-NMR (400 MHz, DMSO-d₆) δ: 2.15 (6H, s), 3.39 (2H, s), 7.14 (2H, d, J=8.5 Hz), 7.31 (2H, d, J=7.5 Hz), 7.44 (1H, s), 7.58 (2H, d, J=8.5 Hz), 7.62 (2H, d, J=7.5 Hz), 7.90 (1H, s), 8.32 (1H, s), 10.57 (1H, s).
ESI-MS m/z: 321 (M+H)⁺.

### Example 55

### 4-(4-Methylpiperazin-1-yl)benzaldehyde 4-(oxazol-5-yl)phenylhydrazone

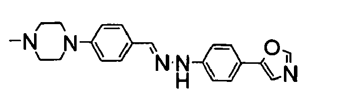

4-(4-Methylpiperazin-1-yl)benzaldehyde (175 mg) was added to an ethanol solution (5 ml) of 4-(oxazol-5-yl)phenylhydrazine (150 mg), followed by heating overnight under reflux. The residue obtained by evaporating the solvent was washed with ethanol diethyl and ether to obtain the title compound (205 mg) as a light brown solid.
¹H-NMR (400 MHz, CDCl₃) δ: 2.36 (3H, s), 2.58 (4H, t, J=4.6 Hz), 3.28 (4H, t, J=4.6 Hz), 6.91 (2H, d, J=8.8 Hz), 7.12 (2H, d, J=8.8 Hz), 7.19 (1H, s), 7.54 (2H, d, J=9.4 Hz), 7.58 (2H, d, J=9.4 Hz), 7.65 (1H, s), 7.84 (1H, s).
ESI-MS m/z: 362 (M+H)⁺.

### Example 56

### 4-(4-tert-Butoxycarbonylpiperazin-1-yl)benzaldehyde 4-(oxazol-5-yl)phenylhydrazone

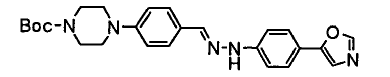

4-(4-*tert*-Butoxycarbonylpiperazin-1-yl)benzaldehyde (363 mg) was added to an ethanol solution (10 ml) of 4-(oxazol-5-yl)phenylhydrazine (200 mg), followed by heating overnight under reflux. Dichloromethane was added to the residue obtained by evaporating the solvent, and the thus precipitated solid was collected by filtration, washed with diethyl ether and then dried to obtain the title compound (427 mg) as a yellow solid.
¹H-NMR (400 MHz, CDCl₃) δ: 1.49 (9H, s), 3.21 (4H, bs), 3.58 (4H, bs), 6.91 (2H, d, J=8.8 Hz), 7.13 (2H, d, J=8.8 Hz), 7.26 (1H, s), 7.55 (2H, d, J=8.8 Hz), 7.57 (2H, d, J=8.8 Hz), 7.62 (1H, s), 7.66 (1H, s), 7.85 (1H, s).
ESI-MS m/z: 448 (M+H)⁺.

### Example 57

### 4-(Piperazin-1-yl)benzaldehyde 4-(oxazol-5-yl)phenylhydrazone

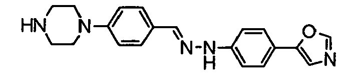

A hydrochloric acid-methanol solution (1.5 ml) was added to a methanol solution (3 ml) of 4-(4-*tert-*butoxycarbonylpiperazin-1-yl)benzaldehyde 4-(oxazol-5-yl)phenylhydrazone (200 mg) at 0°C, followed by stirring overnight at room temperature. The solvent was evaporated and to the thus obtained residue was added a saturated aqueous sodium bicarbonate solution and extracted with a chloroform: methanol (10:1) solution. After drying over sodium sulfate, the solvent was evaporated and the thus obtained residue was washed with diethyl ether and then dried to obtain the title compound (63 mg) as a yellow solid.
¹H-NMR (400 MHz, DMSO-d₆) δ: 2.82 (4H, t, J=5.2 Hz), 3.10 (4H, t, J=5.2 Hz), 6.93 (2H, d, J=8.8 Hz), 7.08 (2H, d, J=8.5 Hz), 7.40 (1H, s), 7.50 (2H, d, J=8.5 Hz), 7.54 (2H, d, J=8.8 Hz), 7.80 (1H, s), 8.30 (1H, s), 10.30 (1H, s).
ESI-MS m/z: 348 (M+H)⁺.

### Example 58

### N-(2-Hydroxyethyl)-4-[4-(oxazol-5-yl)phenylhydrazonomethyl]benzamide

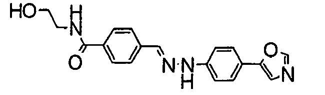

Ethanolamine (42 µl), dimethylaminopyridine (160 mg) and EDC·HCl (162 mg) were added to a DMF-dichloromethane (1:1, v/v) mixed solution (20 ml) of 4-[4-(oxazol-5-yl)phenylhydrazonomethyl]benzoic acid (200 mg) at 0°C, followed by stirring overnight at room temperature. To the reaction solution was added water, extracted with dichloromethane and then dried over sodium sulfate. The solvent was evaporated, the thus obtained residue was purified by silica gel column chromatography, and the fraction obtained from the eluate of dichloromethane:methanol = 10:2 was concentrated under reduced pressure to obtain the title compound (57 mg) as a yellow solid.
¹H-NMR (400 MHz, DMSO-d₆) δ: 3.33 (2H, t, J=5.9 Hz), 3.51 (2H, dt, J=5.6 and 5.9 Hz), 4.73 (1H, t, J=5.9 Hz), 7.19 (2H, d, J=8.5 Hz), 7.46 (1H, s), 7.60 (2H, d, J=8.5 Hz), 7.74 (2H, d, J=8.3 Hz), 7.88 (2H, d, J=8. Hz), 7.93 (1H, s), 8.44 (1H, s), 8.50 (1H, t, J=5.6 Hz), 10.78 (1H, s).
ESI-MS m/z: 351 (M+H)⁺.

### Example 59

### 4-(Morpholinomethyl)benzaldehyde 4-(oxazol-5-yl)phenylhydrazone

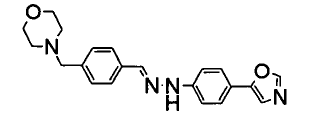

4-(Morpholinomethyl)benzaldehyde (175 mg) was added to an ethanol solution (10 ml) of 4-(oxazol-5-yl)phenylhydrazine (150 mg), followed by heating overnight under reflux. The solvent was evaporated and the thus obtained residue was washed with diethyl ether to obtain the title compound (165 mg) as a yellow solid.
¹H-NMR (400 MHz, DMSO-d₆) δ: 2.50 (4H, s), 3.31 (4H, s), 3.58 (2H, t, J=4.6 Hz), 7.14 (2H, d, J=8.6 Hz), 7.33 (2H, d, J=8.6 Hz), 7.44 (1H, s), 7.58 (2H, d, J=8.6 Hz), 7.62 (2H, d, J=8.6 Hz), 7.90 (1H, s), 8.32 (1H, s), 10,56 (1H, s).
ESI-MS m/z: 363 (M+H)⁺.

### Example 60

### tert-Butyl 4-[4-(oxazol-5-yl)phenylhydrazonomethyl]benzylcarbamate

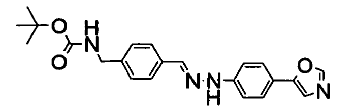

*tert*-Butyl 4-formylbenzylcarbamate (1.35 g) was added to an ethanol solution (30 ml) of 4-(oxazol-5-yl)phenylhydrazine (920 mg), followed by heating overnight under reflux. The solvent was evaporated and the thus obtained residue was washed with diethyl ether to obtain the title compound (1.50 g) as a yellow solid.
¹H-NMR (400 MHz, DMSO-d₆) δ: 1.47 (9H, s), 4.32 (2H, br s), 4.85 (1H, br s), 7.16 (2H, d, J=8.5 Hz), 7.21 (1H, s), 7.30 (2H, d, J=8.1 Hz), 7.57 (2H, d, J=8.5 Hz), 7.61 (2H, d, J=8.1 Hz), 7.71 (1H, s), 7.75 (1H, s), 7.86 (1H, s).
ESI-MS m/z: 393 (M+H)⁺.

### Example 61

### 4-(Aminomethyl)benzaldehyde 4-(oxazol-5-yl)phenylhydrazone

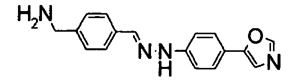

A hydrochloric acid-methanol solution (10 ml) was added to a methanol solution (1 ml) of *tert*-butyl 4-[4-(oxazol-5-yl)phenylhydrazonomethyl]benzylcarbamate (500 mg) at 0°C, followed by stirring overnight at room temperature. The solvent was evaporated and to the thus obtained residue was added a saturated aqueous sodium bicarbonate solution and extracted with a chloroform: methanol (10:1, v/v) solution. After drying over sodium sulfate, the solvent was evaporated and the thus obtained residue was washed with diethyl ether and then dried to obtain the title compound (303 mg) as a yellow solid.
¹H-NMR (400 MHz, CDCl₃) δ: 3.89 (2H, s), 7.15 (2H, d, J=8.5 Hz), 7.21 (1H, s), 7.33 (2H, d, J=8 .1 Hz), 7.56 (2H, d, J=8.5 Hz), 7.63 (2H, d, J=8.1 Hz), 7.71 (1H, s), 7.74 (1H, s), 7.85 (1H, s).
ESI-MS m/z: 293 (M+H)⁺.

### Example 62

### 3-(Dimethylaminomethyl)benzaldehyde 4-(oxazol-5-yl)phenylhydrazone

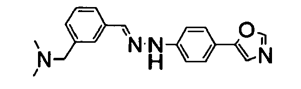

3-(Dimethylaminomethyl)benzaldehyde (204 mg) was added to an ethanol solution (10 ml) of 4-(oxazol-5-yl)phenylhydrazine (200 mg), followed by heating overnight under reflux. The solvent was evaporated and the thus obtained residue was washed with diethyl ether to obtain the title compound (247 mg) as a yellow solid.
¹H-NMR (400 MHz, CDCl₃) δ: 2.28 (6H, s), 3.46 (2H, s), 7.17 (2H, d, J=8.6 Hz), 7.21 (1H, s), 7.27 (1H, d, J=7.5 Hz), 7.34 (1H, t, J=7.5 Hz), 7.56-7.61 (4H, m), 7.72 (1H, s), 7.77 (1H, s), 7.86 (1H, s).
ESI-MS m/z: 321 (M+H)⁺.

### Example 63

### 2-(Dimethylaminomethyl)benzaldehyde 4-(oxazol-5-yl)phenylhydrazone

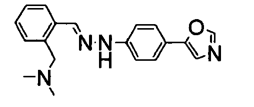

2-(Dimethylaminomethyl)benzaldehyde (166 mg) was added to an ethanol solution (5 ml) of 4-(oxazol-5-yl)phenylhydrazine (163 mg), followed by heating overnight under reflux. The solvent was evaporated and the thus obtained residue was washed with diethyl ether to obtain the title compound (124 mg) as a yellow solid.
¹H-NMR (400 MHz, CDCl₃) δ: 2.25 (6H, s), 3.54 (2H, s), 7.16 (2H, d, J=8.5 Hz), 7.21 (1H, s), 7.22 (1H, t, J=7.8 Hz), 7.26 (1H, d, J=7.8 Hz), 7.33 (1H, t, J=7.8 Hz), 7.56 (2H, d, J=8.5 Hz), 7.85 (1H, s), 7.92 (1H, s), 8.06 (1H, d, J=7.8 Hz), 8.24 (1H, s).
ESI-MS m/z: 321 (M+H)⁺.

### Example 64

### 4-{N-[2-(tert-Butyldiphenylsilyloxy)ethyl]-N-methylaminomethyl}benzaldehyde 4-(oxazol-5-yl)phenylhydrazone

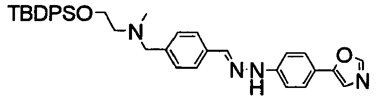

4-{*N*-[2-(*tert-*Butyldiphenylsilyloxy)ethyl]-*N*-methylaminomethyl}benzaldehyde (1.5 g) was added to an ethanol solution (40 ml) of 4-(oxazol-5-yl)phenylhydrazine (560 mg), followed by heating overnight under reflux. The residue obtained by evaporating the solvent was purified by silica gel column chromatography, and the fraction obtained from the eluate of chloroform:methanol = 100:2 was concentrated under reduced pressure. The thus obtained residue was washed with diethyl ether and then dried to obtain the title compound (1.30 g) as a yellow solid.
¹H-NMR (400 MHz, CDCl₃) δ: 1.05 (9H, s), 2.23 (3H, s), 2.63 (2H, t, J=6.1 Hz), 3.57 (2H, s), 3.80 (2H, t, J=6.1 Hz), 7.15 (2H, d, J=8.8 Hz), 7.21 (1H, s), 7.31 (2H, d, J=8.1 Hz), 7.35-7.42 (6H, m), 7.56 (2H, d, J=8.8 Hz), 7.58 (2H, d, J=8.1 Hz), 7.68 (4H, dd, J=1.5 and 7.3 Hz), 7.70 (1H, s), 7.77 (1H, s), 7.85 (1H, s).

### Example 65

### 4-[N-(2-Hydroxyethyl)-N-methylaminomethyl]benzaldehyde 4-(oxazol-5-yl)phenylhydrazone

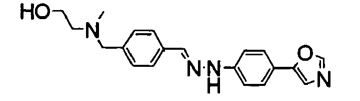

n-Butylammonium fluoride (3.3 ml, 1 M THF solution) was added dropwise to a THF solution (20 ml) of 4-{*N*-[2-(*tert*-butyldiphenylsilyloxy)ethyl]-*N*-methylaminomethyl}benzaldehyde 4-(oxazol-5-yl)phenylhydrazone (1.30 g) at 0°C, followed by stirring overnight at room temperature. The residue obtained by evaporating the solvent was purified by silica gel column chromatography, and the fraction obtained from the eluate of chloroform: methanol = 100:7 was concentrated under reduced pressure. The thus obtained residue was washed with diethyl ether and then dried to obtain the title compound (350 mg) as a yellow solid.
¹H-NMR (400 MHz, CDCl₃) δ: 2.26 (3H, s), 2.63 (2H, t, J=5.1 Hz), 3.60 (2H, s), 3.65 (2H, t, J=5.1 Hz), 7.16 (2H, d, J=8.5 Hz), 2.21 (1H, s), 7.33 (2H, d, J=8 .1 Hz), 7.57 (2H, d, J=8.5 Hz), 7.63 (2H, d, J=8.1 Hz), 7.72 (1H, s), 7.79 (1H, s), 7.86 (1H, s).
ESI-MS m/z: 351 (M+H)⁺.

### Example 66

### N-{4-[4-(Oxazol-5-yl)phenylhydrazonomethyl]phenyl}acetamide

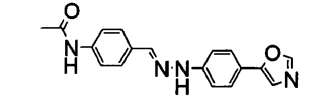

4-Formylphenylacetamide (101 mg) was added to an ethanol solution (5 ml) of 4-(oxazol-5-yl)phenylhydrazine (100 mg), followed by heating overnight under reflux. The residue obtained by evaporating the solvent was collected by filtration and then washed with ethanol to obtain the title compound (127 mg) as a red solid.
¹H-NMR (400 MHz, DMSO-d₆) δ: 2.06 (3H, s), 7.12 (2H, d, J=8.8 Hz), 7.42 (1H, s), 7.57 (2H, d, J=8.5 Hz), 7.59 (2H, d, J=8.8 Hz), 7.62 (2H, d, J=8.5 Hz), 7.85 (1H, s), 8.30 (1H, s), 10.02 (1H, s), 10.47 (1H, s).
ESI-MS m/z: 321 (M+H)⁺.

### Example 67

### 4-[N-(2-Fluoroethyl)-N-methylaminomethyl]benzaldehyde 4-(oxazol-5-yl)phenylhydrazone

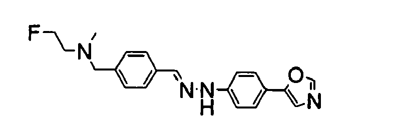

4-[*N*-(2-Fluoroethyl)-*N*-methylaminomethyl]benzaldehyde (198 mg) was added to an ethanol solution (10 ml) of 4-(oxazol-5-yl)phenylhydrazine (162 mg), followed by heating under reflux for three nights. The residue obtained by evaporating the solvent was purified by silica gel column chromatography, and the fraction obtained from the eluate of chloroform:methanol = 100:2 was concentrated under reduced pressure. The thus obtained residue was washed with diethyl ether and then dried to obtain the title compound (66 mg) as yellow amorphous.
¹H-NMR (400 MHz, DMSO-d₆) δ: 2.20 (3H, s), 2.64 (1H, t, J=4.9 Hz), 2.71 (1H, t, J=4.9 Hz), 3.55 (2H, s), 4.49 (2H, t, J=4.9 Hz), 4.61 (2H, t, J=4.9 Hz), 7.14 (2H, d, J=8.3 Hz), 7.33 (2H, d, J=8.3 Hz), 8.43 (1H, s), 7.58 (2H, d, J=7.8 Hz), 7.62 (2H, d, J=7.8 Hz), 7.90 (1H, s), 8.32 (1H, s), 10.55 (1H, s).
ESI-MS m/z: 353 (M+H)⁺.

### Example 68

### 4-[4-(Oxazol-5-yl)phenylhydrazonomethyl]phenylacetic acid

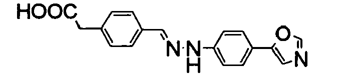

4-Formylphenylacetic acid (220 mg) was added to an ethanol solution (10 ml) of 4-(oxazol-5-yl)phenylhydrazine (214 mg), followed by heating overnight under reflux. The residue obtained by evaporating the solvent was washed with diethyl ether to obtain the title compound (186 mg) as a yellow solid.
¹H-NMR (400 MHz, DMSO-d₆) δ: 3.58 (2H, s), 7.14 (2H, d, J=8.8 Hz), 7.29 (2H, d, J=7.8 Hz), 7.44 (1H, s), 7.58 (2H, d, J=8.8 Hz), 7.61 (2H, d, J=7.8 Hz), 7.89 (1H, s), 8.32 (1H, s), 10.56 (1H, s).
ESI-MS m/z: 322 (M+H)⁺.

### Example 69

### N,N-Dimethyl-2-{4-[4-(oxazol-5-yl)phenylhydrazonomethyl]phenyl}acetamide

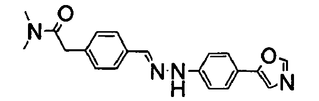

Dimethylamine (171 µl), DMAP (76 mg) and EDC·HCl (72 mg) were added to a DMF solution (5 ml) of 4-[4-(oxazol-5-yl)phenylhydrazonomethyl]phenylacetic acid (100 mg) at 0°C, followed by stirring at room temperature for three nights. To the reaction solution was adde water, extracted with dichloromethane and then dried over sodium sulfate.

The solvent was evaporated, the thus obtained residue was purified by silica gel column chromatography, and the fraction obtained from the eluate of dichloromethane:methanol = 100:1 was concentrated under reduced pressure to obtain the title compound (17 mg) as a yellow solid.
¹H-NMR (400 MHz, CDCl₃) δ: 2.99 (3H, s), 3.02 (3H, s), 3.73 (2H, s), 7.13 (2H, d, J=8.8 Hz), 7.20 (1H, s), 7.25 (2H, d, J=8.1 Hz), 7.54 (2H, d, J=8.8 Hz), 7.58 (2H, d, J=8.1 Hz), 7.60 (1H, s), 7.85 (1H, s), 7.96 (1H, s).

### Example 70

### 4-(4-Methylpiperazin-1-carbonyl)benzaldehyde 4-(oxazol-5-yl)phenylhydrazone

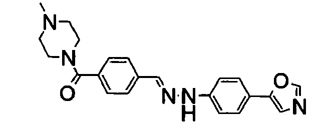

4-Methylpiperazine (64 µl), DMAP (76 mg) and EDC·HCl (120 mg) were added to a DMF solution (5 ml) of 4-[4-(oxazol-5-ylphenyl)hydrazonomethyl]benzoic acid (160 mg) at 0°C, followed by stirring overnight at room temperature. To the reaction solution was added water, extracted with dichloromethane and then dried over sodium sulfate. The solvent was evaporated, the thus obtained residue was purified by silica gel column chromatography, and the fraction obtained from the eluate of dichloromethane:methanol = 10:2 was concentrated under reduced pressure to obtain the title compound (140 mg) as a yellow solid.
¹H-NMR (400 MHz, CDCl₃) δ: 2.33 (3H, s), 2.38 (4H, bs), 3.47 (2H, bs), 3.78 (2H, bs), 7.17 (2H, d, J=8.5 Hz), 7.22 (1H, s), 7.42 (2H, d, J=8.1 Hz), 7.58 (2H, d, J=8.5 Hz), 7.69 (2H, d, J=8.1 Hz), 7.70 (1H, s), 7.86 (1H, s), 7.90 (1H, s).
ESI-MS m/z: 390 (M+H)⁺.

### Example 71

### 4-(Dimethylaminomethyl)benzaldehyde 3-iodo-4-(oxazol-5-yl)phenylhydrazone

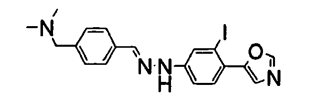

4-(Dimethylaminomethyl)benzaldehyde hydrochloride (133 mg) was added to an ethanol solution (5 ml) of 3-iodo-4-(oxazol-5-yl)phenylhydrazine (200 mg), followed by heating overnight under reflux. To the residue obtained by evaporating the solvent was added 1 N sodium hydroxide and extracted with chloroform-methanol (10:1, v/v). After drying the extract over sodium sulfate, the residue obtained by evaporating the solvent was purified by silica gel column chromatography, and the fraction obtained from the eluate of chloroform: methanol = 100:5 was concentrated under reduced pressure to obtain the title compound (66 mg) as brown amorphous.
¹H-NMR (400 MHz, DMSO-d₆) δ: 2.17 (6H, s), 3.41 (2H, s), 7.15 (1H, d, J=8.3 Hz), 7.32 (2H, d, J=7.3 Hz), 7.42 (1H, d, J=8.3 Hz), 7.52 (1H, s), 7.63 (2H, d, J=7.3 Hz), 7.69 (1H, s), 7.91 (1H, s), 8.41 (1H, s), 10.64 (1H, s).
ESI-MS m/z: 447 (M+H)⁺.

### Example 72

### 4-(4-Methylpiperazin-1-yl)benzaldehyde 3-iodo-4-(oxazol-5-yl)phenylhydrazone

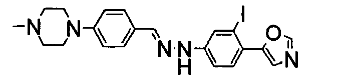

4-(4-Methylpiperazin-1-yl)benzaldehyde (136 mg) was added to an ethanol solution (10 ml) of 3-iodo-4-(oxazol-5-yl)phenylhydrazine (200 mg), followed by heating overnight under reflux. The residue obtained by evaporating the solvent was purified by silica gel column chromatography, and the fraction obtained from the eluate of chloroform:methanol = 100:5 was concentrated under reduced pressure to obtain the title compound (127 mg) as a yellow solid.
¹H-NMR (400 MHz, DMSO-d₆) δ: 2.36 (3H, s), 2.58 (4H, s), 3.29 (4H, s), 6.92 (2H, d, J=8.5 Hz), 7.07 (1H, d, J=9.0 Hz), 7.42 (1H, d, J=9. 0 Hz), 7.63 (2H, d, J=7.3 Hz), 7.69 (1H, s), 7. 91 (1H, s), 8.41 (1H, s), 10.64 (1H, s).
ESI-MS m/z: 488 (N4+H)⁺.

### Example 73

### tert-Butyl N-methyl-N-{2-[3-iodo-4-(oxazol-5-yl)phenylhydrazonomethyl]thiazol-4-ylmethyl}carbamate

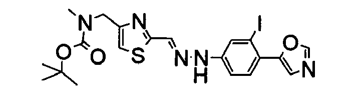

*tert*-Butyl *N*-(2-formylthiazol-4-ylmethyl)-*N*-methylcarbamate (213 mg) was added to an ethanol solution (10 ml) of 3-iodo-4-(oxazol-5-yl)phenylhydrazine (250 mg), followed by heating overnight under reflux. The residue obtained by evaporating the solvent was purified by silica gel column chromatography, and the fraction obtained from the eluate of chloroform: methanol = 100:5 was concentrated under reduced pressure to obtain the title compound (230 mg) as brown amorphous.
¹H-NMR (400 MHz, CDCl₃) δ: 1.26 (9H, s), 2.96 (3H, s), 4.52 (2H, s), 7.01 (1H, s), 7.15 (1H, dd, J=2.2 and 8.5 Hz), 7.47 (1H, d, J=8.5 Hz), 7.64 (1H, s), 7.73 (1H, d, J=2.2 Hz), 7.92 (1H, bs), 7.93 (1H, s).
ESI-MS m/z: 540 (M+H)⁺.

### Example 74

### 4-(N-Methylaminomethyl)thiazol-2-ylcarboxyaldehyde 3-iodo-4-(oxazol-5-yl)phenylhydrazone

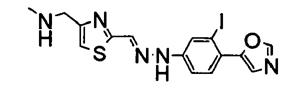

A hydrochloric acid/methanol solution (5 ml) was added to a methanol solution (1 ml) of *tert*-butyl *N-*methyl-*N*-{2-[3-iodo-4-(oxazol-5-yl)phenylhydrazonomethyl]thiazol-4-ylmethyl}carbamate (230 mg) at 0°C, followed by stirring at room temperature for 1 hour. To the residue obtained by evaporating the solvent was added 1 N sodium hydroxide and extracted with chloroform-methanol (10:1, v/v). After drying the extract over sodium sulfate, the residue obtained by evaporating the solvent was purified by silica gel column chromatography, and the fraction obtained from the eluate of chloroform:methanol = 10:1 was concentrated under reduced pressure to obtain the title compound (66 mg) as yellow amorphous.
¹H-NMR (400 MHz, DMSO-d₆) δ: 2.34 (3H, s), 3.77 (2H, s), 7.15 (1H, d, J=8.3 Hz), 7.37 (1H, s), 7.47 (1H, d, J=8.3 Hz), 7.55 (1H, s), 7.67 (1H, s), 8.07 (1H, s), 8.30 (1H, s), 8.43 (1H, s), 11.15 (1H, s).
ESI-MS m/z: 440 (M+H)⁺.

### Example 75

### 4-Pyridinecarboxyaldehyde 2-iodo-4-(oxazol-5-yl)phenylhydrazone

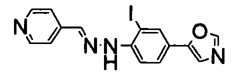

4-Pyridinecarboxyaldehyde (62 µl) was added to an ethanol solution (5 ml) of 2-iodo-4-(oxazol-5-yl)phenylhydrazine (194 mg), followed by heating overnight under reflux. The residue obtained by evaporating the solvent was collected by filtration and washed with ethanol to obtain the title compound (135 mg) as a red solid.
¹H-NMR (400 MHz, CDCl₃) δ: 7.26 (1H, s), 7.54 (2H, d, J=6.9 Hz), 7.57 (1H, d, J=8.1 Hz), 7.59 (1H, dd, J=1.8 and 8.1 Hz), 7.81 (1H, s), 7.88 (1H, s), 8.00 (1H, d, J=1.8 Hz), 8.33 (1H, s), 8.63 (2H, d, J=6.9 Hz).
ESI-MS m/z: 391 (M+H)⁺.

### Example 76

### 4-Pyridinecarboxyaldehyde 3-iodo-4-(oxazol-5-yl)phenylhydrazone

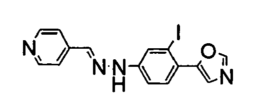

4-Pyridinecarboxyaldehyde (66 µl) was added to an ethanol solution (5 ml) of 3-iodo-4-(oxazol-5-yl)phenylhydrazine (200 mg), followed by heating overnight under reflux. The residue obtained by evaporating the solvent was collected by filtration and washed with ethanol to obtain the title compound (172 mg) as a red solid.
¹H-NMR (400 MHz, CDCl₃) δ: 7.16 (1H, dd, J=2.2,7.1 Hz), 7.49 (1H, d, J=7.1 Hz), 7.52 (2H, d, J=5.8 Hz), 7.65 (2H, s), 7.78 (1H, d, J=2.2 Hz), 7.94 (1H, s), 8.06 (1H, s), 8.62 (2H, d, J=5.8 Hz).
ESI-MS m/z: 391 (M+H)⁺.

### Example 77

### 4-(Dimethylaminomethyl)-3-iodobenzaldehyde 4-(oxazol-5-yl)phenylhydrazone

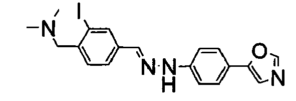

4-(Dimethylaminomethyl)-3-iodobenzaldehyde (310 mg) was added to an ethanol solution (10 ml) of 4-(oxazol-5-yl)phenylhydrazine (170 mg), followed by heating overnight under reflux. The residue obtained by evaporating the solvent was purified by silica gel column chromatography, and the fraction obtained from the eluate of n-hexane:ethyl acetate = 1:3 was concentrated under reduced pressure to obtain the title compound (250 mg) as amorphous.
¹H-NMR (400 MHz, CDCl₃) δ: 2.31 (6H, s), 3.47 (2H, s), 7.16 (2H, d, J=8.5 Hz), 7.22 (1H, s), 7.39 (1H, d, J=8.1 Hz), 7.57 (2H, d, J=8.5 Hz), 7.59 (1H, d, J=8.1 Hz), 7.85 (1H, s), 7.86 (1H, s), 8.12 (1H, s).
ESI-MS m/z: 447 (M+H)⁺.

### Example 78

### N'-[4-(Dimethylaminomethyl)benzylidene]-N-[4-(oxazol-5-yl)phenyl]hydrazinecarboxylic acid tert-butyl ester

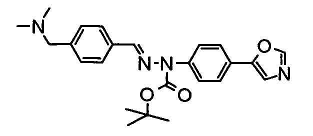

4-(Dimethylaminomethyl)benzaldehyde 4-(oxazol-5-yl)phenylhydrazone (1.10 g) was dissolved in THF (20 ml), (Boc)₂O (875 mg) was added to the solution at room temperature and stirred for 2 hours. The solvent was evaporated, the residue was purified by flash silica gel column chromatography (chloroform:methanol = 20:1 to 9:1), and the thus obtained solid was washed with diethyl ether to obtain the title compound (1.11 g) as a white solid.
¹H-NMR (400 MHz, CDCl₃) δ: 1.50 (9H, s), 2.22 (6H, s), 3.41 (2H, s), 7.26 (4H, m), 7.33 (1H, s), 7.44 (1H, s), 7.58 (2H, d, J=8. Hz), 7.79 (2H, d, J=8. Hz), 7.97 (1H, s).
ESI-MS m/z: 421 (M+H)⁺

### Example 79

### N'-[4-(Dimethylaminomethyl)benzylidene]-N [4-(4-iodooxazol-5-yl)phenyl]hydrazinecarboxylic acid tert-butyl ester

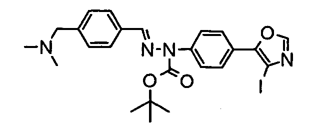

Under argon atmosphere, a 1 M lithium hexamethyldisilazide-THF solution (1.05 ml) was added at -20°C to a mixed solution of THF (8 ml) and 1,3-dimethyl-2-imidazolidinone (4 ml) of *N*'-[4-(dimethylaminomethyl)benzylidene]-*N*-[4-(oxazol-5-yl)phenyl]hydrazinecarboxylic acid *tert*-butyl ester (400 mg), followed by stirring at the same temperature for 2 hours. After cooling the reaction solution to -40°C, a THF solution (2 ml) of iodine (362 mg) was added to the mixture, followed by stirring at 0°C for 1 hour. To the reaction solution was added a saturated aqueous ammonium chloride solution (80 ml) and then extracted with chloroform (80 ml) three times. The organic layer was dried over anhydrous sodium sulfate, the residue obtained by evaporating the solvent was purified by flash silica gel column chromatography (chloroform:methanol = 15:1), and the thus obtained fraction (a mixture of the material and title compound) was recrystallized from methanol:water = 95:5 to obtain the title compound (192 mg) as a white solid.
¹H-NMR (400 MHz, CDCl₃) δ: 1.51 (9H, s), 2.22 (6H, s), 3.41 (2H, s), 7.22-7.35 (5H, m), 7.58 (2H, d, J=8.3 Hz), 7.93 (1H, s), 8.13 (2H, d, J=8.6 Hz).
ESI-MS m/z: 547 (N4+H)⁺.

### Example 80

### 4-(Dimethylaminomethyl)benzaldehyde 4-(4-iodooxazol-5-yl)phenylhydrazone

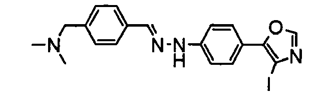

Trifluoroacetic acid (1 ml) was added at room temperature to a dichloromethane solution (3 ml) of *N'*-[4-(*N,N* dimethylaminomethyl)benzylidene]-*N*-[4-(4-iodooxazol-5-yl)phenyl]hydrazinecarboxylic acid *tert*-butyl ester (188 mg), followed by stirring at room temperature for 3 hours. After adding a saturated aqueous sodium bicarbonate solution, the mixture was extracted with chloroform (40 ml) twice and dried over anhydrous sodium sulfate. The solvent was evaporated, and the residue was purified by flash silica gel column chromatography (chloroform:methanol = 9:1) and recrystallized from diethyl ether to obtain the title compound (108.6 mg) as a yellowish brown solid.
¹H-NMR (400 MHz, CDCl₃) δ: 2.29 (6H, s), 3.49 (2H, s), 7.18 (2H, d, J=8.8 Hz), 7.34 (2H, d, J=7.6 Hz), 7.63 (2H, d, J=8.0 Hz), 7.73 (1H, s), 7.77 (1H, s), 7.84 (1H, s), 7.87 (2H, d, J=8.8 Hz).
ESI-MS m/z: 447 (M+H)⁺

### Example 81

### 3-Iodo-4-pyridinecarboxyaldehyde 4-(oxazol-5-yl)phenylhydrazone

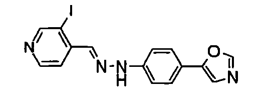

3-Iodo-4-pyridinecarboxyaldehyde (154 mg) was added to an ethanol solution (20 ml) of 4-(oxazol-5-yl)phenylhydrazine (116 mg), followed by heating under reflux for 1.5 hours. The residue obtained by evaporating the solvent was purified by silica gel column chromatography, and the fraction obtained from the eluate of dichloromethane:methanol = 20:1 was concentrated under reduced pressure to obtain the title compound (204 mg) as a yellow solid.
¹H-NMR (400 MHz, DMSO-d₆) δ: 7.23 (2H, d, J=8.5 Hz), 7.50 (1H, s), 7.63 (2H, d, J=8.3 Hz), 7.85 (1H, d, J=5.1 Hz), 8.00 (1H, s), 8.35 (1H, s), 8.43 (1H, d, J=5.1 Hz), 8.86 (1H, s), 11.35 (1H, s).
FAB-MS m/z: 391 (M+H)⁺.

### Example 82

### 2-Iodo-4-pyridinecarboxyaldehyde 4-(oxazol-5-yl)phenylhydrazone

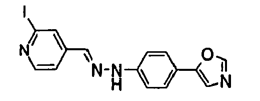

2-Iodo-4-pyridinecarboxyaldehyde (114 mg) was added to an ethanol solution (15 ml) of 4-(oxazol-5-yl)phenylhydrazine (70 mg), followed by heating under reflux for 2 hours. The residue obtained by evaporating the solvent was purified by silica gel column chromatography, and the fraction obtained from the eluate of dichloromethane:methanol = 20:1 was concentrated under reduced pressure to obtain the title compound (139 mg) as a reddish brown solid.
¹H-NMR (400 MHz, DMSO-d₆) δ: 7.23 (2H, d, J=8.8 Hz), 7.50 (1H, s), 7.62 (2H, d, J=8.5 Hz), 7.66 (1H, d, J=5.4 Hz), 7.76 (1H, s), 8.03 (1H, s), 8.30 (1H, d, J=5.1 Hz), 8.36 (1H, s), 11.19 (1H, s).
FAB-MS m/z: 391 (N4+H)⁺.

### Example 83

### 2-Fluoro-4-pyridinecarboxyaldehyde 4-(oxazol-5-yl)phenylhydrazone

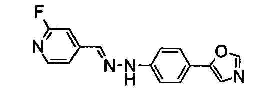

Diisobutylaluminum (5.7 ml) was added dropwise to a tetrahydrofuran solution (10 ml) of 2-fluoro-*N*-methoxy-*N*-methyl-4-pyridinecarboxamide (400 mg) at -78°C, followed by stirring at the same temperature for 1 hour. A saturated aqueous ammonium chloride solution (3.0 ml) was added dropwise to the reaction solution, followed by stirring at room temperature for 1 hour, magnesium sulfate and diethyl ether were added to the mixture and further stirred for 1 hour. After celite filtration, the solvent was evaporated and the thus obtained residue was used in the subsequent reaction without purification.

The above-described residue was dissolved in ethanol, 4-(oxazol-5-yl)phenylhydrazine (380 mg) was added to the solution and heated overnight under reflux. To the residue obtained by evaporating the solvent was added dichloromethane, and the precipitated solid was collected by filtration and then washed with diethyl ether and dried to obtain the title compound (153 mg) as a red solid.
¹H-NMR (400 MHz, DMSO-d₆) δ: 7.24 (2H, d, J=8.5 Hz), 7.35 (1H, s), 7.50 (1H, s), 7.60 (1H, d, J=5.1 Hz), 7.62 (2H, d, J=8.5 Hz), 7.87 (1H, s), 8.19 (1H, d, J=5.1 Hz), 8.35 (1H, s), 11.15 (1H, s).
ESI-MS m/z: 283 (M⁺+H).

### Example 84

### 2-(4-Methylpiperazin-1-yl)-4-pyridinecarboxyaldehyde 4-(oxazol-5-yl)phenylhydrazone

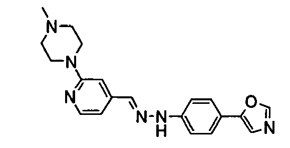

Diisobutylaluminum hydride (5.4 ml) was added dropwise to a THF solution (10 ml) of *N*-methoxy-*N*-methyl-2-(4-methylpiperazin-1-yl)-4-pyridinecarboxamide (540 mg) at - 78°C, followed by stirring at the same temperature for 1 hour. A saturated aqueous ammonium chloride solution (3.0 ml) was added dropwise to the reaction solution, followed by stirring at room temperature for 1 hour, and magnesium sulfate and diethyl ether were added to the mixture, followed by further stirring for 1 hour. After celite filtration, the solvent was evaporated and the thus obtained residue was used in the subsequent reaction without carrying out its separation and purification.

The above-described residue was dissolved in ethanol, 4-(oxazol-5-yl)phenylhydrazine (357 mg) was added to the solution and heated overnight under reflux. To the residue obtained by evaporating the solvent was added dichloromethane, and the precipitated solid was collected by filtration and then washed with diethyl ether and dried to obtain the title compound (127 mg) as a red solid.
¹H-NMR (400 MHz, DMSO-d₆) δ: 2.22 (3H, s), 2.41 (4H, t, J=5.1 Hz), 3.51 (4H, t, J=5.1 Hz), 6.96 (1H, s), 6.97 (1H, d, J=5.1 Hz), 7.18 (2H, d, J=8.5 Hz), 7.46 (1H, s), 7.60 (2H, d, J=8.5 Hz), 7.77 (1H, s), 8.07 (1H, d, J=5.1 Hz), 8.33 (1H, s), 10.85 (1H, s).
ESI-MS m/z: 363 (M+H)⁺.

### Example 85

### 4-Pyridinecarboxyaldehyde 4-(6-iodoimidazo[1,2-a]pyridin-2-yl)phenylhydrazone

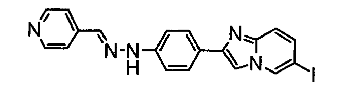

4-(6-Iodoimidazo[1,2-a]pyridin-2-yl)phenylhydrazine (110 mg) was dissolved in ethanol (8 ml), 4-pyridinecarboxyaldehyde (29.4 µl) was added to the solution and heated at 60°C under reflux for 1.5 hours. After evaporation of the solvent, the residue was purified by flash silica gel column chromatography (chloroform:methanol = 20: 1), and the thus obtained solid was washed with diethyl ether to obtain the title compound (39.6 mg) as a yellow solid.
¹H-NMR (400 MHz, DMSO-d₆) δ: 7.18 (2H, d, J=8.8 Hz), 7.38 (2H, s), 7.59 (2H, d, J=6.2 Hz), 7.83 (1H, s), 7.84 (2H, d, J=9.0 Hz), 8.18 (1H, s), 8.53 (2H, d, J=5.8 Hz), 8.86 (1H, s), 10.92 (1H, s).
ESI-MS m/z: 440 (M+H)⁺.

### Example 86

### 4-(4-Methylpiperazin-1-yl)benzaldehyde 4-(6-iodoimidazo[1,2-a]pyridin-2-yl)phenylhydrazone

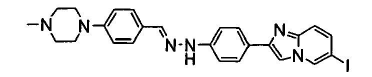

4-(6-Iodoimidazo[1,2-a]pyridin-2-yl)phenylhydrazine (108 mg) was dissolved in ethanol (8 ml), 4-(4-methylpiperazin-1-yl)benzaldehyde (74.2 mg) was added to the solution and heated at 60°C under reflux for 1.5 hours. After evaporation of the solvent, the residue was purified by flash silica gel column chromatography (chloroform:methanol = 20:1), and the thus obtained solid was washed with diethyl ether to obtain the title compound (26.0 mg) as a yellow solid.
¹H-NMR (400 MHz, DMSO-d₆) δ: 2.21 (3H, s), 2.45 (4H, t, J=4.9 Hz), 3.18 (4H, t, J=5.1 Hz), 6.94 (2H, d, J=9.1 Hz), 7.06 (2H, d, J=8.6 Hz), 7.36 (2H, s), 7.49 (2H, d, J=8.8 Hz), 7.77 (2H, d, J=8.6 Hz), 7.79 (1H, s), 8.13 (1H, s), 8.85 (1H, s), 10.22 (1H, s).
ESI-MS m/z: 537 (M+H)⁺.

### Example 87

### (Z) form of 2-[4-(Oxazol-5-yl)phenylhydrazono)phenylacetic acid methyl ester

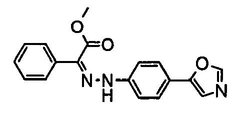

4-(Oxazol-5-yl)phenylhydrazine (102 mg) was dissolved in 60% aqueous solution (10 ml) of acetic acid, phenylglyoxylic acid methyl ester (165 mg) was added to the solution at room temperature and stirred for 2 hours. After evaporation of the solvent, the residue was diluted with ethyl acetate (60 ml), washed with water and dried over anhydrous sodium sulfate. The mixture was concentrated and then purified by flash silica gel column chromatography (hexane:acetone = 2:1 to 1:1) to obtain the title compound (112 mg) as a yellow solid.
¹H-NMR (400 MHz, CDCl₃) δ: 3.89 (3H, s), 7.27 (1H, s), 7.33 (2H, d, J=8.8 Hz), 7.39 (3H, m), 7.62 (2H, d, J=8.8 Hz), 7.63 (2H, J=6.8 Hz), 7.88 (1H, s), 12.5 (1H, s).
ESI-MS m/z: 322 (M+H)⁺.

### Example 88

### (E) form of 2-[4-(oxazol-5-yl)phenylhydrazono]phenylacetic acid methyl ester

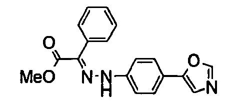

The title compound (6.9 mg) was obtained as a yellow solid, as a by-product of Example 87.
¹H-NMR (400 MHz, CDCl₃) δ: 3.88 (3H, s), 7.19 (2H, d, J=8.6 Hz), 7.25 (1H, s), 7.35 (2H, d, J=7.4 Hz), 7.51 (1H, t, J=7.4 Hz), 7.56 (2H, d, J=7.4 Hz), 7.57 (2H, d, J=8.6 Hz), 7.86 (1H, s), 8.16 (1H, s).
ESI-MS m/z: 322 (M+H)⁺

### Example 89

### 2-[4-(Oxazol-5-ylphenyl)hydrazono]phenylacetic acid

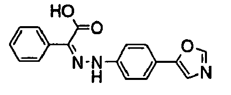

2-[4-(Oxazol-5-ylphenyl)hydrazono]phenylacetic acid methyl ester (73.3 mg) was dissolved in THF (6 ml), 1 M sodium hydroxide (800 µl) was added to teh solution and stirred at room temperature for 15 hours. The mixture was neutralized by adding a 1 M aqueous hydrochloric acid solution (800 µl) and then THF was evaporated. To the residue was added water (30 ml), and extracted with chloroform:methanol = 9:1 (30 ml) three times and dried over anhydrous sodium sulfate. The solvent was evaporated and the thus obtained solid was washed with diethyl ether to obtain the title compound (60.0 mg) as a yellow solid.
¹H-NMR (400 MHz, DMSO-d₆) δ: 7.37 (5H, m), 7.54 (1H, s), 7,65 (4H, m), 8.36 (1H, s), 12.05 (1H, s).
ESI-MS m/z: 308 (N4+H)⁺.

### Example 90

### N,N-dimethyl-2-[4-(oxazol-5-yl)phenylhydrazono]-2-phenylacetamide

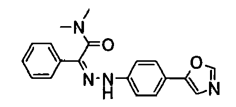

2-[4-(Oxazol-5-ylphenyl)hydrazono]phenylacetic acid (36.5 mg) was dissolved in dichloromethane (6 ml) and DMF (2 ml), NMM (15.7 µl), dimethylamine hydrochloride (11.6 mg) and HOBt (21.8 mg) and then EDC·HCl (27.3 mg) were added to the solution and stirred at room temperature for 2 hours. After evaporation of the solvent, the residue was diluted with ethyl acetate (60 ml), washed with a saturated aqueous ammonium chloride solution, a saturated aqueous sodium bicarbonate solution and saturated brine (20 ml for each) and dried over anhydrous sodium sulfate. The solvent was evaporated, the residue was purified by flash silica gel column chromatography (chloroform:methanol = 40:1 to 20:1), and the thus obtained solid was washed with diisopropyl ether to obtain the title compound (31.6 mg) as a yellow solid.
¹H-NMR (400 MHz, CDCl₃) δ: 2.88 (3H, s), 3.20 (3H, s), 7.21 (2H, d, J=8.3 Hz), 7.23 (1H, s), 7.40 (3H, m), 7.58 (2H, d, J=8.5 Hz), 7.65 (2H, d, J=8.5 Hz), 7.87 (1H, s), 8.44 (1H, s).
ESI-MS m/z: 335 (N4+H)⁺.

### Example 91

### 4-Pyridinecarboxyaldehyde 4-(pyrrolidin-1-ylcarbonyl)phenylhydrazone

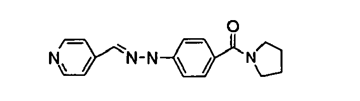

Pyrrolidine (74.7 mg) was added to a dichloromethane solution (20 ml)-DMF solution (20 ml) of 4-(N'-pyridin-4-ylmethylenehydrazino)benzoic acid (241 mg), EDC·HCl (249 mg) and DMAP (244 mg) under ice-cooling, followed by stirring at room temperature for 24 hours. The reaction solution was concentrated under reduced pressure, to the residue was added chloroform (100 ml), and the organic layer was washed with a saturated aqueous ammonium chloride solution, water and a saturated aqueous sodium bicarbonate solution in this order and dried over sodium sulfate. The residue obtained by evaporating the solvent was purified by silica gel column chromatography, and the fraction obtained from the eluate of dichloromethane:methanol = 20:1 to 10:1 was concentrated under reduced pressure to obtain the title compound (276 mg) as a crystalline solid.
¹H-NMR (400 MHz, CDCl₃) δ: 1.66-2.10 (4H, m), 3.41-3.78 (4H, m), 7.11 (2H, m), 7.48 7.53 (4H, m), 7.62 (1H, s), 8.58 (2H, d, J=5.4 Hz), 8.61 (1H, s).
FAB-MS m/z: 295 (M+H)⁺.

### Example 92

### 4-Pyridinecarboxyaldehyde 4-(piperidin-1-ylcarbonyl)phenylhydrazone

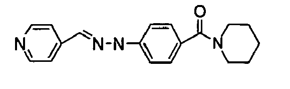

Piperidine (89.4 mg) was added to a dichloromethane solution (20 ml)-DMF solution (20 ml) of 4-(*N*'-pyridin-4-ylmethylenehydrazino)benzoic acid (241 mg), EDC·HCl (249 mg) and DMAP (244 mg) under ice-cooling, followed by stirring at room temperature for 24 hours. The reaction solution was concentrated under reduced pressure, to the residue was added chloroform (100 ml), and the organic layer was washed with a saturated aqueous ammonium chloride solution, water and a saturated aqueous sodium bicarbonate solution in this order and dried over sodium sulfate. The residue obtained by evaporating the solvent was purified by silica gel column chromatography, and the fraction obtained from the eluate of dichloromethane:methanol = 20:1 to 10:1 was concentrated under reduced pressure to obtain the title compound (270 mg) as a crystalline solid.
¹H-NMR (400 MHz, CDCl₃) δ: 1.35-1.75 (6H, m), 3.24-3.90 (4H, m), 7.11 (2H, m), 7.35 (2H, m), 7.49 (2H, dd, J=4.6 Hz, 2.0 Hz), 7.59 (1H, s), 8.58 (2H, d, J=4.6 Hz), 8.59 (1H, s).
FAB-MS m/z: 309 (M+H)⁺.

### Example 93

### 4-Pyridinecarboxyaldehyde 4-(morpholinocarbonyl)phenylhydrazone

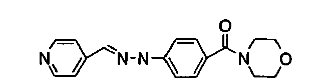

Morpholine (91.5 mg) was added to a dichloromethane solution (20 ml)-DMF solution (20 ml) of 4-(*N'*-pyridin-4-ylmethylenehydrazino)benzoic acid (241 mg), EDC·HCI (249 mg) and DMAP (244 mg) under ice-cooling, followed by stirring at room temperature for 21 hours. The reaction solution was concentrated under reduced pressure, to the residue was added chloroform (100 ml), and the organic layer was washed with a saturated aqueous ammonium chloride solution, water and a saturated aqueous sodium bicarbonate solution in this order and dried over sodium sulfate. The residue obtained by evaporating the solvent was purified by silica gel column chromatography, and the fraction obtained from the eluate of dichloromethane:methanol = 20:1 to 10:1 was concentrated under reduced pressure to obtain the title compound (277 mg) as a crystalline solid.
¹H-NMR (400 MHz, DMSO-d₆) δ: 3.38-3.70 (8H, m), 7.15 (2H, d, J=8.5 Hz), 7.3 (2H, d, J=8.5 Hz), 7.60 (2H, d, J=5.9 Hz), 7.86 (1H, s), 8.55 (2H, d, J=5.9 Hz), 11.02 (1H, s).
FAB-MS m/z: 311 (M+H)⁺.

### Example 94

### 4-Fluorobenzaldehyde 4-(oxazol-5-yl)phenylhydrazone

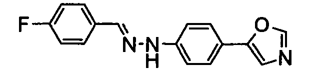

By using 4-fluorobenzaldehyde and carrying out the operation in the same manner as in Example 31, the title compound was obtained as a yellowish brown solid.
¹H-NMR (400 MHz, CDCl₃) δ: 7.05 (2H, t, J=8.8 Hz), 7.11 (2H, d, J=9.0 Hz), 7.19 (1H, s), 7.53 (2H, d, J=8.8 Hz), 7.61 (2H, dd, J=8.8 Hz, 6.0 Hz), 7.63 (1H, s), 7.85 (1H, s), 7.95 (1H, br s).
FAB-MS m/z: 282 (M+H)⁺

### Example 95

### 4-Aminobenzaldehyde 4-(oxazol-5-yl)phenylhydrazone

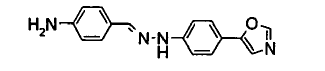

*N*-{4-[4-(Oxazol-5-yl)phenylhydrazonomethyl]phenyl}acetamide (80 mg) was dissolved in a 1 N hydrochloric acid-ethanol solution (10 ml) and stirred at 80°C for 2 hours. After cooling, a saturated aqueous sodium bicarbonate solution was added to the mixture, followed by extraction with ethyl acetate. After drying over sodium sulfate, the solvent was evaporated to obtain the title compound (20 mg) as dark orange powder.
¹H-NMR (400 MHz, DMSO-d₆) δ: 6.63 (2H, d, J=7.8 Hz), 7.06 (2H, d, J=8.5 Hz), 7.37 (2H, d, J=7.8 Hz), 7.39 (1H, s), 7.53 (2H, d, J=8.5 Hz), 7.75 (1H, s), 8.29 (1H, s), 10.21 (1H, br s).
FAB-MS m/z: 279 (M+H)⁺

### Example 96

### 4-[4-(Oxazol-5-yl)phenylhydrazonomethyl]benzenesulfonamide

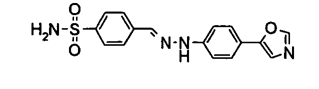

By using the compound obtained in Reference Example 107 and carrying out the operation in the same manner as in Example 35, the title compound was obtained as a yellow solid.
¹H-NMR (400 MHz, DMSO-d₆) δ: 7.19 (2H, d, J=8.5 Hz), 7.36 (2H, br s), 7.47 (1H, s), 7.60 (2H, d, J=8.5 Hz), 7.82 (4H, s), 7.93 (1H, s), 8.34 (1H, s), 10.86 (1H, s).
ESI-MS m/z: 343 (M+H)⁺

### Example 97

### N-{4-[4-(Oxazol-5-yl)phenylhydrazonomethyl]phenyl}methanesulfonamide

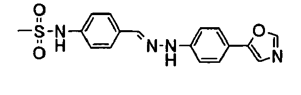

By using the compound obtained in Reference Example 110 and carrying out the operation in the same manner as in Example 35, the title compound was obtained as an orange solid.
¹H-NMR (400 MHz, DMSO-d₆) δ: 3.02 (3H, s), 7.13 (2H, d, J=8.5 Hz), 7.23 (2H, d, J=8.5 Hz), 7.43 (1H, s), 7.57 (2H, d, J=8.5 Hz), 7.63 (2H, d, J=8.5 Hz), 7.86 (1H, s), 8.32 (1H, s), 9.87 (1H, br s), 10.53 (1H, s).
ESI-MS m/z : 357 (M+H)⁺.

### Example 98

### N-{4-[4-(Oxazol-5-yl)phenylhydrazonomethyl]phenyl}-N',N'-dimethylsulfonamide

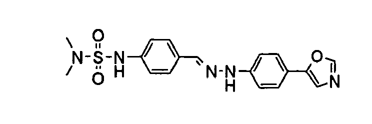

By using the compound obtained in Reference Example 113 and carrying out the operation in the same manner as in Example 35, the title compound was obtained as an orange solid.
¹H-NMR (400 MHz, DMSO-d₆) δ: 2.71 (6H, s), 7.12 (2H, d, J=8.8 Hz), 7.22 (2H, d, J=8.8 Hz), 7.43 (1H, s), 7.57 (2H, d, J=8.8 Hz), 7.59 (2H, d, J=8.8 Hz), 7.84 (1H, s), 8.32 (1H, s), 10.00 (1H, br s), 10.50 (1H, s).
ESI-MS m/z : 386 (M+H)⁺.

### Example 99

### 4-[2-(N,N-Dimethylamino)ethoxy]benzaldehyde 4-(oxazol-5-yl)phenylhydrazone

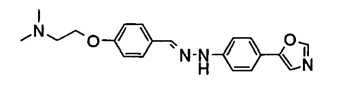

By using the compound obtained in Reference Example 110 and carrying out the operation in the same manner as in Example 35, the title compound was obtained as a brown solid.
Anal.Calcd for C₂₁H₂₂N₄O₂.1.15HCl.1.35H₂O:C,57.65;H,5.95;Cl,9.78;N,13.45.
Found:C,57.71;H,5.95;C1,9.68;N,13.77.

### Example 100

### 2-{4-[4-(Oxazol-5-yl)phenylhydrazonomethyl]phenoxy}acetamide

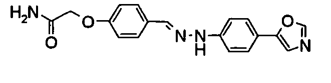

4-Hydroxybenzaldehyde (1.22 g), 2-iodoacetamide (1.85 g) and potassium carbonate (2.76 g) in acetone (40 ml) were heated under reflux for 2 hours. The insoluble material was filtered, and the filtrate was concentrated to obtain the desired compound (1.79 g) as white powder. The compound was used in the subsequent reaction without purification.

4-(Oxazol-5-yl)phenylhydrazine (180 mg) and the above-described compound (180 mg) were dissolved in ethanol (24 ml) and heated under reflex for 4 hours. The solvent was evaporated, the residue was purified by flash silica gel column chromatography, and the title compound (70 mg) was obtained as pale yellow powder from the ethyl acetate eluate.
¹H-NMR (400 MHz, DMSO-d₆) δ: 4.45 (2H, s), 6.97 (2H, d, J=8.8 Hz), 7.10 (2H, d, J=8.8 Hz), 7.40 (1H, br s), 7.42 (1H, s), 7.54 (1H, s), 7.55 (2H, d, J=8.8 Hz), 7.60 (2H, d, J=8.8 Hz), 7.85 (1H, s), 8.31 (1H, s), 10.44 (1H, s).
FAB-MS m/z: 337 (N4+H)⁺.

### Example 101

### N,N-Dimethyl-2-{4-[4-(oxazol-5-yl)phenylhydrazonomethyl]phenoxy}acetamide

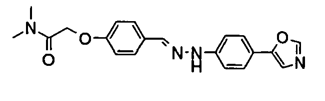

4-Hydroxybenzaldehyde (1.22 g), 2-chloro-*N*,*N*-dimethylacetamide (1.22 g) and potassium carbonate (1.38 g) in acetone (40 ml) were heated under reflux for 3 hours. The insoluble material was filtered, and the filtrate was concentrated to obtain a residue (2.07 g) as pale yellow powder. The residue was used in the subsequent reaction without purification.

4-(Oxazol-5-yl)phenylhydrazine (180 mg) and the above-described compound (180 mg) were dissolved in ethanol (24 ml) and heated under reflex for 4 hours. The solvent was evaporated, the residue was purified by flash silica gel column chromatography, and the title compound (70 mg) was obtained as pale yellow powder from the ethyl acetate eluate.
¹H-NMR (400 MHz, DMSO-d₆) δ: 2.84 (3H, s), 2.99 (3H, s), 4.83 (2H, s), 6.93 (2H, d, J=8.8 Hz), 7.10 (2H, d, J=8.8 Hz), 7.41 (1H, s), 7.55 (2H, d, J=8.6 Hz), 7.57 (2H, d, J=8.6 Hz), 7.84 (1H, s), 8.30 (1H, s), 10.42 (1H, s).
FAB-MS m/z: 365 (N4+H)⁺.

### Example 102

### tert-Butyl {4-[4-(oxazol-5-yl)phenylhydrazonomethyl]phenoxy}acetate

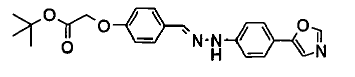

4-Hydroxybenzaldehyde (1.22 g), 2-bromoacetic acid *tert*-butyl ester (1.95 g) and potassium carbonate (2.76 g) in DMF (24 ml) were heated at 90°C for 2 hours. The mixture was extracted with ethyl acetate and washed with 1 N hydrochloric acid twice. The ethyl acetate layer was dried over anhydrous sodium sulfate and then concentrated under reduced pressure to obtain the desired compound (2.34 g) as colorless crystalline powder. The compound was directly used in the subsequent reaction without purification.

4-(Oxazol-5-yl)phenylhydrazine (120 mg) and the above-described compound (160 mg) were dissolved in ethanol (24 ml) and heated under reflex for 4 hours. The solvent was evaporated, the residue was separated and purified by flash silica gel column chromatography, and the title compound (210 mg) was obtained as pale yellow powder from the eluate of n-hexane: ethyl acetate =1:1.
¹H-NMR (400 MHz, DMSO-d₆) δ: 1.42 (9H, s), 4.67 (2H, s), 6.92 (2H, d, J=8.8 Hz), 7.10 (2H, d, J=8.8 Hz), 7.42 (1H, s), 7.55 (2H, d, J=8.8 Hz), 7.59 (2H, d, J=8.8 Hz), 7.85 (1H, s), 8.30 (1H, s), 10.44 (1H, s).
FAB-MS m/z: 394 (M+H)⁺.

### Example 103

### 4-[4-(Oxazol-5-yl)phenylhydrazonomethyl]phenoxyacetic acid

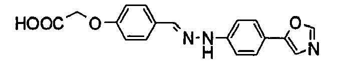

*tert-*Butyl {4-[4-(oxazol-5-yl)phenylhydrazonomethyl]phenoxy}acetate (140 mg) was dissolved in dichloromethane (12 ml), trifluoroacetic acid (12 ml) was added to the solution and stirred at room temperature for 1 hour. The solvent wad evaporated to obtain the title compound (190 mg) as yellowish brown powder.
¹H-NMR (400 MHz, DMSO-d₆) δ: 4.70 (2H, s), 6.93 (2H, d, J=8.5 Hz), 7.10 (2H, d, J=8.5 Hz), 7.41 (1H, s), 7.55 (2H, d, J=8.8 Hz), 7.58 (2H, d, J=8.8 Hz), 7.84 (1H, s), 8.30 (1H, s), 10.43 (1H, s).

### Example 104

### Methyl 2-hydroxy-5-[4-(oxazol-5-yl)phenylhydrazonomethyl]benzoate

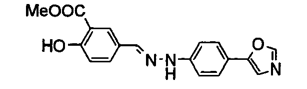

By using the compound obtained in Reference Example 115 and carrying out the operation in the same manner as in Example 35, the title compound was obtained as a red solid.
¹H-NMR (400 MHz, DMSO-d₆) δ: 3.29 (3H, s), 7.04 (1H, d, J=8.5 Hz), 7.12 (2H, d, J=8.1 Hz), 7.43 (1H, s), 7.57 (2H, d, J=8.1 Hz), 7.87 (1H, s), 7.88 (1H, d, J=8.5 Hz), 7.99 (1H, s), 8.32 (1H, s), 10.52 (1H, s), 10.60 (1H, br s).
ESI-MS m/z: 338 (M+H)⁺.

### Example 105

### Methyl 2-hydroxy-3-iodo-5-[4-(oxazol-5-yl)phenylhydrazonomethyl]benzoate

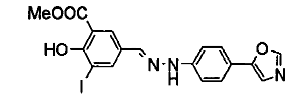

By using the compound obtained in Reference Example 116 and carrying out the operation in the same manner as in Example 35, the title compound was obtained as a red solid.
¹H-NMR (400 MHz, DMSO-d₆) δ: 3.97 (3H, s), 7.14 (2H, d, J=8.5 Hz), 7.43 (1H, s), 7.58 (2H, d, J=8.5 Hz), 7.83 (1H, s), 8.06 (1H, d, J=1.9 Hz), 8.32 (1H, s), 8.36 (1H, d, J=1.8 Hz), 10.63 (1H, s), 11.38 (1H, s).
ESI-MS m/z: 464 (N4+H)⁺.

### Example 106

### 2-Dimethylamino-N-{4-[4-(oxazol-5-yl)phenylhydrazonomethyl]phenyl}acetamide

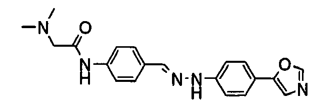

By using the compound obtained in Reference Example 118 and carrying out the operation in the same manner as in Example 35, the title compound was obtained as a yellow solid.
¹H-NMR (400 MHz, DMSO-d₆) δ: 2.28 (6H, s), 3.32 (2H, s), 7.13 (2H, d, J=8.5 Hz), 7.43 (1H, s), 7.57 (2H, d, J=8.8 Hz), 7.60 (2H, d, J=8.5 Hz), 7.70 (2H, d, J=8.8 Hz), 7.84 (1H, s), 8.32 (1H, s), 9.81 (1H, s), 10.51 (1H, s).
ESI-MS m/z: 364 (M+H)⁺.

### Example 107

### 4-(N-Methylaminomethyl)benzaldehyde 4-(oxazol-5-yl)phenylhydrazone

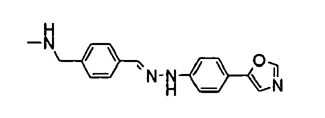

By using the compound obtained in Reference Example 122 and carrying out the operation in the same manner as in Example 47, the title compound was obtained as a yellow solid.
¹H-NMR (400 MHz, DMSO-d₆) δ: 2.27 (3H, s), 3.66 (2H, s), 7.14 (2H, d, J=8.1 Hz), 7.34 (2H, d, J=8.1 Hz), 7.44 (1H, s), 7.58 (2H, d, J=8.3 Hz), 7.61 (2H, d, J=8.3 Hz), 7.89 (1H, s), 8.33 (1H, s), 10.55 (1H, s).
ESI-MS m/z: 307 (M+H)⁺.

### Example 108

### 3-Iodo-4-(piperazin-1-yl)benzaldehyde 4-(oxazol-5-yl)phenylhydrazone

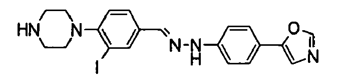

By using the compound obtained in Reference Example 128 and carrying out the operation in the same manner as in Example 47, the title compound was obtained as a yellow solid.
¹H-NMR (400 MHz, DMSO-d₆) δ: 2.87 (8H, br s), 7.10 (1H, d, J=8.3 Hz), 7.13 (2H, d, J=8.8 Hz), 7.43 (1H, s), 7.58 (2H, d, J=8.8 Hz), 7.65 (1H, dd, J=2.0 Hz, 8.3 Hz), 7.80 (1H, s), 8.13 (1H, d, J=2.0 Hz), 8.32 (1H, s), 10.59 (1H, s).
ESI-MS m/z: 474 (M+H)⁺.

### Example 109

### 3-Iodo-4-(N-methylaminomethyl)benzaldehyde 4-(oxazol-5-yl)phenylhydrazone

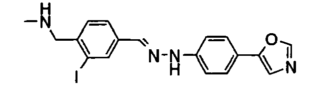

*tert-Butyl* (4-formyl-2-iodobenzyl)methylcarbamate (430 mg) was added to an ethanol solution (10 ml) of 4-(oxazol-5-yl)phenylhydrazine (200 mg), followed by heating overnight under reflux. The residue obtained by evaporating the solvent was purified by silica gel column chromatography, and the fraction obtained from the eluate of n-hexane:ethyl acetate = 2:1 was concentrated under reduced pressure to obtain the desired compound (568 mg) as yellow amorphous. The compound was directly used in the subsequent reaction without further carrying out purification.

A saturated hydrochloric acid methanol solution (5 ml) was added at 0°C to a methanol solution (3 ml) of the above-described amorphous (568 mg), followed by stirring overnight at room temperature. The residue obtained by evaporating the solvent was alkalified by adding a saturated aqueous sodium bicarbonate solution, extracted with a chloroform-methanol (10:1) mixed solvent and then dried over sodium sulfate. The residue obtained by evaporating the solvent was purified by silica gel column chromatography, the fraction obtained from the lower layer eluate of chloroform:methanol:water = 15:3:1 mixed solution was concentrated under reduced pressure, and the residue was collected by filtration, washed with diethyl ether and dried to obtain the title compound (267 mg) as a yellow solid.
¹H-NMR (400 MHz, DMSO-d₆) δ: 2.32 (3H, s), 3.63 (2H, s), 7.16 (2H, d, J=8.8 Hz), 7.43 (1H, d, J=7.8 Hz), 7.44 (1H, s), 7.59 (2H, d, J=8.8 Hz), 7.67 (1H, d, J=7.8 Hz), 7.83 (1H, s), 8.11 (1H, s), 8.33 (1H, s), 10.68 (1H, s).
ESI-MS m/z: 433 (M+H)⁺.

### Example 110

### Thiazole-5-carboxyaldehyde 4-(oxazol-5-yl)phenylhydrazone

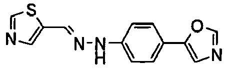

By using thiazole-5-carboxyaldehyde and carrying out the operation in the same manner as in Example 35, the title compound was obtained as yellowish brown crystals.
¹H-NMR (400 MHz, CDCl₃) δ: 7.08 (2H, d, J=8.5 Hz), 7.45 (1H, s), 7.59 (2H, d, J=8.5 Hz), 8.07 (1H, s), 8.16 (1H, s), 8.33 (1H, s), 9.02 (1H, s), 10.79 (1H, s).
FAB-MS m/z: 271 (M+H)⁺.

### Example 111

### 4-(1-Aminoethyl)thiazole-2-carboxyaldehyde 4-(oxazol-5-yl)phenylhydrazone

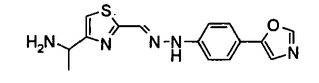

*tert-Butyl* (1-{2-[4-(Oxazol-5-yl)phenylhydrazonomethyl]thiazol-4-yl}ethyl)carbamate (120 mg) was dissolved in dichloromethane (20 ml), trifluoroacetic acid (20 ml) was added to the solution and stirred at room temperature for 1 hour. The solvent was evaporated to obtain the title compound (190 mg) as yellowish brown powder.
¹H-NMR (400 MHz, DMSO-d₆) δ: 1.32 (3H, d, J=7.5 Hz), 4.05 (1H, q, J=7.5 Hz), 7.13 (2H, d, J=8.5 Hz), 7.30 (1H, s), 7.48 (1H, s), 7.63 (2H, d, J=8.5 Hz), 8.04 (1H, s), 8.35 (1H, s), 11.08 (1H, s).
FAB-MS m/z: 414 (M+H)⁺.

### Example 112

### 4-Hydroxymethylthiazole-2-carboxyaldehyde 4-(oxazol-5-yl)phenylhydrazone

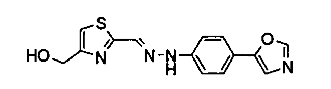

By using the compound obtained in Reference Example 137 and carrying out the operation in the same manner as in Example 47, the title compound was obtained as a yellow solid.
¹H-NMR (400 MHz, DMSO-d₆)δ:4.55 (2H, s), 7.14 (2H, d, J=8.8 Hz), 7.34 (1H, s), 7.47 (1H, s), 7.63 (2H, d, J=8.8 Hz), 8.05 (1H, s), 8.34 (1H, s), 11.09 (1H, s).
ESI-MS m/z: 300 M⁺.

### Example 113

### 2-Hydroxymethylthiazole-4-carboxyaldehyde 4-(oxazol-5-yl)phenylhydrazone

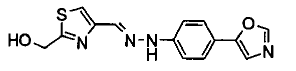

By using the compound obtained in Reference Example 139 and carrying out the operation in the same manner as in Example 35, the title compound was obtained as a yellow solid.
¹H-NMR (400 MHz, DMSO-d₆) δ: 4.75 (2H, d, J=5.9 Hz), 6.10 (1H, t, J=5.9 Hz), 7.12 (2H, d, J=8.5 Hz), 7.44 (1H, s), 7.58 (2H, d, J=8.5 Hz), 7.80 (1H, s), 7.97 (1H, s), 8.33 (1H, s), 10.62 (1H, s).
ESI-MS m/z: 300 M⁺.

### Example 114

### 2-Dimethylamino-4-pyridinecarboxyaldehyde 4-(oxazol-5-yl)phenylhydrazone

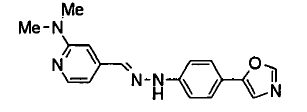

By using the compound obtained in Reference Example 141 and carrying out the operation in the same manner as in Example 35, the title compound was obtained as a red solid.
¹H-NMR (400 MHz, DMSO-d₆) δ: 3.66 (6H, s), 6.77 (1H, s), 6.91 (1H, d, J=8.3 Hz), 7.18 (2H, d, J=8.5 Hz), 7.47 (1H, s), 7.61 (2H, d, J=8.5 Hz), 7.79 (1H, s), 8.05 (1H, d, J=8.3 Hz), 8.34 (1H, s), 10.84 (1H, s).
ESI-MS m/z: 308 (M+H)⁺.

### Example 115

### 6-Fluoro-3-pyridinecarboxyaldehyde 4-(oxazol-5-yl)phenylhydrazone

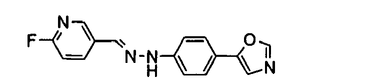

By using the compound obtained in Reference Example 144 and carrying out the operation in the same manner as in Example 35, the title compound was obtained as a red solid.
¹H-NMR (400 MHz, DMSO-d₆) δ: 7.18 (2H, d, J=8.5 Hz), 7.22 (1H, dd, J=2.5 Hz, 8.5 Hz), 7.46 (1H, s), 7.59 (2H, d, J=8.5 Hz), 7.94 (1H, s), 8.32 (1H, dt, J=2.5 Hz, 8.5 Hz), 8.33 (1H, s), 8.46 (1H, s), 10.80 (1H, s).
ESI-MS m/z: 283 (M+H)⁺.

### Example 116

### 6-Dimethylamino-3-pyridinecarboxyaldehyde 4-(oxazol-5-yl)phenylhydrazone

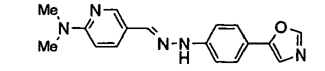

By using the compound obtained in Reference Example 141 and carrying out the operation in the same manner as in Example 35, the title compound was obtained as a yellow solid.
¹H-NMR (400 MHz, DMSO-d₆) δ: 3.07 (6H, s), 6.70 (1H, d, J=8.3 Hz), 7.22 (2H, d, J=7.3 Hz), 7.41 (1H, s), 7.55 (2H, d, J=7. Hz), 7. 81 (1H, s), 7.88 (1H, d, J=8.3 Hz), 8.24 (1H, s), 8.30 (1H, s), 10.33 (1H, s).
ESI-MS m/z: 308 (M+H)⁺.

### Example 117

### 6-(4-Methylpiperazin-1-yl)-3-pyridinecarboxyaldehyde 4-(oxazol-5-yl)phenylhydrazone

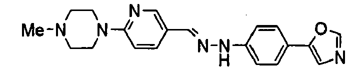

By using the compound obtained in Reference Example 148 and carrying out the operation in the same manner as in Example 35, the title compound was obtained as a yellow solid.
¹H-NMR (400 MHz, DMSO-d₆) δ: 2.22 (3H, s), 2.39 (4H, t, J=4.9 Hz), 3.54 (4H, t, J=4.9 Hz), 6.88 (1H, d, J=8.8 Hz), 7.09 (2H, d, J=8.5 Hz), 7.41 (1H, s), 7.55 (2H, d, J=8.5 Hz), 7.81 (1H, s), 7.90 (1H, dd, J=2.2 Hz, 8.8 Hz), 8.27 (1H, d, J=2.2 Hz), 8.30 (1H, s), 10.39 (1H, s).
ESI-MS m/z: 363 (M+H)⁺.

### Example 118

### 1H-Imidazol-2-ylcarboxyaldehyde 4-(oxazol-5-yl)phenylhydrazone

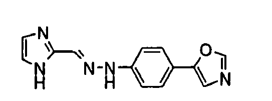

1-Trityl-1H-imidazol-2-ylcarboxyaldehyde (290 mg) was added to an ethanol solution (10 ml) of 4-(oxazol-5-yl)phenylhydrazine (150 mg), followed by heating overnight under reflux. The residue obtained by evaporating the solvent was collected by filtration and washed with diethyl ether to obtain a hydrazone derivative (152 mg) as a mixture of isomers. The mixture was used in the subsequent reaction without purification.

A saturated hydrochloric acid methanol solution (3 ml) was added at 0°C to a methanol solution (5 ml) of the above-described compound (152 mg), followed by stirring overnight at room temperature. The residue obtained by evaporating the solvent was alkalified by adding a saturated aqueous sodium bicarbonate solution, extracted with a chloroform-methanol (10:1) mixed solvent and then dried over sodium sulfate. The residue obtained by evaporating the solvent was purified by silica gel column chromatography, the fraction obtained from the eluate of dichloromethane: methanol =20:1 was concentrated under reduced pressure, and the residue was collected by filtration, washed with diethyl ether and dried to obtain the title compound (38 mg) as a yellow solid.
¹H-NMR (400 MHz, DMSO-d₆) δ: 6.98 (1H, s), 7.19 (1H, s), 7.20 (2H, d, J=8.5 Hz), 7.46 (1H, s), 7.58 (2H, d, J=8.5 Hz), 7.77 (1H, s), 8.32 (1H, s), 10.63 (1H, s), 12.35 (1H, s).
ESI-MS m/z: 474 M+H)⁺.

### Example 119

### 4-(1-Aminoethyl)benzaldehyde 4-(oxazol-5-yl)phenylhydrazone

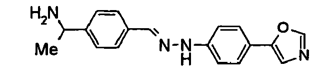

By using the compound obtained in Reference Example 153 and carrying out the operation in the same manner as in Example 47, the title compound was obtained as a yellow solid.
¹H-NMR (400 MHz, DMSO-d₆) δ: 1.25 (3H, d, J=6.6 Hz), 3.32 (2H, s), 3.99 (1H, q, J=6.6 Hz), 7.13 (2H, d, J=8 . 8 Hz), 7. 3 9 (2H, d, J=8 .1 Hz), 7.44 (1H, s), 7.58 (2H, d, J=8.8 Hz), 7.59 (2H, d, J=8.1 Hz), 7.89 (1H, s), 8.32 (1H, s), 10.53 (1H, s).
ESI-MS m/z: 307 (M+H)⁺.

### Example 120

### 2-Hydroxy-3-iodo-5-[4-(oxazol-5-yl)phenylhydrazinomethyl]benzoic acid.

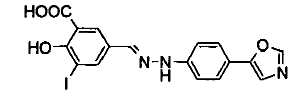

By using the compound obtained in Reference Example 121 and carrying out the operation in the same manner as in Example 35, the title compound was obtained as a red solid.
¹H-NMR (400 MHz, DMSO-d₆) δ: 7.13 (2H, d, J=8.8 Hz), 7.42 (1H, s), 7.58 (2H, d, J=8.8 Hz), 7.82 (1H, s), 8.05 (1H, d, J=2.0 Hz), 8.31 (1H, d, J=2.0 Hz), 8.32 (1H, s), 10.57 (1H, s).
ESI-MS m/z: 450 (M+H)⁺.

### Example 121

### 1-Benzyl-1,2,3,6-tetrahydropyridine-4-carboxyaldehyde 4-(oxazol-5-yl)phenylhydrazone

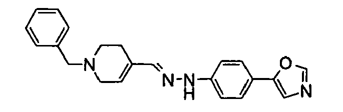

By using the compound obtained in Reference Example 154 and carrying out the operation in the same manner as in Example 35, the title compound was obtained as orange powder.
¹H-NMR (400 MHz, DMSO-d₆) δ: 2.38-2.42 (2H, m), 2.58 (2H, t, J=5.6 Hz), 3.03-3.07 (2H, m), 3.59 (2H, s), 5.92 (1H, s), 7.00 (2H, d, J=8.8 Hz), 7.24-7.28 (1H, m), 7.33 (4H, d, J=4.4 Hz), 7.40 (1H, s), 7.53 (2H, d, J=8.8 Hz), 7.57 (1H, s), 8.30 (1H, s), 10.24 (1H, s).
ESI-MS m/z: 359 (M+H)⁺.

### Example 122

### 6-Iodoimidazo[1,2-a]pyridine-2-carboxyaldehyde 4-(oxazol-5-yl)phenylhydrazone

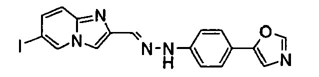

By using the compound obtained in Reference Example 158 and carrying out the operation in the same manner as in Example 35, the title compound was obtained as a brown solid.
¹H-NMR (400 MHz, DMSO-d₆) δ: 7.12 (2H, d, J=8.8 Hz), 7.42 (2H, d, J=8.8 Hz), 7.44 (1H, s), 7.50-7.64 (2H, m), 7.96 (1H, s), 8.16 (1H, s), 8.89 (1H, s), 10.85 (1H, s).
ESI-MS m/z: 430 (M+H)⁺.

### Example 123

### 4-(4-Dimethylaminopiperidin-1-yl)-3-iodobenzaldehyde 4-(oxazol-5-yl)phenylhydrazone

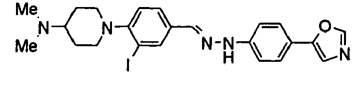

By using the compound obtained in Reference Example 159 and carrying out the operation in the same manner as in Example 35, the title compound was obtained as yellow amorphous.
¹H-NMR (400 MHz, DMSO-d₆) δ:1.60 (2H, q, J=10.4 Hz), 1.87 (2H, d, J=11.2 Hz), 2.23 (6H, s), 2.64 (2H, t, J=11.2 Hz), 3.25 (2H, d, J=10.5 Hz), 3.38 (1H, q, J=11.2 Hz), 7.11 (1H, d, J=8.5 Hz), 7.13 (2H, d, J=8.5 Hz), 7.43 (1H, s), 7.58 (2H, d, J=8.5 Hz), 7.63 (1H, d, J=8.5 Hz), 7.80 (1H, s), 8.13 (1H, s), 8.32 (1H, s), 10.59 (1H, s).
ESI-MS m/z: 516 (M+H)⁺.

### Example 124

### (Z) form of 2-[4-(oxazol-5-yl)phenylhydrazono]pyridin-4-ylacetic acid ethyl ester

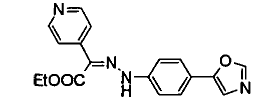

Oxopyridin-4-ylacetic acid ethyl ester (438 mg) was dissolved in 60%aqueous solution (15 ml) of acetic acid, 4-(oxazol-5-yl)phenylhydrazine (164 mg) was added to the solution at room temperature and stirred for 2 hours. The solvent was evaporated, and the residue was diluted with chloroform:methanol = 9:1 (90 ml) and washed with a saturated aqueous sodium bicarbonate solution. After drying over anhydrous sodium sulfate, the solid obtained by evaporating the solvent was recrystallized from ethanol to obtain the title compound (240 mg) as yellowish brown needle crystals.
¹H-NMR (400 MHz, CDCl₃) δ: 1.42 (3H, t, J=7.1 Hz), 4.41 (2H, q, J=7.1 Hz), 7.29 (1H, s), 7.36 (2H, d, J=8.8 Hz), 7.64 (4H, m), 7.89 (1H, s), 8.60 (2H, d, J=5.9 Hz), 12.74 (1H, s).
ESI-MS m/z: 337 (M+H)⁺

### Example 125

### (Z) form of 2-[4-(oxazol-5-yl)phenylhydrazono]pyridin-4-ylacetic acid hydrochloride

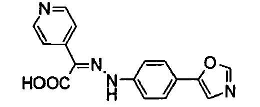

(Z) form of 2-[4-(oxazol-5-yl)phenylhydrazono]pyridin-4-ylacetic acid ethyl ester (204 mg) was dissolved in THF (6 ml), 1 N sodium hydroxide (1 ml) was added to the solution at room temperature and stirred for 2 hours. After adding the aqueous solution of 1 N hydrochloric acid to the reaction mixture, THF was evaporated, and the insoluble material was collected by filtration and washed with water, ethanol and diethyl ether to obtain the free form (163 mg) as a red solid. To an ethanol suspension (5 ml) of the free form (48.1 mg) was added 1 N hydrochloric acid ethanol (0.2 ml) and stirred at room temperature for 3 hours. The solvent was evaporated and the thus obtained solid was washed with diethyl ether to obtain the title compound (43.9 mg) as a red solid.
¹H-NMR (400 MHz, DMSO-d₆) δ: 7.65 (1H, s), 7.66 (2H, d, J=8.8 Hz), 7.75 (2H, d, J=8.8 Hz), 8.36 (2H, d, J=6.8 Hz), 8.42 (1H, s), 8.78 (2H, d, J=6.8 Hz), 13.0 (1H, s).
ESI-MS m/z: 309 (M+H)⁺

### Example 126

### 2-[4-(Oxazol-5-yl)phenylhydrazono]pyridin-4-ylacetamide, a mixture of (E) and (Z) isomers (1:1)

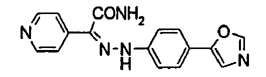

(*Z*) form of 2-[4-(oxazol-5-yl)phenylhydrazono]pyridin-4-ylacetic acid hydrochloride (96 mg) was dissolved in dichloromethane (8 ml) and DMF (2 ml), NMM (76.6 µl), ammonium chloride (17.9 mg) and HOBt (51.2 mg) were added to the mixture at 0°C and stirred for 15 minutes and then EDC·HCl (64.1 mg) was added to the mixture. After stirring at room temperature for 15 hours, the solvent was evaporated, and the residue was diluted with ethyl acetate (90 ml) and washed with a saturated aqueous sodium bicarbonate solution and saturated brine (45 ml for each). After drying the organic layer over anhydrous sodium sulfate, the solvent was evaporated, the residue was purified by flash silica gel column chromatography (chloroform: methanol = 20: 1), and the thus obtained solid was washed with diethyl ether to obtain the title compound (53 mg) as a yellow solid.
¹H-NMR (400 MHz, DMSO-d₆) δ: 7.21 (0.5H, s), 7.30 (1H, d, J=4.7 Hz), 7.43 (1H, d, J=8.0 Hz), 7.52 (2H, m), 7.57 (2H, d, J=7.3 Hz), 7.63 (1H, d, J=8.0 Hz), 7.73 (0.5H, br s), 8.01 (0.5H, br s), 8.13 (0.5H, br s), 8.34 (1H, d, J=4.4 Hz), 8.58 (1H, d, J=4.9 Hz), 8.68 (1H, d, J=4.7 Hz), 9.85 (0.5H, s), 11.01 (0.5H, s).
ESI-MS m/z: 308 (M+H)⁺

### Example 127

### N-(2-Hydroxymethyl)-2-[4-(oxazol-5-yl)phenylhydrazono]pyridin-4-ylacetamide, mixture of (E) and (Z) isomers (3:7)

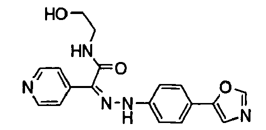

2-[4-(Oxazol-5-yl)phenylhydrazono]pyridin-4-ylacetic acid (62.5 mg) was dissolved in DMF (8 ml), NMM (26.7 µl), ethanolamine (14.6 µl) and HOBt (37.3 mg) were added to the solution at room temperature and then EDC·HCl (46.6 mg) was added to the solution and stirred at room temperature for 2 hours. After evaporation of the solvent, the residue was diluted with chloroform:methanol = 9:1 (60 ml), washed with a saturated aqueous ammonium chloride solution, a saturated aqueous sodium bicarbonate solution and saturated brine (20 ml for each) and dried over anhydrous sodium sulfate. The solvent was evaporated, the residue was purified by flash silica gel column chromatography (chloroform:methanol = 20:1 to 9:1), and the thus obtained solid was washed with diethyl ether to obtain the title compound (47.6 mg) as a yellow solid, as a mixture ofE and Z isomers (3:7).
¹H-NMR (400 MHz, CDCl₃) δ: 3.61 (1.4H, m), 3.83 (0.6H, m), 6.32 (0.7H, br s), 7.25-7.33 (3.0H, m), 7.45 (0.3H, br s), 7.52 (2H, d, J=5.9 Hz), 7.60 (2.OH, d, J=8.5 Hz), 7.87 (1H, s), 8.02 (0.3H, s), 8.66 (1.4H, d, J=5.6 Hz), 8.82 (0.6H, d, J=5.9 Hz), 12.84 (0.7H, s).
ESI-MS m/z: 355 (M+H)⁺

### Example 128

### 4-Pyridinecarboxyaldehyde 4-(oxazol-5-yl)phenylhydrazonyl chloride

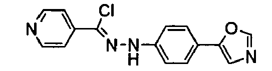

Isonicotinic acid *N'*-4-(oxazol-5-yl)phenylhydrazide (78.3 mg) was dissolved in carbon tetrachloride (10 ml) and acetonitrile (3 ml), triphenylphosphine (183 mg) was added to the solution and stirred at 60°C for 2 hours. The solvent was evaporated, and the thus obtained solid was washed with acetone to obtain the title compound (72.6 mg) as a yellow solid.
¹H-NMR (400 MHz, DMSO-d₆) δ: 6.87 (2H, d, J=8.8 Hz), 7.41 (1H, s), 7.52 (2H, d, J=8.8 Hz), 8.04 (2H, d, J=6.4 Hz), 8.30 (1H, s), 8.89 (2H, d, J=6.2 Hz), 10.89 (1H, s).
ESI-MS m/z: 299 (M+H)⁺

### Example 129

### 4-(Oxazol-5-yl)phenylhydrazonophenylacetonitrile

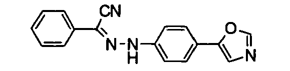

Phenylcyanoacetic acid ethyl ester (1 ml) was added dropwise to 8% potassium hydroxide/ethanol suspension (3.8 ml) of 4-(oxazol-5-yl)benzenediazonium tetrafluoroborate (400 mg) at 0°C, followed by stirring at the same temperature for 2 hours. To the reaction mixture was added water (200 ml) and stirred for 30 minutes, and the insoluble material was colleted by filtration and washed with water. After drying, the thus obtained solid was washed with diethyl ether and hexane to obtain the title compound (403 mg) as a yellow solid.
¹H-NMR (400 MHz, CDCl₃) δ: 7.29 (1H, s), 7.31 (2H, d, J=8.8 Hz), 7.39-7.46 (3H, m), 7.66 (2H, d, J=8.9 Hz), 7.82 (2H, d, J=7.1 Hz), 7.90 (1H, s), 8.82 (1H, s).
ESI-MS m/z: 289 (M+H)⁺

### Example 130

### Benzamide 4-(oxazol-5-yl)phenylhydrazone hydrochloride

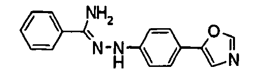

Ethyl benzimidate hydrochloride (515 mg) was added to a pyridine (8 ml) solution of 4-(oxazol-5-yl)phenylhydrazine (405 mg) at room temperature, followed by stirring for 2 hours. Diethyl ether (10 ml) was added to the reaction solution, and the pale red insoluble material was collected by filtration and washed with 1 N hydrochloric acid/ethanol. By further washing with ethanol and diethyl ether, the title compound (339 mg) was obtained as a white solid.
¹H-NMR (400 MHz, DMSO-d₆) δ: 7.00 (2H, d, J=8.6 Hz), 7.52 (1H, s), 7.55-7.68 (4H, m), 7.78 (1H, t, J=7.1 Hz), 7.92 (2H, d, J=7.6 Hz), 8.36 (1H, s), 9.03 (1H, s), 9.58 (1H, br s), 9.92 (1H, br s), 11.79 (1H, br s).
ESI-MS m/z: 279 (M)⁺

### Example 131

### Propan-2-one 4-(oxazol-5-yl)phenylhydrazone

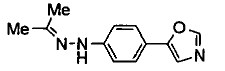

4-(Oxazol-5-yl)phenylhydrazine (200 mg) was dissolved in acetone (8 ml) and stirred for 10 minutes, and then the solvent was evaporated. The thus obtained solid was washed with diethyl ether to obtain the title compound (213 mg) as a yellow solid.
¹H-NMR (400 MHz, CDCl₃) δ: 1.90 (3H, s), 2.07 (3H, s), 7.00 (1H, s), 7.08 (2H, d, J=8.5 Hz), 7.19 (1H, s), 7.53 (2H, d, J=8.5 Hz), 7.84 (1H, s).
ESI-MS m/z: 216 (M+M)⁺

### Example 132

### 2-[4-(Oxazol-5-yl)phenylhydrazono]malononitrile

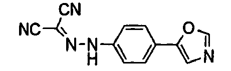

Sodium acetate (274 mg) was added to a mixed solution of methanol (3 ml) and water (6 ml) of malononitrile (106 mg) at 0°C, followed by stirring for 5 minutes, and then 4-(oxazol-5-yl)benzenediazonium tetrafluoroborate (345 mg) was added to the mixture. The mixture was stirred at 0°C for 30 minutes and further at room temperature for 30 minutes, and the insoluble material was collected by filtration and washed with water, ethanol and diethyl ether to obtain the title compound (229 mg) as a yellow solid.
¹H-NMR (400 MHz, DMSO-d₆) δ: 7.55 (2H, d, J=8.8 Hz), 7.67 (1H, s), 7.76 (2H, d, J=8.9 Hz), 8.44 (1H, s), 13.1 (1H, br s).
ESI-MS m/z: 238 (M+H)⁺

### Example 133

### 4-Pyridinecarboxyaldehyde 3-fluoro-4-(oxazol-5-yl)phenylhydrazone

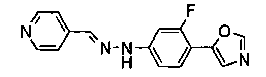

By using the compound obtained in Reference Example 167 and 4-pyridine carboxyaldehyde, and carrying out the operation in the same manner as in Example 35, the title compound was obtained as a red solid.
¹H-NMR (400 MHz, DMSO-d₆) δ: 7.02 (1H, dd, J=2.0 Hz, 8.3 Hz), 7.11 (1H, dd, J=2.0 Hz, 13.5 Hz), 7.35 (1H, d, J=3.4 Hz), 7.63-7.68 (3H, m), 7.89 (1H, s), 8.44 (1H, s), 8.57 (2H, d, J=6.1 Hz), 11.18 (1H, s).
ESI-MS m/z: 282 (M+).

### Example 134

### 4-(1-Aminoethyl)thiazole-2-carboxyaldehyde 3-iodo-4-(oxazol-5-yl)phenylhydrazone

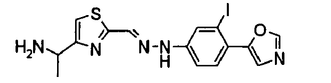

By using the compound obtained in Reference Example 168 and carrying out the operation in the same manner as in Example 47, the title compound was obtained as a yellow solid.
¹H-NMR (400 MHz, DMSO-d₆) δ: 1.32 (3H, d, J=6.3 Hz), 4.05 (1H, d, J=6.3 Hz), 7.15 (1H, dd, J=2.2 Hz, 8.5 Hz), 7.34 (1H, s), 7.48 (1H, d, J=8.5 Hz), 7.56 (1H, s), 7.67 (1H, d, J=2.2 Hz), 8.06 (1H, s), 8.46 (1H, s), 11.14 (1H, s).
ESI-MS m/z: 440 (M+H)⁺.

### Example 135

### 4-(Piperazin-1-yl)benzaldehyde 3-iodo-4-(oxazol-5-yl)phenylhydrazone

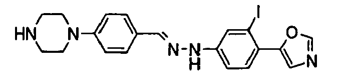

By using the compound obtained in Reference Example 168 and carrying out the operation in the same manner as in Example 47, the title compound was obtained as an orange solid.
¹H-NMR (400 MHz, DMSO-d₆) δ: 2.83 (4H, br s), 3.11 (4H, br s), 6.94 (2H, d, J=8.5 Hz), 7.10 (1H, d, J=7.6 Hz), 7.39 (1H, d, J=7.6 Hz), 7.50 (1H, s), 7.52 (2H, d, J=8.5 Hz), 7.64 (1H, s), 7.82 (1H, s), 8.42 (1H, s), 10.42 (1H, s).
ESI-MS m/z: 474 (M+H)⁺.

### Example 136

### 4-(N-Methylaminomethyl)benzaldehyde 3-iodo-4-(oxazol-5-yl)phenylhydrazone

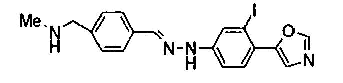

*tert*-Butyl (4-formylbenzyl)methylcarbamate (182 mg) was added to an ethanol solution (10 ml) of 3-iodo-4-(oxazol-5-yl)phenylhydrazine (220 mg), followed by heating overnight under reflux. The residue obtained by evaporating the solvent was purified by silica gel column chromatography, and the fraction obtained from the eluate of chloroform: methanol = 100:3 was concentrated under reduced pressure to obtain the title compound (113 mg) as yellow amorphous. The compound was directly used in the subsequent reaction.

A hydrochloric acid/methanol solution (5 ml) was added to a methanol solution (3 ml) of the above-described amorphous (110 mg) at 0°C, followed by stirring at room temperature for 1 hour. To the residue obtained by evaporating the solvent was added 1 N sodium hydroxide and extracted with chloroform-methanol (10:1, v/v). After drying the extract over sodium sulfate, the residue obtained by evaporating the solvent was purified by silica gel column chromatography, and the fraction obtained from the lower layer eluate of a mixed solution of chloroform:methanol:water = 15:3:1 was concentrated under reduced pressure to obtain the title compound (20 mg) as yellow amorphous.
¹H-NMR (400 MHz, DMSO-d₆) δ: 2.35 (3H, s), 3.82 (2H, s), 7.17 (1H, dd, J=2.2 Hz, 8.6 Hz), 7.37 (2H, d, J=7.5 Hz), 7.43 (1H, d, J=8.6 Hz), 7.52 (1H, s), 7.67 (2H, d, J=7.5 Hz), 7.69 (1H, d, J=2.2 Hz), 7.92 (1H, s), 8.43 (1H, s), 10.71 (1H, s).
ESI-MS m/z: 433 (M+H)⁺.

### Example 137

### 4-Pyridinecarboxyaldehyde 4-iodophenylhydrazone

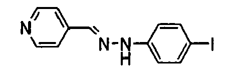

By using 4-iodophenylhydrazine and 4-pyridinecarboxyaldehyde, and carrying out the operation in the same manner as in Example 35, the title compound was obtained as a yellow solid.
¹H-NMR (400 MHz, CDCl₃) δ: 6.94 (2H, d, J=8.6 Hz), 7.48 (2H, d, J=6.1 Hz), 7.53 (2H, d, J=8.5 Hz), 7.68 (1H, s), 8.56 (2H, d, J=6.1 Hz), 9.52 (1H, s).
FAB-MS m/z: 324 (M)⁺.

### Example 138

### 4-Pyridinecarboxyaldehyde 4-(6-bromoimidazo[1,2-a]pyridin-2-yl)phenylhydrazone

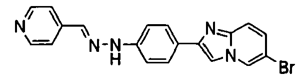

4-(6-Bromoimidazo[1,2-a]pyridin-2-yl)phenylamine (1.50 g) was dissolved in concentrated hydrochloric acid (10 ml) and water (30 ml), and an aqueous solution (5 ml) of sodium nitrite (431 mg) was slowly added dropwise to the solution at 0°C. After stirring for 30 minutes, a concentrated hydrochloric acid solution (5 ml) of tin chloride dihydrate (2.35 g) was added to the mixture, followed by stirring at room temperature for 1 hour. The reaction solution was alkalified by adding 28 wt% aqueous ammonia and extracted with chloroform:methanol = 9:1 (300 ml) twice. By drying the organic layer over anhydrous sodium sulfate and evaporating the solvent, a yellowish brown solid was obtained.

The above-described solid and 4-pyridinecarboxyaldehyde (419 µl) were dissolved in ethanol (20 ml) and heated under reflux for 15 hours. After evaporation of the solvent, the residue was purified by flash silica gel column chromatography (chloroform:methanol = 30:1 to 10:1), and the thus obtained solid was washed with diethyl ether to obtain the title compound (702 mg) as a yellow solid.
¹H-NMR (400 MHz, DMSO-d₆) δ: 7.19 (2H, d, J=8.5 Hz), 7.31 (1H, d, J=9.6 Hz), 7.52 (1H, d, J=9.5 Hz), 7.59 (2H, d, J=5.6 Hz), 7.83 (1H, s), 7.86 (2H, d, J=8.6 Hz), 8.22 (1H, s), 8.53 (2H, d, J=5.6 Hz), 8.84 (1H, s), 10. 93 (1H, s).
ESI-MS m/z: 392 M⁺

### Example 139

### 4-Pyridinecarboxyaldehyde 4-(6-chloroimidazo[1,2-a]pyridin-2-yl)phenylhydrazone

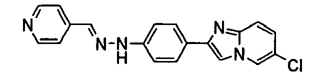

By using the compound obtained in Reference Example 174 and carrying out the operation in the same manner as in Example 35, the title compound was obtained as a yellow solid.
¹H-NMR (400 MHz, DMSO-d₆) δ: 7.19 (2H, d, J=8.8 Hz), 7.25 (1H, dd, J=9.5 Hz, 2.2 Hz), 7.56 (1H, s), 7.57 (1H, d, J=9.5 Hz), 7.59 (2H, d, J=5.9 Hz), 7.83 (1H, s), 7.85 (2H, d, J=8.8 Hz), 8.23 (1H, s), 8.53 (2H, d, J=6.1 Hz), 8.77 (1H, d, J=2.2 Hz), 10.93 (1H, s).
ESI-MS m/z: 348 (M+H)⁺

### Example 140

### 4-Pyridinecarboxyaldehyde 4-(6-fluoroimidazo[1,2=a]pyridin-2-yl)phenylhydrazone

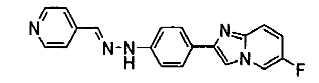

By using the compound obtained in Reference Example 176 and carrying out the operation in the same manner as in Example 138, the title compound was obtained as a yellow solid.
¹H-NMR (400 MHz, DMSO-d₆) δ: 7.26 (2H, d, J=8.3 Hz), 7.29 (1H, dd, J=4.6 Hz, 2.2 Hz), 7.57 (1H, s), 7.58 (2H, d, J=5.8 Hz), 7.82 (1H, s), 7.84 (2H, d, J=8.6 Hz), 8.25 (1H, s), 8.53 (2H, d, J=5.9 Hz), 8.71 (1H, dd, J=4.4 Hz, 2.4 Hz), 10.92 (1H, s).
ESI-MS m/z: 332 (M+H)⁺

### Example 141

### 4-Pyridinecarboxyaldehyde 4-(imidazo[2,1-b]thiazol-6-yl)phenylhydrazone

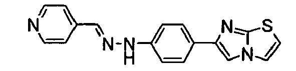

By using the compound obtained in Reference Example 178 and carrying out the operation in the same manner as in Example 138, the title compound was obtained as a yellow solid.
¹H-NMR (400 MHz, DMSO-d₆) δ: 7.16 (2H, d, J=8.5 Hz), 7.22 (1H, d, J=4.4 Hz), 7.59 (2H, d, J=5.4 Hz), 7. 73 (2H, d, J=8.3 Hz), 7.82 (1H, s), 7.91 (1H, d, J=4.4 Hz), 8.07 (1H, s), 8.53 (2H, d, J=5.4 Hz), 10.87 (1H, s).
FAB-MS m/z: 320 (M+H)⁺.

### Example 142

### 4-Pyridinecarboxyaldehyde 4-(imidazo[1,2-a]pyrimidin-2-yl)phenylhydrazone

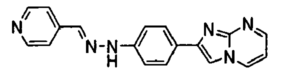

By using the compound obtained in Reference Example 180 and carrying out the operation in the same manner as in Example 35, the title compound was obtained as a yellowish brown solid.
¹H-NMR (400 MHz, DMSO-d₆) δ: 7.01 (1H, dd, J=6.6 Hz, 4.2 Hz), 7.21 (2H, d, J=8.8 Hz), 7.60 (2H, d, J=6.1 Hz), 7. 84 (1H, s), 7.90 (2H, d, J=8.5 Hz), 8.23 (1H, s), 8.47 (1H, dd, J=4.2 Hz, 2.0 Hz), 8.54 (2H, d, J=5.9 Hz), 8.91 (1H, dd, J=6.8 Hz, 2.0 Hz), 10.95 (1H, s).
ESI-MS m/z: 315 (M+H)⁺

### Example 143

### 4-Pyridinecarboxyaldehyde 4-(6-hydroxybenzothiazol-2-yl)phenylhydrazone

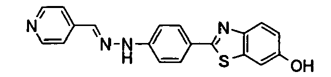

By using the compound obtained in Reference Example 183 and carrying out the operation in the same manner as in Example 138, the title compound was obtained as a yellowish brown solid.
¹H-NMR (400 MHz, DMSO-d₆) δ: 6.94 (1H, d, J=8.8 Hz), 7.24 (2H, d, J=8.6 Hz), 7.35 (1H, s), 7.62 (2H, d, J=4.7 Hz), 7.76 (1H, d, J=8.8 Hz), 7.89 (1H, s), 7.90 (2H, d, J=8.6 Hz), 8.56 (2H, d, J=4.7 Hz), 9.76 (1H, s), 11.14 (1H, s).
ESI-MS m/z: 347 (M+H)⁺

### Example 144

### 4-Pyridinecarboxyaldehyde 4-(6-iodoimidazo[1,2-a]pyrimidin-2-yl)phenylhydrazone

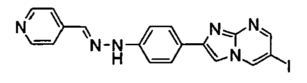

By using the compound obtained in Reference Example 185 and carrying out the operation in the same manner as in Example 138, the title compound was obtained as a brown solid.
¹H-NMR (DMSO-d₆) δ: 7.21 (2H, d, J=7.8 Hz), 7.60 (2H, d, J=4.9 Hz), 7.84 (1H, s), 7.90 (2H, d, J=8.1 Hz), 8.13 (1H, s), 8.54 (3H, m), 9.27 (1H, s), 10.97 (1H, s).
ESI-MS m/z: 441 (M+H)⁺

### Example 145

### 4-Pyridinecarboxyaldehyde 4-(6-tributylstannylimidazo[1,2-a]pyridin-2-yl)phenylhydrazone

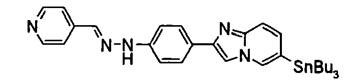

Trifluoroacetic acid *N*-[4-(6-tributylstannylimidazo[1,2-a]pyridin-2-yl)phenyl]-*N*'-pyridin-4-ylmethylenehydrazide (27.3 mg) was dissolved in ethanol (8 ml), and 1 N sodium hydroxide (0.2 ml) was added to the solution at room temperature, followed by stirring for 30 minutes. The reaction solution was diluted with ethyl acetate (90 ml), washed three times with water (30 ml) and dried over anhydrous sodium sulfate. The solvent was evaporated, and the residue was purified by flash silica gel column chromatography (chloroform:methanol = 30:1) to obtain the title compound (12.0 mg) as yellow oil.
¹H-NMR (400 MHz, CDCl₃) δ: 0.91 (9H, t, J=7.3 Hz), 1.14 (6H, m), 1.35 (6H, m), 1.56 (6H, m), 7.13 (1H, d, J=8.8 Hz), 7.20 (2H, d, J=8.5 Hz), 7.51 (2H, d, J=6.2 Hz), 7.58 (1H, m), 7.61 (1H, s), 7.77 (1H, s), 7.90 (2H, d, J=8.6 Hz), 7.97 (1H, s), 8.14 (1H, s), 8.59 (2H, d, J=5.8 Hz).
ESI-MS m/z: 603 (M+H)⁺

### Example 146

### 4-Pyridinecarboxyaldehyde 4-(2-iodovinyl)phenylhydrazone

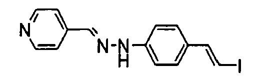

By using the compound obtained in Reference Example 189 and carrying out the operation in the same manner as in Example 47, the title compound was obtained as a reddish brown solid.
¹H-NMR (400 MHz, DMSO-d₆) δ: 6.93 (1H, d, J=15.0 Hz), 7.08 (2H, d, J=8.6 Hz), 7.36 (2H, d, J=8.6 Hz), 7.37 (1H, d, J=15.0 Hz), 7.59 (2H, dd, J=1.5 Hz, 4.7 Hz), 7.82 (1H, s), 8.53 (2H, d, J=1.5 Hz, 4.7 Hz), 10.92 (1H, s).
ESI-MS m/z: 350 (M+H)⁺.

### Example 147

### 4-Pyridinecarboxyaldehyde 4-[1-(2-chloroethyl)-2-methyl-1H-imidazol-4-yl]phenylhydrazone

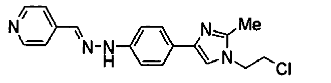

By using the compound obtained in Reference Example 191 and carrying out the operation in the same manner as in Example 138, the title compound was obtained as a yellowish brown solid.
¹H-NMR (400 MHz, CDCl₃) δ: 2.47 (3H, s), 3.77 (2H, t, J=5.4 Hz), 4.21 (2H, t, J=5.4 Hz), 7.09 (1H, s), 7.13 (2H, d, J=6.8 Hz), 7.50 (2H, d, J=4.4 Hz), 7.58 (1H, s), 7.68 (2H, d, J=6.6 Hz), 8.04 (1H, s), 8.58 (2H, d, J=3.9 Hz).
ESI-MS m/z: 340 (M+H)⁺

### Example 148

### 4-Hydroxy-3-methoxybenzaldehyde 4-(imidazol-1-yl)phenylhydrazone

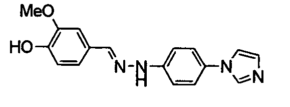

By using the compound obtained in Reference Example 3 and vanillin, and carrying out the operation in the same manner as in Example 35, the title compound was obtained as a pale brown solid.
¹H-NMR (400 MHz, DMSO-d₆) δ: 3.82 (3H, s), 6.77 (1H, d, J=8.0 Hz), 7.01 (1H, d, J=8.0 Hz), 7.04 (1H, s), 7.11 (2H, d, J=8.6 Hz), 7.26 (1H, s), 7.41 (2H, d, J=8.8 Hz), 7.56 (1H, s), 7.78 (1H, s), 8.04 (1H, s), 9.24 (1H, br s), 10.24 (1H, s).
ESI-MS m/z: 309 (M+H)⁺

### Example 149

### 5-Iodo-3,4-dimethoxybenzaldehyde 4-(imidazol-1-yl)phenylhydrazone

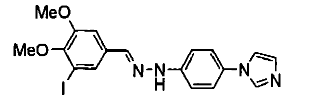

By using the compound obtained in Reference Example 3 and the compound obtained in Reference Example 192, and carrying out the operation in the same manner as in Example 35, the title compound was obtained as a pale brown solid.
¹H-NMR (400 MHz, DMSO-d₆) δ: 3.71 (3H, s), 3.88 (3H, s), 7.05 (1H, s), 7.16 (2H, d, J=8.1 Hz), 7.36 (1H, s), 7.45 (2H, d, J=8.1 Hz), 7.58 (2H, d, J=10.0 Hz), 7.77 (1H, s), 8.06 (1H, s), 10.57 (1H, s).
ESI-MS m/z: 449 (M+H)⁺

### Example 150

### 5-Bromo-4-hydroxy-3-methoxybenzaldehyde 4-(imidazol-1-yl)phenylhydrazone

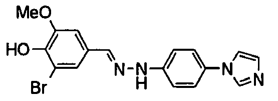

By using the compound obtained in Reference Example 3 and 5-bromovanillin, and carrying out the operation in the same manner as in Example 35, the title compound was obtained as a pale brown solid.
¹H-NMR (400 MHz, DMSO-d₆) δ: 3.89 (3H, s), 7.05 (1H, s), 7.14 (2H, d, J=8.8 Hz), 7.29 (1H, s), 7.34 (1H, s), 7.44 (2H, d, J=9.1 Hz), 7.57 (1H, s), 7.76 (1H, s), 8.06 (1H, s), 9.69 (1H, br s), 10.43 (1H, s).
ESI-MS m/z: 387 M⁺

### Example 151

### 5-Bromo-2-hydroxy-3-methoxybenzaldehyde 4-(imidazol-1-yl)phenylhydrazone

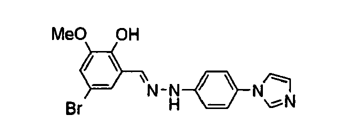

By using the compound obtained in Reference Example 3 and 5-bromo-2-hydroxy-3-methoxybenzaldehyde, and carrying out the operation in the same manner as in Example 35, the title compound was obtained as a reddish brown solid.
¹H-NMR (400 MHz, DMSO-d₆) δ: 3.82 (3H, s), 7.04 (1H, s), 7.05 (1H, s), 7.08 (2H, d, J=8.8 Hz), 7.42 (1H, s), 7.46 (2H, d, J=8.8 Hz), 7.57 (1H, s), 8.06 (1H, s), 8.12 (1H, s), 9.85 (1H, s), 10.65 (1H, s).
ESI-MS m/z: 387 M⁺

### Example 152

### 5-Bromo-3-methoxybenzaldehyde 4-(imidazol-1-yl)phenylhydrazone

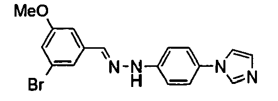

By using the compound obtained in Reference Example 3 and the compound obtained in Reference Example 196, and carrying out the operation in the same manner as in Example 35, the title compound was obtained as yellowish brown crystals.
¹H-NMR (400 MHz, DMSO-d₆) δ: 3.81 (3H, s), 7.05 (1H, d, J=1.2 Hz), 7.06 (1H, s), 7.17 (2H, d, J=9.1 Hz), 7.20 (1H, s), 7.44 (1H, s), 7.46 (1H, d, J=8.9 Hz), 7.58 (1H, d, J=1.5 Hz), 7.80 (1H, s), 8.07 (1H, s), 10.69 (1H, s).
ESI-MS m/z: 371 M⁺

### Example 153

### 4-Hydroxy-3,5-dimethoxybenzaldehyde 4-(imidazol-1-yl)phenylhydrazone

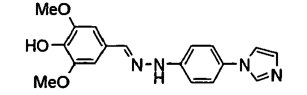

By using the compound obtained in Reference Example 3 and 4-hydroxy-3,5-dimethoxybenzaldehyde, and carrying out the operation in the same manner as in Example 35, the title compound was obtained as a yellowish brown solid.
¹H-NMR (400 MHz, DMSO-d₆) δ: 3.81 (6H, s), 6.93 (2H, s), 7.05 (1H, s), 7.12 (2H, d, J=8.8 Hz), 7.43 (2H, d, J=8.6 Hz), 7.57 (1H, s), 7.77 (1H, s), 8.06 (1H, s), 8.59 (1H, s), 10.30 (1H, s).
ESI-MS m/z: 339 (M+H)⁺

### Example 154

### 3,4-Dihydroxybenzaldehyde 4-(6-iodoimidazo[1,2-a]pyridin-2-yl)phenylhydrazone

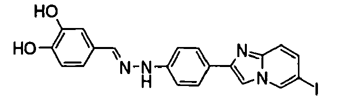

By using the compound obtained in Reference Example 48 and 3,4-dihydroxybenzaldehyde, and carrying out the operation in the same manner as in Example 35, the title compound was obtained as a reddish brown solid.
¹H-NMR (400 MHz, DMSO-d₆) δ: 6.72 (1H, d, J=8.1 Hz), 6.84 (1H, dd, J=8.0 Hz, 2.0 Hz), 7.04 (2H, d, J=8.8 Hz), 7.15 (1H, d, J=1.9 Hz), 7.30-7.38 (1H, m), 7.36 (2H, s), 7.72 (1H, s), 7.77 (2H, d, J=8.8 Hz), 8.13 (1H, s), 8.85 (1H, s), 9.13 (1H, br s), 10.19 (1H, br s).
ESI-MS m/z: 471 (M+H)⁺

### Example 155

### 3-Carboxy-4-hydroxybenzaldehyde 4-(6-iodoimidazo[1,2-a]pyridin-2-yl)phenylhydrazone

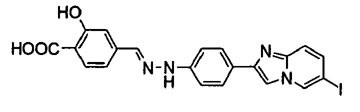

By using the compound obtained in Reference Example 48 and 5-formyl-2-hydroxybenzoic acid, and carrying out the operation in the same manner as in Example 35, the title compound was obtained as a reddish brown solid.
¹H-NMR (400 MHz, DMSO-d₆) δ: 6.98 (1H, d, J=8.8 Hz), 7.10 (2H, d, J=8.6 Hz), 7.40 (2H, s), 7.79 (2H, d, J=8.6 Hz), 7.85-7.87 (2H, m), 7.99 (1H, d, J=2.0 Hz), 8.15 (1H, s), 8.88 (1H, s), 10.41 (1H, s).
ESI-MS m/z: 499 (M+H)⁺

### Example 156

### (E) form of tert-butyl {2-[4-(6-iodoimidazo[1,2-a]pyridin-2-yl)phenylhydrazonomethyl]thiazol-4-ylmethyl}methylcarbamate

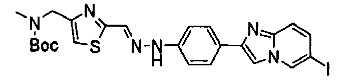

The compound obtained in Reference Example 48 (434 mg) and *tert-butyl* (2-formylthiazol-4-ylmethyl)methylcarbamate (318 mg) were dissolved in ethanol (8 ml) and heated under reflux for 2 hours. The solvent was evaporated, and the thus obtained solid was purified by flash silica gel column chromatography (hexane:acetone = 3:2), and the high polar fraction was concentrated to obtain the title compound (271 mg) as a yellowish brown solid.
¹H-NMR (400 MHz, DMSO-d₆) δ: 1.37 (9H, s), 2.84 (3H, s), 4.41 (2H, br s), 7.11 (2H, d, J=8.9 Hz), 7.27 (1H, s), 7.38 (2H, s), 7.84 (2H, d, J=8.8 Hz), 8.02 (1H, s), 8.17 (1H, s), 8.86 (1H, s), 11.04 (1H, s).
ESI-MS m/z: 589 (M+H)⁺

### Example 157

### (Z) form of tert-butyl {2-[4-(6-iodoimidazo[1,2-a]pyridin-2-yl)phenylhydrazonomethyl]thiazol-4-ylmethyl}methylcarbamate

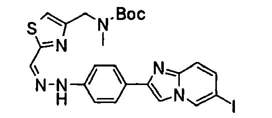

In Example 156, the fraction of the low polarity component was concentrated to obtain the title compound (231 mg) as a yellowish brown solid.
¹H-NMR (400 MHz, DMSO-d₆) δ: 1.33,1.43 (9H, s), 2.89,2.96 (3H, s), 4.60 (2H, s), 7.29 (2H, d, J=7.1 Hz), 7.39 (2H, s), 7.53 (1H, s), 7.57 (1H, s), 7.88 (2H, d, J=8.3 Hz), 8.23 (1H, s), 8.87 (1H, s), 12.91,13.17 (1H, s).
ESI-MS m/z: 589 (M+H)⁺

### Example 158

### 4-(N-methylaminomethyl)thiazol-2-ylcarboxyaldehyde 4-(6-iodoimidazo[1,2-a]pyridin-2-yl)phenylhydrazone

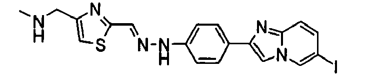

(*E*) form of *tert*-butyl {2-[4-(6-iodoimidazo[1,2-a]pyridin-2-yl)phenylhydrazonomethyl]thiazol-4-ylmethyl}methylcarbamate (83 mg) was dissolved in ethanol (6 ml), and 1 N hydrochloric acid-ethanol (1 ml) was added to the solution, followed by stirring at 50°C for 3 hours. The solvent was evaporated, and to the residue was added 28% aqueous ammonia (30 ml) and extracted with chloroform:methanol = 9:1 (60 ml). After drying the organic layer over anhydrous sodium sulfate, the solvent was evaporated, and the residue was purified by flash silica gel column chromatography (lower layer of chloroform:methanol:water = 7:3:1) to obtain the title compound (58 mg) as a yellowish brown solid.
¹H-NMR (400 MHz, DMSO-d₆) δ: 2.32 (3H, s), 3.73 (2H, s), 7.11 (2H, d, J=8.6 Hz), 7.31 (1H, s), 7.38 (2H, s), 7.84 (2H, d, J=8.8 Hz), 8.03 (1H, s), 8.17 (1H, s), 8.87 (1H, s), 11.02 (1H, s).
ESI-MS m/z: 489 (M+H)⁺

### Example 159

### 4-(1-Aminoethyl)thiazol-2-ylcarboxyaldehyde 4-(6-iodoimidazo[1,2-a]pyridin-2-yl)phenylhydrazone

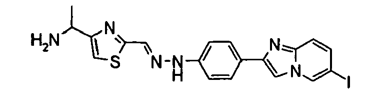

The compound obtained in Reference Example 48 (376 mg) and *tert*-butyl (2-formylthiazol-4-yl)ethylcarbamate (275 mg) were dissolved in ethanol (8 ml) and heated under reflux for 2 hours. The solvent was evaporated to obtain the residue.

The above-described residue was dissolved in methanol (6 ml), saturated hydrochloric acid-methanol (1.5 ml) was added to the solution and stirred at room temperature for 3 hours. After evaporation of the solvent, the residue was alkalified by adding a saturated aqueous sodium bicarbonate solution (100 ml) and extracted with chloroform:methanol = 9:1 (300 ml) twice. After drying over anhydrous sodium sulfate, the solvent was evaporated and the solid was washed with a diethyl ether-ethanol mixed liquid to obtain the title compound (401 mg) as a yellowish brown solid.
¹H-NMR (400 MHz, DMSO-d₆) δ: 1.32 (3H, d, J=6.6 Hz), 2.05-2.30 (2H, br s), 4.03 (1H, q, J=6.8 Hz), 7.11 (2H, d, J=8.6 Hz), 7.27 (1H, s), 7.38 (2H, s), 7.84 (2H, d, J=8.5 Hz), 8.02 (1H, s), 8.86 (1H, s), 11.0 (1H, s).
ESI-MS m/z: 489 (M+H)⁺

### Example 160

### 4-(N-Methylaminomethyl)benzaldehyde 4-(6-iodoimidazo[1,2-a]pyridin-2-yl)phenylhydrazone

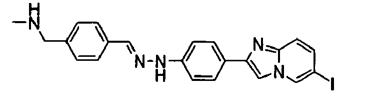

4-(6-Iodoimidazo[1,2-a]pyridin-2-yl)phenylhydrazine (118 mg) and *tert*-butyl (4-formylbenzyl)methylcarbamate (126 mg) were dissolved in ethanol (10 ml) and heated at 70°C for 2 hours under reflux. The solvent was evaporated to obtain the residue.

The above-described residue was dissolved in dichloromethane (6 ml), trifluoroacetic acid (1 ml) was added to the solution and stirred at room temperature for 2 hours. After evaporation of the solvent, the residue was diluted with ethyl acetate (120 ml) and washed with a saturated aqueous sodium bicarbonate solution. After drying the organic layer over anhydrous sodium sulfate, the residue was concentrated under reduced pressure and purified by flash silica gel column chromatography (chloroform:methanol = 9:1 to 7:1) to obtain the title compound (96 mg) as a yellowish brown solid.
¹H-NMR (400 MHz, DMSO-d₆) δ: 2.38 (3H, s), 3.84 (2H, s), 7.13 (2H, d, J=8.5 Hz), 7.37 (2H, s), 7.40 (2H, d, J=7.8 Hz), 7.64 (2H, d, J=8.1 Hz), 7.80 (2H, d, J=8.3 Hz), 7.89 (1H, s), 8.15 (1H, s), 8.86 (1H, s), 10.54 (1H, br s).
ESI-MS m/z: 482 (M+H)⁺

### Example 161

### 4-(1-Aminoethyl)benzaldehyde 4-(6-chloroimidazo[1,2-a]pyridin-2-yl)phenylhydrazone

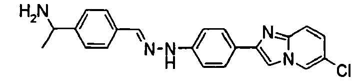

An ethanol solution (8 ml) of 4-(6-chloroimidazo[1,2-a]pyridin-2-yl)phenylhydrazine (235 mg) and 4-(1-di-*tert*-butoxycarbonylaminoethyl)benzaldehyde (318 mg) was heated at 70°C for 3 hours under reflux. The solvent was evaporated, and the residue was purified by flash silica gel column chromatography (chloroform:methanol = 10:1) to obtain a yellowish brown solid.

The above-described solid was dissolved in dichloromethane (6 ml), trifluoroacetic acid (1 ml) was added to the solution and stirred at room temperature for 20 hours. After concentration under reduced pressure, a saturated aqueous sodium bicarbonate solution (50 ml) was added to the mixture, followed by extraction with chloroform:methanol = 5:1 (50 ml). The organic layer was dried over anhydrous sodium sulfate, the solvent was evaporated, and the thus obtained solid was washed with isopropyl alcohol to obtain the title compound (126 mg) as a pale yellow solid.
¹H-NMR (400 MHz, DMSO-d₆) δ: 1.24 (3H, d, J=6.6 Hz), 3.98 (1H, q, J=6.5 Hz), 7.11 (2H, d, J=8.8 Hz), 7.24 (1H, dd, J=9.6 Hz, 2.1 Hz), 7.38 (2H, d, J=8.3 Hz), 7.56 (1H, d, J=9.5 Hz), 7.58 (2H, d, J=8.3 Hz), 7. 81 (2H, d, J=8.5 Hz), 7.87 (1H, s), 8.20 (1H, s), 8.76 (1H, q, J=1.0 Hz), 10.43 (1H, s).
ESI-MS m/z: 390 (M+H)⁺

### Example 162

### 4-(N-Methylaminomethyl)benzaldehyde 4-(6-chloroimidazo[1,2-a]pyridin-2-yl)phenylhydrazone

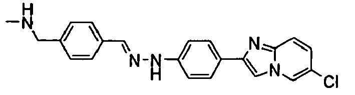

An ethanol solution (8 ml) of 4-(6-chloroimidazo[1,2-a]pyridin-2-yl)phenylhydrazine (148 mg) and 4-(*N*-*tert*-butoxycarbonylaminomethyl)benzaldehyde (143 mg) was heated at 70°C for 3 hours under reflux. The solvent was evaporated, and the residue was purified by flash silica gel column chromatography (chloroform:methanol = 10:1) to obtain a yellowish brown solid.

The above-described solid was dissolved in dichloromethane (8 ml), trifluoroacetic acid (2 ml) was added to the solution and stirred at room temperature for 16 hours. After concentration under reduced pressure, a saturated aqueous sodium bicarbonate solution (50 ml) was added to the mixture, followed by extraction with chloroform:methanol = 5:1 (50 ml) twice. The organic layer was dried over anhydrous sodium sulfate, the solvent was evaporated, and the thus obtained solid was washed with isopropyl alcohol to obtain the title compound (91 mg) as a pale yellow solid.
¹H-NMR (400 MHz, DMSO-d₆) δ: 2.26 (3H, s), 3.63 (2H, s), 7.12 (2H, d, J=8.5 Hz), 7.24 (1H, dd, J=9.5 Hz, 2.2 Hz), 7.33 (2H, d, J=8.3 Hz), 7.57 (1H, d, J=9.8 Hz), 7.60 (2H, d, J=8.1 Hz), 7.81 (2H, d, J=8.5 Hz), 7.87 (1H, s), 8.20 (1H, s), 8.76 (1H, d, J=2.0 Hz), 10.45 (1H, s).
ESI-MS m/z: 390 (M+H)⁺

### Example 163

### 4-Iodobenzaldehyde 4-(pyridin-3-yl)phenylhydrazone

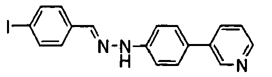

By using the compound obtained in Reference Example 15 and 4-iodobenzaldehyde, and carrying out the operation in the same manner as in Example 35, the title compound was obtained as a yellow solid.
¹H-NMR (400 MHz, DMSO-d₆) δ: 7.19 (2H, d, J=8.8 Hz), 7.42 (1H, dd, J=8.1 Hz, 4.6 Hz), 7.48 (2H, d, J=8.5 Hz), 7.63 (2H, d, J=8.5 Hz), 7.75 (2H, d, J=8.3 Hz), 7.85 (1H, s), 8.00 (1H, s), 8.48 (1H, dd, J=4.6 Hz, 1.5 Hz), 8.85 (1H, d, J=2.0 Hz), 10.64 (1H, s).
EI-MS m/z: 399 (M+).

### Example 164

### 3-Iodo-4-(N-methylaminomethyl)benzaldehyde 4-(pyridin-3-yl)phenylhydrazone

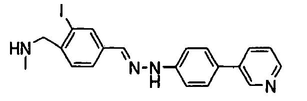

By using the compound obtained in Reference Example 197 and carrying out the operation in the same manner as in Example 47, the title compound was obtained as a yellow solid.
¹H-NMR (400 MHz, DMSO-d₆) δ: 2.32 (3H, s), 3.63 (2H, s), 7.19 (2H, d, J=8.8 Hz), 7.44 (2H, d, J=8.8 Hz), 7.63 (2H, d, J=8.5 Hz), 7.66 (1H, t, J=7.8 Hz), 7.83 (1H, s), 7.90-8.01 (1H, m), 8.12 (1H, d, J=1.5 Hz), 8.47 (1H, dd, J=1.5 Hz, 4.5 Hz), 8.85 (1H, d, J=2.7 Hz), 10.63 (1H, s).
ESI-MS m/z: 443 (M+H)⁺.

### Example 165

### 4- Iodo- 3 -(N-methylaminomethyl)benzaldehyde 4-(oxazol-5-yl)phenylhydrazone

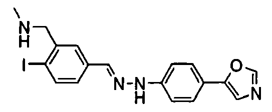

*tert*-Butyl (5-formyl-2-iodobenzyl)methylcarbamate (180 mg) was added to an ethanol solution (10 ml) of 4-(oxazol-5-yl)phenylhydrazine (84 mg), followed by heating overnight under reflux. The residue obtained by evaporating the solvent was purified by silica gel column chromatography, and the fraction obtained from the eluate of n-hexane:ethyl acetate = 1:1 was concentrated under reduced pressure to obtain the title compound (250 mg) as yellow amorphous which was directly used in the subsequent reaction.

A saturated hydrochloric acid methanol solution (3 ml) was added to a methanol solution (2 ml) of the above-described amorphous (250 mg) at 0°C, followed by stirring overnight at room temperature. The residue obtained by evaporating the solvent was alkalified by adding a saturated aqueous sodium bicarbonate solution, extracted with a chloroform-methanol (10:1) mixed solvent and then dried over sodium sulfate. The residue obtained by evaporating the solvent was collected by filtration, washed with diethyl ether and dried to obtain the title compound (85 mg) as a yellow solid.
¹H-NMR (400 MHz, DMSO-d₆) δ: 2.35 (3H, s), 3.64 (2H, s), 7.15 (2H, d, J=8.8 Hz), 7.32 (1H, dd, J=2.0 Hz, 8.3 Hz), 7.45 (1H, s), 7.59 (2H, d, J=8.8 Hz), 7.69 (1H, d, J=2.0 Hz), 7.83 (1H, d, J=8.3 Hz), 7.86 (1H, s), 8.33 (1H, s), 10.68 (1H, s).
ESI-MS m/z: 433 (M+H)⁺.

### Example 166

### 3-Chloro-4-(N-methylaminomethyl)benzaldehyde 4-(oxazol-5-yl)phenylhydrazone

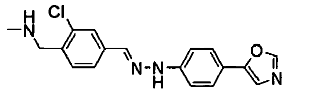

By using the compound obtained in Reference Example 203 and carrying out the operation in the same manner as in Example 165, the title compound was obtained as a yellow solid.
¹H-NMR (400 MHz, DMSO-d₆) δ: 2.35 (3H, s), 3.79 (2H, s), 7.16 (1H, s), 7.17 (2H, d, J=8.5 Hz), 7.45 (1H, s), 7.52 (1H, d, J=8.1 Hz), 7.59 (2H, d, J=8.5 Hz), 7.62 (1H, d, J=8.1 Hz), 7.72 (1H, s), 7.87 (1H, s), 10.73 (1H, s).
ESI-MS m/z: 341 (M+H)⁺.

### Example 167

### 3-Fluoro-4-(N-methylaminomethyl)benzaldehyde 4-(oxazol-5-yl)phenylhydrazone

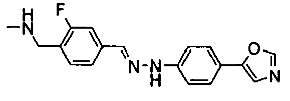

Diisobutylaluminum hydride (3.6 ml, 0.95 M hexane solution) was added dropwise to a THF solution (10 ml) of *tert-*butyl [2-fluoro-4-(*N*-methoxy-*N* methylcarbamoyl)benzyl]methylcarbamate (374 mg) at -78°C, followed by stirring at the same temperature for 30 minutes. A saturated aqueous ammonium chloride solution (5.1 ml) was added dropwise to the reaction solution, followed by stirring at room temperature for 30 minutes. After completion of the reaction, to the reaction solution was added a saturated aqueous ammonium chloride solution and diethyl ether and stirred for 1 hour and then magnesium sulfate was added to the mixture and further stirred for 1 hour. After filtration trough celite, the solvent was evaporated, and the thus obtained residue was purified by silica gel column chromatography, and the fraction obtained from the eluate of n-hexane:ethyl acetate = 10:2 was concentrated under reduced pressure to obtain the residue (257 mg).

The above-described residue (257 mg) was added to an ethanol solution (10 ml) of 4-(oxazol-5-yl)phenylhydrazine (170 mg), followed by heating overnight under reflux. The solvent was evaporated, the thus obtained residue was purified by silica gel column chromatography, and the fraction obtained from the eluate of dichloromethane:methanol = 50:1 was concentrated under reduced pressure to obtain the title compound (289 mg) as yellow amorphous.

A saturated hydrochloric acid methanol solution (5 ml) was added at 0°C to a methanol solution (3 ml) of the above-described amorphous (402 mg), followed by stirring overnight at room temperature. The residue obtained by evaporating the solvent was alkalified by adding a saturated aqueous sodium bicarbonate solution, extracted with a chloroform-methanol (10:1) mixed solvent and then dried over sodium sulfate. The residue obtained by evaporating the solvent was purified by silica gel column chromatography, and the fraction obtained from the organic layer eluate of chloroform:methanol:water = 15:3:1 was concentrated under reduced pressure, collected by filtration, washed with diethyl ether and dried to obtain the title compound (85 mg) as a yellow solid.
¹H-NMR (400 MHz, DMSO-d₆)δ:2.27 (3H, s), 3.67 (2H, s), 7.17 (2H, d, J=8.5 Hz), 7.44-7.46 (4H, m), 7.59 (2H, d, J=8.5 Hz), 7.87 (1H, s), 8.33 (1H, s), 10.68 (1H, s).
ESI-MS m/z: 325 (M+H)⁺.

### Example 168

### 4-(N-Methylaminomethyl)-3-trimethylstannylbenzaldehyde 4-(oxazol-5-yl)phenylhydrazone

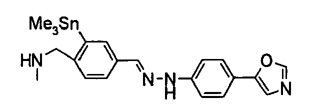

2,2,2-Trifluoro-*N*-methyl-*N*-{4-[4-(oxazol-5-yl)phenylhydrazonomethyl]-2-trimethylstannylbenzyl}acetamide (17.1 mg) was dissolved in ethanol (2.5 ml), 1 N sodium hydroxide (0.1 ml) was added to the solution at 0°C and stirred at room temperature for 2 hours. The reaction solution was diluted with chloroform:methanol = 9:1 (60 ml), washed twice with water (30 ml) and dried over anhydrous sodium sulfate. The solvent was evaporated, and the residue was purified by flash silica gel column chromatography (hexane:acetone = 3:2) to obtain the title compound (9.6 g) as pale yellow oil.
¹H-NMR (400 MHz, CDCl₃)δ:0.32 (9H, s), 2.38 (3H, s), 3.76 (2H, s), 7.15 (2H, d, J=8.5 Hz), 7.21 (1H, s), 7.26 (1H, s), 7.46 (1H, ddd, J=7.8 Hz, 7.1 Hz, 2.4 Hz), 7.57 (2H, d, J=8.5 Hz), 7.67 (1H, ddd, J=8.6 Hz, 7.4 Hz, 1.2 Hz), 7.73 (1H, s), 7.78 (1H, d, J=9.0 Hz), 7.86 (1H, s).
ESI-MS m/z: 470 (M+H)⁺

### Example 169

### Benzimidazole-5-carboxyaldehyde 4-(oxazol-5-yl)phenylhydrazone

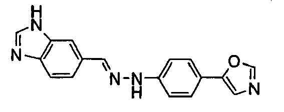

A mixture of isomers, *tert*-butyl 5-formylbenzimidazole-1-carboxylate and *tert*-butyl 6-formylbenzimidazole-1-carboxylate, (257 mg) and 4-(oxazol-5-yl)phenylhydrazine (183 mg) were dissolved in ethanol (10 ml) and heated at 70°C for 2 hours under reflux, and then the reaction solution was evaporated to obtain the residue.

The above-described residue was dissolved in dichloromethane (8 ml) and trifluoroacetic acid (2 ml) was added to the solution at room temperature and stirred for 4 hours. After concentration under reduced pressure, the residue was diluted with ethyl acetate (200 ml) and washed with a saturated aqueous sodium bicarbonate solution and saturated brine (100 ml for each). After drying over anhydrous sodium sulfate, the solvent was evaporated and the residue was purified by flash silica gel column chromatography (chloroform:methanol = 8:1) to obtain the title compound (121 mg) as a pale yellow solid.
¹H-NMR (400 MHz, DMSO-d₆) δ: 7.14 (2H, d, J=8.5 Hz), 7.42 (1H, s), 7.57 (2H, d, J=8.5 Hz), 7.62-7.83 (3H, m), 8.01 (1H, s), 8.23 (1H, s), 8.31 (1H, s), 10.48 (1H, br s), 12.49 (1H, br s).
ESI-MS m/z: 304 (M+H)⁺

### Example 170

### 4-Pyridinecarboxyaldehyde 3-iodophenylhydrazone

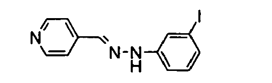

By using the compound obtained in Reference Example 210 and 4-pyridinecarboxyaldehyde, and carrying out the operation in the same manner as in Example 35, the title compound was obtained as brown amorphous.
¹H-NMR (400 MHz, DMSO-d₆)δ:7.02-7.16 (3H, m), 7.49 (1H, s), 7.59 (2H, d, J=6.1 Hz), 7.82 (1H, s), 8.54 (2H, d, J=6.1 Hz), 10.83 (1H, s).

### Example 171

### 6-Dimethylamino-3-pyridinecarboxyaldehyde 4-iodophenylhydrazone

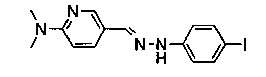

By using the compound obtained in Reference Example 146 and 4-iodophenylhydrazine, and carrying out the operation in the same manner as in Example 35, the title compound was obtained as a brown solid.
¹H-NMR (400 MHz, DMSO-d₆)δ:2.50 (6H, s), 6.69 (1H, d, J=9.1 Hz), 6.86 (2H, d, J=8.8 Hz), 7.46 (2H, d, J=8. 8 Hz), 7. 77 (1H, s), 7.86 (1H, dd, J=2.2 Hz, 9.1 Hz), 8.22 (1H, d, J=2.2 Hz), 10.19 (1H, s).
ESI-MS m/z: 367 (M+H)⁺.

### Example 172

### 4-Dimethylaminobenzaldehyde 3-iodophenylhydrazone

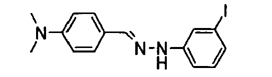

By using the compound obtained in Reference Example 210 and 4-dimethylaminobenzaldehyde, and carrying out the operation in the same manner as in Example 35, the title compound was obtained as yellow powder.
¹H-NMR (400 MHz, DMSO-d₆) δ:2.50 (6H, s), 6.73 (2H, d, J=8.8 Hz), 6.95-7.02 (3H, m), 7.37 (1H, s), 7.47 (2H, d, J=8.8 Hz), 7.76 (1H, s), 10.06 (1H, s).
ESI-MS m/z: 324 (M+H)⁺.

### Example 173

### 4-Dimethylaminobenzaldehyde 4-iodophenylhydrazone

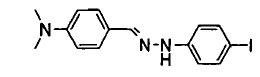

By using 4-iodophenylhydrazine and 4-dimethylaminobenzaldehyde, and carrying out the operation in the same manner as in Example 35, the title compound was obtained as brown amorphous.
¹H-NMR (400 MHz, DMSO-d₆)δ:2.50 (6H, s), 6.72 (2H, d, J=8.8 Hz), 6.85 (2H, d, J=8.8 Hz), 7.45 (4H, d, J=8.8 Hz), 7.76 (1H, s), 10.08 (1H, s).
ESI-MS m/z: 366 (M+H)⁺.

### Example 174

### 1-Benzyl-1,2,3,6-tetrahydropyridine-4-carboxyaldehyde 4-iodophenylhydrazone

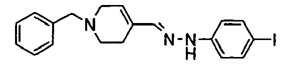

By using 4-iodophenylhydrazine and the compound obtained in Reference Example 156, and carrying out the operation in the same manner as in Example 35, the title compound was obtained as a brown solid.
¹H-NMR (400 MHz, DMSO-d₆)δ:2.37 (2H, br s), 2.57 (2H, t, J=5.8 Hz), 3.03 (2H, d, J=2.9 Hz), 3.58 (2H, s), 5.90 (1H, s), 6.77 (2H, d, J=8.8 Hz), 7.26 (1H, q, J=4.4 Hz), 7.33 (4H, d, J=4.4 Hz), 7.45 (2H, d, J=8.8 Hz), 7.52 (1H, s), 10.11 (1H, s).
ESI-MS m/z: 418 (M+H)⁺.

### Example 175

### 4-(N-Methylaminomethyl)benzaldehyde 4-iodophenylhydrazone

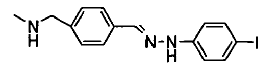

By using 4-iodophenylhydrazine and the compound obtained in Reference Example 121, and carrying out the operation in the same manner as in Example 159, the title compound was obtained as a yellow solid.
¹H-NMR (400 MHz, DMSO-d₆)δ:2.28 (3H, s), 3.66 (2H, s), 6.90 (2H, d, J=8.6 Hz), 7.33 (2H, d, J=8.1 Hz), 7.50 (2H, d, J=8.6 Hz), 7.59 (2H, d, J=8.1 Hz), 7.85 (1H, s), 10.41 (1H, s).
ESI-MS m/z: 366 (M+H)⁺.

### Example 176

### N-[4-(4-Iodophenylhydrazonomethyl)phenyl]acetamide

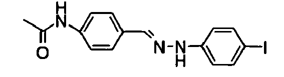

By using 4-iodophenylhydrazine and *N*-(formylphenyl)acetamide, and carrying out the operation in the same manner as in Example 35, the title compound was obtained as a yellow solid.
¹H-NMR (400 MHz, CDCl₃)δ:2.05 (3H, s), 6.88 (2H, d, J=8.8 Hz), 7.48 (2H, d, J=8.8 Hz), 7.56 (2H, d, J=8.8 Hz), 7.60 (2H, d, J=9.0 Hz), 7.80 (1H, s), 10.02 (1H, s), 10.34 (1H, s).
EI-MS m/z: 379 (M)⁺.

### Example 177

### 4-Methylpiperazin-1-ylbenzaldehyde 4-iodophenylhydrazone

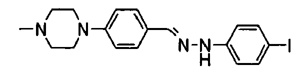

By using 4-iodophenylhydrazine and 4-methylpiperazin-1-ylbenzaldehyde, and carrying out the operation in the same manner as in Example 35, the title compound was obtained as a yellow solid.
¹H-NMR (400 MHz, DMSO-d₆)δ:2.22 (3H, s), 2.44 (4H, t, J=4.9 Hz), 3.18 (4H, t, J=4.9 Hz), 6.86 (2H, d, J=8.6 Hz), 6.94 (2H, d, J=8.8 Hz), 7.46 (2H, d, J=8.8 Hz), 7.48 (2H, d, J=8.6 Hz), 7.77 (1H, s), 10.18 (1H, s).
ESI-MS m/z: 421 (M+H)⁺.

### Example 178

### 4-(N,N-Dimethylaminomethyl)benzaldehyde 4-iodophenylhydrazone

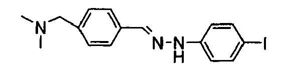

*4-(N,N-*Dimethylaminomethyl)benzaldehyde hydrochloride (240 mg) was added to an ethanol solution (30 ml) of 4-iodophenylhydrazine (296 mg), followed by heating under reflux for 0.5 hour. The solvent was evaporated, and the thus obtained residue was washed with diethyl ether and then a saturated aqueous sodium bicarbonate solution was added to the mixture and extracted with ethyl acetate. The organic layer was washed with water and saturated brine and then dried over sodium sulfate. After evaporation of the solvent, the residue was washed with n-hexane and dried to obtain the title compound (383 mg) as a yellow solid.
¹H-NMR (400 MHz, DMSO-d₆)δ:2.25 (6H, s), 3.43 (2H, s), 6.89 (2H, d, J=8.5 Hz), 7.31 (2H, d, J=8.5 Hz), 7.52 (2H, d, J=8.8 Hz), 7.59 (1H, s), 7.64 (2H, d, J=6.3 Hz), 7.72 (1H, s).
EI-MS m/z: 379 (M)⁺.

### Example 179

### 2-Iodopyridine-4-carboxyaldehyde 4-(imidazo[1,2-a]pyrimidin-2-yl)phenylhydrazone

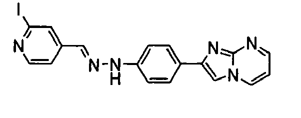

By using the compounds obtained in Reference Example 100 and Reference Example 180, and carrying out the operation in the same manner as in Example 35, the title compound was obtained as a yellowish brown solid.
¹H-NMR (400 MHz, DMSO-d₆) δ: 7.03 (1H, dd, J=6.8 Hz, 4.2 Hz), 7.23 (2H, d, J=8.8 Hz), 7.66 (1H, d, J=5.1 Hz), 7.75 (1H, s), 7.90 (2H, d, J=8.8 Hz), 8.02 (1H, s), 8.24 (1H, s), 8.28 (1H, d, J=5.1 Hz), 8.48 (1H, q, J=2. 0 Hz), 8.93 (1H, dd, J=6. 8 Hz, 2. 0 Hz), 11.12 (1H, s).
ESI-MS m/z: 441 (M+H)⁺

### Example 180

### 2-Iodopyridine-4-carboxyaldehyde 4-(pyridin-3-yl)phenylhydrazone

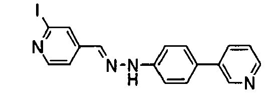

By using the compounds obtained in Reference Example 15 and Reference Example 100, and carrying out the operation in the same manner as in Example 35, the title compound was obtained as a yellow solid.
¹H-NMR (400 MHz, DMSO-d₆) δ: 7.27 (2H, d, J=8.8 Hz), 7.57 (1H, dd, J=7.9 Hz, 5.0 Hz), 7.66 (1H, d, J=5.1 Hz), 7.69 (2H, d, J=8.5 Hz), 7.77 (1H, s), 8.02 (1H, s), 8.19 (1H, d, J=8.1 Hz), 8.29 (1H, d, J=5.1 Hz), 8,55 (1H, dd, J=4.9 Hz, 1.5 Hz), 8.93 (1H, d, J=2.2 Hz), 11.18 (1H, s).
ESI-MS m/z: 401 (M+H)⁺

### Example 181

### 4-(6-Iodoimidazo[1,2-a]pyridin-2-yl)phenylhydrazonomalononitrile

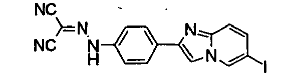

Sodium acetate (227 mg) was added to a methanol (3 ml) and water (6 ml) solution of malononitrile (87.5 mg) at 0°C, followed by stirring for 30 minutes, and then 4-(6-iodoimidazo[1,2-a]pyridin-2-yl)phenyldiazonium tetrafluoroborate (480 mg) was added to the mixture, followed by stirring at room temperature for 20 hours. The insoluble material was collected by filtration and washed with water, and then purified by flash silica gel column chromatography (chloroform:methanol = 30:1) to obtain the title compound (351 mg) as a red solid.
¹H-NMR (400 MHz, DMSO-d₆) δ: 7.45 (2H, d, J=7.1 Hz), 7.52 (2H, d, J=8.5 Hz), 7.97 (2H, d, J=8.5 Hz), 8.31 (1H, s), 8.92 (1H, s).
ESI-MS m/z: 413 (M+H)⁺

### Example 182

### 3-[4-(6-Iodoimidazo[1,2-a]pyridin-2-yl)phenylhydrazonopentane-2,4-dione

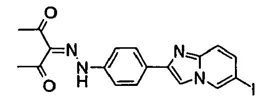

By using acetylacetone and carrying out the operation in the same manner as in Example 181, the title compound was obtained as a yellow solid.
¹H-NMR (400 MHz, DMSO-d₆) δ: 2.48 (6H, s), 7.42 (2H, s), 7.64 (2H, d, J=8.7 Hz), 7.99 (2H, d, J=8.7 Hz), 8.31 (1H, s), 8.90 (1H, s), 14.11 (1H, br s).
ESI-MS m/z: 447 (M+H)⁺

### Example 183

### Methyl cyano[4-(6-iodoimidazo[1,2-a]pyridin-2-yl)phenylhydrazono]acetate, a mixture of (E) and (Z) isomers

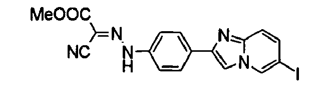

By using methyl cyanoacetate and carrying out the operation in the same manner as in Example 181, the title compound was obtained as a yellow solid of a mixture of (*E*) and (*Z*) isomers.
¹H-NMR (400 MHz, DMSO-d₆) δ: 3.82 (2.4H, s), 3.86 (0.6H, s), 7.42 (2H, s), 7.54 (1.6H, d, J=8.8 Hz), 7.59 (0.4H, d, J=8.6 Hz), 7.97 (1.6H, d, J=8.5 Hz), 7.99 (0.4H, d, J=8.5 Hz), 8.28 (0.8H, s), 8.31 (0.2H, s), 8.90 (1H, s), 12.35 (0.8H, br s), 12.97 (0.2H, br s).
ESI-MS m/z: 446 (M+H)⁺

### Example 184

### Methyl 2-[4-(6-iodoimidazo[1,2-a]pyridin-2-yl)phenylhydrazono]propionate

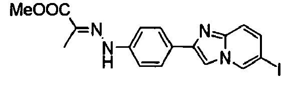

By using the compound obtained in Reference Example 48 and methyl propionate, and carrying out the operation in the same manner as in Example 35, the title compound was obtained as a yellow solid.
¹H-NMR (400 MHz, DMSO-d₆) δ: 2.08 (3H, s), 3.74 (3H, s), 7.32 (2H, d, J=8.8 Hz), 7.39 (2H, s), 7. 8 5 (2H, d, J=8.8 Hz), 8.19 (1H, s), 8.87 (1H, s), 9.96 (1H, s).
ESI-MS m/z: 435 (M+H)⁺

### Test Example 1

### Examination of the effect of drugs on the amyloid formation of amyloid β-protein

In PBS(-), 15 µM of amyloid β-protein (human, 1-40; purchased from Peptide Institute) and 1.6, 8 or 40 µM of each of the agents to be tested shown in Table 4 were incubated at room temperature for 1 day. Thereafter, the quantity of amyloid formation was measured by the Thioflavine T method (Naiki *et al., Lab Invest,* 65, 104-110, 1991). The measured value was converted to a relative value (%) based on the quantity of amyloid formation in the drug-non-added control group, and then 50% inhibition concentration (IC₅₀ value) of amyloid formation was calculated.

**Table 4**

| Drugs to be tested | IC₅₀ value (µM) |
|---|---|
| DDNP | 3.23 |
| Congo Red | 0.87 |
| Example 3 | 2.94 |

| | |
|---|---|
| DDNP: 2-(1,1-dicyanopropen-2-yl)-6-dimethylaminonaphthalene | |

### Test Example 2

### Examination of the effect of drugs on the amyloid formation of various amyloid forming proteins

Amyloid β-protein ((human, 1-40); 10 µM; manufactured by Peptide Institute, β-Amyloid (1-42); 10 µM; purchased from American Peptide), a partial fragment of prion protein (PrP 118-135; 50 µM; purchased from Bachem) or Amyrin (10 µM; purchased from Bachem), as an amyloid forming protein, and the compound of Incentive Example 3 (1.6, 8 or 40 µM) were incubated at room temperature in PBS (-). Samples were collected on the next day in the case of the proteins other than prion protein, or after 10 days of incubation in the case of prion protein, and the quantity of amyloid formation was measured by the Thioflavine T method. The measured value was converted to a relative value (%) based on the quantity of amyloid formation in the Example compound-non-added control group, and then 50% inhibition concentration (IC₅₀ value) of amyloid formation was calculated.

**Table 5**

| Amyloid forming proteins | IC₅₀ (µM) |
|---|---|
| Aβ1-40 | 1.16 |
| Aβ1-42 | 0.70 |
| Amyrin | 2.08 |
| Prion protein | 1.97 |

| | |
|---|---|
| Aβ1-40: Amyloid β-protein (human, 1-40) | |
| Aβ1-42: Amyloid β-protein (human, 1-42) | |

### Test Example 3

### Specific binding to amyloid

The compound of Example 21 having fluorescence was dissolved in TBS at a concentration of 50 µM and incubated with formalin-fixed human Alzheimer disease brain sections (purchased from BioChain). Thereafter, the sections were washed with saturated lithium carbonate/40% ethanol, dried and then observed under a fluorescence microscope. As a result, accumulation of fluorescence was found in the senile plaque.

Thus, it was shown that the compound of the present invention can specifically bind to amyloid in the Alzheimer brain.

While the present invention has been described in detail and with reference to specific embodiments thereof, it will be apparent to one skill in the art that various changes and modifications can be made therein without departing from the spirit and scope thereof

This application is based on Japanese application No. 2003-94257 filed on March 31, 2005, the entire contents of which are incorporated hereinto by reference. All references cited herein are incorporated in their entirety.

### Industrial Applicability

As is evident from Test Examples, the compound (I) of the present invention inhibited amyloid formation of amyloid forming proteins and bonded specifically to amyloid. Accordingly, the compound (I) of the present invention is useful as a preventive and/or therapeutic agent for a disease caused by accumulation of a special fibrous and stable protein aggregate called amyloid.

## Claims

1. A compound represented by the following formula (I): 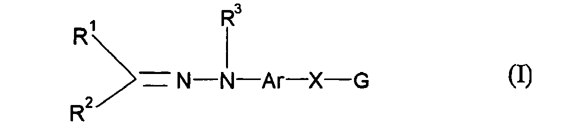
wherein R¹ and R² each independently represents hydrogen, alkyl, alkenyl, alkynyl, aralkyl, amino, alkylamino, cyano, halogen, halogenoalkyl, halogenoalkenyl, halogenoalkynyl, carboxyl, alkoxycarbonyl, carbamoyl, *N*-alkylcarbamoyl, *N*,*N*-dialkylcarbamoyl, *N*-hydroxyalkylcarbamoyl, aryl which may have a substituent, a saturated or unsaturated 5- to 7-membered heterocyclic group which may have a substituent, a saturated or unsaturated bicyclic or tricyclic condensed heterocyclic group which may have a substituent, arylalkenyl which may have a substituent, saturated or unsaturated hetero ring-alkenyl which may have a substituent, or saturated or unsaturated bicyclic or tricyclic condensed hetero ring-alkenyl which may have a substituent, wherein the substituent is one substituent or 2 or 3 substituents, which are the same or different, selected from the following Group (A):
Group (A):
halogen, hydroxyl, alkyl, alkoxy, halogenoalkyl, cyano, nitro, hydroxyalkyl, carboxyl, alkoxycarbonyl, carboxyalkoxy, alkoxycarbonylalkoxy, aralkyloxy, *N*-alkylaminoalkylcarbonyl, *N,N*-dialkylaminoalkylcarbonyl, carboxyalkyl, alkoxycarbonylalkoxy, morpholinocarbonylalkoxy, mercapto, alkylthio, aminosulfonyl, *N*-alkylaminosulfonyl, *N*,*N*-dialkylaminosulfonyl, sulfo, alkylsulfonyl, alkylsulfonylalkyl, tetrazolyl, trialkyltin, trialkylsilyl, aminosulfonylalkyl, *N*-alkylaminosulfonylalkyl, *N*,*N-*dialkylaminosulfonylalkyl, aralkyl, alkylsulfonylamino, *N*-alkylaminosulfonylamino, *N*,*N*-dialkylaminosulfonylamino, *N*-alkylaminoacylamino, *N*,*N-*dialkylaminoacylamino,
a group represented by the following formula (II):
-A¹-Y¹ (II)
wherein A¹ represents a single bond or linear, branched or cyclic alkylene having from 1 to 6 carbon atoms which may be substituted with halogen or hydroxyl; and Y¹ represents a saturated or unsaturated 5- to 7-membered heterocyclic group which may have a substituent,
wherein the substituent on Y¹ is one substituent or 2 or 3 substituents, which are the same or different, selected from the group consisting of halogen, alkyl, halogenoalkyl, carboxyl, alkoxycarbonyl, aminoalkyl, *N*-alkylamino, *N*,*N*-dialkylamino, *N*-alkylaminoalkyl, *N*,*N*-dialkylaminoalkyl, *N*-alkyl-*N*-alkoxycarbonylamino and *N*-alkyl-*N*-alkoxycarbonylaminoalkyl,
a group represented by the following formula (III)
-A² -(C=O)- Y² (III)
wherein A² represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl, in which the alkylene binds to the carbonyl in the group; and Y² represents a saturated or unsaturated 5- to 7-membered heterocyclic group which may have a substituent,
wherein the substituent on Y² represents one substituent or 2 or 3 substituents, which are the same or different, selected from the group consisting of halogen, alkyl, halogenoalkyl, carboxyl, alkoxycarbonyl, aminoalkyl, *N*-alkylamino, *N,N* dialkylamino, *N*-alkylaminoalkyl, *N,N* dialkylaminoalkyl, *N*-alkyl-*N*-alkoxycarbonylamino and *N*-alkyl-*N-*-alkoxycarbonylaminoalkyl,
a group represented by the following formula (IV)
-A³-N(R⁴)(R⁵) (IV)
wherein A³ represents a single bond, linear, branched or cyclic alkylene having from 1 to 6 carbon atoms which may be substituted with halogen or hydroxyl, linear, branched or cyclic-O-alkylene having from 1 to 6 carbon atoms which may be substituted with halogen or hydroxyl, in which the alkylene binds to the nitrogen atom in the group, or linear, branched or cyclic-(C=O)-alkylene having from 1 to 6 carbon atoms which may be substituted with halogen or hydroxyl, in which the alkylene binds to the nitrogen atom in the group; and R⁴ and R⁵ each independently represents hydrogen, alkyl, hydroxyalkyl, halogenoalkyl, acyl, alkoxycarbonyl, alkylsulfonyl, *N*-alkylaminosulfonyl, *N,N*-dialkylaminosulfonyl, *N*-alkylaminoalkylcarbonyl, *N,N* dialkylaminoalkylcarbonyl or alkyldiphenylsilyloxyalkyl, and
a group represented by the following formula (V)
-A⁴-(C=O)-N(R⁶)(R⁷) (V)
wherein A⁴ represents a single bond, linear, branched or cyclic alkylene having from 1 to 6 carbon atoms which may be substituted with halogen or hydroxyl, or linear, branched or cyclic-O-alkylene having from 1 to 6 carbon atoms which may be substituted with halogen or hydroxyl, in which the alkylene binds to the carbonyl in the group; and
R⁶ and R⁷ each independently represents hydrogen, alkyl, hydroxyalkyl, halogenoalkyl, acyl, alkoxycarbonyl, alkylsulfonyl, *N*-alkylaminosulfonyl, *N,N-*dialkylaminosulfonyl, *N*-alkylaminoalkylcarbonyl, *N*,*N*-dialkylaminoalkylcarbonyl or alkyldiphenylsilyloxyalkyl;
R³ represents hydrogen, alkyl which may have substituent, acyl or alkoxycarbonyl;
Ar represents a divalent group derived from aromatic hydrocarbon, a saturated or unsaturated 5- to 7-membered hetero ring or a saturated or unsaturated bicyclic or tricyclic condensed hetero ring, which may have one substituent or 2 or 3 substituents, which are the same or different, selected from the following Group (B):
Group (B):
halogen, hydroxyl group, alkyl, alkoxy, halogenoalkyl, cyano, amino, nitro, alkylamino, hydroxyalkyl, carboxyl, alkoxycarbonyl, carbamoyl, mercapto, alkylthio, aminosulfonyl, *N*-alkylaminosulfonyl, *N,N*-dialkylaminosulfonyl, sulfo, trialkyltin and trialkylsilyl;
X represents a single bond, linear or branched alkylene having from 1 to 3 carbon atoms which may have a substituent, linear or branched alkenylene having from 1 to 3 carbon atoms which may have a substituent, linear or branched alkynylene having from 1 to 3 carbon atoms which may have a substituent or carbonyl; and
G represents halogen, halogenoalkyl, halogenoalkenyl, halogenoalkynyl, alkoxy, alkoxycarbonyl, *N*-alkylamino, *N,N*-dialkylamino, a saturated or unsaturated 5- or 6-membered cyclic hydrocarbon group which may have a substituent, a saturated or unsaturated bicyclic or tricyclic condensed hydrocarbon group which may have a substituent, a saturated or unsaturated 5- to 7-membered heterocyclic group which may have a substituent, or a saturated or unsaturated bicyclic or tricyclic condensed heterocyclic group which may have a substituent, wherein the substituent represents one substituent or 2 or 3 substituents, which are the same or different, selected from the following Group (C):
Group (C):
halogen, hydroxyl, alkyl, alkoxy, halogenoalkyl, halogenoalkenyl, halogenoalkoxy, cyano, amino, nitro, *N*-alkylamino, *N,N-*dialkylamino, *N*-alkylaminoalkyl, *N*,*N*-dialkylaminoalkyl, hydroxyalkyl, carboxyl, carboxyalkyl, alkoxycarbonyl, carbamoyl, mercapto, alkylthio, aminosulfonyl, *N*-alkylaminosulfonyl, *N,N-*dialkylaminosulfonyl, oxo, trialkyltin and trialkylsilyl,
a salt thereof or a solvate thereof

2. The compound represented by formula (I) according to claim 1, R¹ and R² each independently is hydrogen, alkyl, amino, cyano, halogen, halogenoalkenyl, carboxyl, alkoxycarbonyl, carbamoyl, *N*,*N-*dialkylcarbamoyl, *N*-hydroxyalkylcarbamoyl, aryl which may have a substituent, a saturated or unsaturated 5- to 7-membered heterocyclic group which may have a substituent, or a saturated or unsaturated bicyclic or tricyclic condensed heterocyclic group which may have a substituent, a salt thereof or a solvate thereof

3. The compound represented by formula (I) according to claim 1 or 2, wherein R³ is hydrogen, a salt thereof or a solvate thereof.

4. The compound represented by formula (I) according to any one of claims 1 to 3, wherein Ar is phenylene, a salt thereof or a solvate thereof.

5. The compound represented by formula (I) according to any one of claims 1 to 4, wherein X is a single bond or linear or branched alkylene having from 1 to 3 carbon atoms which may have a substituent, a salt thereof or a solvate thereof

6. The compound represented by formula (I) according to any one of claims 1 to 5, wherein G is halogen, halogenoalkenyl, alkoxy, alkoxycarbonyl, *N,N*-dialkylamino, a saturated or unsaturated 5- or 6-membered cyclic hydrocarbon group which may have a substituent, or a saturated or unsaturated 5- to 7-membered heterocyclic group which may have a substituent, a salt thereof or a solvate thereof.

7. The compound represented by formula (I) according to any one of claims 1 to 6, wherein any one of R¹, R², R³, Ar and G is labeled with a radiation-releasing isotope, a salt thereof or a solvate thereof.

8. The compound represented by formula (I) according to claim 7, wherein the radiation-releasing isotope is a radioactive iodine atom, a salt thereof or a solvate thereof

9. A medicament which comprises the compound represented by formula (I) according to any one of claims 1 to 8, a salt thereof or a solvate thereof.

10. A pharmaceutical composition which comprises the compound represented by formula (I) according to any one of claims 1 to 8, a salt thereof or a solvate thereof, and a pharmaceutically acceptable carrier.

11. An agent for inhibiting aggregation and/or deposition of an amyloid protein or an amyloid-like protein, which comprises the compound represented by formula (I) according to any one of claims 1 to 8, a salt thereof or a solvate thereof.

12. An agent for inhibiting and/or treating a conformation disease, which comprises the compound represented by formula (I) according to any one of claims 1 to 8, a salt thereof or a solvate thereof.

13. An agent for preventing and/or treating a disease caused by accumulation of amyloid, which comprises the compound represented by formula (I) according to any one of claims 1 to 8, a salt thereof or a solvate thereof.

14. An agent for preventing and/or treating Alzheimer disease, Down syndrome, Creutzfeldt-Jacob disease, diabetes mellitus type II, dialysis amyloidosis, AA amyloidosis, Gerstmann Straussler Scheinker syndrome, Maxwell's syndrome, localized atrial amyloid, medullary carcinoma of thyroid, skin amyloidosis, localized nodular amyloidosis, AL amyloidosis, AH amyloidosis, familial amyloid polyneuropathy, senile systemic amyloidosis, cerebrovascular amyloidosis, familial Mediterranean fever, Parkinson disease, tauopathy, ALS or CAG repeat disease, which comprises the compound represented by formula (I) according to any one of claims 1 to 8, a salt thereof or a solvate thereof.

15. A radioactive diagnosing agent, which comprises the compound represented by formula (I) according to claim 7 or 8, a salt thereof or a solvate thereof.

16. A method for inhibiting and/or treating a conformation disease, which comprises administering the compound represented by formula (I) according to claim 1, a salt thereof or a solvate thereof.

17. A method for preventing and/or treating a disease caused by accumulation of amyloid, which comprises the compound represented by formula (I) according to claim 1, a salt thereof or a solvate thereof

18. A method for preventing and/or treating Alzheimer disease, Down syndrome, Creutzfeldt-Jacob disease, diabetes mellitus type II, dialysis amyloidosis, AA amyloidosis, Gerstmann Straussler Scheinker syndrome, Maxwell's syndrome, localized atrial amyloid, medullary carcinoma of thyroid, skin amyloidosis, localized nodular amyloidosis, AL amyloidosis, AH amyloidosis, familial amyloid polyneuropathy, senile systemic amyloidosis, cerebrovascular amyloidosis, familial Mediterranean fever, Parkinson disease, tauopathy, ALS or CAG repeat disease, which comprises administering the compound represented by formula (I) according to claim 1, a salt thereof or a solvate thereof.

19. A method for diagnosing accumulation of amyloid, which comprises administering the compound represented by formula (I) according to claim 7, a salt thereof or a solvate thereof; and detecting a radiation-releasing isotope.

20. Use of the compound represented by formula (I) according to any one of claims 1 to 8, a salt thereof or a solvate thereof for the manufacture of an agent for inhibiting and/or treating a conformation disease, which comprises.

21. Use of the compound represented by formula (I) according to any one of claims 1 to 8, a salt thereof or a solvate thereof for the manufacture of an agent for preventing and/or treating a disease caused by accumulation of amyloid.

22. Use of the compound represented by formula (I) according to any one of claims 1 to 8, a salt thereof or a solvate thereof for the manufacture of an agent for preventing and/or treating Alzheimer disease, Down syndrome, Creutzfeldt-Jacob disease, diabetes mellitus type II, dialysis amyloidosis, AA amyloidosis, Gerstmann Straussler Scheinker syndrome, Maxwell's syndrome, localized atrial amyloid, medullary carcinoma of thyroid, skin amyloidosis, localized nodular amyloidosis, AL amyloidosis, AH amyloidosis, familial amyloid polyneuropathy, senile systemic amyloidosis, cerebrovascular amyloidosis, familial Mediterranean fever, Parkinson disease, tauopathy, ALS or CAG repeat disease.

23. Use of the compound represented by formula (I) according to claim 7 or 8, a salt thereof or a solvate thereof for the manufacture of an radioactive diagnosing agent.
